(19) Europäisches Patentamt
European Patent Office
Office européen des brevets

(11) Publication number: **0 371 564 B1**

(12) # EUROPEAN PATENT SPECIFICATION

(45) Date of publication of patent specification: **12.07.95**

(51) Int. Cl.[6]: **C07D 401/06**, C07D 403/06, C07D 403/14, C07D 409/14, C07D 401/14, A61K 31/47, A61K 31/495

(21) Application number: **89203014.9**

(22) Date of filing: **28.11.89**

(54) **(1H-azol-1-ylmethyl)substituted quinoline, quinazoline or quinoxaline derivatives.**

(30) Priority: **29.11.88 GB 8827821**
**29.11.88 GB 8827820**
**29.11.88 GB 8827822**

(43) Date of publication of application:
**06.06.90 Bulletin 90/23**

(45) Publication of the grant of the patent:
**12.07.95 Bulletin 95/28**

(84) Designated Contracting States:
**AT BE CH DE ES FR GB GR IT LI LU NL SE**

(56) References cited:
**EP-A- 0 260 744**
**GB-A- 2 101 115**
**US-A- 4 792 561**

(73) Proprietor: **JANSSEN PHARMACEUTICA N.V.**
**Turnhoutseweg 30**
**B-2340 Beerse (BE)**

(72) Inventor: **Freyne, Eddy Jean Edgard**
**Jan Van Eycklei 8**
**B-2638 Rumst (BE)**
Inventor: **Venet, Marc Gaston**
**21-25 Rue Albert**
**F-75013 Paris (FR)**
Inventor: **Raeymaekers, Alfons Herman Margaretha**
**Aanbeeldstraat 1**
**B-2340 Beerse (BE)**
Inventor: **Sanz, Gerard Charles**
**50 Rue Branly**
**F-95140 Garges les Gonesse (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

In EP-A-260,744 there are described (1H-azol-1-ylmethyl) substituted benzimidazole derivatives which compounds are useful as androgenic hormone biosynthesis inhibitors. The compounds of the present invention differ from the cited art compounds by the fact that they contain a quinoline, quinolinone, quinazoline or quinoxaline moiety in place of an benzimidazole moiety and by their favourable and unexpected pharmaceutical properties. In particular the compounds of the invention suppress the plasma elimination of retinoic acids. Further, some compounds of the invention inhibit the formation of androgens from progestines and/or inhibit the action of the enzyme complex aromatase which catalyses the formation of estrogens from androgenic steroids in mammals.

The present invention is concerned with novel compounds of formula

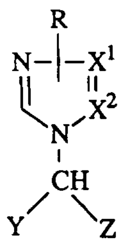

(I)

the pharmaceutical acceptable acid addition salts thereof and the stereochemically isomeric forms thereof, wherein

$-X^1=X^2-$ is a bivalent radical having the formula

$-CH=CH-$ (x),

$-CH=N-$ (y), or

$-N=CH-$ (z);

R is hydrogen or $C_{1-6}$alkyl;

Y is hydrogen, $C_{1-10}$alkyl, $C_{3-7}$cycloalkyl, $Ar^1$, $Ar^2$-$C_{1-6}$alkyl, $C_{2-6}$alkenyl or $C_{2-6}$alkynyl;

Z is a radical of formula

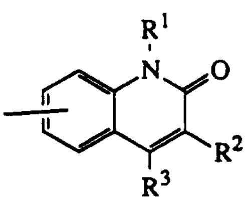

(a-1)

,

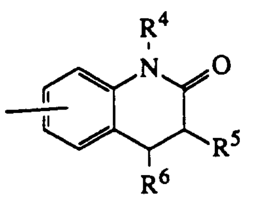

(a-2)

,

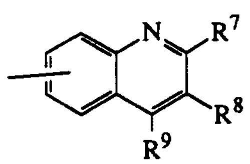

(a-3)

or

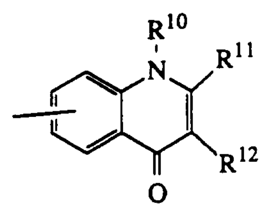

(a-4)

wherein

$R^1$, $R^4$ and $R^{10}$ each independently are hydrogen, $C_{1-6}$alkyl or $Ar^2$-$C_{1-6}$alkyl;

$R^2$, $R^5$, $R^8$ and $R^{12}$ each independently are hydrogen, $C_{1-6}$alkyl or $Ar^2$;

$R^3$, $R^6$ and $R^{11}$ each independently are hydrogen or $C_{1-6}$alkyl;

$R^7$ and $R^9$ each independently are hydrogen, $C_{1-6}$alkyl, $C_{1-6}$alkyloxy, halo, amino, or mono or di($C_{1-6}$alkyl)amino; or

Z is a radical of formula

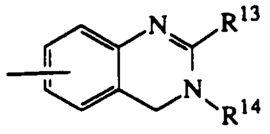,
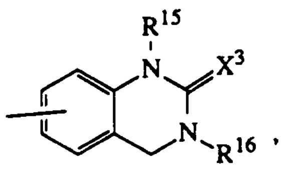,
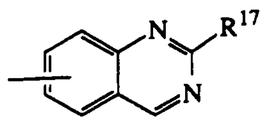,

(b-1) (b-2) (b-3)

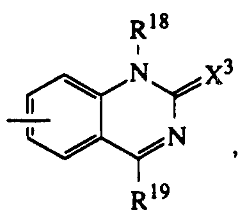,
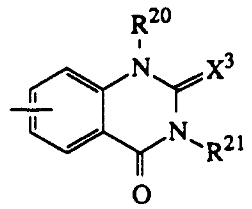
or
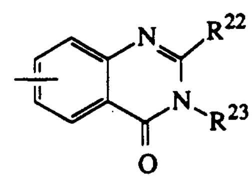

(b-4) (b-5) (b-6)

wherein

$R^{13}$, $R^{17}$ and $R^{22}$ each independently are hydrogen, halo, $C_{1-6}$alkyl, trifluoromethyl, $C_{1-6}$alkyloxy, $Ar^2$, $Ar^2$-$C_{1-6}$alkyl, amino, or mono- or di($C_{1-6}$alkyl)amino;

$R^{14}$, $R^{16}$ and $R^{21}$ each independently are hydrogen, $C_{1-6}$alkyl, $Ar^2$ or $Ar^2$-$C_{1-6}$alkyl;

$R^{15}$, $R^{18}$ and $R^{20}$ each independently are hydrogen, $C_{1-6}$alkyl or $Ar^2$-$C_{1-6}$alkyl;

$R^{19}$ is hydrogen or $C_{1-6}$alkyl;

$R^{23}$ is hydrogen, $C_{1-6}$alkyl, $Ar^2$-$C_{1-6}$alkyl, amino or mono($C_{1-6}$alkyl)amino;

$X^3$ is O or S; or

Z is a radical of formula

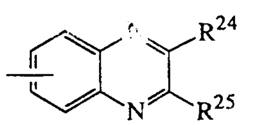,
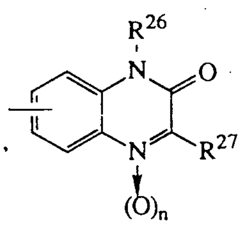,
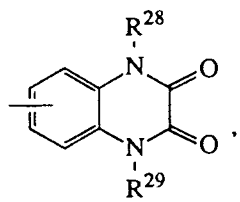,
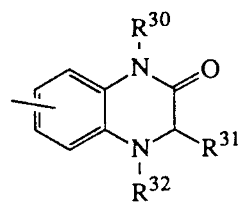,
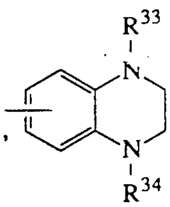

(c-1) (c-2) (c-3) (c-4) (c-5)

wherein

$R^{24}$ is hydrogen, halo, $C_{1-6}$alkyl, $C_{1-6}$alkyloxy, amino, mono- or di($C_{1-6}$alkyl)amino, $Ar^2$ or imidazolyl;

$R^{25}$ is hydrogen, $C_{1-6}$alkyl or $Ar^1$;

$R^{26}$ and $R^{30}$ each independently are hydrogen, $C_{1-6}$alkyl, $Ar^2$-$C_{1-6}$alkyl, amino or mono($C_{1-6}$alkyl)amino;

$R^{27}$ and $R^{31}$ each independently are hydrogen, $C_{1-6}$alkyl, $Ar^1$, $C_{1-6}$alkylcarbonyl, $Ar^2$-carbonyl, $C_{1-6}$alkyloxycarbonyl, carboxyl, $C_{1-6}$alkyloxycarbonyl-$C_{1-4}$alkyl, aminocarbonyl or cyano;

$R^{28}$, $R^{29}$, $R^{32}$, $R^{33}$ and $R^{34}$ each independently are hydrogen, $C_{1-6}$alkyl or $Ar^2$-$C_{1-6}$alkyl;

n is 0 or 1; and

$Ar^1$ is phenyl, substituted phenyl, naphthalenyl, pyridinyl, imidazolyl, triazolyl, thienyl, furanyl or thiazolyl and $Ar^2$ is phenyl or substituted phenyl; said substituted phenyl in $Ar^1$ or $Ar^2$ being phenyl substituted with 1, 2 or 3 substituents each independently selected from halo, hydroxy, trifluoromethyl, $C_{1-6}$alkyl, $C_{1-6}$alkyloxy, cyano, amino, mono- and di($C_{1-6}$alkyl)amino, nitro, carboxyl, formyl and $C_{1-6}$alkyloxycarbonyl.

As used in the foregoing definitions the term halo is generic to fluoro, chloro, bromo and iodo; the term "$C_{1-6}$alkyl" is meant to include straight chained and branched saturated hydrocarbon radicals having from 1 to 6 carbon atoms such as, for example, methyl, ethyl, 1-methylethyl, 1,1-dimethylethyl, propyl, 2-methylpropyl, butyl, pentyl, hexyl; "$C_{1-10}$alkyl" is meant to include the higher homologs of "$C_{1-6}$alkyl"

containing 1-10 carbon atoms; the term "$C_{3-7}$cycloalkyl" is generic to cyclopropyl, cyclobutyl, cyclopentyl, cyclohexyl and cycloheptyl; "$C_{2-6}$ alkenyl" defines straight chained and branched hydrocarbon radicals containing one double bond having from 2 to 6 carbon atoms such as, for example, ethenyl, 2-propenyl, 3-butenyl, 2-butenyl, 2-pentenyl, 3-pentenyl, 3-methyl-2-butenyl; "$C_{2-6}$alkynyl" defines straight chained and branched hydrocarbon radicals containing one triple bond and having from 2 to 6 carbon atoms such as, for example, 2-propynyl, 2-butynyl, 3-butynyl, 2-pentynyl, 3-pentynyl, 4-pentynyl.

It is to be understood that the

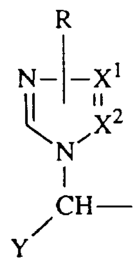

moiety hereinafter referred as the 1H-azol-1-ylmethyl moiety may be substituted on either the 5, 6, 7 or 8 position of the bicyclic ring system, the 6 or 7 position being preferred, the 6 position being most perferred.

Further it should be noted that the compounds of formula (I) wherein Z is a radical of formula (a-1) are denoted as compounds of formula (I-a-1), compounds of formula (a-2) are denoted as compounds of formula (I-a-2), compounds of formula (a-3) are denoted as compounds of formula (I-a-3), compounds of formula (a-4) are denoted as compounds of formula (I-a-4); compounds of formula (I) wherein Z is a radical of formula (b-1) are denoted as compounds of formula (I-b-1); compounds of formula (I) wherein Z is a radical of formula (b-2) are denoted as compounds of formula (I-b-2); compounds of formula (I) wherein Z is a radical of formula (b-3) are denoted as compounds of formula (I-b-3); compounds of formula (I) wherein Z is a radical of formula (b-4) are denoted as compounds of formula (I-b-4); compounds of formula (I) wherein Z is a radical of formula (b-5) are denoted as compounds of formula (I-b-5) and compounds of formula (I) wherein Z is a radical of formula (b-6) are denoted as compounds of formula (I-b-6); compounds of formula (I) wherein Z is a radical of formula (c-1) are denoted as compounds of formula (I-c-1); compounds of formula (I) wherein Z is a radical of formula (c-2) are denoted as compounds of formula (I-c-2); compounds of formula (I) wherein Z is a radical of formula (c-3) are denoted as compounds of formula (I-c-3); compounds of formula (I) wherein Z is a radical of formula (c-4) are denoted as compounds of formula (I-c-4); and compounds of formula (I) wherein Z is a radical of formula (c-5) are denoted as compounds of formula (I-c-5).

The acid addition salts as mentioned hereinabove are meant to comprise the therapeutically active non-toxic acid addition salt forms which the compounds of formula (I) are able to form. The latter can conveniently be obtained by treating the base form with appropriate acids such as, for example, inorganic acids, such as hydrohalic acid, e.g. hydrochloric, hydrobromic, and sulfuric acid, nitric acid, phosphoric acid; or organic acids such as, for example, acetic, hydroxyacetic, propanoic, 2-hydroxypropanoic, 2-oxopropanoic, ethanedioic, propanedioic, butanedioic, (Z)-2-butenedioic, (E)-2-butenedioic 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic 2-hydroxybenzoic, 4-amino-2-hydroxybenzoic acids. Conversely the salt form can be converted by treatment with alkali into the free base form. The term acid addition salt also comprises the hydrates and solvent addition forms which the compounds of formula (I) are able to form. Examples of such forms are e.g. hydrates, alcoholates.

From formula (I) it is evident that the compounds of this invention may have several asymmetric carbon atoms in their structure. Pure isomeric forms of the compounds of formula (I) can be separated from the mixture by conventional separation methods.

Preferably, if a specific stereoisomer is desired, said compound will be synthesized by stereoselective methods of preparation. These methods will advantageously employ enantiomerically pure starting materials.

Further it is evident that the compounds of formula (I) may also contain in their structure a tautomeric system and consequently these compounds can be present in each of their tautomeric forms.

Particular compounds of the present invention are those compounds of formula (I) wherein R is hydrogen or $C_{1-4}$alkyl; and/or Y is hydrogen, $C_{1-6}$alkyl, $C_{3-7}$ cycloalkyl, phenyl, substituted phenyl, pyridinyl, imidazolyl or thienyl; and/or Z is a radical of formula (a-1), (a-2), (a-3) or (a-4) wherein $R^1$, $R^2$, $R^3$,

$R^4$, $R^5$, $R^6$, $R^8$, $R^{10}$, $R^{11}$ and $R^{12}$ each independently are hydrogen or $C_{1-4}$alkyl, and $R^7$ and $R^9$ each independently are hydrogen, $C_{1-4}$alkyl, $C_{1-4}$alkyloxy or halo; and/or Z is a radical of formula (b-1), (b-2), (b-3), (b-4), (b-5), or (b-6) wherein $R^{13}$ is hydrogen, $C_{1-4}$alkyl, trifluoromethyl or phenyl, $R^{14}$ is hydrogen, $C_{1-4}$alkyl, phenyl or phenyl$C_{1-4}$alkyl, $R^{15}$ is hydrogen or $C_{1-4}$alkyl substituted with phenyl, $R^{16}$, $R^{17}$, $R^{18}$, $R^{19}$ and $R^{20}$ each independently are hydrogen or $C_{1-4}$alkyl, $R^{21}$ is hydrogen, $C_{1-4}$alkyl or phenyl-$C_{1-4}$alkyl, $R^{22}$ is hydrogen, amino or mono or di($C_{1-4}$alkylamino), and $R^{23}$ is hydrogen; and/or Z is a radical of formula (c-1), (c-2), (c-3), (c-4) or (c-5) wherein $R^{24}$ is hydrogen, $C_{1-4}$alkyl, halo, $C_{1-4}$alkyloxy, amino, mono- or di-($C_{1-4}$alkyl)amino, phenyl, substituted phenyl or imidazolyl, $R^{25}$ is hydrogen, $C_{1-4}$alkyl, phenyl or substituted phenyl, $R^{26}$ is hydrogen, $C_{1-4}$alkyl, amino, $C_{1-4}$alkylamino or $C_{1-4}$alkyl substituted with phenyl or substituted phenyl; $R^{27}$ is hydrogen, $C_{1-4}$alkyl, $C_{1-4}$alkyloxycarbonyl$C_{1-4}$alkyl, phenyl, substituted phenyl, carboxyl, $C_{1-4}$alkyloxycarbonyl, phenylcarbonyl, substituted phenylcarbonyl, naphthalenyl, thienyl, furanyl, pyridinyl or imidazolyl; $R^{28}$ and $R^{29}$ each independently are hydrogen or $C_{1-4}$alkyl; $R^{30}$ is hydrogen, $C_{1-4}$alkyl, amino or $C_{1-4}$alkyl substituted with phenyl or substituted phenyl; $R^{31}$ is hydrogen, $C_{1-4}$alkyl, phenyl, substituted phenyl, $C_{3-7}$cycloalkyl, naphthalenyl, thienyl, pyridinyl or imidazolyl; $R^{32}$ is hydrogen or $C_{1-4}$alkyl and $R^{33}$ and $R^{34}$ are both hydrogen.

More particular compounds are those particular compounds wherein $-X^1=X^2-$ is a radical having the formula (x) or (y); and Y is hydrogen, $C_{1-4}$alkyl, cyclopropyl, cyclopentyl, cyclohexyl, imidazolyl, pyridinyl, thienyl or phenyl optionally substituted with one or two substituents each independently selected from halo, $C_{1-4}$alkyl, $C_{1-4}$alkyloxy and trifluoromethyl.

Among the compounds of the aforementioned subgroups special emphases is put on

compounds of formula (I) wherein Z is a radical of formula (a-1) wherein $R^1$ and $R^2$ are hydrogen, $R^3$ is hydrogen or $C_{1-4}$alkyl and Y is hydrogen, $C_{1-4}$alkyl or phenyl optionally substituted with one or two halo atoms; and

compounds of formula (I) wherein Z is a radical of formula (a-2) wherein $R^4$, $R^5$ and $R^6$ all being hydrogen, and Y is hydrogen, $C_{1-4}$alkyl, cyclopropyl or phenyl optionally substituted with one or two halo atoms; and

compounds of formula (I) wherein Z is a radical of formula (a-3) wherein $R^7$ is hydrogen, halo or $C_{1-4}$alkyloxy, $R^8$ is hydrogen, $R^9$ is hydrogen, $C_{1-4}$alkyl or $C_{1-4}$alkyloxy and Y is hydrogen, $C_{1-4}$alkyl, cyclopropyl, cyclohexyl, imidazolyl, thienyl or phenyl optionally substituted with one or two substituents each independently selected from halo, $C_{1-4}$alkyl, $C_{1-4}$alkyloxy and trifluoromethyl; and

compounds of formula (I) wherein Z is a radical having the formula (a-4) wherein $R^{10}$ and $R^{12}$ are hydrogen, $R^{11}$ is $C_{1-4}$alkyl and Y is hydrogen; and

compounds of formula (I) wherein Z is a radical of formula (b-2) wherein $R^{15}$ is hydrogen, $R^{16}$ is hydrogen or $C_{1-4}$alkyl and Y is hydrogen, $C_{1-4}$alkyl, pyridinyl or phenyl optionally substituted with one or two halo atoms; and

compounds of formula (I) wherein Z is a radical of formula (b-3) wherein $R^{17}$ is $C_{1-4}$alkyl and Y is phenyl or halophenyl; and compounds of formula (I) wherein Z is a radical of formula (b-5) wherein $R^{20}$ is hydrogen, $R^{21}$ is hydrogen, $C_{1-4}$alkyl or phenyl$C_{1-4}$alkyl and Y is hydrogen, phenyl or halophenyl; and

compounds of formula (I) wherein Z is a radical of formula (b-6) wherein $R^{22}$ is hydrogen or amino, $R^{23}$ is hydrogen and Y is hydrogen, phenyl or halophenyl; and

compounds of formula (I) wherein Z is a radical of formula (c-1) wherein $R^{24}$ is hydrogen, $C_{1-4}$alkyl, $C_{1-4}$alkyloxy, halo, amino, di($C_{1-4}$alkyl)amino, phenyl or imidazolyl, $R^{25}$ is hydrogen, $C_{1-4}$alkyl or phenyl and Y is hydrogen, $C_{1-4}$alkyl, thienyl, imidazolyl or phenyl optionally substituted with one or two substituents selected from halo, $C_{1-4}$alkyl, $C_{1-4}$alkyloxy or trifluoromethyl; and

compounds of formula (I) wherein Z is a radical of formula (c-2) wherein $R^{26}$ is hydrogen, $C_{1-4}$alkyl, amino or $C_{1-4}$alkyl substituted with phenyl and $R^{27}$ is hydrogen, $C_{1-4}$alkyl, carboxyl, $C_{1-4}$alkyloxycarbonyl, naphthalenyl, thienyl, pyridinyl, imidazolyl, phenyl or phenyl substituted with 1, 2 or 3 substituents each independently selected from $C_{1-4}$alkyl, $C_{1-4}$alkyloxy, halo, hydroxy and trifluoromethyl and Y is hydrogen, $C_{1-4}$alkyl, cyclopropyl, cyclopentyl, cyclohexyl, imidazolyl, thienyl, pyridinyl or phenyl optionally substituted with one or two substituents selected from halo, $C_{1-4}$alkyl, $C_{1-4}$alkyloxy and trifluoromethyl.

Preferred compounds of formula (I) wherein Z is a radical of formula (a-1) are those compounds wherein R is hydrogen; $-X^1=X^2-$ is a radical of formula (x) or (y); Y is isopropyl, phenyl or halophenyl; $R^1$ and $R^2$ are both hydrogen; and $R^3$ is methyl.

Most preferred compounds of formula (I) wherein Z is a radical of formula (a-1) are selected from 6-[(4-fluorophenyl)(1H-imidazol-1-yl)methyl]-2(1H)-quinolinone, the pharmaceutically acid addition salts and possible stereoisomeric forms thereof.

Preferred compounds of formula (I) wherein Z is a radical of formula (a-2) are those compounds wherein R is hydrogen; $-X^1=X^2-$ is a radical of formula (x) or (y); Y is cyclopropyl, phenyl or halophenyl and

$R^4$, $R^5$ and $R^6$ are all hydrogen.

Most preferred compounds of formula (I) wherein Z is a radical of formula (a-2) are selected from 6-[(3-chlorophenyl)(1H-imidazol-1-yl)methyl]-3,4-dihydro-2(1H)-quinolinone and 3,4-dihydro-6-[(1H-imidazol-1-yl)-phenylmethyl]-2(1H)-quinolinone, the pharmaceutically acceptable acid addition salts and possible stereoisomers thereof.

Preferred compounds of formula (I) wherein Z is a radical of formula (a-3) are those compounds wherein R is hydrogen; $-X^1=X^2-$ is a radical of formula (x) or (y); Y is phenyl, halophenyl, dihalophenyl, methoxyphenyl or cyclohexyl.

Most preferred compounds of formula (I) wherein Z is a radical of formula (a-3) are selected from 6-[-(1H-1,2,4-triazol-1-yl)[3-(trifluoromethyl)phenyl]methyl]quinoline, the pharmaceutically acceptable acid addition salts and possible stereoisomers thereof.

Preferred compounds of formula (I) wherein Z is a radical of formula (b-2) are those compounds wherein $-X^1=X^2-$ is a radical having the formula (x) or (y); R is hydrogen; $R^{15}$ is hydrogen; $R^{16}$ is hydrogen; and Y is phenyl, halophenyl or propyl.

Most preferred compounds of formula (I) wherein Z is a radical of formula (b-2) are selected from 3,4-dihydro-6-[(1H-imidazol-1-yl)phenylmethyl]-2(1H)-quinazolinone, the pharmaceutically acceptable acid addition salts and possible stereochemically isomeric forms thereof.

Preferred compounds of formula (I) wherein Z is a radical of formula (b-5) are those compounds wherein $-X^1=X^2-$ is a radical having the formula (x) or (y); R is hydrogen; $R^{20}$ is hydrogen; $R^{21}$ is hydrogen, methyl or $C_{1-4}$alkylphenyl; and Y is phenyl or halophenyl.

Most preferred compounds of formula (I) wherein Z is a radical of formula (b-5) are selected from 6-[-(1H-imidazol-1-yl)phenylmethyl]-3-methyl-2,4(1H,3H)-quinazolinedione, the pharmaceutically acceptable acid addition salts and possible stereochemically isomeric forms thereof.

Preferred compounds of formula (I) wherein Z is a radical of formula (b-6) are those compounds wherein $-X^1=X^2-$ is a radical having the formula (x) or (y); R is hydrogen; $R^{22}$ is hydrogen; $R^{23}$ is hydrogen; and Y is phenyl or halophenyl.

Most preferred compounds of formula (I) wherein Z is a radical of formula (b-5) are selected from 6-[-(1H-imidazol-1-yl)phenylmethyl]-4(3H))-quinazolinone, the pharmaceutically acceptable acid addition salts and possible stereochemically isomeric forms thereof.

Preferred compounds of formula (I) wherein Z is a radical of formula (c-1) are those compounds wherein $-X^1=X^2-$ is a radical having the formula (x) or (y); R is hydrogen; $R^{24}$ and $R^{25}$ are both hydrogen and Y is phenyl or halophenyl.

Most preferred compounds of formula (I) wherein Z is a radical of formula (c-1) are selected from 6-[-(1H-imidazol-1-yl)phenylmethyl]quinoxaline and 6-[(4-fluoro phenyl)(1H-imidazol-1-yl)methyl]quinoxaline, the pharmaceutically acceptable acid addition salts and possible stereochemically isomeric forms thereof.

Preferred compounds of formula (I) wherein Z is a radical of formula (c-2) are those compounds wherein $-X^1=X^2-$ is a radical of formula (x) or (y); R is hydrogen; Y is hydrogen, $C_{1-4}$alkyl, cyclopropyl, cyclopentyl or cyclohexyl; $R^{26}$ is hydrogen; $R^{27}$ is hydrogen, $C_{1-4}$alkyl, naphthalenyl, thienyl, pyridinyl, imidazolyl, phenyl or phenyl substituted with 1 or 2 substituents each independently selected from methyl, halo, hydroxy and methoxy; and n is 0.

Other preferred compounds of formula (I) wherein Z is a radical of formula (c-2) are those compounds wherein $-X^1=X^2-$ is a radical of formula (x) or (y); Y is phenyl or halophenyl; $R^{26}$ is hydrogen; $R^{27}$ is hydrogen or $C_{1-4}$alkyl and n is 0.

Most preferred compounds of formula (I) wherein Z is a radical of formula (c-2) are selected from 6-[1-(1H-imidazol-1-yl)-2-methylpropyl]-3-phenyl-2(1H)-quinoxa linone, 6-[1-(1H-imidazol-1-yl)-2-methylpropyl]-3-propyl-2(1H)-quinoxalinone, 3-(3-fluorophenyl)-6-[1-(1H-imidazol-1-yl)-2-methylpropyl]-2(1H)-quinoxalinone, the pharmaceutically acceptable acid addition salts and possible stereochemically isomeric forms thereof.

The compounds of formula (I) can he prepared by N-alkylating an azole of formula (II) or an alkali metal salt thereof with a quinoline, quinolinone, quinazoline or quinoxaline derivative of formula (III).

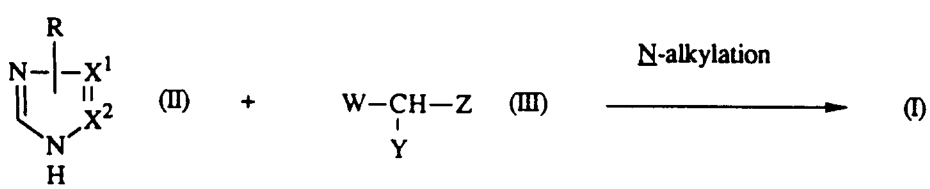

In formula (III) W represents an appropriate reactive leaving group such as, for example, halo, e.g., fluoro, chloro, bromo, iodo or a sulfonyloxy group, e.g. 4-methylbenzenesulfonyloxy, benzenesulfonyloxy, 2-naphthalenesulfonyloxy, methanesulfonyloxy, trifluoromethanesulfonyloxy reactive leaving groups.

The above described N-alkylation is conveniently carried out by stirring the reactants in the presence of a suitable solvent such as, for example, an aromatic hydrocarbon, e.g. benzene, methylbenzene, dimethylbenzene; an ester, e.g. ethyl acetate, γ-butyrolacetone; a ketone, e.g. 2-propanone, 4-methyl-2-pentanone; an ether, e.g. 1,4-dioxane, 1,1'-oxybisethane, tetrahydrofuran; a polar aprotic solvent, e.g. N,N-dimethylformamide, N,N-dimethylacetamide, dimethyl sulfoxide, 1-methyl-2-pyrrolidinone, acetonitrile, hexamethylphosphor triamide, 1,3-dimethyl-3,4,5,6-tetrahydro-2(1H)-pyrimidinone, 1,3-dimethyl-2-imidazolidinone, benzonitrile; or a mixture of such solvents. Somewhat elevated temperatures may be appropriate to enhance the rate of the reaction and in some cases the reaction may even be carried out at the reflux temperature of the reaction mixture.

The addition of an appropriate base such as, for example, an alkali or an earth alkaline metal carbonate, hydrogen carbonate, hydroxide, amide or hydride, e.g. sodium hydroxide, potassium hydroxide, potassium carbonate, sodium hydride or an organic base, such as, for example, N,N-dimethyl-4-pyridinamine, pyridine, N,N-diethylethanamine or N-(1-methylethyl)-2-propanamine may be employed to pick up the acid which is liberated during the course of the reaction. In some instances it may be advantageous to use an excess of the azole (II) or to convert the azole first into a suitable salt form thereof such as, for example, an alkali or earth alkaline metal salt, by reacting (II) with an appropriate base as defined hereinabove and subsequently using said salt form in the reaction with the alkylating reagent of formula (III). Additionally, it may be advantageous to conduct said N-alkylation reaction under an inert atmosphere such as, for example, oxygen-free argon or nitrogen gas. Said alkylation may also be carried out by applying art-known conditions of phase transfer catalysis reactions.

Compounds of formula (I) wherein $-X^1=X^2-$ is a bivalent radical of formula (x), said compounds being represented by formula (I-x), may also be prepared by reacting a quinoline, quinazoline or quinoxaline of formula (III) with a 1-protected imidazole of formula (II-x) following the N-alkylation procedures described hereinabove for the preparation of compounds of formula (I) starting from (II) and (III).

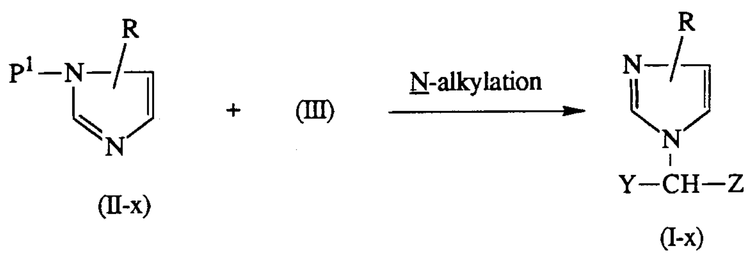


In (II-x) $P^1$ represents a protective group such as, for example, $C_{1-6}$alkylcarbonyl, $C_{1-6}$alkyloxycarbonyl, arylcarbonyl or a tri($C_{1-6}$alkyl)silyl group. In some instances the reaction of (II-x) with (III) first yields a 1-protected imidazolium salt of formula (IV) which may in situ, or if desired, after isolating and further purifying it, be deprotected by stirring it in an aqueous basic or acidic solution.

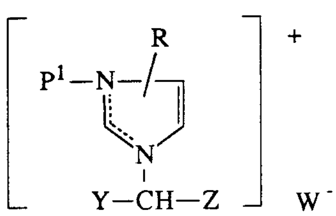
(IV)

In (IV) $W^-$ is an anion arising from an acid such as, for example, hydrochloric acid, hydrobromic acid, methanesulfonic acid, 4-methylbenzenesulfonic acid acids.

Compounds of formula (I) wherein $-X^1=X^2-$ is a bivalent radical of formula (y), said compounds being represented by formula (I-y), can also be prepared by endo-N-alkylation of a triazolamine of formula (II-y) with a quinoline, quinazoline or quinoxaline of formula (III) and subsequent deamination of the thus prepared triazolium salt, wherein $W^-$ is an anion as defined hereinabove.

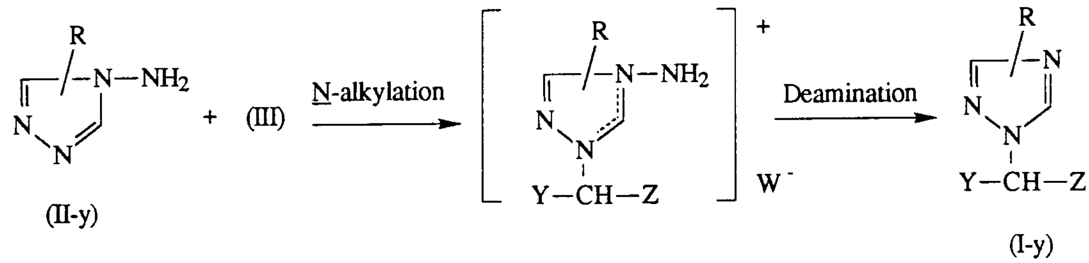


(II-y) (I-y)

The endo-N-alkylation reaction of (II-y) with (III) is carried out according to similar procedures as described hereinabove for the preparation of a compound of formula (I) starting from (III) and (II). Said deamination reaction is conveniently conducted by reaction with an acidic nitrite solution in the presence of an appropriate reductant, or by reaction with an alkylnitrite such as, for example, 1,1-dimethylethylnitrite or isoamylnitrite. Preferably, said deamination reaction is conducted with an aqueous solution of nitrous acid or of a nitrite salt in a suitable acid in the presence of a reducing agent such as, for example, hypophosphorous acid, formic acid, at a lower temperature.

The compounds of formula (I) may also be prepared by reacting an intermediate of formula (V) with a reagent of formula (VI) such as, for example, a 1,1'-carbonylbis[1H-imidazole].

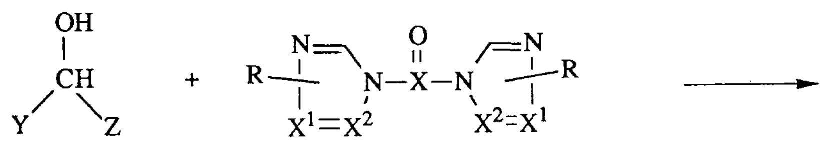

(V) (VI)

In (VI) X represents C or S.

Said reaction may conveniently be conducted in a suitable solvent such as, for example, an ether, e.g. 1,4-dioxane, tetrahydrofuran; a halogenated hydrocarbon, e.g. di- or trichloromethane; a hydrocarbon, e.g. benzene, methylbenzene, dimethylbenzene; N,N-dimethylformamide, N,N-dimethylacetamide, or a mixture of such solvents. In order to enhance the reaction rate, it may be advantageous to heat the reaction mixture.

The compounds of formula (I) may also be prepared by reacting a ketone or aldehyde of formula (VII) with an azole (II) in the presence of formic acid or formamides as reducing agents.

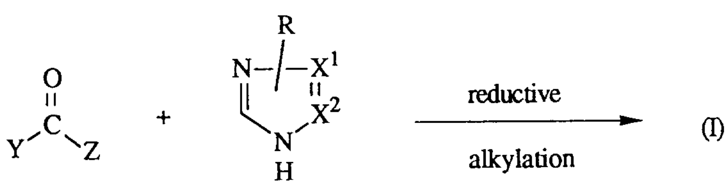


(VII) (II)

Said reductive alkylation can conveniently be conducted by stirring and heating the reagents in formic acid or formamides optionally in the presence of an acid catalyst. An appropriate acid catalyst for using in this reaction is for example a mineral acid such as, hydrochloric acid, sulfuric acid or a sulfonic acid such as, methanesulfonic acid, benzenesulfonic acid, 4-methylbenzenesulfonic acid. It may be appropriate to remove the water which is formed during the reaction by azeotropical distillation, distillation, complexation methods.

In all of the foregoing and following preparations, the reaction products may be isolated from the reaction mixture and, if necessary, further purified according to methodologies generally known in the art such as, for example, extraction, distillation, crystallization, trituration and chromatography.

Some compounds of formula (I-a) can alternatively be prepared under similar conditions as are described in the literature for the preparation of quinolines or quinolinones by cyclizing an intermediate of formula

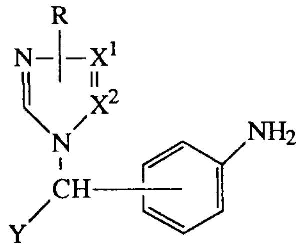

(VIII),

or an appropriate derivative thereof.

For example, the compounds of formula (I-a-1) can be prepared by cyclizing an intermediate of formula (IX).

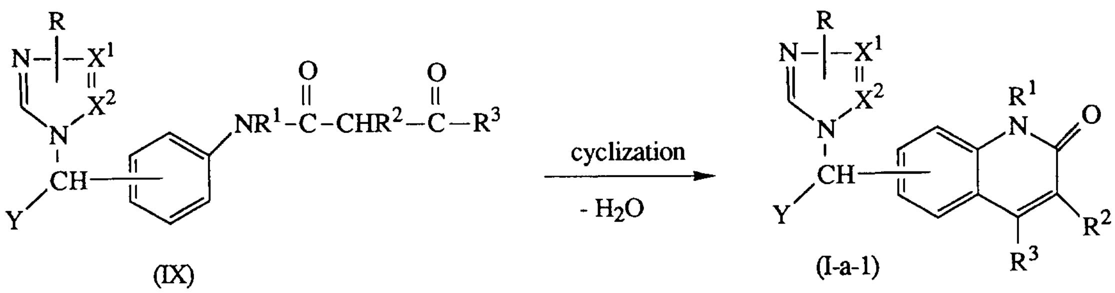


(IX) (I-a-1)

The acid-catalysed cyclization of (IX) can generally be conducted by treating the intermediate amide (IX) with an appropriate acid such as, for example, sulfuric acid, a hydrohalic acid, e.g. hydrochloric acid, polyphosphoric acid strong acids, optionally at an enhanced temperature as described for example in J. Med. Chem. 1986, 29, 2427-2432.

The compounds of formula (I-a-1), may also be obtained by cyclizing an intermediate of formula (X).

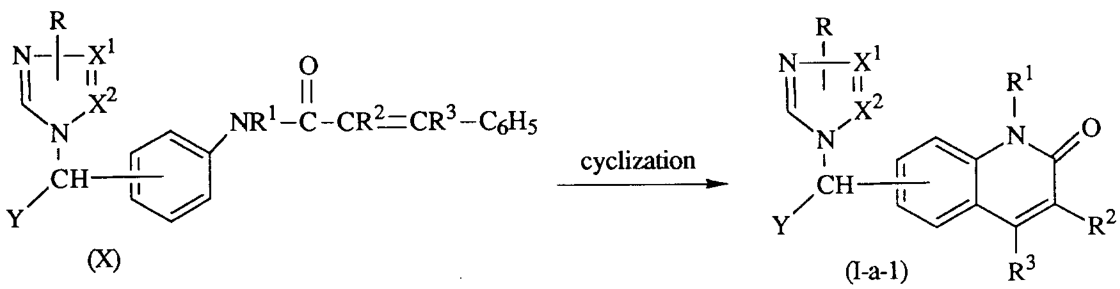


(X) (I-a-1)

The cyclization reaction of (X) may be conducted according to art-known cyclizing procedures as described in, for example, Synthesis 1975, 739. Preferably the reaction is carried out in the presence of a suitable Lewis Acid, e.g. aluminum chloride either neat or in a suitable solvent such as, for example, an aromatic hydrocarbon, e.g. benzene, chlorobenzene, methylbenzene; halogenated hydrocarbons, e.g. trichloromethane, tetrachloromethane; an ether, e.g. tetrahydrofuran, 1,4-dioxane or mixtures of such solvents. Somewhat elevated temperatures, preferably between 70°-100°C, and stirring may enhance the rate of the reaction.

Quinolinones of formula (I-a-1) may also be prepared by cyclizing an intermediate of formula (XI).

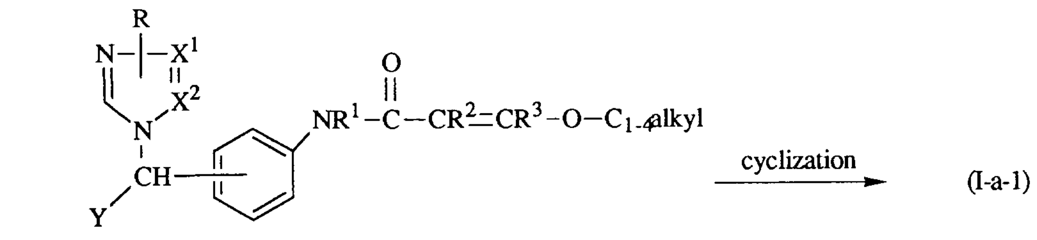


(XI)

The cyclization of (XI) can generally be conducted by treating the intermediate propeneamide (XI) with an appropriate acid such as, for example, sulfuric acid, a hydrohalic acid, e.g. hydrochloric acid, polyphosphoric acid strong acids at room temperature or optionally at an enhanced temperature as described for example in J. Med. Chem. 1989, 32, 1552-1558 or J. Med. Chem. 1988, 31, 2048-2056.

Alternatively the compounds of formula (I) wherein Z is a radical of formula (a-1) or (a-2) may be prepared by cyclizing an intermediate of formula (XII) or (XIII).

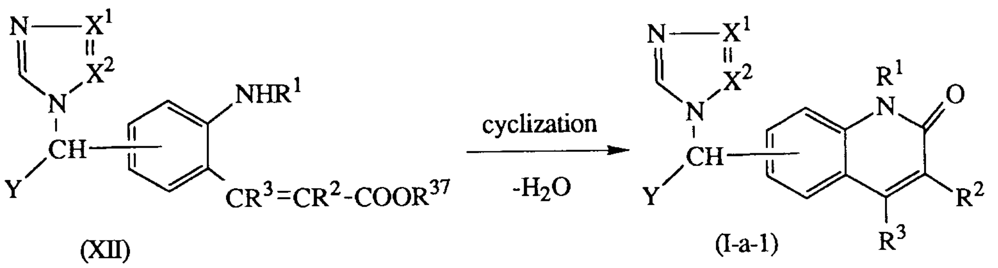


(XII) (I-a-1)

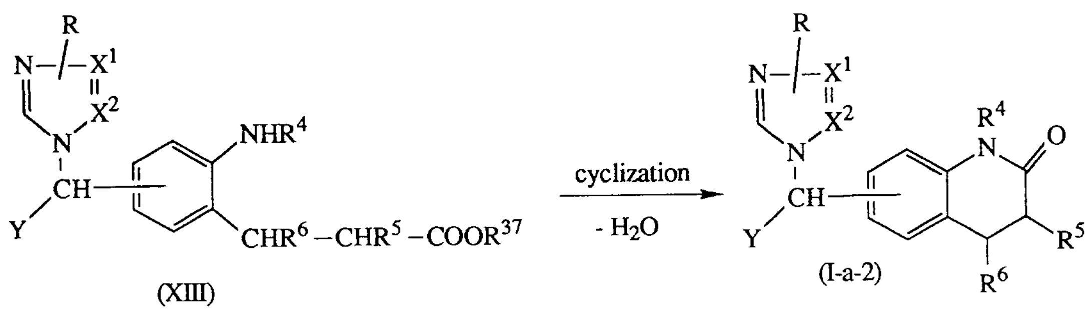


(XIII) (I-a-2)

In (XII) and (XIII) $R^{37}$ represents either a hydrogen or a $C_{1-4}$alkyl group. The above mentioned cyclization reactions may be carried out by stirring and if desired heating the intermediate starting material, optionally in a suitable reaction-inert solvent. Appropriate solvents for said cyclization reactions are for example, aromatic hydrocarbons, e.g. benzene, methylbenzene, dimethylbenzene; halogenated hydrocarbons, e.g. trichloromethane, tetrachloromethane, chlorobenzene; ethers, e.g. 1,1'-oxybisethane, tetrahydrofuran, 1,4-dioxane, 1,2-dimethoxyethane, alkanols, e.g. ethanol, propanol, butanol; ketones, e.g. 2-propanone, 4-methyl-2-pentanone; dipolar aprotic solvents, e.g. N,N-dimethylformamide, dimethylsulfoxide, acetonitrile, methyl acetamide, pyridine, or mixtures of such solvents. The water which is liberated during the cyclization reaction may be removed from the reaction mixture by azeotropical distillation.

Some compounds of formula (I-a-3), can be prepared by cyclizing an intermediate of formula (XIV).

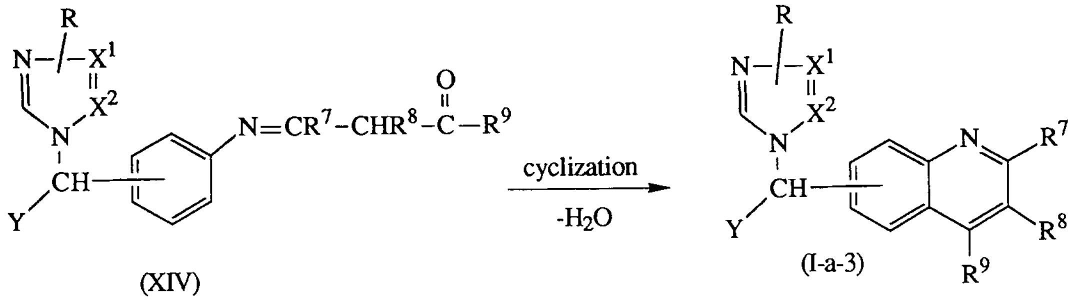


(XIV) (I-a-3)

Said cyclization reaction may conveniently be conducted following similar cyclization procedures as described hereinabove for preparing (I-a-1) from (IX) by cyclizing an intermediate (XIV) in the presence of a suitable dehydrating agent such as, for example, polyphosphoric acid, phosphorous pentoxide, polyphosphate ester, sulfuric acid.

Alternatively the compounds of formula (I-a-3) can be prepared by reacting an aniline of formula (VIII) with an $\alpha,\beta$-unsaturated carbonyl synthon of formula (XV) in the presence of an oxidizing agent.

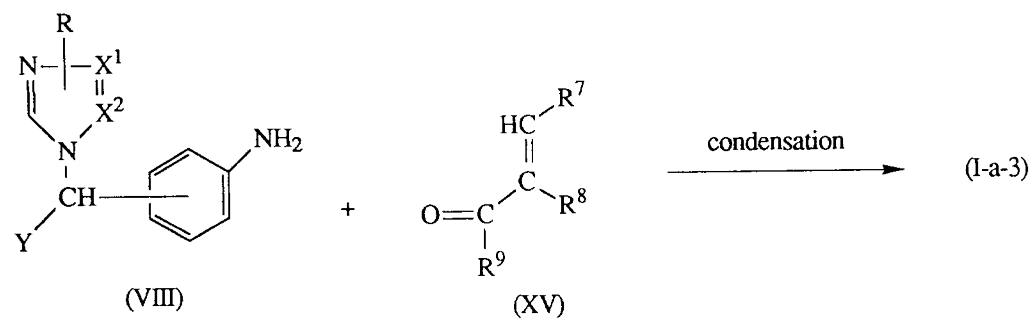


(VIII) + (XV) (I-a-3)

Said reaction may be conducted by heating the reactants in the presence of an acid such as, for example, sulfuric acid, a hydrohalic acid, e.g. hydrochloric acid, polyphosphoric acid strong acids and a mild oxidizing agent. Appropriate oxidizing agents are for example arsenic acid, arsenic oxide, boric acid, ferric chloride, silver nitrate, nitrobenzene, 4-nitrobenzenesulfonic acid or a mixture of 4-nitrobenzoic acid and 4-aminobenzoic acid.

Compounds of formula (I-a-3) may also be prepared by condensing an ortho-acyl aniline of formula (XVI) with a ketone or aldehyde of formula (XVII).

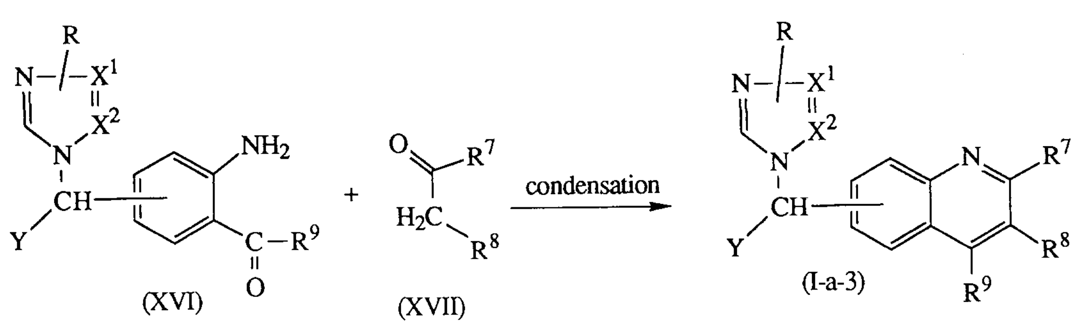


(XVI) + (XVII) (I-a-3)

Said cyclization may convenienlty be conducted by mixing the reactants in a reaction-inert solvent such as, for example, water, an alcohol, e.g. methanol, butanol; an aromatic hydrocarbon, e.g. benzene, methylbenzene, dimethylbenzene, an ester, e.g., ethyl acetate; a halogenated hydrocarbon, e.g., trichloromethane, dichloromethane; or a mixture of such solvents, preferably in the presence of a mineral acid such as, for example, hydrochloric acid, sulfuric acid, a carboxylic acid such as, for example, formic acid, acetic acid, or a sulfonic acid such as, for example, methanesulfonic acid, benzenesulfonic acid, 4-methylbenzenesulfonic

acid or in the presence of a dehydrating agent, such as polyphosphoric acid, phosphorous pentoxide. Somewhat elevated temperatures may he appropriate to enhance the rate of the reaction and in some cases the reaction may he carried out at the reflux temperature of the reaction mixture. It may be appropriate to remove the water which is liberated during the course of the condensation reaction by azeotropical distillation.

The compounds of formula (I) wherein Z is a radical of formula (a-4) and $R^{10}$ is hydrogen, said compounds being represented by (I-a-4-a) can be prepared by cyclizing an intermediate of formula (XVIII).

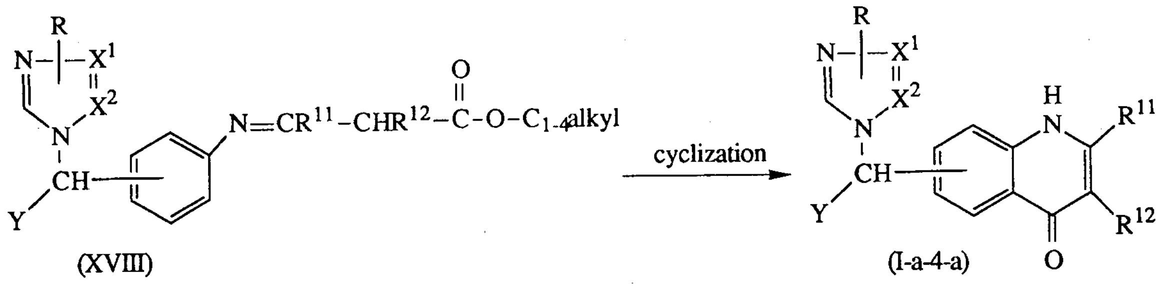


The above mentioned cyclization reaction is preferably accomplished by stirring the intermediate (XVIII) in the presence of a suitable dehydrating agent such as, for example, polyphosphoric acid, phosphorous pentoxide, polyphosphate ester, sulfuric acid, if desired in a reaction inert solvent.

Some compounds of formula (I-b) can alternatively be prepared according to similar procedures as are described in the literature for the preparation of quinazolines and their analogs by cyclizing an appropriate starting compound.

For example, compounds of formula (I-b-1) may be prepared by reacting an intermediate of formula (XIX) with a carboxylic acid of formula (XX) or a functional derivative thereof.

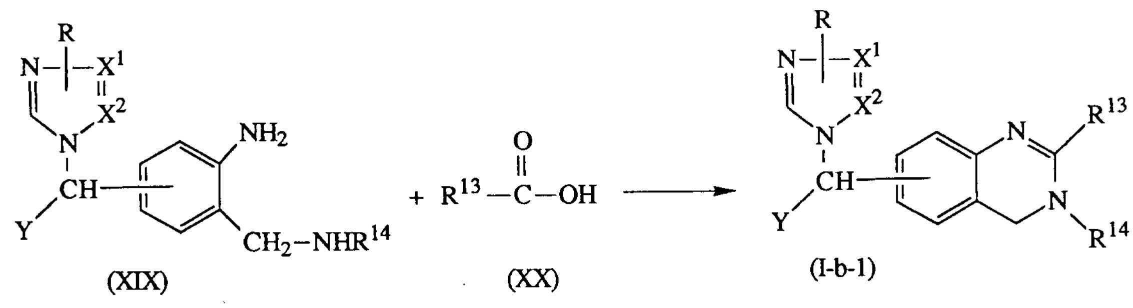

Said functional derivative of (XX) is meant to comprise the halide, anhydride, amide and ester form of (XX), including the ortho and imino ester form thereof. The cyclization of (XIX) and (XX) is preferably carried out by mixing the reactants, optionally in a reaction inert solvent such as, for example, water; a $C_{1-6}$alkanol, e.g. methanol, ethanol, 1-butanol; an ester, e.g. ethyl acetate; a halogenated hydrocarbon, e.g. trichloromethane, dichloromethane; or a mixture of such solvents, preferably in the presence of a mineral acid such as, for example, hydrochloric acid, sulfuric acid, or a carboxylic acid such as, for example, formic acid, acetic acid, or a sulfonic acid such as, for example, methanesulfonic, benzenesulfonic, 4-methylbenzenesulfonic acid or in the presence of an appropriate dehydrating agent such as for example, polyphosphoric acid, phosphorous pentoxide. Somewhat elevated temperatures may be appropriate to enhance the rate of the reaction and in some cases the reaction may even be carried out at the reflux temperature of the reaction mixture. In the instance where (XX) is an acid or the corresponding alkyl ester thereof, the cyclization reaction of (XIX) and (XX) may be conducted in the presence of a suitable dehydrating agent such as, for example, polyphosphoric acid, phosphorous pentoxide, polyphosphate ester. In a preferred method of conducting the above cyclization reaction there is used the imino ester form of (XX) in an acidic medium such as, for example, acetic acid, or a $C_{1-6}$alkanol, whereto an appropriate acid, e.g. hydrochloric acid has been added in case the imino ester is not in the form of an acid addition salt.

The compounds of formula (I-b-1) may also be obtained by cyclizing an intermediate of formula (XXI).

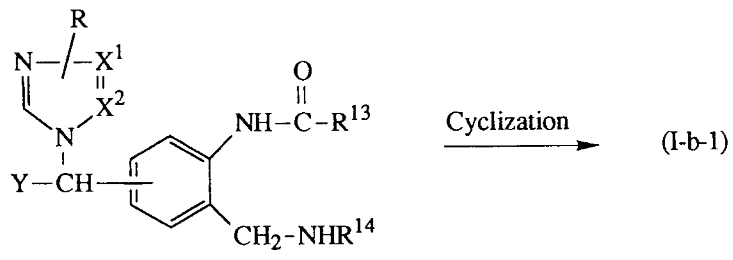


(XXI) (I-b-1)

Said cyclization reaction may conveniently be conducted by heating intermediate (XXI) in an appropriate reaction-inert solvent such as, for example, an aromatic hydrocarbon, e.g., benzene, methylbenzene, dimethylbenzene, a halogenated hydrocarbon, e.g., trichloromethane, tetrachloromethane, an alkanol, e.g., ethanol, propanol, butanol, a ketone, e.g., 2-propanone, 4-methyl-2-pentanone, a dipolar aprotic solvent, e.g., N,N-dimethylformamide N,N-dimethylacetamide, acetonitrile, pyridine, or a mixture of such solvents, and optionally removing the water which is liberated during the course of the cyclization reaction by azeotropical distillation. It may be appropriate to add to the reaction mixture an acid catalyst such as, for example, a mineral acid, e.g., hydrochloric, sulfuric acids, a carboxylic acid, e.g., acetic acid, trifluoroacetic acid, a sulfonic acid, e.g., methanesulfonic, benzenesulfonic or 4-methylbenzenesulfonic acid.

The compounds of formula (I-b-2) may be obtained by reacting an intermediate of formula (XIX-a) with a reagent of formula L-C(=$X^3$)-L (XXII) wherein L represents a reactive leaving group and $X^3$ is oxygen or sulfur.

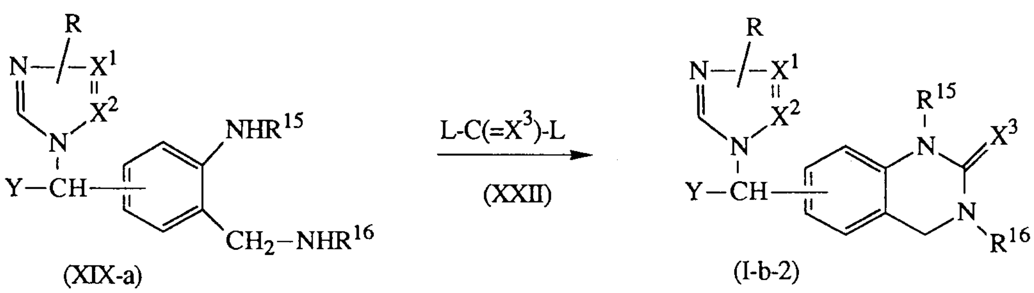


(XIX-a) (I-b-2)

As examples of reagents of formula L-C(=$X^3$)-L (XXII) there may be mentioned urea, thiourea, 1,1'-sulfinylbis[1H-imidazole], 1,1'-carbonylbis[1H-imidazole], alkylcarbonohalidates, e.g., ethyl carbonochloridate, dialkylcarbonates, carbonoic dichloride, carbonothioic dichloride, trichloromethyl chloroformate, carbon disulfide, trifluoromethyl carbonohalidate reagents. Said reaction may be carried out by stirring the reactants, optionally in a reaction-inert solvent such as, for example, an ether, e.g. 1,1'-oxybisethane, tetrahydrofuran; a halogenated hydrocarbon, e.g. dichloromethane, trichloromethane; a hydrocarbon, e.g. benzene, methylbenzene; an alcohol, e.g. methanol, ethanol; a ketone, e.g. 2-propanone, 4-methyl-2-pentanone; a polar aprotic solvent, e.g. N,N-dimethylformamide, N,N-dimethylacetamide, acetonitrile, or a mixture of such solvents. In some instances it may be appropriate to add to the reaction mixture a base such as, for example, an alkali or earth alkaline metal carbonate, hydrogen carbonate, hydroxide or oxide, for example, sodium carbonate, sodium hydrogen carbonate, potassium carbonate, sodium hydroxide, potassium hydroxide, or an organic base, for example, N,N-diethylethanamine, N-(1-methylethyl)-2-propanamine. In order to enhance the reaction rate, it may be advantageous to heat the reaction mixture.

Alternatively, the compounds of formula (I-b-2) can also be prepared by reducing and condensing an intermediate of formula (XXIII) in a reaction-inert solvent.

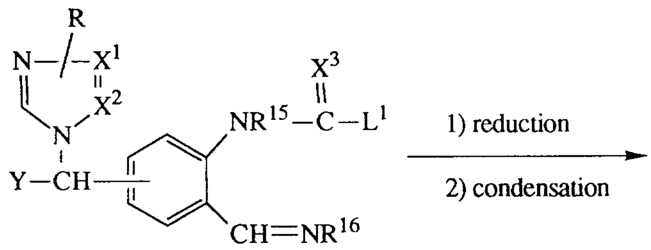


(XXIII) (I-b-2)

In formula (XXIII) $L^1$ represents a reactive leaving group such as, for example, amino or alkyloxy, e.g., methoxy, ethoxy. Reaction-inert solvents are, for example, alkanols, e.g., methanol, ethanol, butanol, aromatic hydrocarbons, e.g., benzene, methylbenzene, halogenated hydrocarbons, e.g. trichloromethane, tetrachloromethane. Said reduction can conveniently be carried out by treating (XXIII) with a reducing agent such as, for example, an alkali metal borohydride, e.g. lithium, potassium or, preferably, sodium borohydride, sodium cyanoborohydride reducing agents.

The compounds of formula (I-b-3), may be prepared by reacting an intermediate of formula (XXIV) with ammonia.

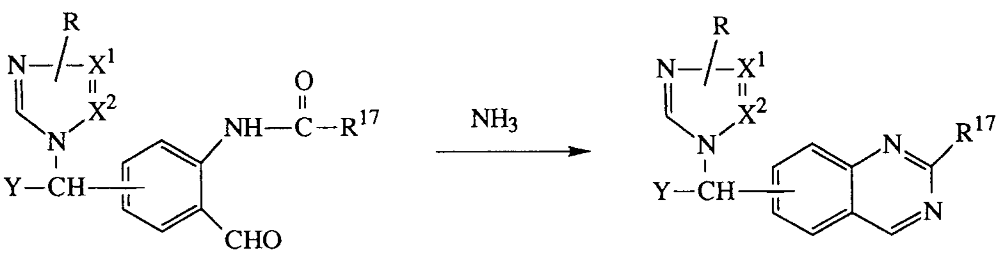


(XXIV) (I-b-3)

Said reaction may conveniently be conducted by stirring the reactants in an appropriate solvent such as, for example, and alkanol, e.g., methanol, ethanol, an ether, e.g., 1,1'-oxybisethane, tetrahydrofuran, 1,4-dioxane, an aromatic hydrocarbon, e.g., benzene, methylbenzene, dimethylbenzene, a halogenated hydrocarbon, e.g. trichloromethane, tetrachloromethane solvents.

In a similar manner, the compounds of formula (I-b-4), may be obtained from an intermediate of formula (XXV) wherein $L^1$ represents a leaving group as defined hereinabove, by reaction with ammonia, following the procedures described hereinabove for the preparation of the compounds of formula (I-b-3) from the intermediates (XXIV).

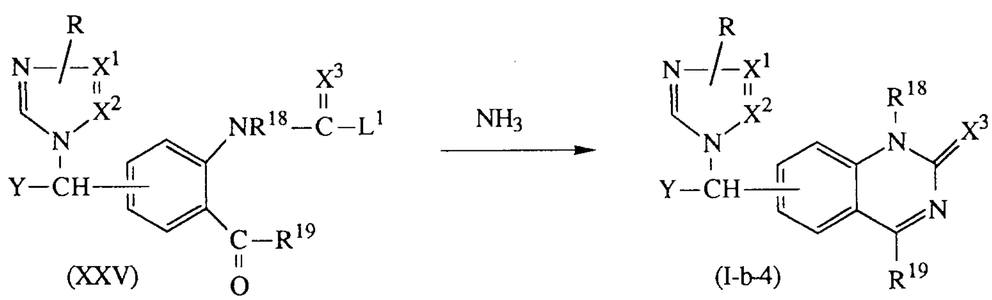


(XXV) (I-b-4)

Compounds of formula (I-b-4) wherein $R^{19}$ is $C_{1-6}$alkyl may be prepared by cyclizing an intermediate of formula (XXVI) in the presence of a suitable dehydrating agent such as, for example, polyphosphoric acid, phosphorous pentoxide. In (XXVI) and (I-b-4) $R^{19-a}$ represents $C_{1-6}$alkyl.

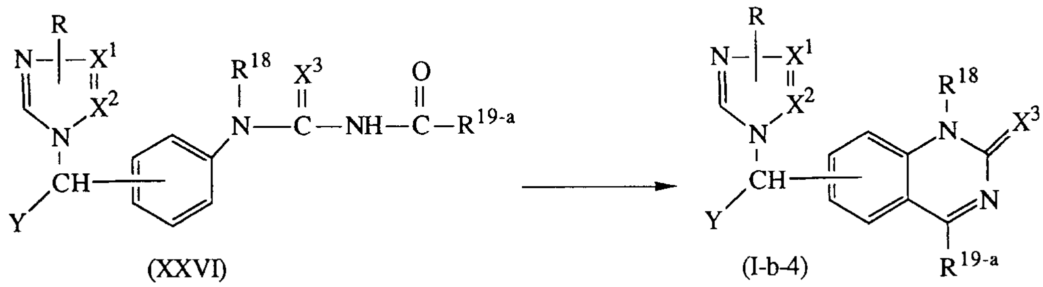

(XXVI) (I-b-4)

The compounds of formula (I-b-5), may be prepared by condensing an intermediate (XXVII) with a reagent L-C(=$X^3$)-L (XXII), as defined hereinabove.

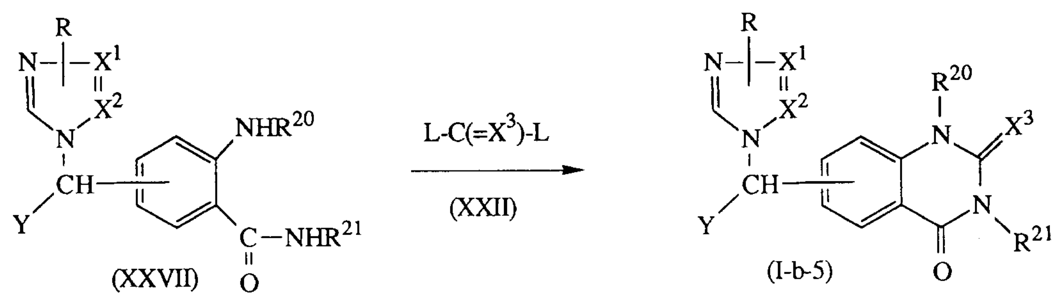


(XXVII) (I-b-5)

Said cyclization reaction may conveniently be conducted following the procedures described hereinabove for the preparation of the compounds of formula (I-b-2) from the intermediates (XIX-a) and the reagent L-C(=$X^3$)-L (XXII).

The compounds of formula (I-b-6), may be prepared by reacting an intermediate (XXVIII) with a carboxylic acid of formula (XIX) or a functional derivative thereof.

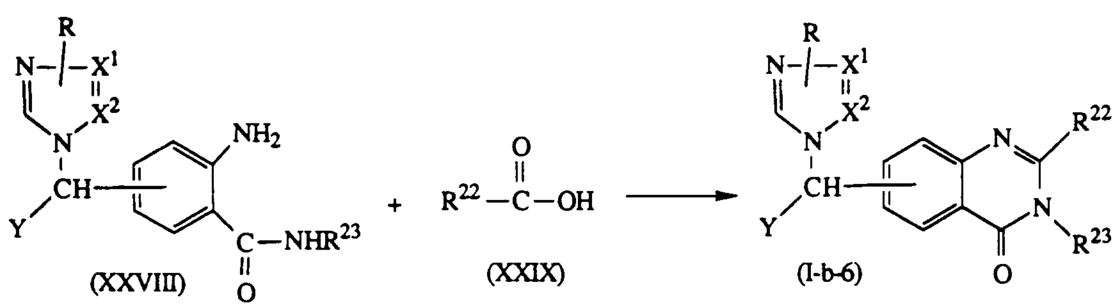

(XXVIII) (XXIX) (I-b-6)

Said functional derivative of (XXIX) is meant to include the halide, anhydride, amide and ester form of (XXIX), including the ortho and imino ester form thereof. The cyclization is carried out according to similar procedures as described herein before for the preparation of (I-b-1) starting from (XIX) and (XX).

The quinoxaline compounds of formula (I-c) can alternatively be prepared under similar conditions as are described in the literature by condensing an appropriate ortho-disubstituted benzene with a two-carbon synthon.

The compounds of formula (I) wherein Z is a radical (c-1) and $R^{24}$ is hydrogen, $C_{1-6}$alkyl or $Ar^2$, said compound being represented by formula (I-c-1-a), may be obtained by condensing an appropriate ortho-benzenediamine of formula (XXX-a) with a 1,2-diketone of formula (XXXI).

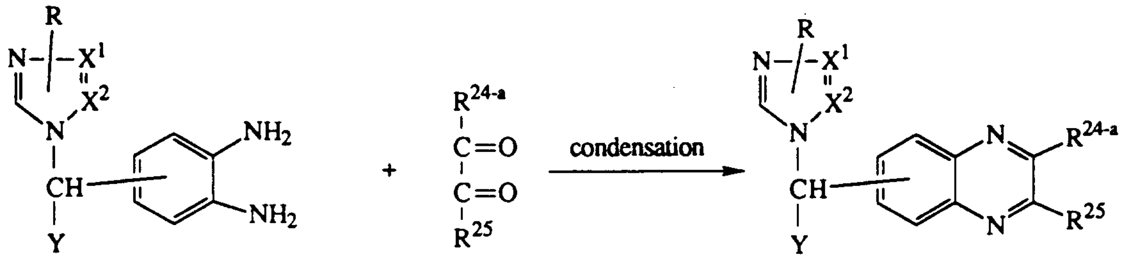


(XXX-a) (XXXI) (I-c-1-a)

In (XXXI) and (I-c-1-a) $R^{24-a}$ represents hydrogen, $C_{1-6}$alkyl or $Ar^2$.

The condensation of the (1H-azol-1-ylmethyl) substituted ortho-diamine of formula (XXX-a) and the 1,2-diketone of formula (XXXI) can be carried out by mixing the reactants in a suitable solvent such as, for example, an alkanol, e.g. methanol, ethanol, propanol; an ether, e.g. tetrahydrofuran, 1,4-dioxane, 1,1'-oxybisbutane; a halogenated hydrocarbon, e.g. trichloromethane, dichloromethane; a dipolar aprotic solvent, e.g., N,N-dimethylformamide, N,N-dimethylacetamide, dimethylsulfoxide; an aromatic hydrocarbon, e.g. benzene, methylbenzene, dimethylbenzene or mixtures of such solvents optionally in the presence of a carboxylic acid, e.g. acetic acid, a mineral acid such as, for example hydrochloric acid, sulfuric acid, or a sulfonic acid such as, for example, methanesulfonic acid, benzenesulfonic acid, 4-methylbenzenesulfonic acid. Somewhat elevated temperatures may be appropriate to enhance the rate of the reaction and in some cases the reaction may even be carried out at the reflux temperature of the reaction mixture. The water which is liberated during the condensation may be removed from the mixture by azeotropical distillation, distillation methods. As suitable 1,2-diketones of formula (XXXI) there may be named for example, ethanedial, diphenylethanedione, 2,3-butanedial two carbon synthons.

The compounds of formula (I) wherein Z is a radical of formula (c-2) and n is 0, said compounds being represented by (I-c-2-a), may be obtained by condensing an appropriate ortho-benzenediamine of formula (XXX-b) with an appropriate α-keto acid of formula (XXXII) or a functional derivative thereof such as, for example, an ester, a halide.

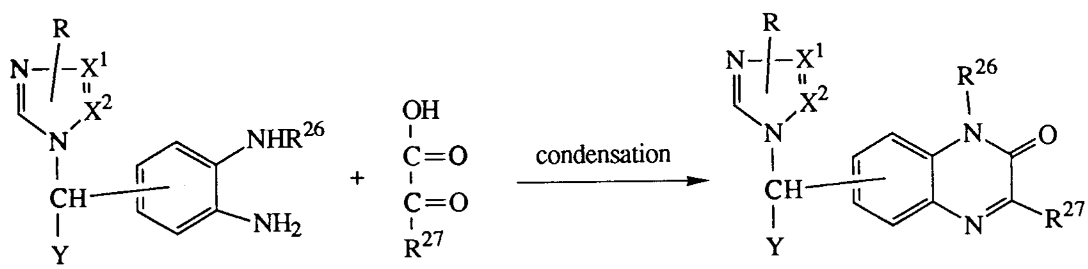


(XXX-b) (XXXII) (I-c-2-a)

The condensation of the (1H-azol-1-ylmethyl) substituted ortho-diamine of formula (XXX-b) and the α-keto acid or ester of formula (XXXII) can be carried out by mixing the reactants in a suitable solvent such as, for example, water, an alkanol, e.g. methanol, ethanol, propanol; an ether, e.g. tetrahydrofuran, 1,4-dioxane, 1,1'-oxybisbutane; a halogenated hydrocarbon, e.g. trichloromethane, dichloromethane; a dipolar aprotic solvent, e.g. N,N-dimethylformamide, N,N-dimethylacetamide, dimethylsulfoxide; an aromatic hydrocarbon, e.g. benzene, methylbenzene, dimethylbenzene; and mixtures of such solvents optionally in the presence of a carboxylic acid, e.g. acetic acid, a mineral acid such as, for example hydrochloric acid, sulfuric acid, or a sulfonic acid such as, for example, methanesulfonic acid, benzenesulfonic acid, 4-methylbenzenesulfonic acid. Somewhat elevated temperatures may be appropriate to enhance the rate of the reaction and in some cases the reaction may even be carried out at the reflux temperature of the mixture. The water which is liberated during the condensation may be removed from the mixture by azeotropical distillation, distillation methods. As representative α-keto acids of formula (XXXII) there may be named 2-oxopentanoic acid, 2-oxoacetic acid, 2-oxopropanoic acid acids. As suitable α-keto esters there may be named for example, ethyl 2-oxopropanoate, ethyl 4-methyl-2-oxopentanoate, ethyl 3-methyl-2-oxobutanoate, methyl β-oxobenzeneacetate, diethyl 2-methyl-3-oxo-1,4-butanedioate, diethyl-1,3-propanedioate esters. As suitable halides

there may be named 2-oxopropanoyl chloride, dichloroacetyl chloride, diethoxyacethyl chloride.

In some instances the reaction of (XXX-b) with (XXXII) first yields an intermediate of formula (XXXIII-a) which may in situ or, if desired, after isolating and purifying it, be cyclized by heating it in the presence of an acid such as, for example, a carboxylic acid, e.g. acetic acid, a mineral acid such as, for example hydrochloric acid, sulfuric acid, or a sulfonic acid such as, for example, methanesulfonic acid, benzenesulfonic acid, 4-methylbenzenesulfonic acid.

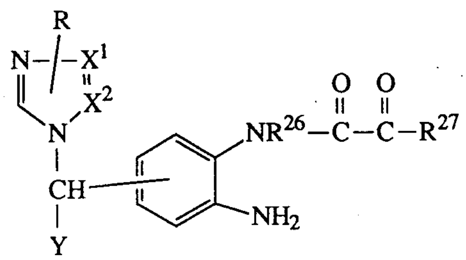

(XXXIII-a)

Alternatively compounds of formula (I-c-2-a) may be prepared by the reduction of an intermediate of formula (XXXIII-b).

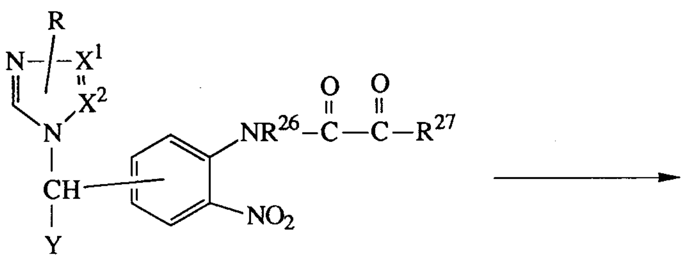

(I-c-2-a)

(XXXIII-b)

The reduction and cyclization of (XXXIII-b) can conveniently be conducted by stirring the starting compound in a reaction inert solvent such as, for example, an alkanol, e.g. methanol, ethanol, propanol, an ester, e.g. ethyl acetate, butylacetate, an aromatic hydrocarbon, e.g. benzene, methylbenzene; a halogenated hydrocarbon, e.g. chloromethane in the presence of hydrogen and an appropriate metal catalyst such as, for example, palladium-on-charcoal, Raney nickel, optionally at an elevated temperature and/or pressure.

The compounds of formula (I) wherein Z is a radical of formula (c-2) wherein n is 1, said compounds being represented by formula (I-c-2-b) may be prepared by cyclizing an ortho-nitroanilide containing a suitable activated methylenegroup of formula (XXXIV-a).

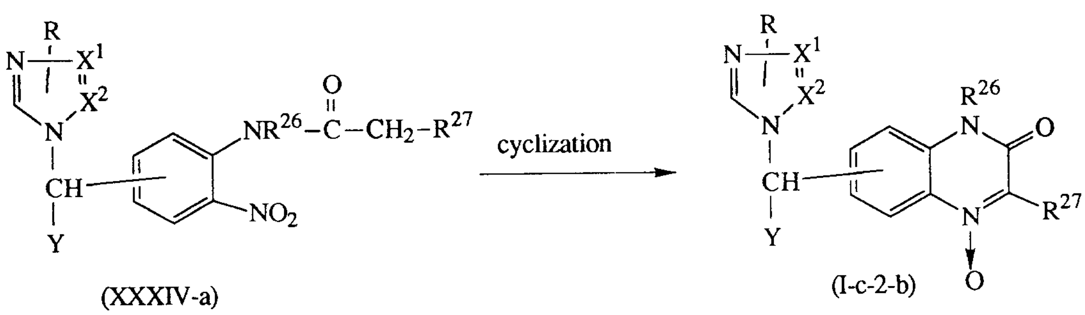


(XXXIV-a) (I-c-2-b)

The base promoted cyclization of (XXXIV-a) can be conducted according to art-known cyclizing procedures as described in, for example, J. Chem. Soc., 1963, 2429; J. Med. Soc., 1966, 2285 and J. Org. Chem., 1968, 30, 201 by stirring, and optionally heating the ortho-nitroanilide (XXXIV-a) in a suitable solvent such as, for example water, an alcohol, e.g. methanol, ethanol; a polar aprotic solvent, e.g. pyridine; a ketone, e.g. propanone; an aromatic hydrocarbon e.g. benzene, dimethylbenzene; a halogenated hydrocarbon, e.g. trichloromethane, tetrachloromethane; an ether, e.g. tetrahydrofuran or a mixture of such solvents, in the

presence of an appropriate base. Suitable bases are for example, an alkaline metal or an earth alkaline metal carbonate, hydrogen carbonate, hydroxide or hydride, e.g. sodium carbonate, sodium hydrogen carbonate, potassium carbonate, sodium hydroxide, sodium hydride, or an organic base such as, for example, a tertiary amine, e.g. N-(1-methylethyl)-2-propanamine. Depending on the reaction conditions and the nature of the activating group $R^{27}$, the obtained 3-substituted quinoxaline-N-oxide of formula (I-c-2-b) may be decomposed to give the corresponding unsubstituted N-oxide wherein $R^{27}$ is hydrogen.

The compounds of formula (I-c-2-b) can also be prepared by cyclizing an ortho anilide of formula (XXXIV-b).

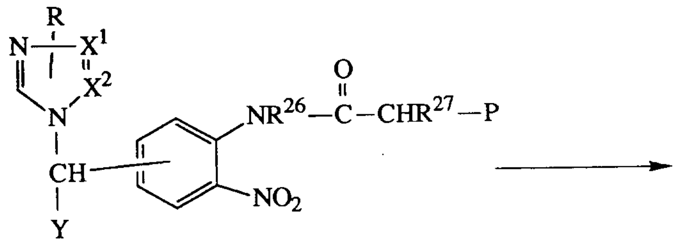

(I-c-2-b)

(XXXIV-b)

In (XXXIV-b) P represents a suitable activating group such as, for example, $C_{1-4}$alkylcarbonyl, arylcarbonyl. The base promoted cyclization of (XXXIV-b) can be carried out according similar procedures as described hereinabove for the cyclization of (XXXIV-a). Similar cyclization procedures are also outlined in J. Chem. Soc. 1963, p. 2431 and J. Chem. Soc. 1964, p. 2666

The quinoxaline-2,3-diones of formula (I-c-3) can be prepared by condensing an intermediate of formula (XXX-c) with oxalic acid (XXXV) or a functional derivative thereof such as, for example an ester or halide.

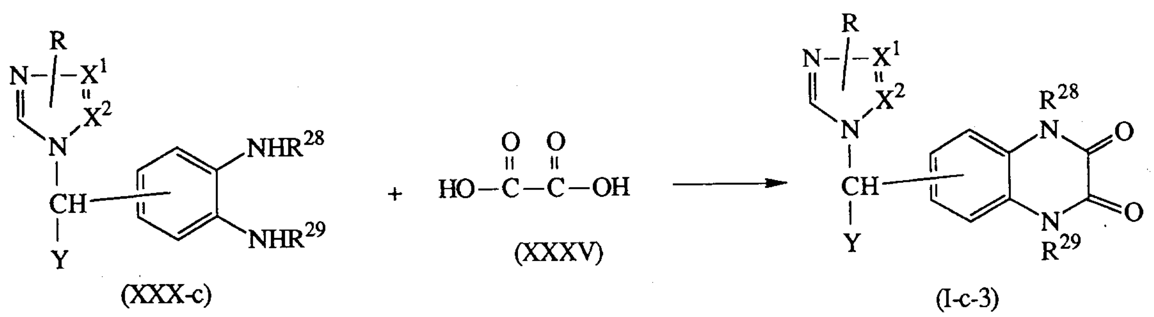

(XXX-c) (XXXV) (I-c-3)

The condensation of (XXX-c) and (XXXV) is conveniently carried out by mixing the reactants, optionally in a reaction inert solvent such as, for example, water; an alkanol, e.g. methanol, ethanol; a halogenated hydrocarbon, e.g. trichloromethane, dichloromethane; an ether, e.g. tetrahydrofuran; a dipolar aprotic solvent, e.g. N,N-dimethylformamide, N,N-dimethylacetamide, dimethylsulfoxide, an aromatic hydrocarbon, e.g. benzene, methylbenzene, dimethylbenzene; an ester, e.g. ethyl acetate or a mixture of such solvents optionally in the presence of a carboxylic acid, e.g. acetic acid, a mineral acid such as, for example hydrochloric acid, sulfuric acid, or a sulfonic acid such as, for example, methanesulfonic acid, benzenesulfonic acid, 4-methylbenzenesulfonic acid. In some instances the reaction may even be carried out in an excess of carboxylic acid, e.g. acetic acid. Somewhat elevated temperatures may be appropriate to enhance the reaction and in some cases the reaction may even be carried out at the reflux temperature of the mixture. The water or acid which is liberated during condensation may be removed by azeotropical distillation, distillation, complexation, salt formation methods.

The compounds of formula (I) wherein Z is a radical of formula (c-4) may be prepared by condensation of an ortho diamine of formula (XXX-d) with an α-halo acid of formula (XXXVI) or the ester form thereof

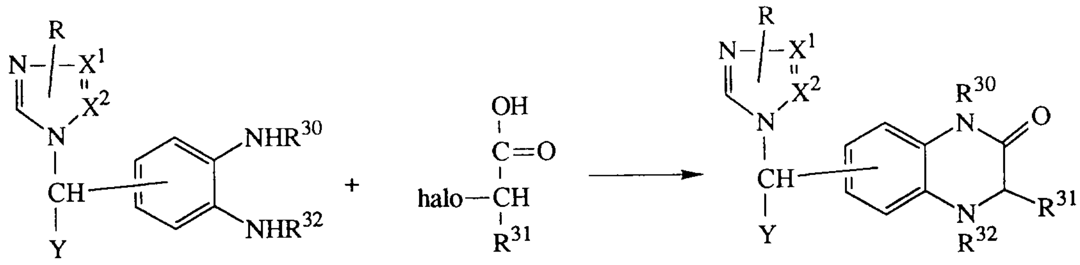

(XXX-d) (XXXVI) (I-c-4)

The above mentioned condensation can be carried out by stirring the reactants at an enhanced temperature in a suitable solvent such as, for example, water; an alkanol, e.g. methanol, ethanol, propanol; an ether, e.g. 1,4-dioxane, 1,1'-oxybisethane, tetrahydrofuran; an ester, e.g. ethylacetate; a halogenated hydrocarbon, e.g. trichloromethane, tetrachloromethane; a dipolar aprotic solvent, e.g. N,N-dimethylformamide, N,N-dimethylacetamide, dimethylsulfoxide, an aromatic hydrocarbon, e.g. benzene, methylbenzene, dimethylbenzene; a ketone, e.g. 2-propanone, 4-methyl-2-pentanone and mixtures of such solvents. The addition of an appropriate base such as, for example, an alkali metal carbonate, hydrogen carbonate or hydroxide, e.g. sodium carbonate, sodium hydrogen carbonate, ammonium hydroxide or an organic base such as, for example, N,N-diethylethanamine, may be utilized to pick up the acid which is liberated during the course of the reaction.

Alternatively the α-ketotetrahydroquinoxalines of formula (I-c-4) wherein $R^{30}$ is hydrogen, said compounds being represented by (I-c-4-a) may be prepared by the reduction of an appropriately substituted ortho-nitrophenylglycine of formula (XXXVII).

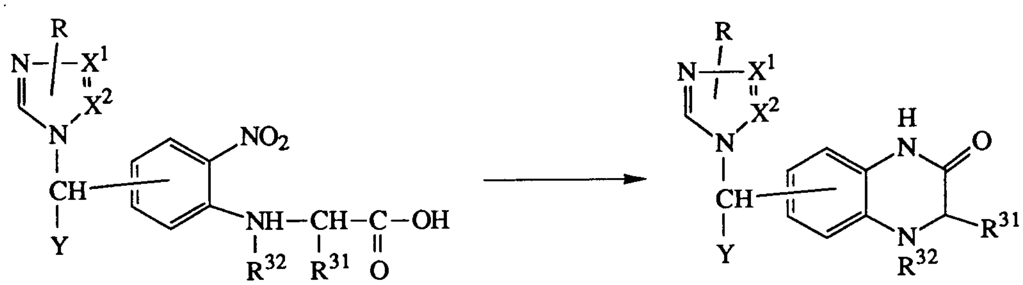

(XXXVII) (I-c-4-a)

The reduction of the ortho-nitrophenylglycine of formula (XXXVII) can conveniently be conducted by stirring the starting material in a reaction-inert solvent such as, for example, an alkanol, e.g. methanol, ethanol, propanol, an ester, e.g. ethyl acetate, butylacetate, an aromatic hydrocarbon, e.g. benzene, methylbenzene; a halogenated hydrocarbon, e.g. chloromethane in the presence of hydrogen and an appropriate metal catalyst such as, for example, palladium-on-charcoal, Raney nickel, optionally at an elevated temperature and/or pressure.

Alternatively the reduction may be carried out with sodium dithionite in the presence of acetic acid or in aqueous alkanol, e.g. an aqueous ethanol solution.

Alternatively, some compounds of formula (I) may also be prepared according to procedures analogous to those described in the literature for the preparation of azoles by cyclizing an appropriate starting material.

The compounds of formula (I-x) may also be prepared, for example, by cyclizing an intermediate of formula (XXXVIII) and desulfurating the thus obtained intermediate of formula (IXL).

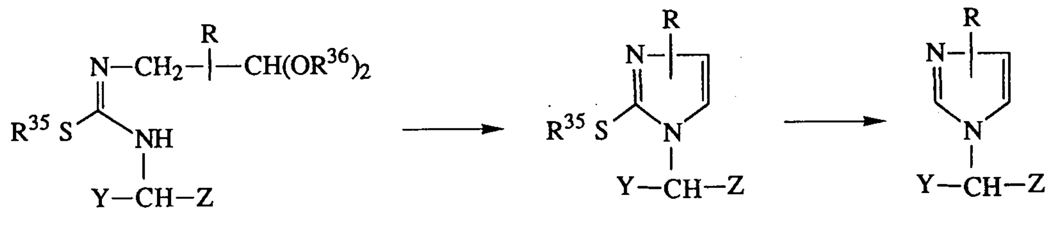

(XXXVIII) (IXL) (I-x)

In formulae (XXXVIII) and (IXL) $R^{35}$ represents hydrogen or $C_{1-6}$alkyl and $R^{36}$ represents $C_{1-6}$alkyl or both $R^{36}$ taken together form a $C_{2-3}$alkanediyl radical.

Said cyclization reaction may conveniently be conducted by stirring and heating intermediate (XXXVIII) in an aqueous acidic solvent, e.g. in aqueous hydrochloric or sulfuric acid. The intermediate (IXL) may be desulfurated following art-known procedures, e.g., by treatment with Raney nickel in the presence of an alkanol, e.g. methanol, ethanol, or by treatment with nitric acid, optionally in the presence of sodium nitrite.

The compounds of formula (I-y) may be prepared from a hydrazine derivative of formula (XL) by reaction with s-triazine following the procedures described in J. Org. Chem., 1956, 1037.

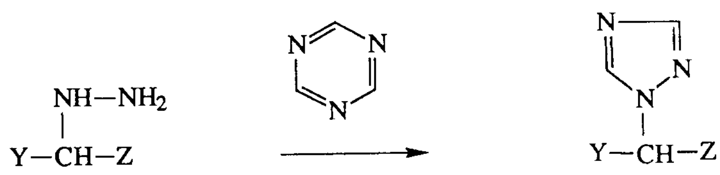

(XL) (I-y)

The intermediate hydrazine (XL) and the corresponding intermediate amine of formula Y-CH($NH_2$)-Z - (XLI) may also advantageously he converted into azoles, wherein $-X^1=X^2-$ is a bivalent radical of formula (x), (y) or (z), following the procedures described in U.S. Pat. No. 4,267,179, incorporated herein by reference.

The compounds of formula (I) can also be converted into each other following art-known functional group transformation procedures.

The compounds of formula (I-a-1) wherein $R^1$ is hydrogen may be converted into compounds of formula (I-a-3) wherein $R^7$ is halo by treatment with a halogenating agent such as, for example, thionyl chloride, pentachlorophosphorane, phosphoryl chloride, sulfuryl chloride. The thus obtained compounds of formula (I-a-3) wherein $R^7$ is halo may further be converted into compounds of formula (I-a-3) wherein $R^7$ is $C_{1-6}$alkyloxy by reacting the starting compound with an appropriate alcohol, preferably an alkali metal or earth alkaline metal salt of said alcohol.

Depending on the nature of the substituents the compounds of formula (I-a-1) may also be converted into compounds of formula (I-a-2), by a selective hydrogenation of the starting compound with an appropriate reducing agent such as, for example with a nobel catalyst, such as platinum-on-charcoal, palladium-on-charcoal. Dehydrogenation of the compounds of formula (I-a-2) may result in a compound of formula (I-a-1). The dehydrogenation may be accomplished by stirring and optionally heating the starting compound with alkaline peroxide, ammoniacal silver nitrate, 2,3-dichloro-5,6-dicyano-p-benzoquinone manganese(IV)oxide, bromine in the presence of bromobenzen in suitable reaction-inert solvent. Suitable solvents for said dehydrogenation are, for example, water, alkanols, e.g. methanol, ethanol, ketones, e.g. 2-propanone, halogenated hydrocarbons, e.g. trichloromethane, tetrachloromethane, ethers, e.g. 1,1-oxybisethane, dipolar aprotic solvents, e.g. N,N-dimethylformamide, N,N-dimethylacetamide, pyridine, or a mixture of such solvents. Some compounds of formula (I) may also be N-alkylated or N-aminated according to art known procedures.

The compounds of formula (I-b-4) wherein $R^{18}$ and $R^{19}$ are both hydrogen may be converted into compounds of formula (I-b-3) wherein $R^{17}$ is halo by treatment with a halogenating agent such as, for example, phosphoryl chloride, thionylchloride, pentachlorophosphorane, sulfurylchloride. The thus obtained compounds of formula (I-b-3) wherein $R^{17}$ is halo may further be converted into compounds wherein $R^{17}$ is

$C_{1-6}$alkyloxy by reacting the starting compound with an appropriate alcohol, preferably an alkali metal or earth alkaline metal salt of said alcohol. According to the same functional group transformation reactions, the compounds of formula (I-b-2) wherein $R^{15}$ is hydrogen may be converted into the corresponding compounds of formula (I-b-1). The compounds of formula (I-b-3) can also be obtained by oxidizing a compound of formula (I-b-1) with an appropriate oxidizing reagent in a suitable reaction-inert solvent. Appropriate oxidizing reagents are, for example, permanganate or manganese(IV)oxide, silver oxide, silver nitrate, tert. butylhydroperoxide, hypochlorite, chromic acid, ferric chloride, ferric cyanide, lead tetra-acetate. Suitable solvents for said oxidation reactions are, for example, water, alkanols, e.g. methanol, ethanol, ketones, e.g. 2-propanone, 4-methyl-2-pentanone, halogenated hydrocarbons, e.g. trichloromethane, tetrachloromethane, ethers, e.g. 1,1'-oxybisethane, tetrahydrofuran, 1,4-dioxane, dipolar aprotic solvents, e.g. N,N-dimethylformamide, N,N-dimethylacetamide, pyridine, or a mixture of such solvents. Following analogous oxidation procedures the compounds of formula (I-b-4) may be obtained from the compounds of formula (I-b-2).

The deoxygenation of the N-oxide of formula (I-c-2) can be carried out by stirring and, if desired, heating the starting compounds in a suitable solvent in the presence of hydrogen or hydrazine and an appropriate metal catalyst such as, for example, Raney nickel, Raney cobalt, platinum-on-charcoal, palladium-on-charcoal metal catalysts. Suitable solvents are water, an alkanol, e.g. methanol, ethanol, an ether, e.g. tetrahydrofuran, and mixtures of such solvents whereto an appropriate base has been added such as, for example, an alkali metal carbonate, hydrogen carbonate or hydroxide, e.g. sodium carbonate, sodium hydrogen carbonate, sodium hydroxide. Alternatively the deoxygenation of the N-oxide of formula (I-c-2-b) may be carried out with sodium dithionite in the presence of acetic acid or in an aqueous alkanol, e.g. an aqueous ethanol solution. It further proved possible to accomplish the deoxygenation by stirring the N-oxide in the presence of zinc and acetic acid.

The $\alpha$-ketotetrahydroquinoxalines of formula (I-c-4) may also be converted to a quinoxaline of formula (I-c-2) according to art-known dehydrogenation procedures as described for example J. Chem. Soc., 1953, 2816. For example, the dehydrogenation of the compounds of formula (I-c-4) can be carried out by heating the starting compound in an aqueous alkaline solution optionally in the presence of an appropriate oxidant such as, for example, peroxide, silver nitrate or manganese(IV) oxide.

Compounds of formula (I-c-2) wherein $R^{26}$ is hydrogen and n is 0 may also be converted into the corresponding compounds of formula (I-c-1) wherein $R^{24}$ is halo by treatment with a halogenating agent such as, for example, thionyl chloride, phosphoryl chloride, pentachloro phosphorane, sulfuryl chloride. The thus obtained compounds wherein $R^{24}$ is halo may further be converted into quinoxalines of formula (I-c-1) wherein $R^{24}$ is $C_{1-6}$alkyloxy or mono or di($C_{1-6}$alkyl)amino by reacting the starting compounds with an appropriate amine or alcohol, preferably an alkali metal or earth alkaline metal salt of said alcohol. Some compounds of formula (I-c) may also be N-alkylated or N-aminated according to art known procedures.

Compounds of formula (I-c-5) can be obtained by reducing the corresponding compounds of formula (I-c-1) with an appropriate reducing agent such as, for example, an alkali metal borohydride, e.g. lithium, potassium or preferably, sodium borohydride, sodium cyanoborohydride reducing agents in a reaction inert solvent.

A number of intermediates and starting materials in the foregoing preparations are known compounds which may be prepared according to art-known methodologies of preparing said or similar compounds. Some intermediates of the previous reaction schemes are novel and have especially been developed for conversion into compounds of the present invention.

Intermediates of formula (III), (V) and (VII-a) wherein Y is other than hydrogen may be prepared from an appropriately substituted quinoline or quinolinone, quinazoline or quinoxaline derivative of formula (XLII) according to the following reaction sequence.

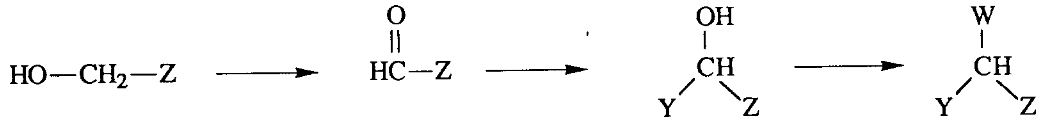

(XLII) (VII-a) (V) (III)

The hydroxymethyl moiety in the starting intermediate of formula (XLII) is first converted into a formyl moiety with a suitable oxidant, e.g. manganese(IV) oxide or potassium permanganate, and subsequently reacted with a metal alkyl, e.g. methyllithium, butyllithium, metal aryl, e.g. phenyllithium, or with a complex

metal alkyl or aryl in a suitable solvent, e.g. tetrahydrofuran, 1,1'-oxybisethane to form the secundary alcohols (V). The desired intermediates of formula (III) may then be obtained by converting the alcohol function of the intermediate of formula (V) into an appropriate leaving group W following standard procedures as known in the art. For example, halides are generally prepared by the reaction of (V) with an appropriate halogenating agent such as, for example, thionyl chloride, sulfuryl chloride, pentachlorophosphorane, pentabromophosphorane, phosphorylchloride, hydrochloric acid, hydrobromic acid halogenating agents. The intermediates of formula (III) wherein Y is hydrogen can be obtained directly from the intermediates of formula (XLII) following the procedure described hereinabove for converting (V) into (III).

Some intermediates of formula (III) wherein Y is other than hydrogen may also be prepared by acylating a quinoline or quinolinone, quinazoline or quinoxaline of formula (XLIV) with an appropriate reagent of formula (XLIII) according to art-known Friedel-Crafts acylation reaction procedures, reducing the thus obtained ketone (VII-b) with an appropriate reductant, e.g. sodium borohydride in a suitable solvent such as water; an alcohol e.g. methanol, ethanol or mixtures thereof with tetrahydrofuran optionally in the presence of sodium hydroxide and subsequently converting the alcohol function into an appropriate leaving group as described hereinbefore.

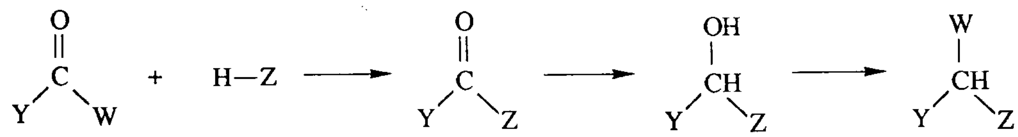

(XLIII) (XLIV) (VII-b) (V) (III)

Some intermediates of formula (III) may also be prepared by cyclizing an appropriate benzaldehyde or ketone derivative of the general formula (XLV) according to similar cyclization procedures as described hereinabove for the synthesis of the compounds of formula (I-a-1), (I-a-2), (I-a-3), (I-a-4), (I-b-1), (I-b-2), (I-b-3), (I-b-4), (I-b-5), (I-b-6), (I-c-1), (I-c-2), (I-c-3), (I-c-4) or (I-c-5) reducing the thus obtained quinoline, quinazoline, quinoxaline or quinolinone with an appropriate reductant, e.g. sodium borohydride, sodium cyanoborohydride reagents and subsequently converting the alcohol function of (V) in an appropriate leaving group. Depending on the cyclization procedure it may be useful to protect the ketone or aldehyde group according to art known procedures e.g. by acetalization.

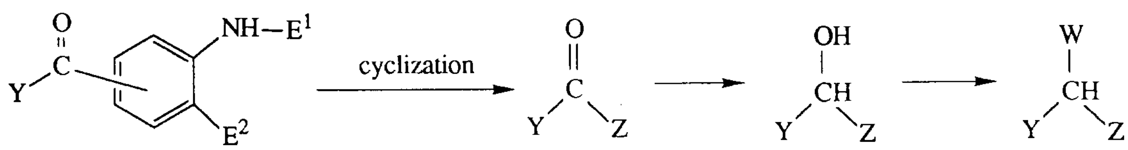


(XLV) (VII) (V) (III)

In (XLV) the meanings of $E^1$ and $E^2$ are selected in such a manner to enable a cyclization reaction. For example, as appropriate intermediates of formula (XLV) there may be mentioned:

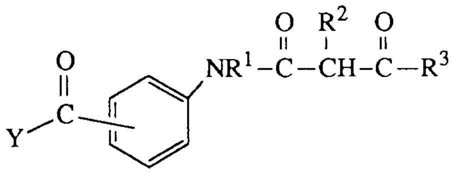

(XLV-a)

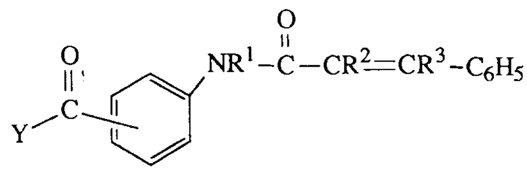

(XLV-b)

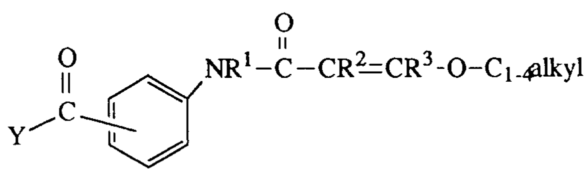

(XLV-c)

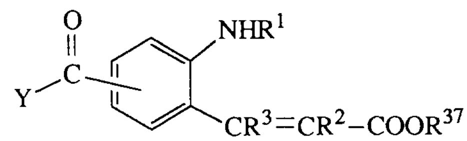

(XLV-d)

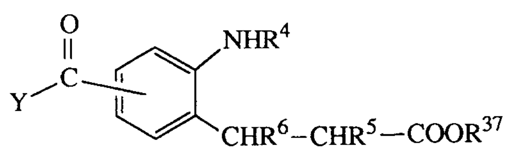

(XLV-e)

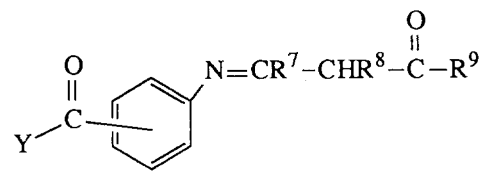

(XLV-f)

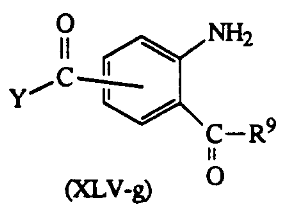

**(XLV-g)**

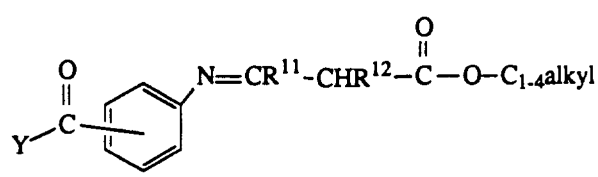

**(XLV-h)**

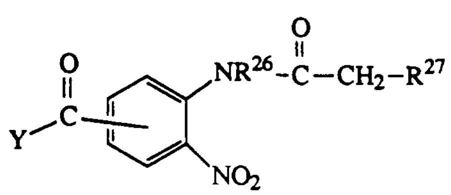

**(XLV-i)**

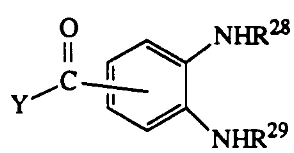

**(XLV-j)**

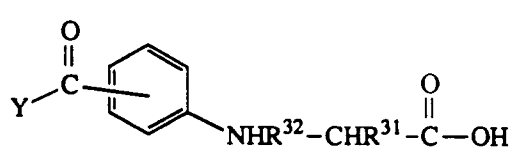

**(XLV-k)**

More particular intermediates to prepare quinoline compounds may be prepared according the following procedures.

Intermediates of formula (IX), (X)and (XI) can conveniently be prepared by reacting an aniline (VIII-a) with respectively a carboxylic acid of formula (XLVI-a), (XLVI-b) or (XLVI-c) or a functional derivative thereof.

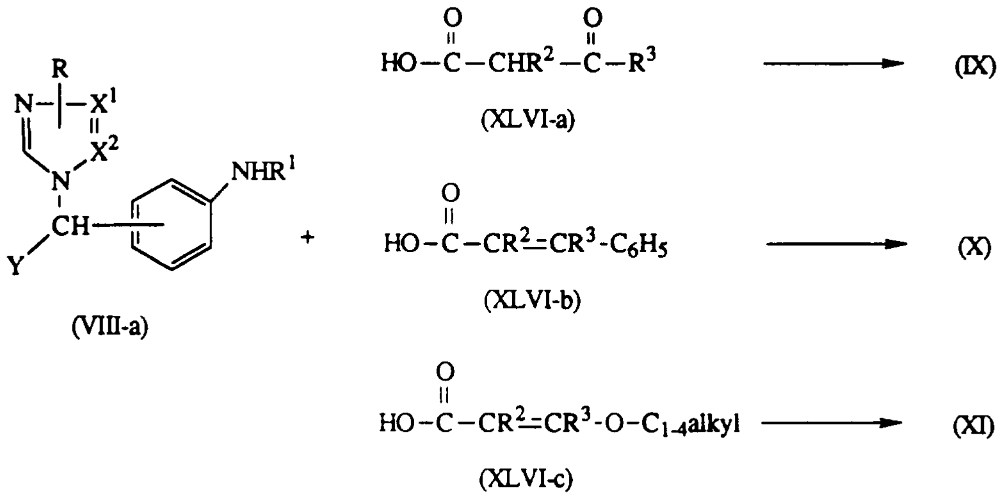

(VIII-a) (XLVI-a) (IX) (XLVI-b) (X) (XLVI-c) (XI)

Said functional derivatives of formula (XLVI-a), (XLVI-b) and (XLVI-c) are meant to comprise the halide, anhydride, amide and ester forms of (XLVI-a), (XLVI-b) and (XLVI-c). (XLVI-a) may also be in the form of a reactive lactone such as, for example, 4-methylene-2-oxetanone.

Functional derivatives may be prepared following art-known procedures, for example, by reacting the carboxylic acid of formula (XLVI) with thionyl chloride, phosphorous trichloride, polyphosphoric acid, phosphoryl chloride, or by reacting the carboxylic acid of formula (XLVI) with an acyl halide, e.g. acetyl chloride, ethyl carbonochloridate. Or the intermediates (VIII-a) and (XLVI) may be coupled in the presence of a suitable reagent capable of forming amides, e.g. 1,1'-carbonylbis[1H-imidazole], dicyclohexylcarbodiimide, 2-chloro-1-methylpyridinium iodide.

Said amidation reactions may conveniently be carried out by stirring the reactants in a suitable reaction-inert solvent, such as, for example, a halogenated hydrocarbon, e.g. dichloromethane, trichloromethane, an aromatic hydrocarbon, e.g. methylenzene, an ether, e.g. 1,1'-oxybisethane, tetrahydrofuran or a dipolar aprotic solvent, e.g. N,N-dimethylformamide N,N-dimethylacetamide. The addition of a suitable base may be appropriate, in particular a tertiary amine such as, N,N-diethylethanamine. The water, the alcohol or the acid which is liberated during the course of the reaction may be removed from the reaction mixture according methodologies generally known in the art such as, for example, azeotropical distillation, complexation and salt formation.

When a reactive lactone of formula (XLVI-a) is used the reaction may be carried out according to similar procedures as outlined in Organic Synthesis, Willy New York, 1955, Collect. Vol. III page 10.

The intermediate of formula (XII) and/or (XIII) can be prepared by reducing the nitro derivative of formula (XLVII) in the presence of hydrogen and a suitable metal catalyst such as, for example, palladium-on-charcoal, platinum oxide catalysts. The nitro derivative of formula (XLVII) in turn can be prepared from an aldehyde of formula (XLVIII) by reacting the latter with a phosphorous ylide of formula (IL) or with an ylide of formula (L) prepared from a phosphonate.

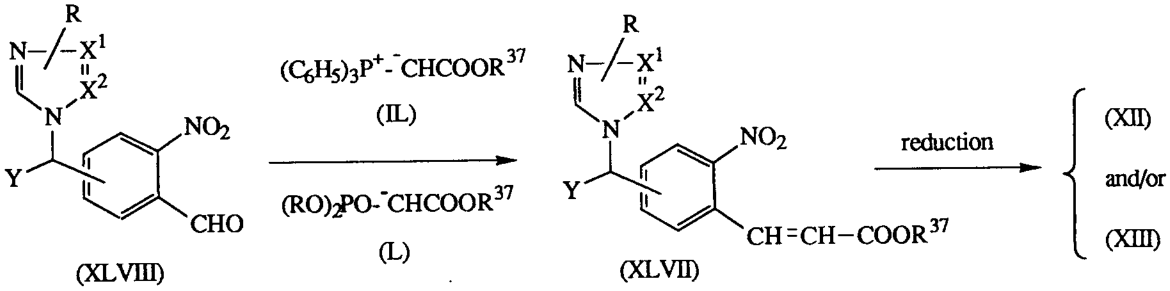


(XLVIII) (XLVII) (XII) and/or (XIII)

In formula (XLVII) $R^{37}$ represents hydrogen or $C_{1-4}$alkyl.

The reaction of (XLVIII) with (IL) or (L) can conveniently he conducted by treating a phosphonium salt or a phosphonate with an appropriate base such as, for example, butyllithium, methyllithium, sodium amide, sodium hydride, a sodium or potassium alkoxide, sulfinylbis(methane) sodium salt bases, under an inert

atmosphere and in a reaction-inert solvent such as for example, a hydrocarbon, e.g. hexane, heptane, cyclohexane; an ether, e.g. 1,1'-oxybisethane, tetrahydrofuran, 1,2-dimethoxyethane; a dipolar aprotic solvent, e.g. dimethylsulfoxide, hexamethylphosphor triamide, solvents.

The starting intermediate (XLVIII) wherein the 1H-azole-1-ylmethyl moiety is substituted in the para position can for example be prepared according the following reaction sequence.

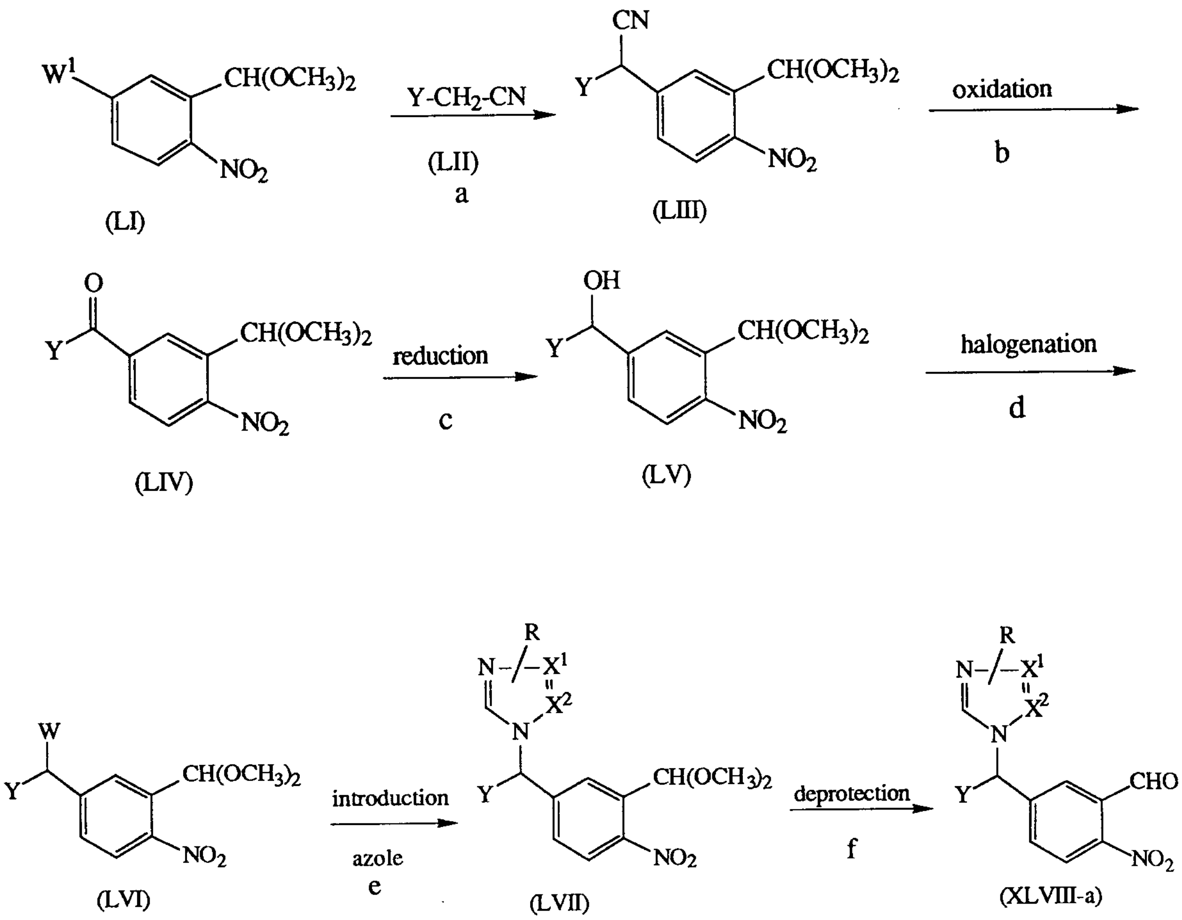


In formula (LI) $W^1$ represents a reactive leaving group such as, for example, halo, e.g. chloro or fluoro, nitro, 4-methylbenzenesulfonyloxy, phenyloxy, alkyloxy groups.

a) The aromatic nucleophilic substitution on a nitrobenzene of formula (LI) with a cyanide of formula (LII) can be conducted by stirring the reactants in the presence of a base in a reaction inert solvent such as for example, a dipolar aprotic solvent, e.g. N,N-dimethylformamide, N,N-dimethylacetamide, hexamethylphosphoric triamide, pyridine, 1,3-dimethyl-3,4,5,6-tetrahydro-2(1H)-pyrimidinone, 1,3-dimethylimidazolidinone, 1,1,3,3-tetramethylurea, 1-methyl-2-pyrrolidinone, nitrobenzene solvents; or mixtures thereof. Appropriate bases are sodium hydride, sodium amide, sulfinylbis(methane) sodium salt bases. It may be advantageous to add to the reaction mixture a crown ether, e.g. 1,4,7,10,13,16-hexaoxacyclooctadecane or a complexing agent such as for example, tris[2-(2-methoxyethoxy)]-ethanamine. Somewhat elevated temperatures may enhance the rate of the reaction.

b) The oxidation of the cyanide of formula (LIII) can be accomplished following art-known oxidation procedures as described in J. Org. Chem., 1975, 40, 267.

c) The reduction of the aldehyde or ketone of formula (LIV) can be carried out by stirring the latter with an appropriate reductant, e.g. sodium borohydride in a suitable solvent, e.g. methanol, ethanol.

d) The halogenation of the alcohol of formula (LV) can he accomplished by reacting the alcohol with a suitable halogenating agent, e.g., thionyl chloride, methanesulfonyl chloride.

e) The introduction of the azole can be carried out according procedures outlined hereinbefore for the synthesis of (I) from (II) and (III).

f) The deprotection of the carboxaldehyde group of (LVII) can easily be conducted following art-known methods of hydrolyzing acetals, e.g. by acid hydrolysis in an aqueous medium.

The intermediates of formula (XIV) and (XVIII) may be prepared by reacting an aniline of formula (VIII) with a 1,3-dicarbonyl of formula $R^7$-C(=O)-$CHR^8$-C(=O)-$R^9$ (LVIII) or $R^{11}$-C(=O)-$CHR^{12}$-C(=O)-O-$C_{1-4}$alkyl (LIX) in a reaction-inert solvent in the presence of an appropriate acid catalyst such as, for example, a sulfonic acid, e.g. methanesulfonic acid, benzenesulfonic acid, 4-methylbenzenesulfonic acid acids.

The starting compounds of formula (VIII) can easily be prepared according to procedures described in U.S. Patent No. 4,859,684 corresponding to EP-A-260,744 incorporated herein by reference for the process of preparing the intermediate of formula (VIII).

More particularly intermediates to prepare quinazoline compounds may be prepared as follows.

The intermediates of formula (XIX) can generally be prepared from amides, ureas or carbamates of formula (XXI-a) following art-known hydrolysis procedures, for example, by treating said amides, ureas or carbamates (XXI-a) with an acidic or basic aqueous solution, optionally at an enhanced temperature.

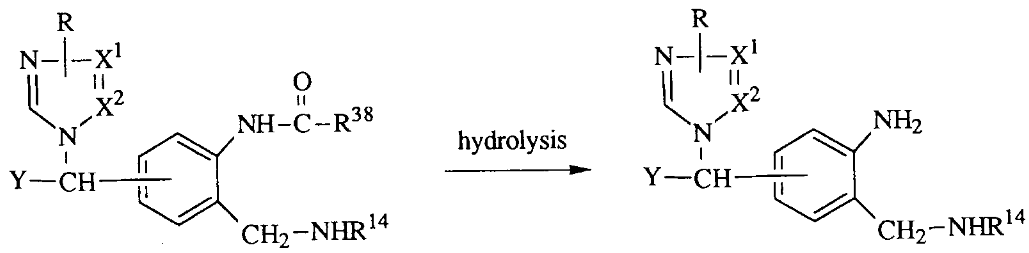


(XXI-a) (XIX)

In formula (XXI-a) and hereinafter $R^{38}$ represents either $C_{1-6}$alkyl, trifluoromethyl, $Ar^2$ or $Ar^2$-$C_{1-6}$alkyl; or $C_{1-6}$alkyloxy, amino or mono- or di($C_{1-6}$alkyl)amino.

The intermediates of formula (XXI-a) can be prepared by reducing an imine of formula (XXIII-a) following art-known reduction procedures such as, for example, reduction with an alkali metal borohydride, e.g. lithium, potassium, or preferably, sodium borohydride, sodium cyanoborohydride reagents, in a reaction-inert solvent such as, for example, an alkanol, e.g. methanol, ethanol.

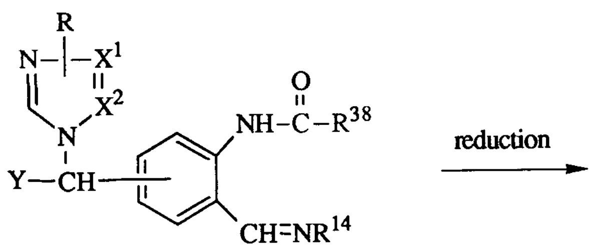


(XXI-a)

(XXIII-a)

The imines of formula (XXIII-a) in turn are prepared from an aldehyde of formula (XXIV-a) by reaction with an amine of formula $R^{14}$-$NH_2$ in a reaction-inert solvent in the presence of an appropriate acid catalyst such as, for example, a sulfonic acid, e.g. methanesulfonic, benzenesulfonic, 4-methylbenzenesulfonic acid acid catalysts.

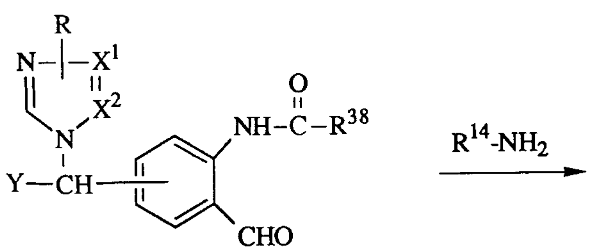


(XXIII-a)

(XXIV-a)

The aldehydes of formula (XXIV-a) can prepared from a derivative of formula (LX) wherein P represents a protected carboxaldehyde group or a protected hydroxymethyl group, by hydrolysis of the protective group and in the case of the hydroxymethyl group, oxidation to the carboxaldehyde group.

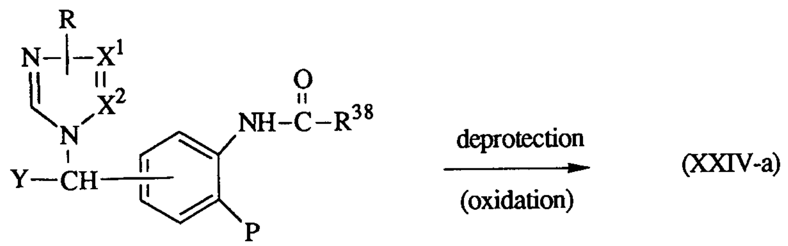


(LX)

Examples of suitable protective groups for hydroxymethyl are, for example, tetrahydropyranyl, 2-methoxyethoxymethyl, 2-methoxypropyl, 2-acetoxypropyl, 1-ethoxyethyl; a trialkylsilyl group, e.g. trimethylsilyl, tert. butyldimethylsilyl groups. Examples of suitable protective groups for carboxaldehyde are acyclic acetals formed with $C_{1-6}$alkanols such as methanol, ethanol; or cyclic acetals formed with diols such as, 1,2-ethanediol, 1,3-propanediol. Said deprotection reactions can easily be conducted following art-known methods of hydrolyzing acetals and silyl ethers, e.g. by acid hydrolysis in aqueous media.
Said oxidation of a hydroxymethyl to a carboxaldehyde group can conveniently be conducted by oxidation with a suitable oxidizing agent such as, for example, manganese (IV) oxide; permanganate salts, e.g. potassium permanganate; dimethylsulfoxide with a dehydrating reagent, e.g. oxalylchloride, sulfur trioxide, dicyclohexylcarbodiimide. Suitable solvents for said oxidation are, for example, water, halogenated hydrocarbons, e.g. dichloromethane, trichloromethane, tetrachloromethane.

The protected intermediates of formula (LX) are generally prepared from ketones of formula (LXI) following reaction sequences as described hereinabove for the conversion of ketones of formula (VII) into compounds of formula (I).

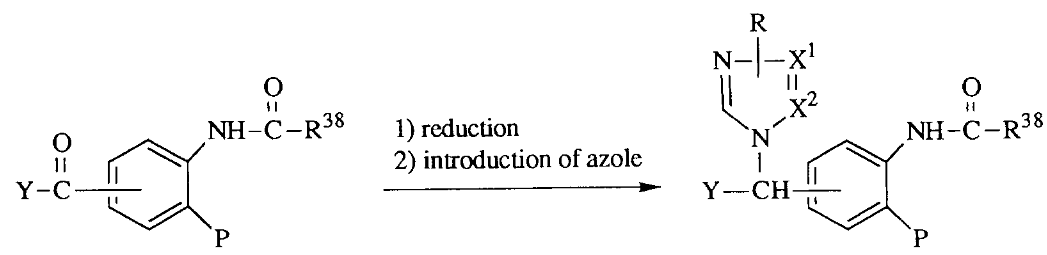


(LXI) (LX)

The intermediates of formula (LXI) are obtained from a suitably substituted nitrobenzene (LXII) by reduction following art-known nitro-to-amino reduction procedures, e.g. catalytic hydrogenation with Raney nickel, palladium-on-charcoal; and subsequently acylating the thus obtained aniline with a $C_{1-6}$alkanoic halide or anhydride, a $C_{1-6}$alkylcarbonohalidate, e.g. ethyl carbonochloridate, 1,1,-dimethylethyl carbonochloridate acylating reagents.

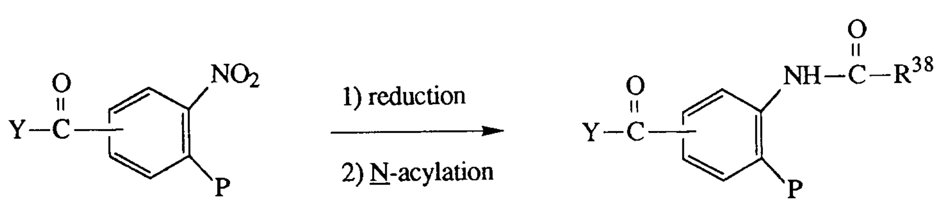


(LXII) (LXI)

The intermediates of formula (XXVII) wherein $R^{20}$ and $R^{21}$ are hydrogen or those of formula (XXVIII) wherein $R^{23}$ is hydrogen, said intermediates being represented by formula (LXIV), may, for example, be prepared from an appropriately substituted nitrobenzenamine of formula (LXII) by converting the latter into the corresponding nitrobenzenenitrile by diazotation and subsequent reaction with a cyanide salt e.g. copper cyanide and/or sodium cyanide, and reducing the thus obtained nitrobenzenenitrile under a hydrogen atmosphere, in the presence of an appropriate catalyst such as, for example, Raney nickel.

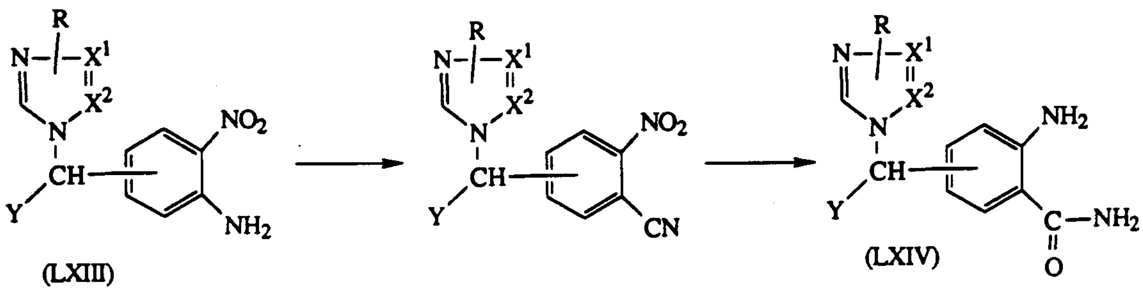

(LXIII) (LXIV)

The intermediates of formula (LXIV) can also be obtained from a ketone of formula (LXV) following the reaction sequences as described hereinabove for the conversion of ketones of formula (VII) into compounds of formula (I).

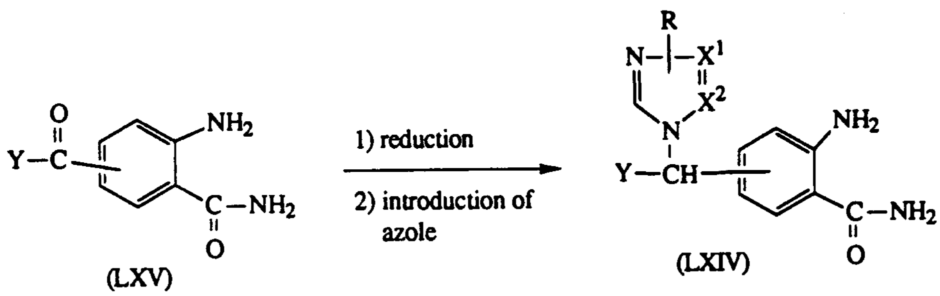


(LXV) (LXIV)

The intermediate ketones of formula (LXV) can be prepared from a suitably substituted 2-nitrobenzaldehyde of formula (LXVI) by reacting the aldehyde with hydroxylamine or an acid addition salt thereof and dehydrating the intermediate oxime to a benzenenitrile of formula (LXVII). The thus obtained nitrile is further hydrolyzed to an amide group and the nitro group reduced to an amino group following art-known hydrolysis and reduction procedures.

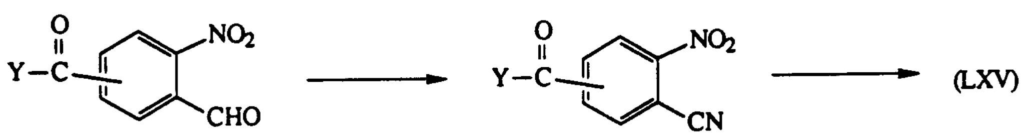

(LXVI) (LXVII)

The intermediates of formula (XXVII) and (XXVIII) wherein $R^{20}$ is hydrogen, said intermediates being represented by formula (LXVIII) can alternatively be prepared from a ketone of formula (LXIX) following the reaction sequences described hereinabove for the conversion of ketones of formula (VII) into compounds of formula (I).

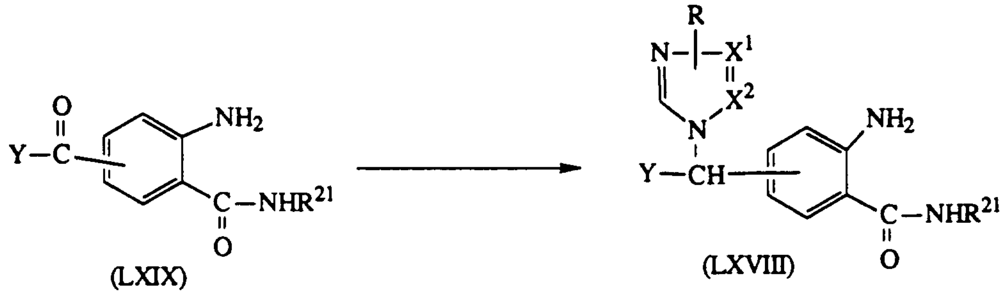

(LXIX) (LXVIII)

The intermediates of formula (LXIX) inturn can be obtained from a 2-nitrobenzoic acid of formula (LXX) by N-acylation of an amine $R^{21}$-$NH_2$ following art-known amidation procedures and reduction of the nitro group to an amino group according to procedures described hereinabove, for example, in the conversion of (LXII) into (LXI).

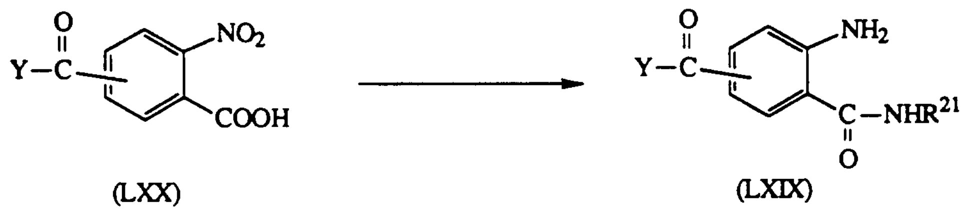

(LXX) (LXIX)

More particular intermediates to prepare quinoxaline compounds may be prepared according the following procedures. The intermediates of formulae (XXXIII-b), (XXXIV-a) and (XXXIV-b) can conveniently be prepared by reacting an intermediate (LXXII) with a carboxylic acid of formula (LXXI-a), (LXXI-b) or (LXXI-c) or a functional derivative thereof.

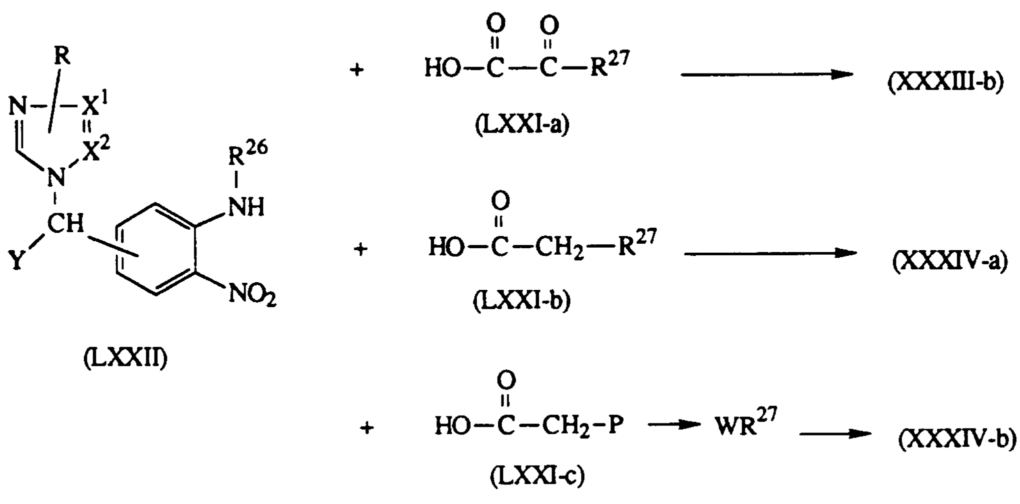

(LXXI-a) (XXXIII-b)

(LXXI-b) (XXXIV-a)

(LXXII)

(LXXI-c) (XXXIV-b)

Said functional derivative of (LXXI-a), (LXXI-b) or (LXXI-c) are meant to comprise the halide, a symmetrical or mixed anhydride, amide and ester forms of (LXXI-a), (LXXI-b) or (LXXI-c). In the instance where $R^{27}$ represents a $C_{1-4}$alkylcarbonyl group in formula (LXXI-b) the hydroxyl group taken together with $R^{27}$ may also form a reactive lactone such as, for example, 4-methylene-2-oxetanone.

Functional derivatives may be prepared following art-known procedures, for example, by reacting the carboxylic acid of formula (LXXI) with thionyl chloride, phosphorous trichloride, polyphosphoric acid, phosphoryl chloride, or by reacting the carboxylic acid of formula (LXXI) with an acyl halide, e.g. acetyl chloride, ethyl carbonochloridate. Or the intermediates (LXXI) and (LXXII) may be coupled in the presence of a suitable reagent capable of forming amides, e.g. dicyclohexylcarbodiimide, 1,1'-biscarbonyl[1H-imidazole], 2-chloro-1-methylpyridinium iodide.

Said amidation reactions may conveniently be carried out by stirring the reactants in a suitable reaction-inert solvent, such as, for example, a halogenated hydrocarbon, e.g. dichloromethane, trichloromethane, an aromatic hydrocarbon, e.g. methylbenzene, an ether, e.g. 1,1'-oxybisethane, tetrahydrofuran or a dipolar aprotic solvent, e.g. N,N-dimethylformamide N,N-dimethylacetamide. The addition of a suitable base may be appropriate, in particular a tertiary amine such as, N,N-diethylethanamine. The water, the alcohol or the acid which is liberated during the course of the reaction may be removed from the reaction mixture according methodologies generally known in the art such as, for example, azeotropical distillation, complexation and salt formation.

Intermediates of formula (XXX) and (LXXII) can easily be prepared according to procedures described in U.S. Patent 4,859,684 corresponding to EP-A-2609744 and U.S. Serial No. 223,486 corresponding to EP-A-0,293,278 incorporated herein by reference for the process of preparing the intermediates of formula (XXX) and (XXXIV-α).

The intermediate hydrazines (XL) and amines (XLI) may conveniently be prepared from a ketone of formula (VII) by reaction with either an acid addition salt thereof, or with hydroxylamine or hydrazine or an acid addition salt or a solvate thereof, and reducing the thus obtained oxime or hydrazone, for example, by catalytic hydrogenation in the presence of hydrogen and an appropriate hydrogenation catalyst, e.g. Raney nickel.

The intermediates of formula (XXXVIII) can be prepared from an amine of formula (XLI) by reaction with a reagent of formula (LXXIII) and optionally S-alkylating the thus obtained thiourea with a $C_{1-6}$alkylhalide.

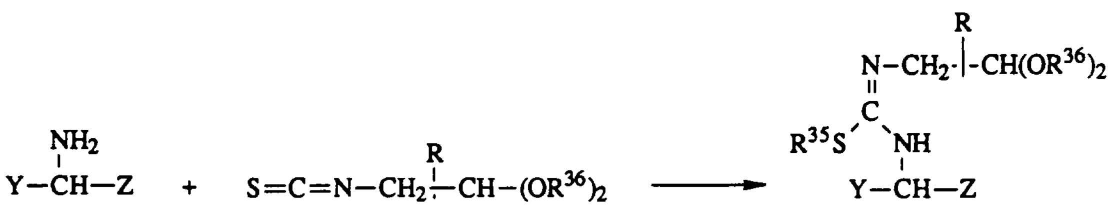

(XLI) (LXXIII) (XXXVIII)

The compounds of formula (I) and some of the intermediates in this invention have an asymmetric carbon atom in their structure. This chiral center may be present in a R- and a S-configuration, this R- and S-notation being in correspondence with the rules described in Pure Appl. Chem., 1976, 45, 11-30.

Pure stereochemically isomeric forms of the compounds of this invention may be obtained by the application of art-known procedures. Diastereoisomers may be separated by physical separation methods such as selective crystallization and chromatographic techniques, e.g. counter current distribution, and enantiomers may be separated from each other by the selective crystallization of their diastereomeric salts with optically active acids.

Pure stereochemically isomeric forms may also be derived from the corresponding pure stereochemically isomeric forms of the appropriate staring materials, provided that the reaction occurs stereospecifically.

The compounds of the present invention, their pharmaceutically acceptable acid addition salts and their possible stereochemically isomeric forms have useful pharmacological properties. For example, they suppress the plasma elimination of retinoids, such as, all-trans-retinoic acid, 13-cis retinoic acid and their derivatives. The latter results in more sustained/higher tissue concentrations of retinoic acid and improved control of the differentiation and growth of various cell types. In addition some compounds inhibit the formation of androgens from progestines and/or inhibit the action of the enzyme complex aromatase which catalyses the formation of estrogens from androgenic steroids in mammals. A number of compounds also show an inhibitory action on the biosynthesis of thromboxane $A_2$.

Said property of the compounds of the invention to delay the metabolism of retinoic acid can easily be evidenced in various in *vivo* experiments. A particular test procedure is described hereinafter as the "Metabolism of endogenous or exogenously administered all-trans-retinoic acid" test and demonstrates the suppression of the plasma elimination of endogenous or exogenously administered all-trans-retinoic acid. As such, the compounds of formula (I) can be used to control the rate of growth and differentiation of various cell types which effects are known to be affected by retinoids. The ability of retinoids, such as, 13-cis-retinoic acid, all-trans-retinoic acid and their derivatives to modulate differentiation and proliferation in several cell types whether they are of epithelial or mesenchymal origin is extensively studied and reviewed in J. Clin. Chem. Clin, Biochem., 26, 479-488 (1983); Pharmacological Reviews 36, 935-1005, (1984), Arch. Dermatol. 117, 160-180; (1981) and Journal of Medicinal Chemistry 25, 1269-1277, (1982).

In view of their capability to delay the metabolism of retinoic acid the compounds can thus be used in the treatment of disorders which are characterized by an increased proliferation and/or abnormal differentiation of epithelial cells. In particular the compounds of the invention can be used for treatment of carcinoma which is essentially a derailment of cellular differentiation, occurring in epithelial tissues. Other uses include, in addition to cancer treatment, the treatment of a variety of disorders of keratinization such as, for example, acne, psoriasis, lamellar ichthyosis, plantar warts, callosites, acanthosis nigricans, lechen planus, molluscum, melasma, corneal epithelial abrasion, geograpic tongue, Fox-Fordyce disease, cuteneous mestatic melanoma and heloids, epidermolytic hyperkeratosis, Darier's disease, pityriasis rubra pilaris, congenital ichthyosiform erythroderma, hyperkeratosis palmaris et plantaris, and similar diseases.

The anti-tumor activity may be demonstrated in several retinoic acid-sensitive and insensitive cell lines and solid tumors such as, for example, in Ta3-Ha induced mamma tumors in female mice.

The inhibition of androgen and/or estrogen formation can be demonstrated by analyzing the effects of the compounds of the invention on the conversion of progestins into androgens in the presence of testicular microsomes or on the conversion of androstenedione into estrone and estradiol in the presence of human placental microsomes. The in *vivo*-inhibition of androgen or estrogen formation can, for example, be demonstrated by measuring the suppression of the plasma testosterone or estrogen concentration in dogs, rats or mice. A number of relevant tests have been described in EP-A-260,744 and EP-A-293,978, both incorporated herein by reference.

In view of their capability to inhibit the biosynthesis of estrogens and/or androgens the compounds can be used in the treatment of estrogen or androgen dependent disorders such as, for example, breast cancer, endometriosis, endometrial cancer, polycystic ovarian disease, benign breast disease, prostatic cancer and hirsutism.

The beneficial effect of androgen inhibitors in these disorders, especially in the treatment of prostatic cancer, is described in, e.g., Journal of Urology 132, 61-63 (1984). The beneficial effect of aromatase inhibitors in these disorders, especially in the treatment of breast cancer, is described in, e.g. Cancer Research, 42, Suppl. 8 : 3261s (1982).

In view of the usefulness of the subject compounds it is evident that the present invention provides a method for treating mammals suffering from disorders which are characterized by an increased proliferation and/or abnormal differentiation of normal, preneoplastic or neoplastic cells, whether they are epithelial or mesenchymal; whether they are of ectodermal, endodermal or mesodermal origin; or whether they are estrogen dependent, androgen dependent or nonestrogen and nonandrogen dependent. Said method comprises the systemic or topical administration to the latter of an amount, effective to treat said disorders, of a compound of formula (I), a pharmaceutically acceptable acid-addition salt, or a possible stereochemically isomeric form thereof. In particular the present invention provides a method in which the growth and differentiation in said normal, preneoplastic and neoplastic cells is sensitive to the actions of retinoids.

Those of skill in treating disorders which are characterized by an excessive proliferation and/or abnormal differentiation of tissues could determine the effective amount from the test results presented hereinafter. In general it is contemplated than an effective amount would be from 0.001 mg/kg to 50 mg/kg body weight and more preferably from 0.01 mg/kg to 10 mg/kg body weight.

The subject compounds may be formulated into various pharmaceutical forms for administration purposes. As appropriate compositions there may be cited all compositions usually employed for systemically or topically administering drugs. To prepare the pharmaceutical compositions of this invention, an effective amount of the particular compound, optionally in acid-addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols in the case of oral liquid preparations such as suspensions, syrups, elixirs and solutions; or solid carriers such as starches, sugars, kaolin, lubricants, binders, disintegrating agents in the case of powders, pills, capsules, and tablets. Because of their ease in administration, tablets and capsules represents the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents may be employed. Also included are solid form preparations which are intended to be

converted, shortly before use, to liquid form preparations. In the compositons suitable for percutaneous administration, the carrier optionally comprises a penetration enhancing agent and/or a suitable wetting agent, optionally combined with suitable additives of any nature in minor proportions, which additives do not introduce a significant deleterious effect on the skin.

As appropriate compositions for topical application there may be cited all compositions usually employed for topically administering drugs, e.g., creams, gellies, dressings, shampoos, tinctures, pastes, ointments, salves, powders. Application of said compositions may be by aerosol e.g. with a propellent such as nitrogen carbon dioxide, a freon, or without a propellent such as a pump spray, drops, lotions, or a semisolid such as a thickened composition which can be applied by a swab. In particular compositions, semisold compositions such as salves, creams, gellies, ointments will conveniently be used.

It is especially advantageous to formulate the aforementioned pharmaceutical compositions in dosage unit form for ease of administration and uniformity of dosage. Dosage unit form as used in the specification and claims herein refers to physically discreate units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powders packets, wafers, injectable solutions or suspensions, teaspoonfuls, tablespoonfuls, and segregated multiples thereof.

Other such compositions are preparations of the cosmetic type, such as toilet waters, packs, lotions, skin milks or milky lotions. Said preparations contain, besides the active ingredient, components usually employed in such preparations. Examples of such components are oils, fats, waxes, surfactants, humectants, thickening agents, antioxidants, viscosity stabilizers, chelating agents, buffers, preservatives, perfumes, dyestuffs, lower alkanols. If desired, further ingredients may be incorporated in the compositions, e.g. antiinflammatory agents, antibacterials, antifungals, disinfectants, vitamins, sunscreens, antibiotics, or other anti-acne agents.

Examples of oils comprise fats and oils such as olive oil and hydrogenated oils; waxes such as beeswax and lanolin; hydrocarbons such as liquid paraffin, ceresin, and squalane; fatty acids such as stearic acid and oleic acid; alcohols such as cetyl alcohol, stearyl alcohol, lanolin alcohol, and hexadecanol; and esters such as isopropyl myristate, isopropyl palmitate and butyl stearate. As examples of surfactants there may be cited anionic surfactants such as sodium stearate, sodium cetylsulfate, polyoxyethylene lauryl-ether phosphate, sodium N-acyl glutamate; cationic surfactants such as stearyldimethyl-benzylammonium chloride and stearyltrimethylammonium chloride; ampholytic surfac-tants such as alkylaminoethylglycine hydrochloride solutions and lecithin; and nonionic surfactants such as glycerin monostearate, sorbitan monostearate, sucrose fatty acid esters, propylene glycol monostearate, polyoxyethylene oleylether, polyethylene glycol monostearate, polyoxyethylene sorbitan monopalmitate, polyoxyethylene coconut fatty acid monoethanolamide, pelyoxyethylene polyoxypropylene glycol (e.g. the materials sold under the trademark "Pluronic"), polyoxyethylene castor oil, and polyoxyethylene lanolin. Examples of humectants include glycerin, 1,3-butylene glycol, and propylene glycol; examples of lower alcohols include ethanol and isopropanol; examples of thickening agents include xanthan gum, hydroxypropyl cellulose, hydroxypropyl methyl cellulose, polyethylene glycol and sodium carboxymethyl cellulose; examples of antioxidants comprise butylated hydroxytoluene, butylated hydroxyanisole, propyl gallate, citric acid and ethoxyquin; examples of chelating agents include disodium edetate and ethanehydroxy diphosphate; examples of buffers comprise citric acid, sodium citrate, boric acid, borax, and disodium hydrogen phosphate; and examples of preservatives are methyl parahydroxybenzoate, ethyl parahydroxybenzoate, dehydroacetic acid, salicylic acid and benzoic acid.

For preparing ointments, creams, toilet waters, skin milks, typically from 0.01 to 10% in particular from 0.1 to 5% and more in particular from 0.2 to 2.5% of the active ingredient will be incorporated in said compositions. In ointments or creams, the carrier for example consists of 1 to 20%, in particular 5 to 15% of a humectant, 0.1 to 10% in particular from 0.5 to 5% of a thickener and water; or said carrier may consist of 70 to 99%, in particular 20 to 95% of a surfactant, and 0 to 20%, in particular 2.5 to 15% of a fat; or 80 to 99.9% in particular 90 to 99% of a thickener; or 5 to 15% of a surfactant, 2-15% of a humectant, 0 to 80% of an oil, very small (<2%) amounts of preservative, colouring agent and/or perfume, and water. In a toilet water, the carrier for example consists of 2 to 10% of a lower alcohol, 0.1 to 10% or in particular 0.5 to 1% of a surfactant, 1 to 20%, in particular 3 to 7% of a humectant, 0 to 5% of a buffer, water and small amounts (<2%) of preservative, dyestuff and/or perfume. In a skin milk, the carrier typically consists of 10-50% of oil, 1 to 10% of surfactant, 50-80% of water and 0 to 3% of preservative and/or perfume. In the afore-mentioned preparations, all % symbols refer to weight by weight percentage. The humectant, surfactant, oil, etc... referred to in said preparations may be any such component used in the cosmetic arts but preferably will be one or more of the components mentioned hereinabove.

Further, when in the above compositions one or more of the components make up the major part of the composition, the other ingredients can evidently be not present at their indicated maximum concentration and therefore will make up the remainder of the composition.

Particular compositions for use in the method of the present invention are those wherein the active ingredient is formulated in liposome-containing compositions. Liposomes are artificial vesicles formed by amphiphatic molecules such as polar lipids, for example, phosphatidyl cholines, ethanolamines and serines, sphingomyelins, cardiolipins, plasmalogens, phosphatidic acids and cerebiosides. Liposomes are formed when suitable amphiphathic molecules are allowed to swell in water or aqueous solutions to form liquid crystals usually of multilayer structure comprised of many bilayers separated from each other by aqueous material (also referred to as coarse liposomes). Another type of liposome known to be consisting of a single bilayer encapsulating aqueous material is referred to as a unilamellar vesicle. If water-soluble materials are included in the aqueous phase during the swelling of the lipids they become entrapped in the aqueous layer between the lipid bilayers.

In a further aspect of the invention there are provided particular pharmaceutical or cosmetical compositions which comprise an inert carrier, an effective amount of a compound of formula (I) an acid addition salt or a stereochemically isomeric form thereof and an effective amount of a retinoic acid, a derivative thereof or a stereochemically isomeric form thereof. Said retinoic acid containing compositions are particularly useful for treating acne or for retarding the effects of aging of the skin and generally improve the quality of the skin, particularly human facial skin. A pharmaceutical or cosmetical composition containing retinoic acid or a derivative thereof as the active ingredient in intimate admixture with a dermatologically acceptable carrier can be prepared according to conventional compounding techniques, such as those known for topical application of retinoic acid and its derivatives. Conventional pharmaceutical compounding techniques for topical application of retinoic acid are described for example in, U.S. Pat. Nos. 3,906,108 and 4,247,547, which are incorporated herein by reference. Preferred composition for topical application are in form of a cream, ointment or lotion comprising from 0.005 to 0.5% (particularly from 0.01 to 0.1%) all-trans-retinoic acid, 13-cis-retinoic acid or a derivative thereof and from 0.1 to 5% of a compound of formula (I) and, a dermatologically acceptable acid addition salt thereof or a stereochemically isomeric form thereof, in a semi-solid or liquid diluent or carrier.

These preferred compositions should preferably be non-irritating and as far as possible they should be odorless and non-toxic. For convenience in applying to the skin, the composition usually contain, besides water or an organic solvent, several of certain organic emollients, emulsifiers for the aqueous and/or non aqueous phases of the compositions, wetting agents preservatives and agents that facilitate the penetration and remainence of the active agents in the skin.

The following examples are intended to illustrate the scope of the present invention. Unless otherwise stated all parts therein are by weight.

Experimental part

A-a) Preparation of the intermediates in the synthesis of quinoline derivatives of formula (I-a)

Example 1-a

α) A mixture of 8.6 parts of 7-quinolinemethanol, 20 parts of manganese(IV)oxide and 130 parts of dichloromethane was stirred for 24 hours at room temperature. The reaction mixture was filtered over diatomaceous earth and the filtrate was evaporated. The residue was purified by column chromatography (silica gel ; $CH_2Cl_2/CH_3OH$ 98:2). The eluent of the desired fraction was evaporated, yielding 8 parts (94.2%) of 7-quinolinecarboxaldehyde; mp. 56 °C (interm. 1-a).

β) To a stirred mixture of 1.25 parts of magnesium, 14 parts of 1,1'-oxybisethane and 8 parts of bromobenzene was added a solution of 8 parts of intermediate 1-a, namely 7-quinolinecarboxaldehyde, in 72 parts of tetrahydrofuran, keeping the temperature between 0 °C and 5 °C. After stirring for 12 hours at room temperature, the reaction mixture was poured into 300 parts of ice-water. The product was extracted with 1,1'-oxybisethane (3x70 parts). The combined extracts were dried, filtered and evaporated. The residue was purified by column chromatography (silica gel; $CH_2Cl_2/CH_3OH$ 98:2). The eluent of the desired fraction was evaporated, yielding 3.2 parts (26.6%) of α-phenyl-7-quinolinemethanol; mp. 118 °C (interm. 2-a).

In a similar manner there were also prepared the intermediates listed in Table 1-a.

Table 1-a

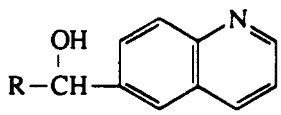

| Int. No. | R | physical data (mp. in °C) |
|---|---|---|
| 3-a | $C_6H_5$ | 98 |
| 4-a | 3-$ClC_6H_4$ | 112 |
| 5-a | 3-$FC_6H_4$ | 94 |
| 6-a | 4-$ClC_6H_4$ | 148 |
| 7-a | 3-$CH_3C_6H_4$ | 122 |
| 8-a | 3-$CH_3OC_6H_4$ | 142 |
| 9-a | 3,4-di(F)$C_6H_3$ | - |
| 10-a | 3,4-di($CH_3$)$C_6H_3$ | 114 |
| 11-a | 3-$CF_3C_6H_4$ | - |
| 12-a | 4-$FC_6H_4$ | 128 |
| 13-a | 4-$CH_3OC_6H_4$ | 164 |
| 14-a | 4-$CH_3C_6H_4$ | 135 |
| 15-a | c.$C_6H_{11}$ | 118 |

Example 2-a

α) A mixture of 34 parts of 6-quinolinemethanol, 70 parts of manganese(IV)oxide and 300 parts of trichloromethane was stirred for 24 hours at room temperature. The reaction mixture was filtered over diatomaceous earth and the filtrate was evaporated, yielding 27.7 parts (82.7%) of 6-quinolinecarboxaldehyde; mp. 72 °C (interm. 16-a).

β) To a stirred and cooled (-5/0 °C) solution of 5.4 parts of thiophene in 21.3 parts of 1,1'-oxybisethane were added portionwise 43.5 parts of a solution of n. butyllithium in hexanes 1.6M. After stirring for 20 min. at 0 °C, there was added a solution of 5 parts of intermediate 16-a, namely 6-quinolinecarboxaldehyde, in 71.2 parts of tetrahydrofuran. Stirring at 0 °C was continued for 1 hour and then the reaction mixture was poured into 200 parts of ice-water. The product was extracted with 1,1'-oxybisethane and the extract was dried, filtered and evaporated. The residue was purified by column chromatography (silica gel ; $CH_2Cl_2$/$CH_3OH$ 95:5). The eluent of the desired fraction was evaporated, yielding 2.4 parts (31.1%) of α-(2-thienyl)-6-quinolinemethanol (interm. 17-a).

Example 3-a

α) To a stirred amount of 45.3 parts of aluminiumtrichloride were added dropwise 6.9 parts of N,N-dimethylformamide. After stirring for 5 min. at 70 °C, there were added portionwise 5 parts of 3,4-dihydroquinolin-2(1H)-one and, after another 5 min., 4.7 parts of benzoylchloride. Stirring at 70 °C was continued for 2 hours and then the reaction mixture was carefully poured into ice-water. There were added 50 ml of HCl 12N and the whole was stirred for 15 min. The precipitate was filtered off and boiled in 2-propanol. The product was filtered off, washed with 2-propanol and 2,2'-oxybispropane and dried in vacuo at 60 °C, yielding 6.3 parts (73.8%) of 6-benzoyl-3,4-dihydro-2(1H)-quinolinone; mp. 211.0 °C (interm. 18-a).

β) To a suspension of 27.3 parts of intermediate 18-a, namely 6-benzoyl-3,4-dihydro-2(1H)-quinolinone, in 790 parts of methanol were added 115 parts of an aqueous sodium hydroxide solution 1N. After stirring for 10 min., there were added at once 4.54 parts of sodium tetrahydroborate. Stirring was continued over weekend at room temperature. There were added 110 ml of HCl 1N and 1000 parts of water. The precipitate was filtered off, stirred in water for 15 min and then taken up in a mixture of methanol and methylbenzene. This solution was evaporated and the residue was co-evaporated with methylbenzene. The product was filtered off and dried at 70 °C, yielding 21.9 parts (78.6%) of 3,4-dihydro-6-(hydroxyphenylmethyl)-2(1H)-quinolinone; mp. 175.0 °C (interm. 19-a).

In a similar manner there were also prepared:

6-[(3-chlorophenyl)hydroxymethyl]-3,4-dihydro-2(1H)-quinolinone; mp. 181.1 °C (interm. 20-a);
3,4-dihydro-6-(1-hydroxyethyl)-2(1H)-quinolinone; mp. 174.5 °C (interm. 21-a); and
3,4-dihydro-6-[hydroxy(isopropyl)methyl]-2(1H)-quinolinone; mp. 194.4 °C (interm. 22-a).

Example 4-a

A mixture of 3.2 parts of intermediate 2, namely α-phenyl-7-quinolinemethanol, 8 parts of thionyl chloride and 65 parts of dichloromethane was stirred for 4 hours at room temperature. The reaction mixture was evaporated and the residue was poured into water. The product was extracted with dichloromethane (3x39 parts) and the combined extracts were dried, filtered and evaporated, yielding 3.4 parts (98.5%) of 7-(chlorophenylmethyl)quinoline (interm. 23-a).

In a similar manner there were also prepared the intermediates listed in Table 2-a.

Table 2-a

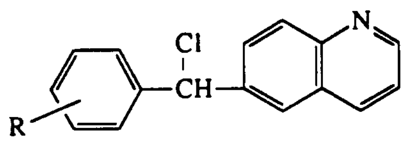

| Int. No. | R |
|---|---|
| 24-a | 3-Cl |
| 25-a | H |
| 26-a | 3-F |
| 27-a | 4-Cl |
| 28-a | $3-CH_3$ |
| 29-a | $3-CH_3O$ |

| Int. No. | R |
|---|---|
| 30-a | 3,4-di(F) |
| 31-a | 3,4-di($CH_3$ |
| 32-a | 3-$CF_3$ |
| 33-a | 4-$CH_3$ |
| 34-a | 4-F |
| 35-a | 4-$CH_3O$ |

Example 5-a

To a stirred mixture of 2 parts of intermediate 21-a, namely 3,4-dihydro-6-(1-hydroxyethyl)-2(1H)-quinolinone in 8.9 parts of tetrahydrofuran were added 1.62 parts of thionyl chloride. Stirring at room temperature was continued overnight. The reaction mixture was evaporated and the residue was co-evaporated with methylbenzene, yielding 2.3 parts (93.4%) of 6-(1-chloroethyl)-3,4-dihydro-2(1H)-quinolinone hydrochloride (interm. 36-a).

Example 6-a

A mixture of 20 parts of intermediate 19-a, namely 3,4-dihydro-6-(hydroxyphenylmethyl)-2(1H)-quinolinone and 355 parts of a solution of hydrobromic acid in acetic acid 30% was stirred overnight at room temperature. The reaction mixture was evaporated and the residue was stirred in ethyl acetate. The product was filtered off, washed with ethyl acetate and 2,2'-oxybispropane and dried in vacuo at 35 ° C, yielding 23 parts (67.2%) of 6-[bromophenylmethyl]-3,4-dihydro-2(1H)-quinolinone hydrobromide dihydrate; mp. 119.5 ° C (interm. 37-a).
In a similar manner there were also prepared:
6-[bromo(3-chlorophenyl)methyl]-3,4-dihydro-2(1H)-quinolinone hydrobromide (interm. 38-a); and
6-[bromocyclohexylmethyl]quinoline (interm. 39-a).

Example 7-a

α) To a stirred and cooled (0 ° C) amount of 55.2 parts of sulfuric acid were added portionwise 13 parts of 1-(2-methyl-1-phenylpropyl)-1H-imidazole mononitrate. After stirring for 1/2 hour at 0 ° C, the reaction mixture was poured into ice-water. The whole was basified with ammonia and extracted with dichloromethane. The extract was dried, filtered and evaporated, yielding 12 parts (97.8%) of 1-[2-methyl-1-(4-nitrophenyl)propyl]-1H-imidazole (interm. 40-a).

β) A mixture of 12 parts of intermediate 40-a, namely 1-[2-methyl-1-(4-nitrophenyl)propyl]-1H-imidazole, and 79 parts of methanol was hydrogenated for 1 hour at room temperature and $2.10^5$ Pa with 3 parts of Raney nickel. The catalyst was filtered off and the filtrate was evaporated, yielding 12 parts (100%) of 4-[1-(1H-imidazol-1-yl)-2-methylpropyl]benzenamine (interm. 41-a).

Example 8-a

α) To a stirred solution of 88.7 parts of 1-[chlorophenylmethyl]-4-nitrobenzene in 790 parts of acetonitrile were added 121.8 parts of 1H-imidazole. After stirring for 24 hours at reflux temperature, the reaction mixture was evaporated. The residue was taken up in methylbenzene. This solution was washed with $K_2CO_3$ (aq.), dried, filtered and evaporated. The residue was purified by column chromatography (silica gel; $CH_2Cl_2/CH_3OH$ 98:2). The eluent of the desired fraction was evaporated, yielding 53 parts (53%) of 1-[(4-nitrophenyl)phenylmethyl]-1H-imidazole (interm. 42-a).

β) A solution of 39 parts of intermediate 42-a, namely 1-[(4-nitrophenyl)phenylmethyl]-1H-imidazole, in 240 parts of ethanol was hydrogenated at $3.10^5$ Pa and at room temperature with 20 parts of Raney nickel. After the calculated amount of hydrogen was taken up, the catalyst was filtered off and the filtrate was evaporated, yielding 34.6 parts (99.1%) of 4-[(1H-imidazol-1-yl)phenylmethyl]-benzenamine (interm. 43-a).

In a similar manner there were also prepared:

4-[(4-fluorophenyl)(1H)-imidazol-1-yl)methyl]benzenamine (interm. 44a); and

4-[(4-chlorophenyl)(1H)-imidazol-1-yl)methyl]benzenamine (interm. 45-a).

Example 9-a

α) To a stirred and cooled (0 °C) mixture of 5 parts of N-[4-[(3-chlorophenyl)hydroxymethyl]phenyl]-acetamide, 66.5 parts of dichloromethane and 5.5 parts of N,N-diethylethanamine was added a solution of 3.1 parts of methanesulfonyl chloride in 13.3 parts of dichloromethane under a nitrogen atmosphere. After stirring for 1 hour, the reaction mixture was evaporated, yielding 8 parts (100%) of 4-(acetylamino)-α-(3-chlorophenyl)benzenemethanol methanesulfonate(ester) (interm. 46-a).

β) A mixture of 8 parts of intermediate 46-a, namely 4-(acetylamino)-α-(3-chlorophenyl) benzenemethanol methanesulfonate(ester), 10 parts of 1H-imidazole and 39.5 parts of acetonitrile was stirred for 2 hours at reflux temperature. The reaction mixture was evaporated and the residue was extracted with ethyl acetate. The extract was washed with $NaHCO_3$ (aq.), dried, filtered and evaporated, yielding 18 parts (100%) of N-[4-[(3-chlorophenyl)(1H-imidazol-1-yl)methyl]phenyl]acetamide (interm. 47-a).

γ) A mixture of 80 parts of intermediate 47-a, namely N-[4-[(3-chlorophenyl) (1H-imidazol-1-yl)methyl]-phenyl]acetamide, 150 ml of an aqueous hydrochloric acid solution 2N and 15.8 parts of methanol was stirred for 2 hours at reflux temperature. The reaction mixture was evaporated and the residue was basified. The product was extracted with dichloromethane and the extract was dried, filtered and evaporated. The residue was purified by column chromatography (silica gel ; $CH_2Cl_2/CH_3OH$ 98:2). The eluent of the desired fraction was evaporated, yielding 14.1 parts (20.2%) of 4-[(3-chlorophenyl) (1H-imidazol-1-yl)methyl]benzenamine (interm. 48-a).

In a similar manner there was also prepared:

4-[(3-fluorophenyl)(1H-imidazol-1-yl)methyl]benzenamine (interm. 49-a).

Example 10-a

To a stirred and cooled (0 °C) mixture of 14.6 parts of 4-[(4-chlorophenyl)(1H-imidazol-1-yl)methyl]-benzenamine, 60.9 parts of benzene and 6.86 parts of pyridine was added a solution of 10.6 parts of 3-phenyl-2-propenoyl chloride in 17.4 parts of benzene under a nitrogen atmosphere. After stirring overnight at room temperature, the reaction mixture was basified and extracted with ethyl acetate. The extract was washed with water, dried, filtered and evaporated. The residue was crystallized from dichloromethane. The product was filtered off and dried, yielding 17.7 parts (83.8%) of N-[4-[(4-chlorophenyl) (1H-imidazol-1-yl)-methyl]phenyl]-3-phenyl-2-propenamide; mp. 244 °C(interm. 50-a).

In a similar manner there were also prepared the intermediates listed in Table 3-a :

Table 3-a

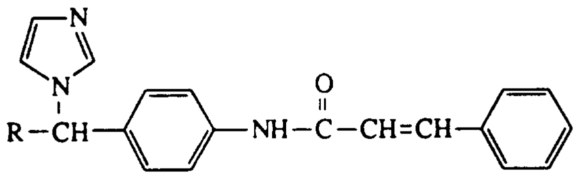

| Int. No. | R | physical data (mp. in °C) |
|---|---|---|
| 51-a | $C_6H_5$ | - |
| 52-a | $i.C_3H_7$ | 217 |
| 53-a | $3\text{-}ClC_6H_4$ | - |
| 54-a | $3\text{-}FC_6H_4$ | - |
| 55-a | $4\text{-}FC_6H_4$ | - |
| 56-a | H | 195 |

Example 11-a

To a stirred solution of 10 parts of 4-[(1H-imidazol-1-yl)methyl]benzenamine in 180 parts of 1,2-dichloroethane were added dropwise 3.9 parts of 4-methylene-2-oxetanone. After stirring for 1/2 hour at room temperature, the precipitate was filtered off, washed with 1,2-dichioroethane and dried, yielding 9.8 parts (74.8%) of N-[4-(1H-imidazol-1-ylmethyl)phenyl]-3-oxobutanamide; mp. 175 ° C (interm. 57-a).
In a similar manner there were also prepared the intermediates listed in Table 4a :

Table 4-a

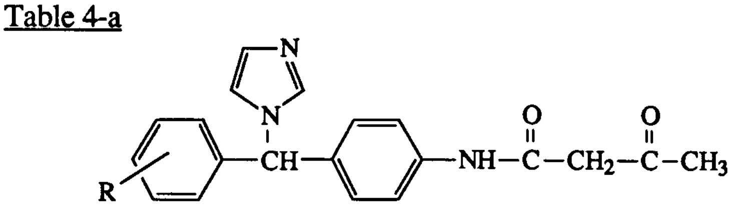

| Int. No. | R |
|---|---|
| 58-a | H |
| 59-a | 4-F |
| 60-a | 4-Cl |
| 61-a | 3-Cl |
| 62-a | 3-F |

B-a) Preparation of the final quinoline and quinolinone compounds of formula (I-a)

Example 12-a

A mixture of 3.4 parts of 7-[chlorophenylmethyl]quinoline, 4.5 parts of 1H-imidazole and 72 parts of N,N-dimethylformamide was stirred for 6 hours at 80 ° C. The reaction mixture was evaporated to dry and the residue was taken up in water. The product was extracted three times with 65 parts of dichloromethane. The combined extracts were dried, filtered and evaporated. The residue was purified by column chromatograhpy over silica gel using a mixture of dichloromethane and methanol (95:5 by volume) as eluent. The pure fractions were collected and the eluent was evaporated. The residue was crystallized from a mixture of 2,2'-oxybispropane and 2-propanone. The product was filtered off and dried, yielding 1.27 parts (33.2%) of 7-[-(1H-imidazol-1yl)phenylmethyl]quinoline; mp. 110.7 ° C (compound 36-a).

Example 13-a

A mixture of 12.3 parts of 6-(chloromethyl)quinoline, 9.5 parts of 1H-imidazole, 19.2 parts of potassium carbonate and 135 parts of N,N-dimethylformamide was stirred for 3 hours at 80 ° C. After evaporation to dry, the residue was taken up in water and further purified according to similar procedures as described in example 12-a, yielding 10 parts (48%) of 6-(1H-imidazol-1-ylmethyl)quinoline dihydrochloride; mp. 254.6 ° C (compound 29-a).

Example 14-a

A mixture of 5.34 parts of 6-[chloro(4-chlorophenyl)methyl]quinoline, 6.4 parts of 1H-1,2,4-triazole, 1.26 parts of potassium carbonate and 79 parts of acetonitrile was stirred for 8 hours at reflux temperature. After evaporation to dry, the residue was taken up in water and was further purified according to similar procedures as described in example 12-a, yielding 3 parts (49.2%) of 6-[(4-chlorophenyl)(4H-1,2,4-triazol-4-yl)methyl]quinoline hemihydrate; mp. 87.8 ° C (compound 45-a).

Example 15-a

A mixture of 2.3 parts of 6-(1-chloroethyl)-3,4-dihydro-2(1H)-quinolinone hydrochloride, 24 parts of acetonitrile, 7.7 parts of dimethyl sulfoxide and 3.8 parts of 1H-imidazole was stirred overnight at 60-70 ° C. The reaction mixture was poured into water, extracted and further purified according similar procedures as described in example 12-a, yielding 1.2 parts (53.5%) of 3,4-dihydro-6-[1-(1H-imidazol-1-yl)ethyl]-2(1H)-quinolinone; mp. 184.8 ° C (compound 21-a).

Example 16-a

A mixture of 15 parts of α-phenyl-6-quinolinemethanol, 21 parts of 1,1'-carbonylbis[1H-imidazole] and 135 parts of N,N-dimethylformamide was stirred for 12 hours at room temperature. After evaporation to dry, the residue was stirred for 20 minutes at room temperature in a mixture of 140 parts of 1,1'-oxybisethane and 200 parts of water. The mixture was filtered and the filtrate was extracted with trichloromethane and water. The separated organic phase was dried, filtered and evaporated. The residue was purified by column over silica gel, first using a mixture of dichloromethane and methanol (98:2 by volume) and then a mixture of ethyl acetate and cyclohexane (70:30 by volume) as eluents. The pure fractions were collected and the eluent was evaporated. The residue was converted into the sulfate salt in 8 parts of 2-propanone and ethanol at 0 ° C. The salt was filtered off and crystallized from a mixture of 2-propanol and methanol. The product was filtered off and dried, yielding 1.33 parts (5.1%) of 6-[(1H-imidazol-1-yl)phenyl-methyl]quinoline sulfate(1:1),monohydrate; mp. 135 ° C (compound 33-a).

Example 17-a

7 Parts of N-[4-(1H-imidazol-1-ylmethyl)phenyl]-3-oxobutanamide were added dropwise to 73.6 parts of concentrated sulfuric acid (exothermic reaction, the temperature rose to 90 ° C). Upon complete addition, the mixture was stirred for 1 hour at 70 ° C. The reaction mixture was poured into crushed ice and the whole was neutralized with an ammonium hydroxide solution to pH 9. The precipitated product was filtered off and taken up in water. The whole was extracted with dichloromethane. The aqueous layer was concentrated.

The crystallized product was filtered off, washed with 2-propanone and dried in vacuo at 100 °C, yielding 2.25 parts (34.8%) of 6-(1H-imidazol-1-ylmethyl)-4-methyl-2(1H)-quinolinone; mp. 266.0 °C (compound 1-a).

Example 18-a

To a stirred and heated (100 °C) solution of 10 parts of 4-(1H-imidazol-1-ylmethyl)benzenamine in 50 parts of poly phosphoric acid were added 15 parts of ethyl 3-oxobutanoate. The whole was stirred for 4 hours at 140 °C. 100 Parts of water were added to the mixture and the whole was neutralized with potassium carbonate. The product was extracted with a mixture of ethyl acetate and methanol. The extract was dried, filtered and concentrated. The concentrate was crystallized from a mixture of 2-propanone and methanol. The product was filtered off and dried, yielding 2 parts (14.4%) of 6-(1H-imidazol-1-ylmethyl)-2-methyl-4(1H)quinolinone; mp. 245.5 °C (decomp.) (compound 77-a).

Example 19-a

To a stirred solution of 10.5 parts of N-[4-[(1H-imidazol-1-yl)phenylmethyl]phenyl]-3-phenyl-2-propenamide in 110 parts of chlorobenzene were added 18.5 parts of aluminium chloride. The reaction mixture was stirred for 3 hours at 120 °C. After cooling to room temperature, the product was extracted with ethyl acetate. The extract was dried, filtered and evaporated. The residue was purified by column chromatography over silica gel using a mixture of dichloromethane and methanol (90:10 by volume) as eluent. The pure fractions were collected and the eluent was evaporated. The residue was crystallized from a mixture of 2-propanone and methanol. The product was filtered off and dried, yielding 1.3 parts (15.4%) of 6-[(1H-imidazol-1-yl)phenylmethyl]-2(1H)-quinolinone; mp. 226.9 °C (compound 2-a).

Example 20-a

To a stirred solution of 2 parts of sodium in 24 parts of 1-propanol was added a solution of 5.2 parts of 2-chloro-6-(1H-imidazol-1-ylmethyl)-4-methylquinoline in 16 parts of 1-propanol at room temperature under nitrogen atmosphere. After stirring for 2 hours at reflux temperature, the mixture was evaporated. The residue was taken up in a potassium carbonate solution and the product was extracted with ethyl acetate. The extract was dried, filtered and evaporated. The residue was purified by column chromatograhpy over silica gel using a mixture of dichloromethane and methanol (98:2 by volume) as eluent. The pure fractions were collected and the eluent was evaporated. The residue was crystallized from 2-propanone. The product was filtered off and dried, yielding 1.8 parts (31.9%) of 6-(1H-imidazol-1-ylmethyl)-4-methyl-2-propoxyquinoline; mp. 137.9 °C (compound 35-a).

Example 21-a

A solution of 13 parts of 6-(1H-imidazol-1-ylmethyl)-4-methyl-2(1H)-quinolinone in 55 parts of phosphoryl chloride was stirred for 1 hour at room temperature. After evaporation, the residue was purified by column chromatography over silica gel using a mixture of trichloromethane and methanol (95:5 by volume) as eluent. The pure fractions were collected and the eluent was evaporated. The residue was crystallized from a mixture of acetonitrile and 2,2'-oxybispropane. The product was filtered off and dried, yielding 1.75 parts (12.5%) of 2-chloro-6-(1H-imidazol-1-ylmethyl)-4-methylquinoline; mp. 120.6 °C (compound 34-a).

All the other compounds listed in tables 5-a to 8-a were obtained by analogous methods of preparation as described in examples 12-a to 21-a, the actual method of preparation being indicated in column 2 (Ex. No.).

Table 5-a

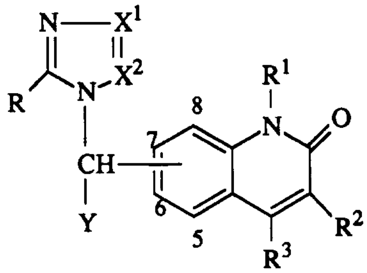

| Comp. No. | Ex. No. | R | $X^1=X^2$ | Y | p | $R^1$ | $R^2$ | $R^3$ | mp./salt |
|---|---|---|---|---|---|---|---|---|---|
| 1-a | 17-a | H | -CH=CH- | H- | 6 | H- | H- | $-CH_3$ | 266.0 |
| 2-a | 19-a | H | -CH=CH- | $C_6H_5$- | 6 | H- | H- | -H | 226.9 |
| 3-a | 17-a | H | -CH=CH- | $C_6H_5$- | 6 | H- | H- | $-CH_3$ | 209.3 |
| 4-a | 17-a | H | -CH=CH- | 4-F-$C_6H_4$- | 6 | H- | H- | $-CH_3$ | 215.6 |
| 5-a | 17-a | H | -CH=CH- | 4-Cl-$C_6H_4$- | 6 | H- | H- | $-CH_3$ | 137.6/0.5$H_2O$ |
| 6-a | 17-a | H | -CH=CH- | 3-Cl-$C_6H_4$- | 6 | H- | H- | $-CH_3$ | 164.3/0.5$H_2O$ |
| 7-a | 17-a | H | -CH=CH- | 3-F-$C_6H_4$- | 6 | H- | H- | $-CH_3$ | 192.9 |
| 8-a | 19-a | H | -CH=CH- | i-$C_3H_7$- | 6 | H- | H- | -H | 165.7 |
| 9-a | 19-a | H | -CH=CH- | 4-Cl-$C_6H_4$- | 6 | H- | H- | -H | 180.1 |
| 10-a | 19-a | H | -CH=CH- | 3-Cl-$C_6H_4$- | 6 | H- | H- | -H | 212.2 |
| 11-a | 19-a | H | -CH=CH- | 3-F-$C_6H_4$- | 6 | H- | H- | -H | 210.6 |
| 12-a | 19-a | H | -CH=CH- | 4-F-$C_6H_4$- | 6 | H- | H- | -H | 253.7 |
| 13-a | 14-a | H | -N=CH- | H- | 6 | H- | H- | -H | >300/$H_2O$ |
| 14-a | 19-a | H | -CH=CH- | H- | 6 | H- | H- | -H | 229.6 |

In the previous and following tables p indicates the position of the 1H-azol-1-ylmethyl moiety on the quinoline ring.

Table 6-a

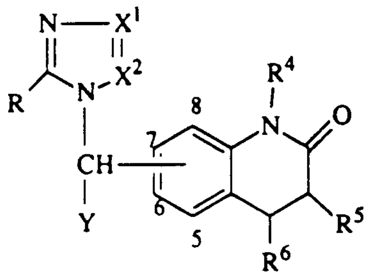

| Comp. No. | Ex. No. | R | $-X^1=X^2-$ | Y | p | $R^4$ | $R^5$ | $R^6$ | mp./salt |
|---|---|---|---|---|---|---|---|---|---|
| 15-a | 15-a | H | -CH=N- | $C_6H_5$- | 6 | H- | H- | H- | 220.1 |
| 16-a | 15-a | H | -CH=CH- | $C_6H_5$- | 6 | H- | H- | H- | 223.9 |
| 17-a | 15-a | H | -N=CH- | $C_6H_5$- | 6 | H- | H- | H- | 187.8 |
| 18-a | 15-a | H | -N=CH- | 3-Cl-$C_6H_4$- | 6 | H- | H- | H- | 170.6/$HNO_3$ |
| 19-a | 15-a | H | -CH=N- | 3-Cl-$C_6H_4$- | 6 | H- | H- | H- | 110.1/$HNO_3$ |

| Comp. No. | Ex. No. | R | $-X^1=X^2-$ | Y | p | $R^4$ | $R^5$ | $R^6$ | mp./salt |
|---|---|---|---|---|---|---|---|---|---|
| 20-a | 15-a | H | -CH=CH- | $3-Cl-C_6H_4-$ | 6 | H- | H- | H- | 189.5 |
| 21-a | 15-a | H | -CH=CH- | $-CH_3$ | 6 | H- | H- | H- | 184.8 |
| 22-a | 15-a | H | -CH=N- | $-CH_3$ | 6 | H- | H- | H- | 172.3 |
| 23-a | 15-a | H | -N=CH- | $-CH_3$ | 6 | H- | H- | H- | 220.3 |
| 24-a | 16-a | H | -CH=CH- | $c-C_3H_5-$ | 6 | H- | H- | H- | 168.7 |
| 25-a | - | H | -CH=CH- | $i.C_3H_7-$ | 6 | H- | H- | H- | - |
| 26-a | - | H | -N=CH- | $i.C_3H_7-$ | 6 | H- | H- | H- | - |
| 27-a | - | H | -CH=N- | $i.C_3H_7-$ | 6 | H- | H- | H- | - |
| 28-a | - | H | -CH=CH- | $3-Cl-C_6H_4-$ | 6 | H- | $CH_3-$ | $CH_3-$ | - |

Table 7-a

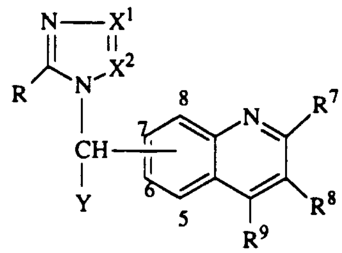

| Comp. No. | Ex. No. | R | $-X^1=X^2-$ | Y | p | $R^7$ | $R^8$ | $R^9$ | mp./salt |
|---|---|---|---|---|---|---|---|---|---|
| 29-a | 13-a | H | -CH=CH- | -H | 6 | H | H | H | 254.6/2 HCl |
| 30-a | 13-a | H | -CH=CH- | -H | 8 | H | H | H | $167.8/(COOH)_2$ |
| 31-a | 13-a | H | -CH=CH- | -H | 7 | H | H | H | $163.8/2(COOH)_2$ |
| 32-a | 13-a | H | -CH=CH- | -H | 5 | H | H | H | $216.4/0.5(COOH)_2$ |
| 33-a | 16-a | H | -CH=CH- | $C_6H_5-$ | 6 | H | H | H | $79.8/H_2SO_4/H_2O$ |
| 34-a | 21-a | H | -CH=CH- | -H | 6 | Cl- | H | $CH_3-$ | 120.6 |
| 35-a | 20-a | H | -CH=CH- | -H | 6 | $C_3H_7-O-$ | H | $CH_3-$ | 137.9 |
| 36-a | 12-a | H | -CH=CH- | $C_6H_5-$ | 7 | H | H | H | 110.7 |
| 37-a | 20-a | H | -CH=CH- | -H | 6 | $i-C_3H_7-O-$ | H | $CH_3-$ | 111.1 |
| 38-a | 20-a | H | -CH=CH- | -H | 6 | $CH_3-O-$ | H | $CH_3-$ | 142.6 |
| 39-a | 21-a | H | -CH=CH- | -H | 6 | $CH_3-$ | H | Cl- | 103.7 |
| 40-a | 20-a | H | -CH=CH- | -H | 6 | $CH_3-$ | H | $CH_3O-$ | 116.9 |
| 41-a | 14-a | H | -CH=CH- | $3-Cl-C_6H_4-$ | 6 | H | H | H | 120.7 |

| Comp. No. | Ex. No. | R | $-X^1=X^2-$ | Y | p | $R^7$ | $R^8$ | $R^9$ | mp./salt |
|---|---|---|---|---|---|---|---|---|---|
| 42-a | 14-a | H | -CH=CH- | 3-F-$C_6H_4$- | 6 | H | H | H | 98.9 |
| 43-a | 14-a | H | -N=CH- | $C_6H_5$- | 6 | H | H | H | 173.2 |
| 44-a | 14-a | H | -CH=N- | $C_6H_5$- | 6 | H | H | H | 115.0/ $H_2O$/HCl |
| 45-a | 14-a | H | -N=CH- | 4-Cl-$C_6H_4$- | 6 | H | H | H | 87.8/0.5$H_2O$ |
| 46-a | 14-a | H | -CH=N- | 3-Cl-$C_6H_4$- | 6 | H | H | H | 120.7 |
| 47-a | 14-a | H | -CH=CH- | 3-$CH_3$-$C_6H_4$- | 6 | H | H | H | 124.7 |
| 48-a | 14-a | H | -CH=N- | 4-Cl-$C_6H_4$- | 6 | H | H | H | 201.8/HCl/ 0.5$H_2O$ |
| 49-a | 14-a | H | -CH=CH- | 3-$CH_3O$-$C_6H_4$- | 6 | H | H | H | 121.3 |
| 50-a | 14-a | H | -N=CH- | 3-Cl-$C_6H_4$- | 6 | H | H | H | 161.1 |
| 51-a | 14-a | H | -CH=CH- | 3,4-$F_2$-$C_6H_3$- | 6 | H | H | H | 108.5 |
| 52-a | 14-a | H | -CH=CH- | 3,4-$(CH_3)_2$-$C_6H_3$- | 6 | H | H | H | 122.1 |
| 53-a | 14-a | H | -CH=N- | 3,4-$(CH_3)_2$-$C_6H_3$- | 6 | H | H | H | 127.5 |
| 54-a | 16-a | H | -CH=CH- | 1<u>H</u>-imidazol-1-yl | 6 | H | H | H | 193.8/* |
| 55-a | 16-a | H | -CH=CH- | 2-thienyl | 6 | H | H | H | 124.7 |
| 56-a | 14-a | H | -CH=CH- | 3-$CF_3$-$C_6H_4$- | 6 | H | H | H | 133.9 |
| 57-a | 14-a | H | -CH=CH- | 4-$CH_3$-$C_6H_4$- | 6 | H | H | H | 133.9/* |
| 58-a | 14-a | H | -N=CH- | 3-F-$C_6H_4$- | 6 | H | H | H | 165.0 |
| 59-a | 14-a | H | -CH=N- | 3,4-$F_2$-$C_6H_3$- | 6 | H | H | H | 104.2 |
| 60-a | 14-a | H | -CH=N- | 4-F-$C_6H_4$- | 6 | H | H | H | 135.1/* |
| 61-a | 14-a | H | -N=CH- | 3-$CH_3$-$C_6H_4$- | 6 | H | H | H | 118.0 |
| 62-a | 14-a | H | -CH=N- | 4-$CH_3$-$C_6H_4$- | 6 | H | H | H | 164.5/* |
| 63-a | 14-a | H | -CH=N- | 4-$OCH_3$-$C_6H_4$- | 6 | H | H | H | 151.1/* |
| 64-a | 14-a | H | -N=CH- | 3-$OCH_3$-$C_6H_4$- | 6 | H | H | H | 142.0 |
| 65-a | 14-a | H | -N=CH- | 3,4-$F_2$-$C_6H_3$- | 6 | H | H | H | 149.5 |
| 66-a | 14-a | H | -CH=N- | 3-$CF_3$-$C_6H_4$- | 6 | H | H | H | 142.9 |
| 67-a | 14-a | H | -CH=CH- | c.$C_6H_{11}$- | 6 | H | H | H | 285.0/2 HCl |
| 68-a | 14-a | H | -CH=N- | 3-$OCH_3$-$C_6H_4$- | 6 | H | H | H | 150.0/* |
| 69-a | 14-a | $CH_3$ | -CH=CH- | $C_6H_5$- | 6 | H | H | H | 52.6/0.5$H_2O$ |
| 70-a | 14-a | H | -CH=N- | 3-$CH_3$-$C_6H_4$- | 6 | H | H | H | 117.9 |
| 71-a | - | H | -CH=CH- | c.$C_3H_5$- | 6 | H | H | H | - |

* = $(COOH)_2$

| Comp. No. | Ex No. | R | $-X^1=X^2-$ | Y | p | $R^7$ | $R^8$ | $R^9$ | mp./salt |
|---|---|---|---|---|---|---|---|---|---|
| 72-a | - | H | -CH=CH- | $CH_3-C\equiv C-$ | 6 | H | H | H | - |
| 73-a | - | H | -CH=CH- | $CH_3$-CH=CH- | 6 | H | H | H | - |
| 74-a | - | H | -CH=CH- | 3-pyridinyl | 6 | H | H | H | - |
| 75-a | - | H | -CH=N- | $3,4Cl_2-C_6H_3-$ | 6 | H | H | H | - |
| 76-a | - | H | -CH=CH- | $3,4Cl_2-C_6H_3-$ | 6 | H | H | H | - |

Table 8-a

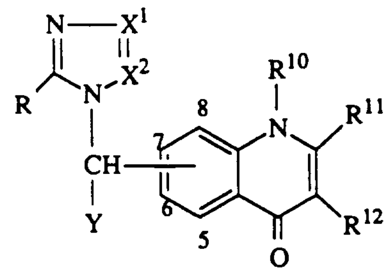

| Comp. No. | Ex. No. | R | $-X^1=X^2-$ | Y | p | $R^{10}$ | $R^{11}$ | $R^{12}$ | mp./salt |
|---|---|---|---|---|---|---|---|---|---|
| 77-a | 18-a | H | -CH=CH- | -H | 6 | H | $-CH_3$ | -H- | 245.5 (decomp) |

C-a) Pharmacological Examples

The useful pharmacological properties of the compounds of the present invention can for example be demonstrated by the following experiment.

Example 22-a

Metabolism of exogenous all-trans-retinoic acid

Male Wistar rats weighing 200~210 g were orally treated with vehicle (PEG 200) or with 40 mg/kg of a compound of formula (I-a). One hour later, the animals were anesthetized with ether and injected intrajugularly with 0.50 ml saline solution containing 20 $\mu$g of all-trans-retinoic acid. Two hours after this injection, rats were killed by decapitation and blood was collected on heparin. Blood samples were centrifuged (1000 g, 15 min) and plasma was recovered to determine the quantity of plasmatic all-trans-retinoic acid. The samples were analyzed by means of HPLC with UV-detection at 350 nm. Quatification was achieved by peak area integration and external standardization. Under the conditions used, plasma concentrations of the retinoic acid in vehicle-pretreated animals were not detectable (<0.5 ng/ml), whereas compound nos. 2-a, 3-a, 4-a, 5-a, 6-a, 7-a, 8-a, 9-a, 10-a, 11-a, 12-a, 15-a, 16-a, 20-a, 21-a, 24-a, 33-a, 41-a, 42-a, 55-a and 67-a enhanced the recovery of all-trans-retinoic acid from the plasma to a least 10 ng/ml after dosing with 40 mg/kg.

Example 23-a

Metabolism of endogenous all-trans-retinoic acid

Male Wistar rats weighing 200~210 g were orally treated with vehicle (PEG 200) or with 40 mg/kg of a compound of formula (I-a).Two hours after drug administration, the rats were killed by decapitation and blood was collected on heparin. Blood samples were centrifuged (1000 g, 15 min) and plasma was recovered to determine the quantity of plasmatic all-trans-retinoic acid. The samples were analyzed by means of HPLC with UV-detection at 350 nm. Quatification was achieved by peak area integration and external standardization. Under the conditions used, plasma concentrations of the retinoic acid in vehicle-pretreated animals were not detectable (<0.5 ng/ml), whereas compound nos. 2-a, 3-a, 4-a, 7-a, 8-a, 11-a, 12-a, 16-a, 19-a, 20-a, 24-a, 33-a, 41-a, 42-a, 46-a, 48-a, 49-a, 51-a, 55-a, 56-a, 59-a, 60-a, 66-a, 67-a, 68-a, 69-a and 70-a enhanced the recovery of all-trans-retinoic acid from the plasma to a least 1 ng/ml.

A-b) Preparation of the intermediates in the synthesis of quinazoline derivatives of formula (I-b)

Example 1-b

To a vigorously stirred amount of 45 parts of aluminiumtrichloride were added dropwise 7.05 parts of N,N-dimethylformamide. After stirring for 5 min. at 70 °C, there were added 5 parts of benzoyl chloride and, dropwise, 4.7 parts of 3,4-dihydro-2(1H)-quinazolinone. Stirring was continued for 2 hours at 70 °C. The reaction mixture was poured into ice-water and there were added 63.5 parts of HCl. The precipitate was filtered off and recrystallized from 2-methoxyethanol, yielding 6.5 parts (76.4%) of 6-benzoyl-3,4-dihydro-2-(1H)-quinazolinone; mp. 264.8 °C (interm. 1-b). In a similar manner there were also prepared the intermediates listed in Table 1-b.

Table 1-b

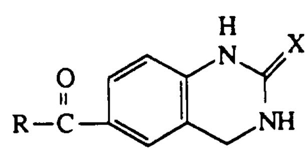

| Interm. No. | R | X | Physical data (mp. in °C) |
|---|---|---|---|
| 2-b | (3-pyridinyl) | O | 256.4 / .HCl |
| 3-b | $3-ClC_6H_4$ | O | >260 (decomp.) |
| 4-b | $i.C_3H_7$ | O | 291.0 |
| 5-b | $CH_3$ | O | 240.1 |
| 6-b | $c.C_3H_5$ | O | 269.3 |
| 7-b | $C_6H_5$ | S | 264.7 |

Example 2-b

α) A mixture of 14.7 parts of 5-chloro-2-nitrobenzaldehyde, 13.3 parts of thrimethoxymethane, 0.15 parts of 4-methylbenzenesulfonic acid and 64 parts of 2-propanol was stirred at reflux temperature until completion of the reaction. After cooling, there was added $Na_2CO_3$ and stirring was continued for 5 min. The reaction mixture was filtered and the filtrate was evaporated, yielding 18.3 parts (99.7%) of 4-chloro-2-(dimethoxymethyl)-1-nitrobenzene (interm. 8-b).

β) To a solution of 9.55 parts of benzeneacetonitrile in 90 parts of N,N-dimethylacetamide were added 7.6 parts of a dispersion of sodium hydride in mineral oil (50%). The mixture was stirred until $H_2$-evolution ceased. Then there were added 1.28 parts of 2-(2-methoxyethoxy)-N,N-bis[2-(2-methoxyethoxy)ethyl]-ethanamine and, dropwise, a solution of 18.3 parts of intermediate 8-b, namely 4-chloro-2-(dimethoxymethyl)-1-nitrobenzene, in 27 parts of N,N-dimethylacetamide. The whole was stirred at room temperature for a while and was then poured into ice-water. After neutralizing, the product was extracted with dichloromethane. The extract was dried, filtered and evaporated, yielding 28.1 parts (100%) of 3-(dimethoxymethyl)-4-nitro-α-phenylenzeneacetonitrile (interm. 9-b).

γ) A mixture of 26.7 parts of intermediate 9-b, namely 3-(dimethoxymethyl)-4-nitro-α-phenylbenzeneacetonitrile,12.3 parts of potassium carbonate and 360 parts of N,N-dimethylacetamide was stirred at room temperature while bubbling air through it. The reaction mixture was poured into water and the whole was extracted with dichloromethane. The extract was dried, filtered and evaporated and the residue was purified by column chromatography (silica gel ; $CHCl_3$/hexane 80:20). The eluent of the desired fraction was evaporated, yielding 18.1 parts (67.3%) of [3-(dimethoxymethyl)-4-nitrophenyl]-phenylmethanone (interm. 10-b).

δ) A mixture of 19 parts of intermediate 10-b, namely [3-(dimethoxymethyl)-4-nitrophenyl]-phenylmethanone, 40 parts of an aqueous hydrochloric acid solution 5N and 120 parts of trichloromethane was stirred overnight at room temperature and for 4 hours at reflux temperature. After cooling, the organic layer was separated, basified with $NH_4OH$ (aq.), washed with water, dried, filtered and evaporated. The residue was crystallized from a mixture of 2,2'-oxybispropane and ethyl acetate. The product was filtered off, washed successively with a mixture of 2,2'-oxybispropane and ethyl acetate and with 2,2'-oxybispropane and dried in vacuo at 50 ° C, yielding 7.61 parts (49.0%) of 5-benzoyl-2-nitrobenzaldehyde; mp. 96.7 ° C (interm. 11-b)

ε) A mixture of 19 parts of intermediate 11-b, namely 5-benzoyl-2-nitrobenzaldehyde, 6.18 parts of hydroxylamine monohydrochloride, 474 parts of ethanol and 7.76 parts of sodium hydrogen carbonate was refluxed for 14 hours. The reaction mixture was filtered and the filtrate was evaporated. The residual oil was stirred in water. The solid was filtered off and recrystallized from a mixture of ethyl acetate and hexane. The product was filtered off, washed successively with a mixture of ethyl acetate and hexane and with 2,2'-oxybispropane and dried in vacuo at 60 ° C, yielding 16.6 parts (82.4%) of (E + Z)-5-benzoyl-2-nitrobenzaldehyde, oxime; mp. 135.0 ° C (interm. 12-b).

ζ) A mixture of 17.5 parts of intermediate 12-b, namely (E + Z)-5-benzoyl-2-nitrobenzaldehyde, oxime, and 162 parts of acetic anhydride was refluxed for 48 hours. The reaction mixture was evaporated and the residue was taken up in water. After basifying with $NaHCO_3$, the product was extracted with dichloromethane. The extract was dried, filtered and evaporated and the residue was purified by column chromatography (silica gel ; $CHCl_3$/hexane 80:20). The eluent of the desired fraction was evaporated and the residue was co-evaporated with ethyl acetate. The product was crystallized successively from a mixture of ethyl acetate and 2,2'-oxybispropane and from ethyl acetate. The product was filtered off, washed with a mixture of ethyl acetate and 2,2'-oxybispropane and dried in vacuo at 50 ° C, yielding 5.30 parts (32.4%) of 5-benzoyl-2-nitrobenzonitrile; mp. 121.8 ° C (interm. 13-b).

η) A solution of 8.9 parts of intermediate 13-b, namely 5-benzoyl-2-nitrobenzonitrile, 166 parts of sulfuric acid and 10 parts of water was heated at 90 ° C for 1 3/4 hours. The reaction mixture was poured into ice-water. The precipitate was filtered off and recrystallized from methanol. The product was filtered off, washed with methanol and 2,2'-oxybispropane and dried in vacuo at 60-70 ° C, yielding 5.23 parts (54.8%) of 5-benzoyl-2-nitrobenzamide; mp. 244.3 ° C (intermediate 14-b).

θ) A mixture of 7.76 parts of intermediate 14-b, namely 5-benzoyl-2-nitrobenzamide, 2 parts of a solution of thiophene in methanol 4% and 198 parts of methanol was hydrogenated overnight at normal pressure and at 50 ° C with 2 parts of palladium-on-charcoal catalyst 10%. The catalyst was filtered off and washed with tetrahydrofuran. The combined filtrates were evaporated and the residue was co-evaporated with methylbenzene. The residue was purified by column chromatography (silica gel ; $CHCl_3/CH_3OH/CH_3OH(NH_3)$ 90:5:5). The eluent of the desired fraction was evaporated and the residue was taken up in methanol. This solution was concentrated and the product was filtered off, washed with methanol and 2,2'-oxybispropane and dried in vacuo at 60 ° C, yielding 2.84 parts (41.0%) of 2-amino-5-benzoylbenzamide; mp. 225.2 ° C (interm. 15-b).

ι) A mixture of 5 parts of intermediate 15-b, namely 2-amino-5-benzoylbenzamide, 5.53 parts of trimethoxymethane and 61 parts of formic acid was refluxed for 4-5 hours. The reaction mixture was evaporated and the residue was taken up in water. After basifying with $NH_4OH$ (aq.), the product was extracted with a mixture of $CHCl_3$, $CH_3OH$ and $CH_3OH(NH_3)$ (90:5:5). The extract was dried, filtered and

evaporated and the residue was crystallized from acetonitrile. The solid was filtered off* and purified by column chromatography (silica gel ; $CHCl_3/CH_3OH$ 95:5). The eluent of the desired fraction was evaporated and the residue was stirred in ethyl acetate. The product was filtered off, washed with ethyl acetate and 2,2'-oxybispropane and dried in vacuo at 70 °C, yielding 0.53 parts (10.1%) of product; mp. 215.5 °C. *The mother liquor was evaporated and the residue was treated similarly as above, yielding an additional 0.69 parts (13.2%) of product; mp. 214.3 °C. Total yield : 1.22 parts (23.3%) of 6-benzoyl-4-(3H)-quinazolinone (interm. 16-b).

Example 3-b

α) To a solution of 22.8 parts of potassium hydroxide, 39.2 parts of pyridine and 89 parts of tetrahydrofuran were added 11.7 parts of benzeneacetonitrile and 16.7 parts of 2-nitrobenzoic acid. After stirring for 2 hours at room temperature, the reaction mixture was diluted with 200 parts of water while cooling on ice. The whole was acidified with HCl and then the tetrahydrofuran layer was separated. There were added 183 parts of 2,2'-oxybispropane and the mixture was stirred overnight. The precipitate was filtered off and dried, yielding 12.7 parts (47.7%) of product. Evaporation of the filtrate yielded an additional 17 parts (63.8%) of product. Total yield : 29.7 parts (100%) of 3-(cyanophenylmethylene)-6-(hydroxyimino)-1,4-cyclohexadiene-1-carboxylic acid; mp. 230.7 °C (intermediate 17-b).

β) To a solution of 16.2 parts of potassium hydroxide, 150 parts of water and 5.72 parts of intermediate 17-b, namely 3-(cyanophenylmethylene)-6-(hydroxyimino)-1,4-cyclohexadiene-1-carboxylic acid, was added a solution of 16.25 parts of hydrogen peroxide in 16 parts of water. After stirring for 1 hour at room temperature, the reaction mixture was acidified with HCl while cooling on ice. The product was extracted with dichloromethane and the extract was dried, filtered and evaporated. The residue was recrystallized from methylbenzene, yielding 3.7 parts (63.5%) of 5-benzoyl-2-nitrobenzoic acid; mp. 168.6 °C (interm. 18-b).

γ) To solution of 8.5 parts of intermediate 18-b, namely 5-benzoyl-2-nitrobenzoic acid, in 66.5 parts of dichloromethane were added 5.3 parts of 1,1'-carbonylbis[1H-imidazole]. After stirring for 1 hour at room temperature, there were added 9.8 parts of benzenemethanamine. Stirring at room temperature was continued for 8 hours. The reaction mixture was diluted with 100 parts of water and acidified with HCl. The organic layer was separated, dried, filtered and evaporated. The residue was purified twice by column chromatography (silica gel ; $CHCl_3/CH_3OH$ 98:2 ; $CH_3COOC_2H_5/C_6H_5CH_3$ 10:90). The eluent of the desired fractions was evaporated and the residue was crystallized from methylbenzene, yielding 8.1 parts (72.5%) of 5-benzoyl-2-nitro-N-(phenylmethyl)benzamide; mp. 167.4 °C (interm. 19-b).

δ) A mixture of 6 parts of intermediate 18-b, namely 5-benzoyl-2-nitrobenzoic acid, 5.24 parts of thionyl chloride and 89.4 parts of trichloromethane was stirred for 1 hour at reflux temperature. The reaction mixture was used as such for further synthesis. Yield: 6.37 parts (100%) of 5-benzoyl-2-nitrobenzoyl chloride (interm. 20-b).

ε) Methanamine was bubbled through a solution of 23.17 parts of intermediate 20-b, namely 5-benzoyl-2-nitrobenzoyl chloride, in 178 parts of tetrahydrofuran at 0 °C for 15 min. and at room temperature for 30 min. The reaction mixture was evaporated and the residue was stirred with HCl 1N for 1 hour. The product was extracted with dichloromethane and the extract was dried, filtered and evaporated. The residue was purified by column chromatography (silica gel ; $CHCl_3/CH_3OH$ 98:2). The eluent of the desired fraction was evaporated and the residue was crystallized from methylbenzene, yielding 7 parts (30.8%) of 5-benzoyl-N-methyl-2-nitrobenzamide; mp. 137.6 °C (interm. 21-b).

ζ) A mixture of 6.5 parts of intermediate 21-b, namely 5-benzoyl-N-methyl-2-nitrobenzamide, 2 parts of a solution of thiophene in methanol 4% and 97 parts of 2-methoxyethanol was hydrogenated at normal pressure and at 50 °C with 2 parts of palladium-on-charcoal catalyst 10%. After the calculated amount of hydrogen was taken up, the catalyst was filtered off and the filtrate was evaporated. The residue was recrystallized from 2-propanol, yielding 4.64 parts (79.3%) of 2-amino-5-benzoyl-N-methylbenzamide; mp. 140.5 °C (interm. 22-b).

η) A solution of 5.3 parts of intermediate 22-b, namely 2-amino-5-benzoyl-N-methylbenzamide, 4 parts of 1,1'-carbonylbis[1H-imidazole], 107 parts of tetrahydrofuran and a catalytic amount of sodium hydride was stirred for 17 hours at reflux temperature. The precipitate was filtered off and dried in vacuo, yielding 3.5 parts (59.5%) of product. The filtrate was evaporated and the residue was washed with water and ethyl acetate and dried, yielding an additional 1.5 parts (25.5%) of product. Total yield : 5.0 parts (85.0%) of 6-benzoyl-3-methyl-2,4-(1H,3H)-quinazolinedione; mp. 250.6 °C (interm. 23-b).

In a similar manner there were also prepared :

6-benzoyl-2,4(1H,3H)-quinaxolinedione; mp. >300 °C (interm.24-b);

6-benzoyl-3-(phenylmethyl)-2,4(1H,3H)-quinazolinedione; mp. 237.9 °C (interm 25-b); and 6-benzoyl-2,3-dihydro-3-(phenylmethyl)-2-thioxo-4(1H)-quinazolinone; mp. 255.1 °C (interm. 26-b).

Example 4-b

To a mixture of 4.35 parts of intermediate 2-b, namely 3,4-dihydro-6-(3-pyridinylcarbonyl)-2(1H)-quinazolinone monohydrochloride, 63.2 parts of methanol, 1.2 parts of sodium hydroxide and 15 parts of water were added portionwise 0.6 parts of sodium tetrahydroborate. After stirring for 2 hours at room temperature, there was added a mixture of 2.1 parts of acetic acid in 25 parts of water. The precipitate was filtered off, washed with water, 2-propanol and 1,1'-oxybisethane and dried, yielding 3.7 parts (96.6%) of 3,4-dihydro-6-[hydroxy(3-pyridinyl)methyl]-2(1H)-quinazolinone; mp. 272.0 °C (interm. 27-b).
In a similar manner there were also prepared the intermediates listed in Tables 2-b and 3-b.

Table 2-b

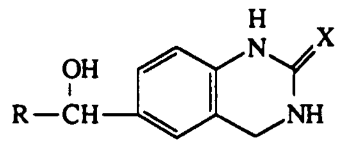

| Interm. No. | R | X | Physical data (mp. in °C) |
|---|---|---|---|
| 28-b | $C_6H_5$ | O | 214.3 |
| 29-b | $3\text{-}ClC_6H_4$ | O | 266.4 |
| 30-b | $i.C_3H_7$ | O | 274.9 |
| 31-b | $CH_3$ | O | 275.5 |
| 32-b | $C_6H_5$ | S | 239.7 |
| 33-b | $c.C_3H_5$ | O | 225 |

Table 3-b

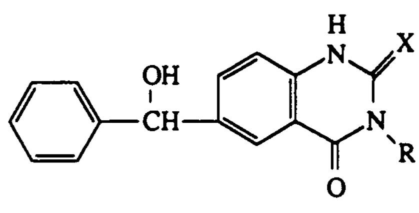

| Interm. No. | R | X | Physical data (mp. in °C) |
|---|---|---|---|
| 34-b | $CH_2\text{-}C_6H_5$ | O | 191.8 |
| 35-b | $CH_2\text{-}C_6H_5$ | S | 178.1 |
| 36-b | H | O | - |
| 37-b | $CH_3$ | O | 261.0 |

In a similar manner there was also prepared :
6-(hydroxyphenylmethyl)-4(3H)-quinazolinone; mp. 204.8 °C (interm. 38-b).

Example 5-b

A mixture of 3 parts of intermediate 27-b, namely 3,4-dihydro-6-[hydroxy(3-pyridinyl)methyl]-2(1H)-quinazolinone, and 40.5 parts of thionyl chloride was stirred for 10 min. at room temperature and for 15 min. at reflux temperature. The reaction mixture was evaporated and the residue was co-evaporated with methylbenzene. The residue was dried in vacuo at 60 °C for 24 hours, yielding 3.1 parts (99.9%) of 6-[chloro(3-pyridinyl)methyl]-3,4-dihydro-2(1H)-quinazolinone monohydrochloride (intermediate 39-b). In a similar manner there were also prepared the intermediates listed in Tables 4-b and 5-b.

Table 4-b

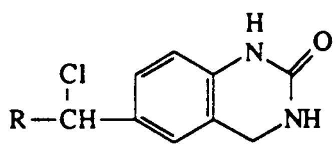

| Interm. No. | R | Physical data |
|---|---|---|
| 40-b | $C_6H_5$ | - |
| 41-b | $i.C_3H_7$ | HCl |
| 42-b | $CH_3$ | HCl |
| 43-b | $3-ClC_6H_4$ | - |

Table 5-b

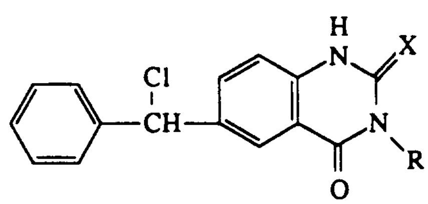

| Interm. No. | R | X | Physical data |
|---|---|---|---|
| 44-b | $CH_2-C_6H_5$ | S | - |
| 45-b | H | O | - |
| 46-b | $CH_2-C_6H_5$ | O | - |
| 47-b | $CH_3$ | O | - |

Example 6-b

A mixture of 4 parts of intermediate 38-b, namely 6-(hydroxyphenylmethyl)-4(3H)-quinazolinone, and 67.7 parts of a solution of hydrobromic acid in acetic acid 30% was stirred for 24 hours at room temperature. The reaction mixture was evaporated and the residue was co-evaporated with methylbenzene, yielding 6.5 parts (100%) of 6-(bromophenylmethyl)-4(3H)-quinazolinone monohydrobromide (interm. 48-b).
In a similar manner there were also prepared:
6-(bromophenylmethyl)-3-(phenylmethyl)-2,4(1H,3H)-quinazolinedione (interm. 49-b).
6-(bromophenylmethyl)-3,4-dihydro-2(1H)-quinazolinethione (interm. 50-b).

Example 7-b

α) To a stirred solution of 7.5 parts of 4-amino-3-nitro-α-phenylbenzenemethanol, 0.1 parts of a dispersion of sodium hydride in mineral oil (50%) and 90 parts of tetrahydrofuran were added 6.4 parts of 1,1'carbonylbis[1H-imidazole]. After stirring for 1 hour at reflux temperature, the reaction mixture was evaporated. The residue was purified by column chromatography (silica gel ; $CHCl_3/CH_3OH$ 93:7). The eluent of the desired fraction was evaporated and the residue was crystallized from methylbenzene. The product was dried for 2 hours at 80 °C, yielding 6.33 parts (71%) of 4-[(1-H-imidazol-1-yl)phenylmethyl]-2-nitrobenzenamine (interm. 51-b).

β) To 200 ml of cooled (0-5 °C) HCl 5N were added 19.3 parts of intermediate 51-b, namely 4-[(1H-imidazol-1-yl)phenylmethyl]-2-nitrobenzenamine, while stirring. When a homogeneous solution was obtained, there was added dropwise a solution of 4.75 parts of sodium nitrite in 40 parts of water at 0-5 °C. Stirring at 0-5 °C was continued for 1/2 hour and then the mixture was added dropwise to a cooled (0-5 °C) solution of 5.8 parts of copper(I)cyanide, 6.42 parts of sodium cyanide and 127.1 parts of $Na_2CO_3$ -(aq.) in a mixture of 700 parts of water and 298 parts of trichloromethane. The whole was left overnight to warm up to room temperature and then there were added $NH_4OH$ (aq.) and 447 parts of trichloromethane. After heating at 50 °C for 15 min. and subsequent cooling, the organic layer was separated, dried, filtered and evaporated. The residue was purified twice by column chromatography (silica gel ; $CHCl_3/CH_3OH$ ($NH_3$) 97.5:2.5 ; $CHCl_3/CH_3OH$ 97.5:2.5). The eluent of the desired fractions was evaporated, yielding 14.6 parts (73.2%) of 4[(1H-imidazol-1-yl)phenylmethyl]-2-nitrobenzonitrile (interm. 52-b).

γ) A solution of 6 parts of intermediate 52-b, namely 4-[(1H-imidazol-1-yl)phenylmethyl]-2-nitrobenzonitrile, in 316 parts of methanol saturated with ammonia was hydrogenated at room temperature and normal pressure with 3 parts of Raney nickel. After the calculated amount of hydrogen was taken up, the catalyst was filtered off and the filtrate was evaporated. The residue was co-evaporated with a mixture of methanol and methylbenzene, yielding 4.8 parts (82.9%) of 2-amino-4-[(1H-imidazol-1-yl)phenylmethyl]-benzamide (interm. 53-b).

Example 8-b

α) A mixture of 25 parts of 5-chloro-2-nitrobenzenemethanol, 13.3 parts of 3,4-dihydro-2H-pyran, 0.28 parts of dichloromethane and 300 parts of 4-methylbenzenesulfonic acid was stirred and for 2 hours at reflux temperature. After cooling, there was added $Na_2CO_3$ and the whole was stirred for 10 min. The reaction mixture was filtered and the filtrate was evaporated. The residue was co-evaporated with methylbenzene and was further purified by column chromatography (silica gel ; $CHCl_3$). The eluent of the desired fraction was evaporated and the residue was co-evaporated with methylbenzene, yielding 36 parts (99.6%) of 2-[(5-chloro-2-nitrophenyl)methoxy]tetrahydro-2H-pyran (interm. 54-b).

β) To a mixture of 7.13 parts of a dispersion of sodium hydride in mineral oil (50%) and 94 parts of N,N-dimethylacetamide was added dropwise a solution of 9.1 parts of benzeneacetonitrile in 18.8 parts of N,N-dimethylacetamide. After the hydrogen evolution had ceased, there were added 1.28 parts of tris-2,2,2-(2-methoxyethoxy)ethanamine and a solution of 20.2 parts of intermediate 54-b, namely 2-[(5-chloro-2-nitrophenyl)methoxy]-tetrahydro-2H-pyran, in 28.2 parts of N,N-dimethylacetamide. After 15 minutes, the reaction mixture was poured into ice-water and the whole was neutralized. The product was extracted with dichloromethane and the extract was dried, filtered and evaporated, yielding 26.2 parts (100%) of 4-nitro-α-phenyl-3-[[(tetrahydro-2H-pyran-2-yl)oxy]methyl]benzeneacetonitrile (interm. 55-b).

γ) A mixture of 26.2 parts of intermediate 55-b, namely 4-nitro-α-phenyl-3-[[(tetrahydro-2H-pyran-2-yl)-oxy]methyl]benzeneacetonitrile, 10.2 parts of potassium carbonate and 376 parts of N,N-dimethylacetamide was stirred at room temperature while air was bubbled through. The reaction mixture

was poured into water and the product was extracted with 2,2'-oxybispropane. The extract was dried, filtered and evaporated, yielding 25 parts (98.6%) of [4-nitro-3-[[(tetrahydro-2H-pyran-2-yl)oxy]methyl]-phenyl] phenylmethanone (interm. 56-b).

δ) A mixture of 20 parts of intermediate 56-b, namely [4-nitro-3-[[(tetrahydro-2H-pyran-2-yl)oxy]methyl]-phenyl] phenylmethanone, 2 parts of a solution of thiophene in methanol 4% and 395 parts of methanol was hydrogenated overnight at normal pressure and room temperature with 2 parts of palladium-on-charcoal catalyst 10%. Tetrahydrofuran was added to dissolve the precipitated reaction product. The catalyst was filtered off and the filtrate was evaporated. The residue was recrystallized from methanol. The product was filtered off, washed with methanol and 2,2'-oxybispropane and dried in vacuo at 60 °C, yielding 14.93 parts (82.0%) of [4-amino-3-[[(tetrahydro-2H-pyran-2-yl)oxy]methyl]phenyl] phenylmethanone; mp. 164.0 °C (interm. 57-b).

ε) To a stirred and cooled (10 °C) solution of 13.7 parts of intermediate 57-b, namely [4-amino-3-[[-(tetrahydro-2H-pyran-2-yl)oxy]methyl]phenyl] phenylmethanone, in 147 parts of pyridine were added dropwise 7.14 parts of ethyl chloroformate. After stirring for 1 hour at 10 °C, the reaction mixture was poured into 700 parts of water. The product was extracted with dichloromethane and the extract was washed with water (3x), dried, filtered and evaporated. The residue was co-evaporated with ethanol (3x) and was then crystallized from ethanol. The product was filtered off, washed with ethanol and 2,2'-oxybispropane and dried in vacuo at 60 °C, yielding 14.05 parts (83.5%) of ethyl [4-benzoyl-2-[[-(tetrahydro-2H-pyran-2-yl)oxy]methyl]phenyl]carbamate; mp. 115.9 °C (interm. 58-b).

ζ) To a solution of 2 parts of intermediate 58-b, namely ethyl [4-benzoyl-2-[[(tetrahydro-2H-pyran-2-yl)-oxy]methyl]phenyl]carbamate, 11.9 parts of ethanol and 22.3 parts of tetrahydrofuran were added 0.2 parts of sodium tetrahydroborate. The mixture was stirred at room temperture for 1 hour and at 40-50 °C until completion of the reaction. The solvent was evaporated and the residue was taken up in water. The product was extracted with dichloromethane and the extract was dried, filtered and evaporated. The residue was co-evaporated with methylbenzene and was used as such for further reaction. Yield : 2 parts (100%) of ethyl [4-(hydroxyphenylmethyl)-2-[[(tetrahydro-2H-pyran-2-yl)oxy]methyl]phenyl]carbamate. (interm. 59-b).

η) To a refluxing solution of 2 parts of intermediate 59-b, namely ethyl [4-(hydroxyphenylmethyl)-2-[[-(tetrahydro-2H-pyran-2-yl)oxy]methyl]phenyl]carbamate, in 33.3 parts of dichloromethane was added dropwise a solution of 1.8 parts of 1,1'-carbonylbis[1H-imidazole] in 20 parts of dichloromethane. After stirring for 3 days at reflux temperature, the reaction mixture was cooled and washed with water (2x). The organic layer was dried, filtered and evaporated. The residue was purified by column chromatography (silica gel ; $CHCl_3/C_2H_5OH$ 98:2). The eluent of the desired fraction was evaporated and the residue was left to crystallize. The crystallized product was filtered off, stirred in hexane and dried in vacuo, yielding 0.67 parts (41.1%) of ethyl [4-[(1H-imidazol-1-yl)phenylmethyl]-2-[[(tetrahydro-2H-pyran-2-yl)oxy]methyl]-phenyl]carbamate; mp. 132.2 °C (interm. 60-b).

θ) A solution of 10.5 parts of intermediate 60-b, namely ethyl [4-[(1H-imidazol-1-yl)phenylmethyl]-2-[[-(tetrahydro-2H-pyran-2-yl)oxy]methyl]phenyl]carbamate, 5.5 parts of 4-methylbenzenesulfonic acid and 198 parts of ethanol was stirred over weekend at room temperature and for a short time at 50-60 °C. After cooling, there were added ethanol and $Na_2CO_3$. The whole was stirred for 15 min. and was then filtered. The filtrate was evaporated and the residue was partitioned between dichloromethane and water. The organic layer was separated and the aqueous layer was re-extracted with dichloromethane. The combined dichloromethane layers were dried, filtered and evaporated. The residue was purified by column chromatography (silica gel ; $CHCl_3/CH_3OH$ 95:5). The eluent of the desired fraction was evaporated and the residue was co-evaporated with methylbenzene. The crystallized product was filtered off, stirred in 2,2'-oxybispropane and dried in vacuo, yielding 4.65 parts (46.0%) of ethyl [2-(hydroxymethyl)-4-[(1H-imidazol-1-yl)phenylmethyl]phenyl]carbamate; mp. 143.1 °C (interm. 61-b).

ι) To a solution of 5.9 parts of intermediate 61-b, namely ethyl [2-(hydroxymethyl)-4-[(1H-imidazol-1-yl)-phenylmethyl]phenyl]carbamate, in 798 parts of dichloromethane were added 21.5 parts of manganese-(IV)oxide and a catalytic amount of $KMnO_4$. After stirring for 18 hours at room temperature, the reaction mixture was filtered over diatomaceous earth and the filtrate was evaporated. The residue was co-evaporated with methylbenzene and was further purified by column chromatography (silica gel ; $CHCl_3/CH_3OH$ 95:5). The eluent of the desired fraction was evaporated and the residue was taken up in ethyl acetate. This solution was concentrated and left to crystallize. The crystallized product was filtered off, stirred in 2,2'-oxybispropane and dried in vacuo, yielding 2.85 parts (48.8%) of ethyl [2-formyl-4-[(1H-imidazol-1-yl)phenylmethyl]phenyl]carbamate; mp. 107.0 °C (interm. 62-b).

κ) A solution of 2 parts of intermediate 62-b, namely ethyl [2-formyl-4-[(1H-imidazol-1-yl)phenylmethyl]-phenyl]carbamate in 32.4 parts of 1-butanol were added to the residue. The whole was stirred at room

temperature overnight while saturating with methanamine. The solvent was evaporated and the residue was co-evaporated with methylbenzene, yielding 1.7 parts (82.3%) of ethyl [4-[(1H-imidazol-1-yl)-phenylmethyl]-2-[(methylimino)methyl]phenyl]carbamate (interm. 63-b).

Example 9-b

α) A mixture of 68 parts of intermediate 10-b, namely [3-(dimethoxymethyl)-4-nitrophenyl] phenylmethanone, 4 parts of a solution of thiophene in methanol 4%, 20 parts of calcium oxide and 474 parts of methanol was hydrogenated for 24 hours at normal pressure and room temperature with 6 parts of palladium-on-charcoal catalyst 10%. The catalyst was filtered off and the filtrate was evaporated. The residue was co-evaporated with methylbenzene, yielding 59.1 parts (96.8%) of [4-amino-3-(dimethoxymethyl)phenyl] phenylmethanone (interm. 64-b).

β) To a stirred and cooled (10 °C) solution of 22.1 parts of intermediate 64-b, namely [4-amino-3-(dimethoxymethyl)phenyl] phenylmethanone, in 147 parts of pyridine were added dropwise 12.1 parts of acetyl chloride. After stirring for 1/2 hour at 10 °C and overnight at room temperature, the reaction mixture was poured into water. The product was extracted with 2,2'-oxybispropane and dichloromethane. The combined extracts were dried, filtered and evaporated. The residue was dissolved in dichloromethane. This solution was washed successively with HCl 0.1N, ammonia and water and was then dried, filtered and evaporated, yielding 27.5 parts (100%) of N-[-4-benzoyl-2-(dimethoxymethyl)phenyl]-acetamide (interm. 65-b).

γ) To a stirred solution of 25.6 parts of intermediate 65-b, namely N-[4-benzoyl-2-(dimethoxymethyl)-phenyl]acetamide, in 119 parts of methanol were added portionwise 10.72 parts of sodium tetrahydroborate. Stirring was continued for a while at 60 °C and over weekend at room temperature. The reaction mixture was evaporated and the residue was taken up in water. The product was extracted with dichloromethane (2x) and the combined extracts were dried, filtered and evaporated. The residue was purified by column chromatography (silica gel ; $CH_3COOC_2H_5$/hexane 50:50 → 60:40). The eluent of the desired fraction was evaporated, yielding 13.1 parts (50.8%) of N-[2-(dimethoxymethyl)-4-(hydroxyphenylmethyl)phenyl]acetamide (interm. 66-b).

δ) To a refluxing solution of 13.1 parts of intermediate 66-b, namely N-[2-(dimethoxymethyl)-4-(hydroxyphenylmethyl)phenyl]acetamide, in 200 parts of dichloromethane was added dropwise a solution of 7.07 parts of 1,1'-carbonylbis[1H-imidazole] in 106.4 parts of dichloromethane. After refluxing for 3 1/2 hours and stirring at room temperature overnight, the reaction mixture was washed with water (2x), dried, filtered and evaporated, yielding 16.6 parts (100%) of N-[2-(dimethoxymethyl)-4-[(1H-imidazol-1-yl)-phenylmethyl]phenyl]acetamide (interm. 67-b).

ε) A solution of 2.5 parts of intermediate 67-b, namely N-[2-(dimethoxymethyl)-4-[(1H-imidazol-1-yl)-phenylmethyl]phenyl]acetamide, 52.5 parts of acetic acid and 10 parts of water was stirred for 1/2 hour at reflux temperature. The reaction mixture was evaporated and the residue was co-evaporated with methylbenzene, yielding 2.2 parts (100%) of N-[2-formyl-4-[(1H-imidazol-1-yl)phenylmethyl]phenyl]-acetamide (interm. 68-b).

Example 10-b

A solution of 13 parts of N-[6-(bromomethyl)-4-hydroxy-2-quinazolinyl]-2,2-dimethylpropanamide, 15.5 parts of 1H-imidazole and 80 parts of acetonitrile was stirred for 4 hours at reflux temperature. The reaction mixture was evaporated and the residue was extracted with ethyl acetate. The extract was washed with $NaHCO_3$ (aq.), dried, filtered and evaporated. The residue was purified by column chromatography (silica gel ; $CH_2Cl_2/CH_3OH$ 98:2). The eluent of the desired fraction was evaporated, yielding 4.3 parts (34.7%) of N-[4-hydroxy-6-(1H-imidazol-1-ylmethyl)-2-quinazolinyl]-2,2-dimethylpropanamide (interm. 69-b).

B-b) Preparation of the final quinazoline compounds of formula (I-b)

Example 11-b

A mixture of 3.6 parts of 6-(chlorophenylmethyl)-3,4-dihydro-2(1H)-quinazolinone, 5.3 parts of 1H-imidazole, 60 parts of acetonitrile and 27.5 parts of dimethyl sulfoxide was stirred for 4 hours at reflux temperature. After concentration, the residue was washed twice with water, dissolved in a mixture of trichloromethane and methanol (90:10 by volume), dried, filtered and evaporated. The residue was purified by column chromatography over silica gel using a mixture of trichloromethane and methanol, saturated with

ammonia (95:5 by volume) as eluent. The pure fractions were collected and the eluent was evaporated. The residue was crystallized from a 45 parts of ethyl acetate and a few drops of water. The product was filtered off, washed with ethyl acetate and 2,2'-oxybispropane and dried, yielding 2.3 parts (58.1%) of 3,4-dihydro-6-[(1H-imidazol-1-yl)phenylmethyl]-2(1H)-quinazolinone; mp. 222.5 °C (comp. 16-b).

Example 12-b

A mixture of 5.8 parts of 6-(chlorophenylmethyl)-2,4(1H,3H)-quinazolinedione, 10 parts of 1H-1,2,4-triazole and 158 parts of acetonitrile was stirred for 1 hour at room temperature and for 2 hours at reflux temperature. The solvent was evaporated and the residue was washed with water. The precipitate was filtered off and purified by column chromatography (silica gel ; $CH_2Cl_2/CH_3OH$ 90:10). The eluent of the first fraction was evaporated and the residue was washed with ethyl acetate and dried, yielding 2.2 parts (34.1%) of 6-[phenyl(1H-1,2,4-triazol-1-yl)methyl]-2,4(1H,3H)-quinazolinedione; mp. 280.9 °C (comp. 40-b).

Example 13-b

To a stirred and cooled (15 °C) solution of 2.5 parts of 1H-1,2,4-triazole in 70 parts of 1,4-dioxane was added dropwise 1 part of thionyl chloride under nitrogen atmosphere. After stirring for 10 minutes at 20 °C, a solution of 2 parts of 6-(cyclopropylhydroxymethyl)-3,4-dihydro-2(1H)-quinazolinone in 80 parts of 1,4-dioxane was added portionwise to the previous mixture at 20-25 °C. After stirring overnight at room temperature, the precipitated product was filtered off, washed with 1,4-dioxane and purified by column chromatography over silica gel using a mixture of dichloromethane, methanol and methanol, saturated with ammonia (90:5:5 by volume) as eluent. The eluent of the desired fraction was evaporated and the residue was further purified, first by column chromatography (HPLC) over silica gel using a mixture of dichloromethane and methanol (96:4 and 97.5:2.5 by volume) and then by column chromatography (RP 18) using a mixture of water and methanol (80:20 by volume) as eluents. The eluent of the desired fraction was evaporated and the residue was stirred in 2,2'-oxybispropane. The product was filtered off, washed with 2,2'-oxybispropane and dried in vacuo at 60 °C, yielding 0.04 parts (1.7%) of 6-[cyclopropyl(1H-1,2-4-triazol-1-yl)methyl]-3,4-dihydro-2(1H)-quinazolinone; mp. 184.4 °C (comp. 25-b).

Example 14-b

A solution of 13 parts of N-[6-(bromomethyl)-4-hydroxy-2-quinazolinyl]-2,2-dimethylpropanamide and 15.5 parts of 1H-imidazole in 80 parts of acetonitrile was stirred for 4 hours at reflux temperature. The reaction mixture was concentrated and the concentrate was extracted with ethyl acetate. The extract was washed with a diluted sodium hydrogen carbonate solution, dried, filtered and evaporated. The residue was purified by column chromatography over silica gel using a mixture of dichloromethane and methanol (98:2 by volume) was eluent. The pure fractions were collected and the eluent was evaporated. A solution of 6.5 parts of the residue in 75 parts of a hydrochloric acid solution 3 N was stirred for 2 hours at reflux temperature. The reaction mixture was evaporated to dry and the residue was dissolved in a potassium carbonate solution 40%. The product was extracted with a mixture of dichloromethane and ethanol. The extract was dried, filtered and evaported. The residue was purified by column chromatography over silica gel using a mixture of dichloromethane, methanol and ammonium hydroxide (90:10:0.1 by volume) as eluent. The pure fractions were collected and the eluent was evaporated. The residue was converted into the hydrochloride salt in ethanol. The salt was filtered off and crystallized from methanol. The product was filtered off and dried, yielding 2.3 parts (23.8%) of 2-amino-6-(1H-imidazol-1-ylmethyl)-4-quinazolinol dihydrochloride; mp. >300 °C (comp. 45-b).

Example 15-b

To a stirred mixture of 1.7 parts of ethyl [4-[(1H-imidazol-1-yl)phenylmethyl]-2-[(methylimino)methyl]-phenyl]carbamate and 31.6 parts of ethanol were added 1 part of sodium tetrahydroborate (portionwise) and 55.3 parts of methanol. After stirring for 7 hours at 40-50 °C, the reaction mixture was evaporated. The residue was taken up in water (to which 0.29 parts of acetic acid were added) and the whole was basified with $NH_4OH$. The product was extracted with dichloromethane and the extract was dried, filtered and evaporated. The residue was purified by column chromatography (silica gel ; $CHCl_3/CH_3OH$ 95:5). The eluent of the desired fraction was evaporated and the residue was crystallized from ethyl acetate, yielding 0.3 parts (20.1%) of 3,4-dihydro-6-[(1H-imidazol-1-yl)phenylmethyl]-3-methyl-2(1H)-quinazolinone; mp.

173.8 °C (comp. 23-b).

Example 16-b

A solution of 2.2 parts of N-(2-formyl-4-[(1H-imidazol-1-yl)phenylmethyl]phenyl]acetamide and 40 parts of methanol, saturated with ammonia was stirred for 1 hour at room temperature. After evaporation, dichloromethane was added. The solution was filtered and the filtrate was evaporated. The residue was purified by column chromatography over silica gel first using a mixture of dichloromethane and methanol (97:3 by volume) and then a mixture of dichloromethane and methanol (94:6 by volume) as eluents. The eluent of the desired fraction was evaporated and the residue was crystallized from 21 parts of 1,1'-oxybisethane. The product was filtered off and dried, yielding 0.3 parts (14.7%) of 6-[(1H-imidazol-1-yl)-phenylmethyl]-2-methylquinazoline; mp. 129.6 °C (comp. 32-b).

Example 17-b

To a stirred solution of 4.6 parts of 2-amino-4-[(1H-imidazol-1-yl)phenylmethyl]benzamide in 90 parts of tetrahydrofuran were added 3.52 parts of 1,1'-carbonylbis[1H-imidazole]. The mixture was stirred first overnight at room temperature and then for 48 hours at reflux temperature. The solution was concentrated under reduced pressure and the concentrate was treated with water. The product was extracted with dichloromethane. A white product was precipitated in the dichloromethane layer. This product was filtered off and crystallized from hot acetonitrile. The product was filtered off, washed with acetonitrile and 2,2'-oxybispropane and dried in vacuo at 60-70 °C, yielding 1.77 parts (35.4%) of 7-[(1H-imidazol-1-yl)-phenylmethyl]-2,4(1H,3H)-quinazolinedione; mp. 287.2 °C (comp. 35-b).

Example 18-b

A mixture of 4.2 parts of 2-amino-4-[(1H-imidazol-1-yl)phenylmethyl]benzamide, 97 parts of trimethoxymethane and 1.3 parts of formic acid was stirred for 5 hours at reflux temperature and overnight at room temperature. The solvent was evaporated and the residue was dissolved in methanol. The solution was basified with ammonious methanol and then evaporated. The residue was purified twice by column chromatography (silica gel ; $CHCl_3/CH_3OH$ 95:5 $CHCl_3/CH_3OH$ 90:10). The eluent of the desired fraction was evaporated and the residue was crystallized from acetonitrile. The product was filtered off, washed with 2,2'-oxybispropane and dried, yielding 1 part (23.6%) of 7-[(1H-imidazol-1-yl)phenylmethyl]-4(3H)-quinazolinone; mp. 205.0 °C (comp. 47-b).

Table 6-b

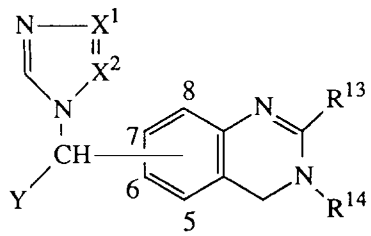

| Comp. No. | Ex. No. | $X^1=X^2$ | Y | p* | $R^{13}$ | $R^{14}$ |
|---|---|---|---|---|---|---|
| 1-b | - | CH=CH | $C_6H_5$- | 6 | H- | $CH_3$- |
| 2-b | - | CH=CH | $C_6H_5$- | 6 | H- | $C_6H_5$-$CH_2$- |
| 3-b | - | CH=CH | $C_6H_5$- | 6 | H- | $C_6H_5$- |
| 4-b | - | CH=CH | $C_6H_5$- | 6 | $CH_3$- | $CH_3$- |
| 5-b | - | CH=CH | $C_6H_5$- | 6 | $CH_3$- | $C_6H_5$-$CH_2$- |
| 6-b | - | CH=CH | $C_6H_5$- | 6 | $CH_3$- | $C_6H_5$- |
| 7-b | - | CH=CH | $C_6H_5$- | 6 | $C_6H_5$- | $CH_3$- |
| 8-b | - | CH=CH | $C_6H_5$- | 6 | $C_6H_5$- | $C_6H_5$-$CH_2$- |
| 9-b | - | CH=CH | $C_6H_5$- | 6 | $C_6H_5$- | $C_6H_5$- |
| 10-b | - | CH=CH | $C_6H_5$- | 6 | $CH_3$- | H- |
| 11-b | - | CH=C($CH_3$) | $C_6H_5$- | 6 | H- | $C_6H_5$-$CH_2$- |
| 12-b | - | C($CH_3$)=CH | $C_6H_5$- | 6 | H- | $C_6H_5$-$CH_2$- |

| Comp. No. | Ex. No. | $X^1=X^2$ | Y | p* | $R^{13}$ | $R^{14}$ |
|---|---|---|---|---|---|---|
| 13-b | - | CH=CH | $C_6H_5$- | 6 | $F_3C$- | H- |
| 14-b | - | CH=CH | $C_6H_5$- | 6 | $CH_3O$- | H- |
| 15-b | | CH=CH | $C_6H_5$- | 6 | Cl- | H- |

* In the previous and following tables p indicates the position of the 1H-azol-1-ylmethyl moiety on the quinazoline moiety.

Table 7-b

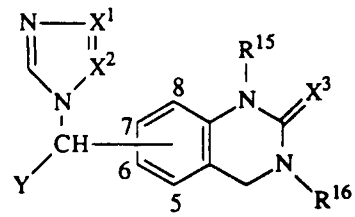

| Comp. No. | Ex. No. | $X^1=X^2$ | Y | p | $R^{15}$ | $R^{16}$ | $X^3$ | mp.(°C) / base / salt |
|---|---|---|---|---|---|---|---|---|
| 16-b | 11-b | CH=CH | $C_6H_5$- | 6 | H- | H- | 0 | 222.5 |
| 17-b | 11-b | CH=CH | 3-pyridinyl | 6 | H- | H- | 0 | 240.7 |
| 18-b | 11-b | CH=CH | i.$C_3H_7$- | 6 | H- | H- | 0 | >260 (dec.)/HCl |
| 19-b | 11-b | CH=N | $C_6H_5$- | 6 | H- | H- | S | 264.3 |
| 20-b | 11-b | CH=CH | $C_6H_5$- | 6 | H- | H- | S | 251.7 |
| 21-b | 11-b | CH=CH | $CH_3$- | 6 | H- | H- | 0 | 177.0 |
| 22-b | 12-b | CH=CH | 3-Cl-$C_6H_4$- | 6 | H- | H- | 0 | 209.3 |
| 23-b | 15-b | CH=CH | $C_6H_5$- | 6 | H- | $CH_3$- | O | 173.8 |
| 24-b | 12-b | CH=N | 3-Cl-$C_6H_4$- | 6 | H- | H- | 0 | 209.2 |
| 25-b | 13-b | CH=N | c.$C_3H_5$- | 6 | H- | H- | 0 | 184.4 |
| 26-b | - | CH=CH | 3,4$Cl_2$-$C_6H_3$- | 6 | H- | H- | 0 | |
| 27-b | - | CH=CH | c.$C_3H_5$- | 6 | H- | H- | 0 | |
| 28-b | - | CH=CH | 2-thienyl | 6 | H- | H- | 0 | |
| 29-b | - | CH=CH | imidazolyl | 6 | H- | H- | 0 | |
| 30-b | - | CH=CH | $CH_3O$-$C_6H_4$- | 6 | H- | H- | 0 | |
| 31-b | - | CH=CH | $C_6H_5$- | 6 | $C_6H_5$-$CH_2$- | $C_3H_7$- | 0 | |

Table 8-b

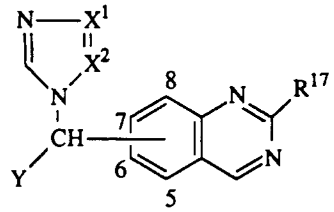

| Comp. No. | Ex. No. | $X^1=X^2$ | Y | p | $R^{17}$ | mp.(°C) / base / salt |
|---|---|---|---|---|---|---|
| 32-b | 16-b | CH=CH | $C_6H_5$- | 6 | $CH_3$- | 129.6 |
| 33-b | - | CH=CH | $C_6H_5$- | 6 | H- | |

Table 9-b

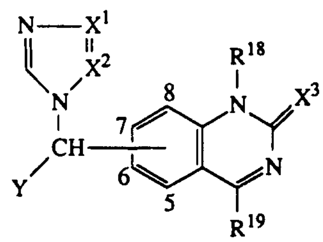

| Comp. No. | Ex. No. | $X^1=X^2$ | Y | p | $R^{18}$ | $R^{19}$ | $X^3$ | mp.(°C) / base / salt |
|---|---|---|---|---|---|---|---|---|
| 34-b | - | CH=CH | $C_6H_5$- | 6 | H- | $CH_3$- | 0 | |

Table 10-b

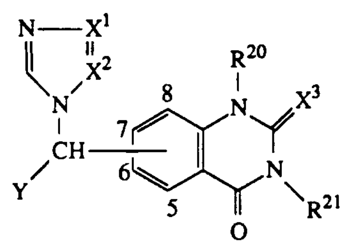

| Comp. No. | Ex. No | $X^1=X^2$ | Y | p | $R^{20}$ | $R^{21}$ | $X^3$ | mp.(°C) / base / salt |
|---|---|---|---|---|---|---|---|---|
| 35-b | 17-b | CH=CH | $C_6H_5$- | 7 | H- | H- | 0 | 287.2 |
| 36-b | 12-b | N=CH | $C_6H_5$- | 6 | H- | $C_6H_5$-$CH_2$- | 0 | 219.8 |
| 37-b | 12-b | CH=CH | $C_6H_5$- | 6 | H- | $C_6H_5$-$CH_2$- | S | 257.3 |

| Comp. No. | Ex. No | $X^1=X^2$ | Y | p | $R^{20}$ | $R^{21}$ | $X^3$ | mp.(°C) / base / salt |
|---|---|---|---|---|---|---|---|---|
| 38-b | 12-b | CH=CH | $C_6H_5$- | 6 | H- | $C_6H_5$-$CH_2$- | 0 | 263.5 |
| 39-b | 12-b | CH=N | $C_6H_5$- | 6 | H- | $C_6H_5$-$CH_2$- | 0 | 204.4 |
| 40-b | 12-b | CH=N | $C_6H_5$- | 6 | H- | H- | 0 | 280.9 |
| 41-b | 12-b | N=CH | $C_6H_5$- | 6 | H- | H- | 0 | 248.0 |
| 42-b | 12-b | CH=CH | $C_6H_5$- | 6 | H- | $CH_3$- | 0 | >300 (dec.) |
| 43-b | 12-b | CH=CH | $C_6H_5$- | 6 | H- | H- | 0 | >300 (dec.) |
| 44-b | - | N=CH | $C_6H_5$- | 6 | H- | H- | 0 | |

Table 11-b

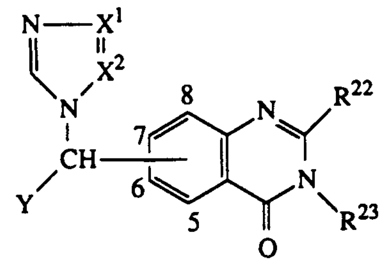

| Comp. No. | Ex. No. | $X^1=X^2$ | Y | p | $R^{22}$ | $R^{23}$ | mp.(°C) / base / salt |
|---|---|---|---|---|---|---|---|
| 45-b | 14-b | CH=CH | H- | 6 | $NH_2$- | H- | >300/2HCl |
| 46-b | 11-b | CH=CH | $C_6H_5$- | 6 | H- | H- | 208.1 |
| 47-b | 18-b | CH=CH | $C_6H_5$- | 7 | H- | H- | 205.0 |

C-b) Pharmacological Examples.

The useful pharmacological properties of the compounds of the present invention can for example be demonstrated by the following experiment.

Example 19-b

Metabolism of exogenous all-trans-retinoic acid

Male Wistar rats weighing 200~210 g were orally treated with vehicle (PEG 200) or with 40 mg/kg of a compound of formula (I-b). One hour later, the animals were anesthetized with ether and injected intrajugularly with 0.50 ml saline solution containing 20 µg of all-trans-retinoic acid. Two hours after this injection, rats were killed by decapitation and blood was collected on heparin. Blood samples were centrifuged (1000 g, 15 min) and plasma was recovered to determine the quantity of plasmatic all-trans-retinoic acid. The samples were analyzed by means of HPLC with UV-detection at 350 nm. Quatification was achieved by peak area integration and external standardization. Under the conditions used, plasma concentrations of the retinoic acid in vehicle-pretreated animals were not detectable (<0.5 ng/ml), whereas

compound nos. 16-b, 18-b, 19-b, 22-b, 24-b, 42-b and 46-b enhanced the recovery of all-trans-retinoic acid from the plasma to a least 10 ng/ml after dosing with 40 mg/kg.

Example 20-b

Metabolism of endogenous all-trans-retinoic acid

Male Wistar rats weighing 200~210 g were orally treated with vehicle (PEG 200) or with 40 mg/kg of a compound of formula (I-b). Two hours after drug administration, the rats were killed by decapitation and blood was collected on heparin. Blood samples were centrifuged (1000 g, 15 min) and plasma was recovered to determine the quantity of plasmatic all-trans-retinoic acid. The samples were analyzed by means of HPLC with UV-detection at 350 nm. Quatification was achieved by peak area integration and external standardization. Under the conditions used, plasma concentrations of the retinoic acid in vehicle-pretreated animals were not detectable (<0.5 ng/ml), whereas compound nos. 18-b, 19-b, 20-b, 24-b, 38-b, 42-b, 43-b and 46-b enhanced the recovery of all-trans-retinoic acid from the plasma to a least 1 ng/ml.

A-c) Preparation of the intermediates in the synthesis of quinoxaline derivatives of formula (I-c)

Example 1-c

A mixture of 10 parts of 5-methylquinoxaline, 10 parts of 1,3-dibromo-5,5-dimethylimidazolidine-2,4-dione, 1.7 parts of benzenecarboperoxoic acid and 318 parts of tetrachloromethane was stirred for 16 hours at reflux temperature under 2 lamps of 250 Watt. The reaction mixture was cooled and the organic layer was decanted. The product was filtered off and dried, yielding 15.5 parts (100%) of 5-(bromomethyl)-quinoxaline (interm. 1-c).

Example 2-c

α) To a stirred solution of 30 parts of (3,4-diaminophenyl) phenylmethanone in 240 parts of methanol were added 30 parts of an ethanedial solution in water 40%. The reaction mixture was stirred for 3 hours at reflux temperature. After cooling to room temperature, the precipitated product was filtered off, washed with methanol and dried, yielding 20 parts (59.3%) of phenyl (6-quinoxalinyl)methanone; mp. 120 °C (interm. 2-c).

β) To a stirred and cooled (5 °C) solution of 20 parts of intermediate 2-c, namely phenyl (6-quinoxalinyl)-methanone in 160 parts of methanol were added portionwise 3.2 parts of sodium tetrahydroborate. Upon completion, the reaction mixture was poured into water and the product was extracted with dichloromethane. The extract was washed with water, dried, filtered and evaporated to dry, yielding 20 parts (100%) of α-phenyl-6-quinoxalinemethanol as an oily residue (interm. 3-c).

γ) To a stirred and cooled (0 °C) mixture of 12 parts of intermediate 3-c, namely α-phenyl-6-quinoxalinemethanol, 213 parts of dichloromethane and 15.4 parts of N,N-diethylethanamine was added a solution of 8.8 parts of methanesulfonyl chloride in 26.6 parts of dichloromethane under nitrogen atmosphere. After stirring overnight at room temperature, the reaction mixture was evaporated, yielding 54 parts (100%) of α-phenyl-6-quinoxalinemethanol methanesulfonate (ester) as an oily residue (interm. 4-c).

Example 3-c

α) To a stirred mixture of 6.9 parts of 3,4-diaminobenzenemethanol, 1 part of N,N-diethylethanamine and 75 parts of water were added 2.9 parts of a solution of ethanedial in water 40% at about 55 °C. The whole was stirred for 1 hour at 55-60 °C. The solvent was evaporated and the residue was purified by column chromatography over silica gel using a mixture of trichloromethane and methanol (95:5 by volume) as eluent. The pure fractions were collected and the eluent was evaporated. The residue was converted into the hydrochloride salt in 2-propanol and ethanol. The product was filtered off and dried, yielding 6.5 parts (66.1%) of 6-quinoxalinemethanol monohydrochloride; mp. >300 °C (interm. 5-c).

β) To a stirred solution of 10 parts of intermediate 5-c, namely 6-quinoxalinemethanol in 133 parts of dichloromethane were added 20 parts of manganese(IV) oxide. After stirring for 3 hours at room temperature, the reaction mixture was filtered and the filtrate was evaporated, yielding 6.6 parts (67.3%) of 6-quinoxalinecarboxaldehyde; mp. 134 °C (interm. 6-c).

Example 4-c

α) To a stirred and refluxed Grignard complex previously prepared starting from 55.1 parts of 1-bromopropane, 10.9 parts of magnesium and tetrahydrofuran was added a solution of 25 parts of N-(4-formylphenyl)acetamide in 225 parts of dry tetrahydrofuran. After stirring for 1 hour at room temperature, the reaction mixture was poured into ice water and a saturated ammonium chloride solution. The organic layer was decanted (and set aside) and the remaining phase was extracted with ethyl acetate. The combined organic layers were dried, filtered and evaporated. The residue was purified by column chromatography over silica gel using a mixture of trichloromethane and methanol (98:2 by volume) as eluent. The pure fractions were collected and the eluent was evaporated, yielding 20 parts (64.3%) of N-[4-(1-hydroxybutyl)phenyl]acetamide as a residue (interm. 7-c).

β) A mixture of 10 parts of intermediate 7-c, namely N-[4-(1-hydroxybutyl)phenyl]acetamide, 16.2 parts of 1,1'-carbonylbis[1H-imidazole] and 135 parts of tetrahydrofuran was stirred for 17 hours at room temperature. The reaction mixture was evaporated and the residue was taken up in trichloromethane. The organic phase was washed with a potassium carbonate solution 10% in water, dried, filtered and evaporated. The residue was purified by column chromatography (HPLC) over silica gel using a mixture of trichloromethane and methanol (95:5 by volume) as eluent. The pure fractions were collected and the eluent was evaporated, yielding 5.8 parts (45.0%) of N-[4-[1-(1H-imidazol-1-yl)butyl]phenyl]acetamide; mp. 186 ° C (interm. 8-c);

γ) To a stirred and cooled (0 ° C) mixture of 2.57 parts of intermediate 8-c, namely N-[4-[1-(1H-imidazol-1-yl)butyl]phenyl]acetamide and 23.0 parts of concentrated sulfuric acid were added portionwise 1.01 parts of potassium nitrate. Upon complete addition, stirring was continued for 30 minutes at 0 ° C. The reaction mixture was poured into crushed ice and treated with ammonium hydroxide to pH 10. The product was extracted with trichloromethane. The extract was dried, filtered and evaporated, yielding 3 parts (99.4%) of N-[4-[1-(1H-imidazol-1-yl)butyl]-2-nitrophenyl]acetamide as a residue (interm. 9-c).

δ) A mixture of 11.5 parts of intermediate 9-c, namely N-[4-[1-(1H-imidazol-1-yl)butyl]-2-nitrophenyl]-acetamide and 150 parts of a hydrochloric acid solution 3 N was stirred for 3 hours at reflux temperature. The reaction mixture was poured into crushed ice and the whole was neutralized with concentrated ammonium hydroxide. The product was extracted with dichloromethane. The extract was dried, filtered and evaporated, yielding 8.8 parts (88.9%) of 4-[1-(1H-imidazol-1-yl)butyl]-2-nitrobenzenamine as a residue (interm. 10-c).

In a similar manner there were also prepared :
4-[1-(1H-imidazol-1-yl)propyl]-2-nitrobenzenamine as a residue (interm. 11-c);
4-[1-(1H-imidazol-1-yl)-3-methylbutyl]-2-nitrobenzenamine as a residue (interm. 12-c); and
4-[1-(1H-imidazol-1-yl)-2-methylpropyl]-2-nitrobenzenamine (interm. 13-c).

Example 5-c

α) To a stirred and cooled mixture (ice bath, 0 ° C) of 30 parts of 1-(4-amino-3-nitrophenyl)-2-methyl-1-propanone and 390 parts of dichloromethane were added dropwise 33 parts of acetyl chloride. Upon complete addition, the reaction mixture was stirred for 12 hours at room temperature. The whole was poured into water and after the addition of sodium carbonate, stirring was continued for 15 minutes. The separated organic layer was dried, filtered and evaporated, yielding 36 parts (100%) of N-[4-(2-methyl-1-oxopropyl)-2-nitrophenyl]acetamide (interm. 14-c).

β) To a stirred and cooled (ice water, 10 ° C) solution of 30 parts of intermediate 14-c, namely N-[4-(2-methyl-1-oxopropyl)-2-nitrophenyl]acetamide in 240 parts of methanol were added portionwise 4.5 parts of sodium tetrahydroborate. Upon completion, stirring was continued for 1 hour. The reaction mixture was evaporated and the residue was extracted with dichloromethane (3 x 104 parts). The combined extracts were washed with water, dried, filtered and evaporated, yielding 32 parts (100%) of N-[4-(1-hydroxy-2-methylpropyl)-2-nitrophenyl]acetamide (interm. 15-c).

χ) To a stirred solution of 36 parts of intermediate 15-c, namely N-[4-(1-hydroxy-2-methylpropyl)-2-nitrophenyl]acetamideand 390 parts of dichloromethane were added 35.0 parts of N,N-diethylethanamine. After cooling to 0 ° C, 20.0 parts of methanesulfonyl chloride were added dropwise to the previous mixture. Upon completion, stirring was continued for 12 hours at room temperature. The whole was poured into water and sodium carbonate was added while stirring. The product was extracted with dichloromethane. The extract was dried, filtered and evaporated, yielding 39 parts (100%) of N-[4-(1-chloro-2-methylpropyl)-2-nitrophenyl]acetamide (interm. 16-c).

δ) A mixture of 40 parts of intermediate 16-c, namely N-[4-(1-chloro-2-methylpropyl)-2-nitrophenyl]-acetamide,51 parts of 1H-1,2-4-triazole, 50 parts of potassium carbonate and 400 parts of acetonitrile was stirred for 2 hours at reflux temperature. After cooling, the whole was evaporated to dry and the residue was taken up in 300 parts of water. The product was extracted with dichloromethane. The extract was dried, filtered and evaporated. The residue was purified by column chromatograhpy over silica gel using a mixture of dichloromethane and methanol (98:2 by volume) as eluent. The pure fractions were collected and the eluent was evaporated, yielding 22 parts (49.0%) of N-[4-[2-methyl-1-(1H-1,2,4-triazol-1-yl)propyl]-2-nitrophenyl]acetamide as an oil (interm. 17-c).

ε) A mixture of 20 parts of intermediate 17-c, namely N-[4-[2-methyl-1-(1H-1,2,4-triazol-1-yl)propyl]-2-nitrophenyl]acetamide and 200 parts of a hydrochloric acid solution 2 N was stirred for 12 hours at room temperature. The reaction mixture was poured into 500 parts of water and the whole was neutralized with a concentrated potassium carbonate solution. The product was extracted with dichloromethane (3 x 130 parts). The combined extracts were dried, filtered and evaporated to dry. The residue was crystallized from 2,2'-oxybispropane, yielding 18.5 parts (97.7%) of 4-[2-methyl-1-(1H-1,2,4-triazol-1-yl)propyl]-2-nitrobenzenamine; mp. 206 °C (interm. 18-c).

In a similar manner there were also prepared :

Table 1-c

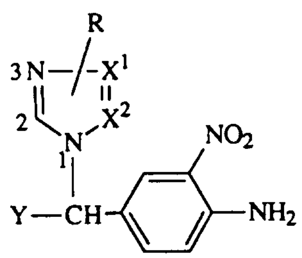

| Int. No. | $-X^1=X^2-$ | R | Y |
|---|---|---|---|
| 19-c | -CH=N- | H | $-C_6H_5$ |
| 20-c | -CH=CH- | $2-CH_3$ | $i-C_3H_7$ |
| 21-c | -CH=N- | H | $C_2H_5$ |
| 22-c | -CH=N- | H | $CH_2-CH(CH_3)_2$ |
| 23-c | -N=CH- | H | $i-C_3H_7$ |
| 24-c | -CH=N- | H | $C_4H_9$ |
| 25-c | -CH=N- | H | $C_3H_7$ |

Example 6-c

α) 1.61 Parts of sodium tetrahydroborate were added dropwise to a stirred solution of 11 parts of (4-amino-3-nitrophenyl) (2-fluorophenyl)methanone in 120 parts of methanol. Upon complete addition, stirring was continued for 1 hour at room temperature. The reaction mixture was poured into water and the product was extracted three times with 75 parts of trichloromethane. The combined extracts were dried, filtered and evaporated, yielding 11.3 parts (100%) of 4-amino-α-(2-fluorophenyl)-3-nitrobenzenemethanol as a residue (interm. 26-c).

β) A mixture of 11.1 parts of intermediate 26-c, namely 4-amino-α-(2-fluorophenyl)-3-nitrobenzenemethanol, 13.7 parts of 1,1'-carbonylbis[1H-imidazole] and 90 parts of N,N-dimethylformamide was stirred for 12 hours at room temperature. After evaporation to dry, the residue was taken up in dichloromethane. The organic phase was washed with 50 parts of water, dried, filtered and evaporated. The residue was purified by column chromatography over silica gel using a mixture of dichloromethane

and methanol (95:5 by volume) as eluent. The pure fractions were collected and the eluent was evaporated, yielding 6.5 parts (49.5%) of 4-[(2-fluorophenyl)(1H-imidazol-1-yl)methyl]-2-nitrobenzenamine; mp. 176 °C (interm. 27-c).

In a similar manner there were also prepared:

Table 2-c

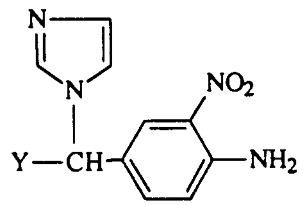

| Int. No. | Y | physical data |
|---|---|---|
| 28-c | 3-pyridinyl | 164.1°C |
| 29-c | phenyl | - |
| 30-c | 2-thienyl | - |
| 31-c | 4-fluorophenyl | 147.4°C |
| 32-c | 3-chlorophenyl | - |
| 33-c | 3,4-dichlorophenyl | 152°C |
| 34-c | cyclopropyl | - |
| 35-c | 4-methoxyphenyl | - |
| 36-c | butyl | - |
| 37-c | 3-fluorophenyl | - |
| 38-c | 2-chlorophenyl | - |
| 39-c | 4-methylphenyl | - |
| 40-c | 3-$CF_3$-phenyl | - |
| 41-c | 4-chlorophenyl | - |
| 42-c | cyclohexyl | - |
| 43-c | cyclopentyl | - |
| 44-c | 4-[$CH(CH_3)_2$]-phenyl | - |

Example 7-c

α) To a stirred solution of 30 parts of 1-(4-chloro-3-nitrophenyl)-2-methyl-1-propanone in 240 parts of methanol was added a solution of 20 parts of methanamine in 160 parts of methanol. After stirring for 12 hours at 60 °C, the reaction mixture was evaporated to dry, yielding 30 parts (100%) of 2-methyl-1-[4-(methylamino)-3-nitrophenyl]-1-propanone as a residue (interm. 45-c).

β) To a stirred solution of 30 parts of intermediate 45-c, namely 2-methyl-1-[4-(methylamino)-3-nitrophenyl]-1-propanonein 320 parts of methanol were added dropwise 15 parts of sodium tetrahydroborate (the temperature was kept at 20 °C). Upon complete addition, stirring was continued for 30 minutes at room temperature. The reaction mixture was poured into water and the product was extracted with trichloromethane. The extract was dried, filtered and evaporated to dry, yielding 30 parts

(100%) of 4-(methylamino)-α-(1-methylethyl)-3-nitrobenzenemethanol as an oily residue (interm. 46-c).

γ) To a stirred solution of 30 parts of intermediate 46-c, namely 4-(methylamino)-α-(1-methylethyl)-3-nitrobenzenemethanol in 270 parts of dry tetrahydrofuran were added 43.4 parts of 1,1'-carbonylbis[1H-imidazole]. After stirring for 24 hours at room temperature, the reaction mixture was evaporated to dry. The residue was taken up in trichloromethane and a potassium carbonate solution 10%. The separated organic layer was dried, filtered and evaporated to dry. The residue was purified by column chromatography over silica gel using a mixture of trichloromethane and methanol (99:1 by volume) as eluent. The pure fractions were collected and the eluent was evaporated, yielding 15 parts (40.9%) of 4-[1-(1H-imidazol-1-yl)-2-methylpropyl]-N-methyl-2-nitrobenzenamine as a residue (interm. 47-c).

Example 8-c

To a stirred and cooled (0 °C) solution of 7 parts of 4-[1-(1H-imidazol-1-yl)propyl]-2-nitrobenzenamine in 126 parts of 1,2-dichloroethane were added 9.6 parts of 2-methylbenzeneacetyl chloride. After stirring for 12 hours at room temperature, the reaction mixture was poured into ice water and the product was extracted with dichloromethane. The extract was dried, filtered and evaporated in vacuo. The oily residue was purified by column chromatography over silica gel using a mixture of dichloromethane and methanol (98:2 by volume) as eluent. The eluent of the desired fraction was evaporated, yielding 9.6 parts (89.3%) of N-[4-[1-(1H-imidazol-1-yl)propyl]-2-nitrophenyl]-2-methylbenzeneacetamide; mp. 122 °C (interm. 48-c).

In a similar manner there were also prepared:

Table 3-c

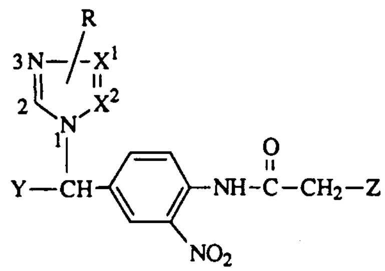

| Int. No. | $-X^1=X^2-$ | R | Y | Z | mp. |
|---|---|---|---|---|---|
| 49-c | -CH=N- | H | $C_6H_5$ | $C_6H_5$ | - |
| 50-c | -CH=CH- | H | i-$C_3H_7$ | 2-F-$C_6H_4$ | - |
| 51-c | -CH=CH- | H | i-$C_3H_7$ | 4-$CH_3$-$C_6H_4$ | 114°C |
| 52-c | -CH=CH- | 2-$CH_3$ | i-$C_3H_7$ | $C_6H_5$ | - |
| 53-c | -CH=CH- | H | i-$C_3H_7$ | 4-Br-$C_6H_4$ | - |
| 54-c | -CH=CH- | H | i-$C_3H_7$ | 3,4-$F_2$-$C_6H_3$ | - |
| 55-c | -CH=CH- | H | i-$C_3H_7$ | 4-$OCH_3$-$C_6H_4$ | - |
| 56-c | -CH=CH- | H | 2,4-$Cl_2$-$C_6H_3$ | $C_6H_5$ | - |
| 57-c | -CH=CH- | H | i-$C_3H_7$ | 3,4-$(OCH_3)_2$-$C_6H_3$ | - |
| 58-c | -CH=CH- | H | i-$C_3H_7$ | 2,4-$Cl_2$-$C_6H_3$ | - |
| 59-c | -CH=CH- | H | i-$C_3H_7$ | 2-naphthalenyl | 144°C |
| 60-c | -CH=CH- | H | i-$C_3H_7$ | 3,4,5-$(OCH_3)_3$-$C_6H_2$ | - |
| 61-c | -CH=CH- | H | i-$C_3H_7$ | 2-thienyl | - |
| 62-c | -CH=CH- | H | i-$C_3H_7$ | 2-$OCH_3$-$C_6H_4$ | - |
| 63-c | -CH=CH- | H | i-$C_3H_7$ | 1-naphthalenyl | - |
| 64-c | -CH=CH- | H | i-$C_3H_7$ | 2-Cl-$C_6H_4$ | - |
| 65-c | -CH=CH- | H | i-$C_3H_7$ | 3-OH-$C_6H_4$ | - |
| 66-c | -CH=CH- | H | i-$C_3H_7$ | 3-Br-$C_6H_4$ | - |
| 67-c | -CH=CH- | H | i-$C_3H_7$ | 3-thienyl | - |
| 68-c | -CH=N- | H | i-$C_3H_7$ | 2-thienyl | - |
| 69-c | -CH=CH- | H | i-$C_3H_7$ | 2Cl,6F-$C_6H_3$ | - |
| 70-c | -CH=CH- | H | i-$C_3H_7$ | 3Br,4OH-$C_6H_3$ | - |
| 71-c | -CH=N- | H | $C_2H_5$ | $C_6H_5$ | - |
| 72-c | -CH=N- | H | $CH_2$-$CH(CH_3)_2$ | $C_6H_5$ | - |
| 73-c | -N=CH- | H | i-$C_3H_7$ | 4-F-$C_6H_4$ | - |
| 74-c | -CH=N- | H | $C_4H_9$ | 3-F-$C_6H_4$ | - |
| 75-c | -CH=CH- | H | i-$C_3H_7$ | 3Cl,4OH-$C_6H_3$ | - |

| Int. No. | $-X^1=X^2-$ | R | Y | Z | mp. |
|---|---|---|---|---|---|
| 76-c | -N=CH- | H | $i\text{-}C_3H_7$ | $C_6H_5$ | - |
| 77-c | -CH=N- | H | $C_3H_7$ | $2\text{-}CH_3\text{-}C_6H_4$ | - |
| 78-c | -N=CH- | H | $i\text{-}C_3H_7$ | $3\text{-}Cl\text{-}C_6H_4$ | - |
| 79-c | -CH=N- | H | $CH_2\text{-}CH(CH_3)_2$ | $2\text{-}CH_3\text{-}C_6H_4$ | - |
| 80-c | -CH=N- | H | $C_2H_5$ | $2\text{-}CH_3\text{-}C_6H_4$ | - |
| 81-c | -N=CH- | H | $i\text{-}C_3H_7$ | $3\text{-}F\text{-}C_6H_4$ | - |
| 82-c | -CH=N- | H | $i\text{-}C_3H_7$ | $2\text{-}F\text{-}C_6H_4$ | - |
| 83-c | -CH=N- | H | $C_4H_9$ | $2\text{-}CH_3\text{-}C_6H_4$ | - |
| 84-c | -CH=N- | H | $C_3H_7$ | $3\text{-}F\text{-}C_6H_4$ | - |

Example 9-c

To a stirred and cooled (0 ° C) mixture of 8 parts of 4-(1H-imidazol-1-yl)methyl]-2-nitrobenzenamine and 106 parts of dichloromethane were added 3.4 ml of 4-methylene-2-oxetanone. After stirring for 1 hour at 0 ° C, another portion of 3.4 ml of 4-methylene-2-oxetanone was added and stirring was continued for 1 hour at this low temperature. The reaction mixture was diluted with 8 parts of methanol and evaporated to dry. The residue was crystallized from 2-propanone, yielding 7.6 parts (68.7%) of N-[4-(1H-imidazol-1-yl)methyl]-2-nitrophenyl]-3-oxobutanamide;mp. 172 ° C (interm. 85-c).
In a similar manner there were also prepared :

Table 4-c

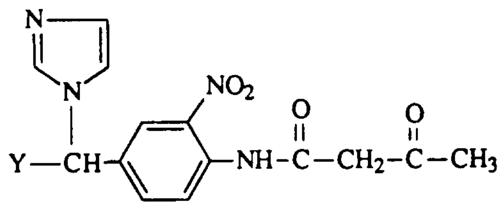

| Int. No. | Y | mp. |
|---|---|---|
| 86-c | $i\text{-}C_3H_7$ | 85°C |
| 87-c | $3\text{-}Cl\text{-}C_6H_4$ | - |
| 88-c | $c\text{-}C_6H_{11}$ | - |
| 89-c | $4\text{-}Cl\text{-}C_6H_4$ | - |
| 90-c | $c\text{-}C_5H_9$ | - |

| Int. No. | Y | mp. |
|---|---|---|
| 91-c | $CH_3$ | 134°C |
| 92-c | $C_2H_5$ | - |
| 93-c | $4\text{-F-}C_6H_4$ | - |
| 94-c | $2\text{-F-}C_6H_4$ | - |
| 95-c | $3\text{-F-}C_6H_4$ | - |

Example 10-c

A solution of 10 parts of 4-[1-(1H-imidazol-1-yl)-2-methylpropyl]-2-nitrobenzenamine, 20 parts of ethyl β-oxobenzenepropanoate and 174 parts of benzene was stirred for 36 hours at reflux temperature. After cooling, the reaction mixture was evaporated. The residue was purified by column chromatograhpy over silica gel using a mixture of dichloromethane and methanol (98:2 by volume) as eluent. The pure fractions were collected and the eluent was evaporated, yielding 7 parts (44.8%) of N-[4-[1-(1H-imidazol-1-yl)-2-methylpropyl]-2-nitrophenyl]-β-oxobenzenepropanamide; mp. 106 °C (interm. 96-c).
In a similar manner there were also prepared :
N-[4-[1-(1H-imidazol-1-yl)ethyl]-2-nitrophenyl]-β-oxobenzenepropanamide; mp. 172 °C (interm. 97-c);
N-[4-(1H-imidazol-1-ylmethyl)-2-nitrophenyl]-β-oxobenzenepropanamide; mp. 98 °C (interm. 98-c); and
N-[4-[(1H-imidazol-1-yl)phenylmethyl]-2-nitrophenyl]-β-oxobenzenepropanamide (interm. 99-c).

Example 11-c

To a stirred solution of 13 parts of 4-[1-(1H-imidazol-1-yl)-2-methylpropyl]-2-nitrobenzenamine in 195 parts of dichloromethane was added a solution of 19 parts of 3-chlorobenzeneacetyl chloride in 65 parts of dichloromethane. After stirring for 4 hours at room temperature, 10.1 parts of N,N-diethylethanamine were added. The reaction mixture was washed with water, dried, filtered and evaporated, yielding 39 parts (100%) of 3-chloro-N-[4-[1-(1H-imidazol-1-yl)-2-methylpropyl]-2-nitrophenyl]benzeneacetamide as an oily residue (interm.100-c).
In a similar manner there were also prepared :

Table 5-c

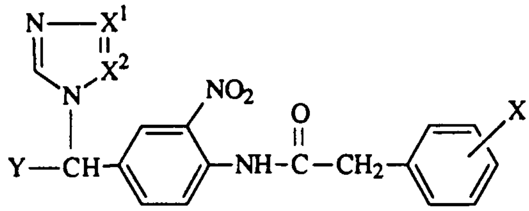

| Int. No. | $-X^1=X^2-$ | Y | X |
|---|---|---|---|
| 101-c | -CH=CH- | $i-C_3H_7$ | 4-Cl |
| 102-c | -CH=CH- | $i-C_3H_7$ | 4-F |
| 103-c | -CH=N- | $i-C_3H_7$ | H |
| 104-c | -CH=CH- | $C_4H_9$ | H |
| 105-c | -CH=CH- | $i-C_3H_7$ | 3-F |
| 106-c | -CH=CH- | $4-Cl-C_6H_4$ | H |
| 107-c | -CH=CH- | $3-Cl-C_6H_4$ | H |
| 108-c | -CH=CH- | $i-C_3H_7$ | $2-CH_3$ |
| 109-c | -CH=CH- | $i-C_3H_7$ | $3-OCH_3$ |
| 110-c | -CH=CH- | $i-C_3H_7$ | $3-CH_3$ |
| 111-c | -CH=CH- | $i-C_3H_7$ | $4-OC_2H_5$ |

Example 12-c

To a stirred and cooled (5 °C) solution of 10 parts of 4-[2-methyl-1-(1H-1,2,4-triazol-1-yl)propyl]-2-nitrobenzenamine and 6.7 parts of pyridine in 195 parts of dichloromethane was added a solution of 14.4 parts of 4-chlorobenzeneacetyl chloride in 39 parts of dichloromethane under nitrogen atmosphere. After stirring overnight at room temperature, the reaction mixture was washed with water, dried, filtered and evaporated. The residue was purified by column chromatography over silica gel using a mixture of dichloromethane and methanol (99:1 by volume) as eluent. The eluent of the desired fraction was evaporated, yielding 11 parts (69.9%) of 4-chloro-N-[4-[2-methyl-1-(1H-1,2,4-triazol-1-yl)propyl]-2-nitrophenyl]benzeneacetamide (interm. 112-c).
In a similar manner there were also prepared :
4-fluoro-N-[4-[2-methyl-1-(1H-1,2,4-triazol-1-yl)propyl]-2-nitrophenyl]benzeneacetamide (interm. 113-c);
3-fluoro-N-[4-[2-methyl-1-(1H-1,2,4-triazol-1-yl)propyl]-2-nitrophenyl]benzeneacetamide (interm. 114-c);
N-[4-[2-methyl-1-(1H-1,2,4-triazol-1-yl)propyl]-2-nitrophenyl]-3-thiopheneacetamide (interm. 115-c); and
3-chloro-N-[4-[2-methyl-1-(1H-1,2,4-triazol-1-yl)propyl]-2-nitrophenyl]benzeneacetamide (interm. 116-c).

Example 13-c

A mixture of 31 parts of 4-[1-(1H-imidazol-1-yl)-2-methylpropyl]-2-nitrobenzenamine and 240 parts of ethanol was hydrogenated at $0.5.10^5$ Pa in a Parr apparatus and at room temperature with 30 parts of Raney nickel catalyst. After the calculated amount of hydrogen was taken up, the catalyst was filtered off over diatomaceous earth and the filtrate was evaporated, yielding 27.4 parts (100%) of 4-[1-(1H-imidazol-1-yl)-2-methylpropyl]-1,2-benzenediamine as a residue (interm. 117-c).
In a similar manner there were also prepared :

Table 6-c 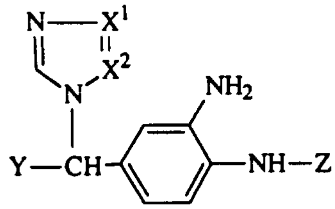

| Int. No. | $-X^1=X^2-$ | Z | Y |
|---|---|---|---|
| 118-c | -CH=CH- | H | $C_6H_5$ |
| 119-c | -CH=CH- | H | 3-pyridinyl |
| 120-c | -CH=CH- | H | 2-thienyl |
| 121-c | -CH=CH- | H | 4-F-$C_6H_4$ |
| 122-c | -CH=CH- | H | 3-Cl-$C_6H_4$ |
| 123-c | -CH=CH- | H | c-$C_3H_5$ |
| 124-c | -CH=CH- | H | $CH_3$ |
| 125-c | -CH=CH- | H | $C_4H_9$ |
| 126-c | -CH=CH- | H | $C_2H_5$ |
| 127-c | -CH=CH- | H | $CH_2$-$CH(CH_3)_2$ |
| 128-c | -CH=CH- | $CH_3$ | i-$C_3H_7$ |
| 129-c | -CH=CH- | H | $C_3H_7$ |
| 130-c | -CH=CH- | H | 3-F-$C_6H_4$ |
| 131-c | -CH=CH- | H | 2-Cl-$C_6H_4$ |
| 132-c | -CH=CH- | H | 2-F-$C_6H_4$ |
| 132-c | -CH=CH- | H | 4-$CH_3$-$C_6H_4$ |
| 133-c | -CH=CH- | H | 3-$CF_3$-$C_6H_4$ |

| Int. No. | $-X^1=X^2-$ | Z | Y |
|---|---|---|---|
| 134-c | -CH=CH- | H | 4-Cl-$C_6H_4$ |
| 135-c | -CH=N- | H | i-$C_3H_7$ |
| 136-c | -CH=CH- | H | c-$C_6H_{11}$ |
| 137-c | -CH=CH- | H | c-$C_5H_9$ |
| 138-c | -CH=CH- | H | 4-(i-$C_3H_7$)-$C_6H_4$ |

B-c) Preparation of the final quinoxaline compounds of formula (I-c)

Example 14-c

A mixture of 15.5 parts of 5-(bromomethyl)quinoxaline, 23.5 parts of 1H-imidazole and 160 parts of acetonitrile was stirred for 1.5 hours at reflux temperature. The reaction mixture was evaporated and the residue was taken up in water. The product was extracted three times with 65 parts of dichloromethane. The combined extracts were dried, filtered and evaporated. The residue was purified by column chromatography over silica gel using a mixture of dichloromethane and methanol (95:5 by volume) as eluent. The pure fractions were collected and the eluent was evaporated. The residue was crystallized from a mixture of 2-propanone and 2,2'-oxybispropane. The product was filtered off and dried, yielding 3 parts (20.5%) of 5-(1H-imidazol-1-ylmethyl)quinoxaline; mp. 121.2 °C (comp. 41-c).

Example 15-c

A mixture of 10.4 parts of α-phenyl-6-quinoxalinemethanol methanesulfonate(ester), 12 parts of 1H-1,2,4-triazole and 79 parts of acetonitrile was stirred overnight at reflux temperature. The reaction mixture was evaporated and the residue was extracted with ethyl acetate. The extract was dried, filtered and evaporated. The residue was purified by column chromatography (silica gel ; $CH_2Cl_2/CH_3OH$ 96:4). The eluent of the desired fraction was evaporated and the residue was crystallized from a mixture of 2-propanol and 2,2'-oxybispropane, yielding 0.9 parts (9.5%) of 6-[phenyl(4H-1,2,4-triazol-4-yl)methyl]quinoxaline; mp. 98.1 °C (comp. 50-c).

Example 16-c

A mixture of 7.8 parts of 6-quinoxalinecarboxaldehyde and 24.3 parts of 1,1'-carbonylbis-1H-imidazole was stirred for 1 hour at 100 °C. After cooling, the reaction mixture was partitioned between water and ethyl acetate. The organic layer was dried, filtered and evaporated. The residue was purified by column chromatography (silica gel ; $CH_2Cl_2/CH_3OH/NH_4OH$ 85:15:1). The eluent of the desired fraction was evaporated and the residue was converted into the 4-methylbenzenesulfonate(1:2) salt in 2-propanone, yielding 7 parts (23.0%) of 6-[di(1H-imidazol-1-yl)methyl]quinoxaline 4-methylbenzenesulfonate(1:2); mp. 240.3 °C (comp. 51-c).

Example 17-c

α) A mixture of 3.76 parts of 4-(1H-imidazol-1-ylmethyl)-1,2-benzenediamine, 4.5 parts of diphenylethanedione and 80 parts of ethanol was stirred for 4 hours at reflux temperature. The reaction mixture was concentrated and the concentrate was purified by column chromatography over silica gel using a mixture of trichloromethane and methanol (95:5 by volume) as eluent. The pure fractions were collected and the eluent was evaporated. The residue was crystallized from a mixture of 1,1'-oxybisethane and ethanol. The product was filtered off and dried, yielding 4 parts (55%) of 6-(1H-imidazol-1-ylmethyl)-2,3-diphenylquinoxaline; mp. 159.3 °C (comp. 8-c).

β) 6-[(1H-imidazol-1-yl)(phenyl)methyl]quinoxaline; mp. 126.8 °C (comp. 5-c) was prepared following substantially the same procedures as in example 17α except that ethanedial was used in place of diphenylethanedione, and 4-[(1H-imidazol-1-yl)phenylmethyl]-1,2-benzenediamine in place of 4-(1H-imidazol-1-ylmethyl)-1,2-benzenediamine.

γ) 6-[(1H-imidazol-1-yl)(phenyl)methyl]-2,3-dimethylquinoxaline monohydrate; mp. 82.9 °C (comp. 6-c) was prepared following substantially the same procedures as in example 17-c-β except that 2,3-butanedione was used in place of ethanedial.

Example 18-c

α) A mixture of 8.1 parts of 4-[(3-fluorophenyl)(1H-imidazol-1-yl)methyl]-1,2-benzenediamine, 5 parts of ethanedial and 80 parts of methanol was stirred at reflux temperature. Upon complete reaction, the mixture was evaporated to dry and the residue was taken up in water. The product was extracted with trichloromethane. The extract was dried, filtered and evaporated. The residue was purified by column chromatography over silica gel using a mixture of dichloromethane and methanol (98:2 by volume) was eluent. The pure fractions were collected and the eluent was evaporated. The residue was crystallized

from 2,2'-oxybispropane. The product was filtered off and dried, yielding 6.35 parts (74.5%) of 6-[(3-fluorophenyl)(1H-imidazol-1-yl)methyl]quinoxaline; mp. 109.7 °C (comp. 15-c).

β) 6-[(3-fluorophenyl)-(1H-imidazol-1-yl)methyl]-2,3-dimethylquinoxaline; mp. 81.4 °C (comp. 19-c) was prepared following substantially the same procedures as in example 18-c-α except that 2,3-butanedione was used in place of ethanedial.

Example 19-c

To a cooled (0-5 °C) solution of 4.5 parts of 6-[1-(1H-imidazol-1-yl)-2-methylpropyl]-quinoxaline in 79 parts of methanol were added portionwise 4.5 parts of sodium tetrahydroborate. After stirring for 3 hours at 0-5 °C, water was added. The product was extracted with dichloromethane and the extract was dried, filtered and evaporated. The residue was purified by column chromatography (silica gel ; $CH_2Cl_2/CH_3OH/NH_4OH$ 90:10:0.1). The eluent of the desired fraction was evaporated and the residue was converted into the ethanedioate (2:5) salt in ethanol, yielding 1 part (11.5%) of 1,2,3,4-tetrahydro-6-[1-(1H-imidazol-1-yl)-2-methylpropyl]quinoxaline ethanedioate(2:5) hemihydrate; mp. 145.6 °C (comp. 1-c).

Example 20-c

α) A mixture of 9.1 parts of 4-[(3-chlorophenyl)(1H-imidazol-1-yl)methyl]-1,2-benzenediamine, 3.7 parts of ethyl 2-oxopropanoate and 160 parts of methanol was stirred for 30 minutes at reflux temperature. The reaction mixture was evaporated to dry. The residue was crystallized from a mixture of 36 parts of 2-propanone and 4 parts of methanol. After stirring for 30 minutes at room temperature, the precipitated product was filtered off (the filtrate was set aside) and recrystallized from a mixture of methanol and dichloromethane. The product was filtered off and dried, yielding 3 parts (28.1%) of 6-[(3-chlorophenyl)-(1H-imidazol-1-yl)methyl]-3-methyl-2(1H)-quinoxalinone; mp. 270.9 °C (comp. 73-c). The filtrate (see above) was evaporated and the residue was purified by column chromatograhpy over silica gel using a mixture of dichloromethane and methanol (95:5 by volume) as eluent. The pure fractions were collected and the eluent was evaporated. The residue was taken up in a mixture of 2-butanone and 2-propanone and the whole was allowed to stand for a few days. The precipitated product was filtered off and dried, yielding 1.4 parts (11.5%) of 7-[(3-chlorophenyl) (1H-imidazol-1-yl)methyl]-3-methyl-2(1H)-quinoxalinone hemihydrate; mp. 201.9 °C (comp. 81-c).

β) 6-[1-(1H-imidazol-1-yl)-2-methylpropyl]-3-(2-methylpropyl)-2(1H)-quinoxalinone; mp. 197.4 °C (comp. 101-c) and 7-[1-(1H-imidazol-1-yl)-2-methylpropyl]-3-(2-methylpropyl)-2(1H)-quinoxalinone; mp. 173.5 °C (comp. 102-c) were prepared following substantially the same procedures as in example 20α except that ethyl 4-methyl-2-oxopentanoate was used in place of ethyl 2-oxopropanoate, and 4-[1-(1H-imidazol-1-yl)-2-methylpropyl]-1,2-benzenediamine in place of 4-[(3-chlorophenyl)-(1H-imidazol-1-yl)methyl]-1,2-benzenediamine.

γ) 7-[1-(1H-imidazol-1-yl)-2-methylpropyl]-3-(1-methylethyl)-2(1H)-quinoxalinone; mp. 186.7 °C (comp. 106-c) and 6-[1-(1H-imidazol-1-yl)-2-methylpropyl]-3-(1-methylethyl)-2(1H)-quinoxalinone; mp. 187.4 °C (comp. 117-c) were prepared following substantially the same procedures as in example 20-c-β except that ethyl 3-methyl-2-oxobutanoate was used in place of ethyl 4-methyl-2-oxopentanoate.

δ) 6-[1-(1H-imidazol-1-yl)-2-methylpropyl]-3-phenyl-2(1H)quinoxalinone; mp. 209.6 °C (comp. 89-c) and 7-[1-(1H-imidazol-1-yl)-2-methylpropyl]-3-phenyl-2(1H)quinoxalinone; mp. 281.0 °C (comp. 91-c) were prepared following substantially the same procedures as in example 2-c-β except that methyl α-oxobenzenacetate was used in place of ethyl 4-methyl-2-oxopentanoate.

ε) ethyl 3,4-dihydro-7-(1H-imidazol-1ylmethyl)-α-methyl-3-oxo-2-quinoxalineacetate; mp. 208.1 °C (comp. 87-c) and ethyl 3,4-dihydro-6-(1H-imidazol-1ylmethyl)-α-methyl-3-oxo-2-quinoxalineacetate; mp. 223.4 °C (comp. 88-c) were prepared following substantially the same procedures as in example 20-c-α except that diethyl 2-methyl-3-oxo-1,4-butanedioate was used in place of ethyl 2-oxopropanoate, and 4-[(1H-imidazol-1-yl)methyl]-1,2-benzenediamine in place of 4-[(3-chlorophenyl) (1H-imidazol-1-yl)methyl]-1,2-benzenediamine.

Example 21-c

α) To a stirred and cooled (0 °C) solution of 9.1 parts of 4-[(3-chlorophenyl) (1H-imidazol-1-yl)methyl]-1,2-benzenediamine in 80 parts of acetic acid and 20 parts of water were added portionwise 5.8 parts of ethyl 4-methyl-2-oxopentanoate. Upon completion, stirring was continued for 4 hours at room temperature. The reaction mixture was poured into 100 parts of water and the whole was neutralized with a

sodium hydroxide solution 3N. The product was extracted with dichloromethane. The extract was dried, filtered and evaporated. The residue was purified by column chromatography over silica gel using a mixture of dichloromethane and methanol (95:5 by volume) as eluent. The pure fractions were collected and the eluent was evaporated. The residue was crystallized from a mixture of 2-propanone and 1,1'-oxybisethane. The product was filtered off (the filtrate was set aside) and dried, yielding 1.5 parts (12.6%) of 6-[(3-chlorophenyl) (1H-imidazol-1-yl)methyl]-3-(2-methylpropyl)-2(1H)-quinoxalinone; mp. 209.7 °C (comp. 178-c)

The filtrate (see above) was evaporated and the residue was further purified by column chromatography (HPLC) over silica gel using a mixture of dichloromethane and methanol (95:5 by volume) as eluent. The pure fractions were collected and the eluent was evaporated. The residue was crystallized twice, yielding 1.2 parts (10.0%) of 7-[(3-chlorophenyl)(1H-imidazol-1-yl)methyl]-3-(2-methylpropyl)-2(1H)-quinoxalinone; mp. 215.2 °C (comp. 177-c).

β) 6-[(3-chlorophenyl)(1H-imidazol-1-yl)methyl]-3-(1-methylethyl)]-2(1H)-quinoxalinone; mp. 188.8 °C (comp. 181-c) and 7-[(3-chlorophenyl)(1H-imidazol-1-yl)methyl]-3-(1-methylethyl)]-2(1H)-quinoxalinone (comp. 313-c) were prepared following substantially the same procedures as in example 21-c-α except that methyl 3-methyl-2-oxobutanoate was used in place of ethyl 4-methyl-2-oxopentanoate.

Example 22-c

A mixture of 8.8 parts of 4-[1-(1H-imidazol-1-yl)-2-methylpropyl]-1,2-benzenediamine, 3 parts of 2-oxoacetic acid monohydrate and 80 parts of methanol was stirred for 4 hours at reflux temperature. The reaction mixture was evaporated to dry and the residue was purified by column chromatography over silica gel using a mixture of dichloromethane and methanol (98:2 by volume) as eluent. The fractions with the two isomers were collected and the eluent was evaporated. The isomers were separated by crystallization; first from a mixture of 2-butanone and 2-propanone and then from 2-butanone. The first product was filtered off and dried, yielding 1.25 parts (12.3%) of 7-[1-(1H-imidazol-1-yl)-2-methylpropyl]-2(1H)-quinoxalinone; mp. 246.3 °C (comp. 90-c).

The second product was filtered off and dried, yielding 0.5 parts (4.9%) of 6-[1-(1H-imidazol-1-yl)-2-methylpropyl]-2(1H)-quinoxalinone; mp. 193.9 °C (comp. 94-c).

Example 23-c

α) To a stirred and cooled (0 °C) mixture of 9 parts of 4-[1-(1H-imidazol-1-yl)-2-methylpropyl]-1,2-benzenediamine in 80 parts of acetic acid and 20 parts of water were added 5 parts of 2-oxopentanoic acid. The reaction mixture was stirred for 12 hours at room temperature. The whole was poured into ice water and neutralized with a sodium hydroxide solution 3 N. The product was extracted three times with 130 parts of dichloromethane. The combined extracts were dried, filtered and evaporated. For obtaining 6-[1-(1H-imidazol-1yl)-2-methylpropyl]-3-propyl-2(1H)-quinoxalinone, the residue was purified by column chromatography (HPLC) over silica gel using a mixture of dichloromethane and methanol (98:2 by volume) as eluent. The pure fractions were collected and the eluent was evaporated. The residue was crystallized from a mixture of 2-butanone and 1,1'-oxybisethane. The precipitated product was filtered off and stirred in cold 4-methyl-2-pentanone. After filtration, the product was recrystallized from a mixture of methanol and 2-propanone, yielding 1.6 parts (13.2%) of the above product; mp. 259.7 °C (comp. 100-c).

For obtaining 7-[1-(1H-imidazol-1yl)-2-methylpropyl]-3-propyl-2(1H)-quinoxalinone, the residue was purified by column chromatography over silica gel using a mixture of dichloromethane, 2-propanol and ammonium hydroxide (90:10:0.1 by volume) as eluent. The pure fractions were collected and the eluent was evaporated. The residue was crystallized from a mixture of acetonitrile and 1,1'-oxybisethane. The precipitated product was filtered off and recrystallized first three times from a mixture of methanol and acetonitrile and then twice from a mixture of methanol, ethyl acetate and 2-propanol, yielding 1.45 parts (12.0%) of the above product; mp. 176.0 °C (comp. 140-c).

β) 3-ethyl-6-[1-(1H-imidazol-1-yl)-2-methylpropyl]-2(1H)-quinoxalinone; mp. 203.7 °C (comp. 104-c) and 3-ethyl-7-[1-(1H-imidazol-1-yl)-2-methylpropyl]-2(1H)-quinoxalinone (comp. 314-c) were prepared following substantially the same procedures as in example 23-c-α were used except that 2-oxobutanoic acid was used in place of 2-oxopentanoic acid.

Example 24-c

A solution of 15 parts of 4-[1-(1H-imidazol-1-yl)-2-methylpropyl]-1,2-benzenediamine and 12.5 parts of diethyl 2-oxo-1,3-propanedioate in 80 parts of ethanol was stirred for 2 hours at reflux temperature. The reaction mixture was evaporated and the residue was purified by column chromatography over silica gel using a mixture of dichloromethane and methanol (95:5 by volume) as eluent. The pure fractions were collected and the eluent was evaporated. The residue was crystallized from a mixture of acetonitrile and ethanol. The product was filtered off and dried, yielding 3.1 parts (14.0%) of ethyl 3,4-dihydro-7-[1-(1H-imidazol-1-yl)-2-methylpropyl]-3-oxo-2-quinoxalinecarboxylate; mp. 229.7 °C (comp. 107-c). The filtrate of the crystallization was evaporated and the residue was recrystallized from a mixture of acetonitrile and ethanol. The product was filtered off and dried, yielding 2.2 parts (10.0%) of ethyl 3,4-dihydro-6-[1-(1H)-imidazol-1-yl)-2-methylpropyl]-3-oxo-2-quinoxalinecarboxylate; mp. 184.8 °C (comp. 116-c).

Example 25-c

A mixture of 7 parts of N-[4-[1-(1H-imidazol-1-yl)-2-methylpropyl]-2-nitrophenyl]-β-oxobenzenepropanamide, 7.03 parts of potassium carbonate and 70 parts of water was stirred for 1.5 hours at reflux temperature. After cooling, the whole was treated with a hydrochloric acid solution 3 N to pH 7. The product was extracted with dichloromethane (3 x 104 parts). The combined extracts were dried, filtered and evaporated. The residue was taken up in ethanol. The product was filtered off and dried, yielding 5.1 parts (73.0%) of 3-benzoyl-6-[1-(1H-imidazol-1-yl)-2-methylpropyl]-2(1H)-quinoxalinone, $N^4$-oxide monohydrate; mp. 210.0 °C (comp. 154-c).

Example 26-c

A mixture of 12 parts of N-[4-[(3-chlorophenyl)(1H-imidazol-1-yl)methyl]-2-nitrophenyl]-3-oxobutanamide and 120 parts of a sodium hydroxide solution 6.5% was stirred for 15 minutes at 80 °C. After cooling, the product was obtained, yielding 10.25 parts (100%) of 6-[(3-chlorophenyl)(1H-imidazol-1-yl)methyl]-2(1H)-quinoxalinone, $N^4$-oxide (comp. 175-c).

Example 27-c

To a stirred mixture of 11 parts of 4-chloro-N-[4-[2-methyl-1-(1H-1,2,4-triazol-1-yl)propyl]-2-nitrophenyl]-benzeneacetamide and 49 parts of pyridine were added 3.6 parts of 2-methyl-2-propanol, potassium salt under a nitrogen atmosphere. After stirring for 1 hour at room temperature, the reaction mixture was poured into ice-water. The whole was neutralized with HCl 3N and the product was extracted with dichloromethane. The extract was dried, filtered and evaporated. The residue was purified by column chromatography (silica gel ; $CH_2Cl_2/CH_3OH$ 98:2). The eluent of the desired fraction was evaporated, yielding 6.6 parts (61.8%) of 3-(4-chlorophenyl)-6-[2-methyl-1-(1H-1,2,4-triazol-1-yl)propyl]-2(1H)-quinoxalinone, $N^4$-oxide (comp. 221-c).

Example 28-c

A solution of 14.5 parts of 3-fluoro-N-[4-[1-(1H-imidazol-1-yl)-2-methylpropyl]-2-nitrophenyl]-benzeneacetamide in 49 parts of pyridine and 10 parts of a potassium hydroxide solution 20% was stirred for 1 hour at 85 °C. The reaction mixture was poured into crushed ice and neutralized with a sulfuric acid solution 2N. After evaporation, the residue was purified by column chromatography over silica gel using a mixture of dichloromethane, methanol and ammonium hydroxide (95:5:0.5 by volume) as eluent. The pure fractions were collected and the eluent was evaporated. The residue was crystallized from a mixture of dichloromethane and 2,2'-oxybispropane. The product was filtered off and dried, yielding 4.3 parts (67.6%) of 3-(3-fluorophenyl)-6-[1-(1H-imidazol-1-yl)-2-methylpropyl]-2(1H)-quinoxalinone, $N^4$-oxide; mp. 212.9 °C (comp. 166-c).

Example 29-c

A mixture of 5 parts of 4-(1H-imidazol-1-ylmethyl)-1,2-benzenediamine, 4 parts of diethyl ethanedioiate and 40 parts of methanol was stirred for 4 hours at room temperature. The precipitated product was filtered off and dried, yielding 4 parts (62.3%) of 6-(1H-imidazol-1-ylmethyl)-2,3(1H,4H)-quinoxalinedione; mp.

>300 ° C (comp. 315-c).

Example 30-c

A solution of 3 parts of 6-[1-(1H-imidazol-1-yl)pentyl]-3-phenyl-2(1H)-quinoxalinone, $N^4$-oxide in 80 parts of methanol was hydrogenated over night at $2.10^5$ Pa and at room temperature with 0.5 parts of Raney nickel catalyst. The catalyst was filtered off over diatomaceous earth and the filtrate was evaporated. The residue was crystallized from acetonitrile, yielding 2.5 parts (87.2%) of 6-[1-(1H-imidazol-1-yl)pentyl]-3-phenyl-2(1H)-quinoxalinone; mp. 192.4 ° C (comp. 162-c).

Example 31-c

A mixture of 10.25 parts of 6-[(3-chlorophenyl)(1H-imidazol-1-yl)methyl]-2(1H)-quinoxalinone, $N^4$-oxide, 120 parts of a sodium hydroxide solution 6.5% and 120 parts of water was hydrogenated for 1 hour in a Parr apparatus at $3.10^5$ Pa and at room temperature with 10 parts of a Raney nickel catalyst under nitrogen atmosphere. The whole was filtered over diatomaceous earth and the filtrate was treated with a hydrochloric acid solution 3 N to pH 7. The product was extracted with a mixture of dichloromethane and methanol. The extract was dried, filtered and evaporated. The residue was purified by column chromatography over silica gel using a mixture of dichloromethane and methanol (96:4 by volume) as eluent. The pure fractions were collected and the eluent was evaporated. The residue was crystallized from a mixture of 2-butanone, 2-propanone and 1,1'-oxybisethane. The product was filtered off and dried, yielding 0.95 parts (9.7%) of 6-[(3-chlorophenyl)(1H-imidazol-1-yl)methyl]-2(1H)-quinoxalinone; mp. 253.0 ° C (comp. 176-c).

Example 32-c

A mixture of 3.9 parts of 3-(3,4-dimethoxyphenyl)-6-[1-(1H-imidazol-1-yl)-2-methylpropyl]-2(1H)-quinoxalinone, $N^4$-oxide, 3.3 parts of sodium dithionate, 55.3 parts of ethanol and 30 parts of water was refluxed for 1/2 hour. After cooling, the reaction mixture was partitioned between water and dichloromethane. The organic layer was dried, filtered and evaporated. The residue was purified by column chromatography (silica gel ; $CH_2Cl_2/CH_3OH$ 95:5). The eluent of the desired fraction was evaporated and the residue was crystallized from methanol and ethyl acetate, yielding 2.1 parts (56.4%) of 3-(3,4-dimethoxyphenyl)-6-[1-(1H-imidazol-1-yl)-2-methylpropyl]-2(1H)-quinoxalinone; mp. 242.6 ° C (comp. 236-c).

Example 33-c

A mixture of 3 parts of 7-(1H-imidazol-1-ylmethyl)-3-methyl-2(1H)-quinoxalinone, 0.3 parts of a sodium hydroxide dispersion 50% and 28 parts of N,N-dimethylformamide was stirred for 1.5 hours at room temperature. 2 Parts of iodomethane were added and stirring was continued for 12 hours at room temperature under nitrogen atmosphere. The reaction mixture was evaporated to dry and the residue was taken up in water and potassium carbonate. The product was extracted with dichloromethane. The extract was dried, filtered and evaporated. The residue was purified by column chromatography over silica gel using a mixture of dichloromethane and methanol (98:2 by volume) as eluent. The pure fractions were collected and the eluent was evaporated. The residue was crystallized from a mixture of 2,2'-oxybispropane and 2-propanone. The product was filtered off and dried, yielding 2.2 parts (66.8%) of 7-(1H-imidazol-1-ylmethyl)-1,3-dimethyl-2(1H)-quinoxalinone hemihydrate; mp. 128.6 ° C (comp. 79-c).

Example 34-c

A mixture of 5 parts of 6-[(1H-imidazol-1-yl)phenylmethyl]-3-methyl-2(1H)-quinoxalinone, 3.3 parts of sodium hydroxide and 30 parts of water was stirred for 1 hour at room temperature. 5 Parts of hydroxylamine-O-sulfonic acid were added and the reaction mixture was stirred for 4 hours at 20 ° C. The product was extracted with dichloromethane (3 x 65 parts). The combined extracts were dried, filtered and evaporated. The residue was purified by column chromatography over silica gel using a mixture of dichloromethane and methanol (95:5 by volume) as eluent. The pure fractions were collected and the eluent was evaporated. The residue was crystallized from a mixture of 2-propanone and 1,1'-oxybisethane. The product was filtered off and dried, yielding 2 parts (38.2%) of 1-amino-6-[(1H-imidazol-1-yl)phenylmethyl]-3-methyl-2(1H)-quinoxalinone; mp. 192.8 ° C (comp. 85-c).

Example 35-c

A solution of 4.25 parts of ethyl 3,4-dihydro-7-[1-(1H)-imidazol-1-yl)-2-methylpropyl]-3-oxo-2-quinoxalinecarboxylate in 20 parts of a sodium hydroxide solution 1 N was stirred for 4 hours at room temperature. The reaction mixture was treated with a diluted sulfuric acid solution to pH 5.5. After concentration, the residue was crystallized from pyridine. The product was filtered off and dried, yielding 1 part (24.6%) of 3,4-dihydro-7-[1-(1H-imidazol-1-yl)-2-methylpropyl]-3-oxo-2-quinoxalinecarboxylic acid; mp. 237.5 °C (comp. 129-c).

Example 36-c

A mixture of 6 parts of 7-(1H-imidazol-1-ylmethyl)-3-methyl-2(1H)-quinoxalinone and 40 parts of phosphoryl chloride was stirred for 2 hours at reflux temperature. The reaction mixture was evaporated to dry. The residue was taken up in 300 parts of ice water and the whole was neutralized with potassium carbonate. The product was extracted three times with 65 parts of dichloromethane. The combined extracts were dried, filtered and evaporated. The residue was purified by column chromatography over silica gel using a mixture of dichloromethane and methanol (98:2 by volume) as eluent. The pure fractions were collected and the eluent was evaporated. The residue was crystallized from 1,1'-oxybisethane. The product was filtered off and dried, yielding 1.6 parts (59.4%) of 3-chloro-6-(1H-imidazol-1-ylmethyl)-2-methylquinoxaline; mp. 115.8 °C (comp. 22-c).

Example 37-c

A solution of 0.3 parts of sodium in 24 parts of 1-propanol was added to 2.4 parts of 3-chloro-6-(1H-imidazol-1-ylmethyl)-2-methylquinoxaline. The whole was stirred for 2 hours at reflux temperature. The reaction mixture was evaporated and the residue was extracted three times with 65 parts of dichloromethane. The combined extracts were dried, filtered and evaporated. The residue was purified by column chromatography over silica gel using a mixture of dichloromethane and methanol (98:2 by volume) as eluent. The pure fractions were collected and the eluent was evaporated. The residue was crystallized from a mixture of pentane and 2,2'-oxybispropane. The product was filtered off and dried, yielding 1.5 parts (57.1%) of 6-(1H-imidazol-1-ylmethyl)-2-methyl-3-propoxyquinoxaline; mp. 85.5 °C (comp. 24-c).

Example 38-c

A mixture of 5.5 parts of 3-chloro-6-[1-(1H-imidazol-1-yl)-2-methylpropyl]-2-methylquinoxaline, 9 parts of an aqueous N-methylmethanamine solution 40% and 48 parts of methanol was stirred for 12 hours at 140 °C. After cooling, the reaction mixture was evaporated to dry and the residue was purified by column chromatography over silica gel using a mixture of dichloromethane and methanol (98:2 by volume) as eluent. The pure fractions were collected and the eluent was evaporated. The residue was crystallized from petroleum ether. The product was filtered off and dried, yielding 0.9 parts (15.9%) of 7-[1-(1H-imidazol-1-yl)-2-methylpropyl]-N,N,3-trimethyl-2-quinoxazolinamine; mp. 116.7 °C (comp. 47-c).

All other compounds listed in tables 7-c to 11-c were obtained by analogous methods of preparation as described in examples 14-c to 38-c, the actual method of preparation being indicated in column 2 (Ex. No.)

Table 7-c

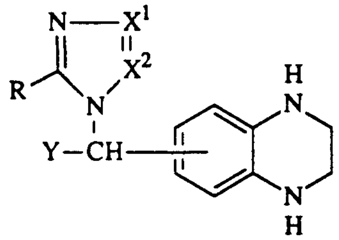

| Comp. No. | Ex. No | R | $-X^1=X^2-$ | Y | mp.(°C)/base / salt |
|---|---|---|---|---|---|
| 1-c | 19-c | H- | -CH=CH- | $i-C_3H_7-$ | 145.6°C/$0.5H_2O$/$2.5(COOH)_2$ |
| 2-c | - | H- | -CH=CH- | H- | - |
| 3-c | - | H- | -CH=CH- | $C_6H_5-$ | - |
| 4-c | - | H- | -CH=CH- | $4Cl-C_6H_4-$ | - |

Table 8-c

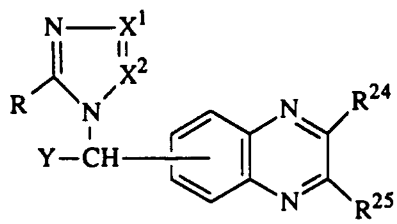

| Comp. No. | Ex. No. | R | $-X^1=X^2-$ | Y | p | $R^{24}$ | $R^{25}$ | mp.(°C) / base / salt |
|---|---|---|---|---|---|---|---|---|
| 5-c | 17-c | H- | -CH=CH- | $C_6H_5-$ | 6 | H- | H- | 126.8 |
| 6-c | 17-c | H- | -CH=CH- | $C_6H_5-$ | 6 | $CH_3-$ | $CH_3-$ | 82.9 / $H_2O$ |
| 7-c | 17-c | H- | -CH=CH- | $CH_3-$ | 6 | H- | H- | 135.1 |
| 8-c | 17-c | H- | -CH=CH- | H- | 6 | $C_6H_5-$ | $C_6H_5-$ | 159.3 |
| 9-c | 18-c | H- | -CH=CH- | $i-C_3H_7$. | 6 | H- | H- | 128.6 |
| 10-c | 18-c | H- | -CH=CH- | H- | 6 | H- | H- | 144.1 |
| 11-c | 18-c | H- | -CH=CH- | $4F-C_6H_4-$ | 6 | H- | H- | 131.6 |

| Comp. No. | Ex. No. | R | $-X^1=X^2-$ | Y | p | $R^{24}$ | $R^{25}$ | mp.(°C) / base / salt |
|---|---|---|---|---|---|---|---|---|
| 12-c | 18-c | H- | -CH=CH- | $C_2H_5$- | 6 | H- | H- | 72.4 |
| 13-c | 18-c | H- | -CH=CH- | 2-thienyl | 6 | H- | H- | 93.7 |
| 14-c | 18-c | H- | -CH=CH- | 4F-$C_6H_4$- | 6 | $CH_3$- | $CH_3$- | 82.0 / $H_2O$ |
| 15-c | 18-c | H- | -CH=CH- | 3F-$C_6H_4$- | 6 | H- | H- | 109.7 |
| 16-c | 18-c | H- | -CH=CH- | 2F-$C_6H_4$- | 6 | H- | H- | 79.7 |
| 17-c | 18-c | H- | -CH=CH- | $CH_3$- | 6 | $CH_3$- | $CH_3$- | 76.2/0.5$H_2O$ |
| 18-c | 18-c | H- | -CH=CH- | 3Cl-$C_6H_4$- | 6 | H- | H- | 70.3/0.5$H_2O$ |
| 19-c | 18-c | H- | -CH=CH- | 3F-$C_6H_4$- | 6 | $CH_3$- | $CH_3$- | 81.4 |
| 20-c | 36-c | H- | -CH=CH- | $C_6H_5$- | 6 | Cl- | $CH_3$- | 164.7 |
| 21-c | 37-c | H- | -CH=CH- | $C_6H_5$- | 6 | $CH_3O$- | $CH_3$- | 117.7 |
| 22-c | 36-c | H- | -CH=CH- | H- | 7 | Cl- | $CH_3$- | 115.8 |
| 23-c | 37-c | H- | -CH=CH- | H- | 7 | $CH_3O$- | $CH_3$- | 151.8 |
| 24-c | 37-c | H- | -CH=CH- | H- | 7 | $C_3H_7O$- | $CH_3$- | 85.5 |
| 25-c | 37-c | H- | -CH=CH- | H- | 7 | i-$C_3H_7O$- | $CH_3$- | 109.6 |
| 26-c | 36-c | H- | -CH=CH- | H- | 6 | Cl- | $CH_3$- | - |
| 27-c | 37-c | H- | -CH=CH- | H- | 6 | $C_3H_7O$- | $CH_3$- | 132.0 |
| 28-c | 37-c | H- | -CH=CH- | H- | 6 | i-$C_3H_7O$- | $CH_3$- | 117.5 |
| 29-c | 37-c | H- | -CH=CH- | H- | 7 | 1H-imidazolyl | $CH_3$- | 164.1 |
| 30-c | 18-c | H- | -CH=CH- | 2F-$C_6H_4$- | 6 | $CH_3$- | $CH_3$- | 94.5 |
| 31-c | 37-c | H- | -CH=CH- | H- | 6 | $CH_3O$- | $CH_3$- | 150.7 |
| 32-c | 37-c | H- | -CH=CH- | $C_6H_5$- | 6 | $C_3H_7O$- | $CH_3$- | 125.6 |
| 33-c | 36-c | H- | -CH=CH- | H- | 7 | Cl- | H- | - |
| 34-c | 37-c | H- | -CH=CH- | H- | 7 | $CH_3O$- | H- | 121.0 |
| 35-c | 18-c | H- | -CH=CH- | 2Cl-$C_6H_4$- | 6 | H- | H- | 114.7 |
| 36-c | 36-c | H- | -CH=CH- | $C_6H_5$- | 7 | Cl- | $CH_3$- | - |
| 37-c | 37-c | H- | -CH=CH- | $C_6H_5$- | 7 | $CH_3O$- | $CH_3$- | 131.2 |
| 38-c | 36-c | H- | -CH=CH- | H- | 6 | Cl- | H- | - |
| 39-c | 37-c | H- | -CH=CH- | H- | 6 | $CH_3O$- | H- | 123.2 |
| 40-c | 18-c | H- | -CH=CH- | 4$CH_3$-$C_6H_4$- | 6 | H- | H- | 132.9 |
| 41-c | 14-c | H- | -CH=CH- | H- | 5 | H- | H- | 121.2 |
| 42-c | 18-c | H- | -CH=CH- | 3$CF_3$-$C_6H_4$- | 6 | H- | H- | 96.5 / $H_2O$ |
| 43-c | 36-c | H- | -CH=CH- | i-$C_3H_7$- | 6 | Cl- | $CH_3$- | - |
| 44-c | 38-c | H- | -CH=CH- | i-$C_3H_7$- | 6 | $NH_2$- | $CH_3$- | 238.2 |

| Comp. No. | Ex. No. | R | $-X^1=X^2-$ | Y | p | $R^{24}$ | $R^{25}$ | mp.(°C) / base / salt |
|---|---|---|---|---|---|---|---|---|
| 45-c | 36-c | H- | -CH=CH- | $i-C_3H_7-$ | 7 | Cl- | $CH_3-$ | - |
| 46-c | 37-c | H- | -CH=CH- | $i-C_3H_7-$ | 7 | $CH_3O-$ | $CH_3-$ | 124.6 |
| 47-c | 38-c | H- | -CH=CH- | $i-C_3H_7-$ | 7 | $(CH_3)_2N-$ | $CH_3-$ | 116.7 |
| 48-c | 15-c | $CH_3-$ | -CH=CH- | $C_6H_5-$ | 6 | H- | H- | 134.5 |
| 49-c | 15-c | H- | -CH=N- | $C_6H_5-$ | 6 | H- | H- | 96.0 |
| 50-c | 15-c | H- | -N=CH- | $C_6H_5-$ | 6 | H- | H- | 98.1 |
| 51-c | 16-c | H- | -CH=CH- | 1<u>H</u>-imidazol-1-yl- | 6 | H- | H- | 240.3 / 2* |
| 52-c | - | H- | -CH=N- | $C_6H_5-$ | 6 | $CH_3-$ | $CH_3-$ | - |
| 53-c | - | H- | -CH=N- | $i-C_3H_7-$ | 6 | $CH_3-$ | $CH_3-$ | - |
| 54-c | - | $CH_3-$ | -CH=CH- | $i-C_3H_7-$ | 6 | H- | H- | - |
| 55-c | - | H- | -CH=CH- | $CH_3-C\equiv C-$ | 6 | H- | H- | - |
| 56-c | - | H- | -CH=CH- | $CH_3-CH=CH-$ | 6 | H- | H- | - |

* = 4-methylbenzenesulfonate

In the previous and following tables p indicates the position of the 1H-azol-1-ylmethyl moiety on the quinoxaline ring.

Table 9-c

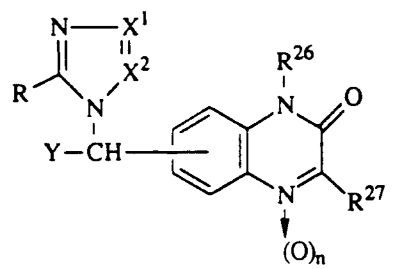

| Comp. No. | Ex. No. | R | $X^1=X^2$ | Y | p | $R^{26}$ | $R^{27}$ | n | mp.(°C) / base/salt |
|---|---|---|---|---|---|---|---|---|---|
| 57-c | 20-c | H- | -CH=CH- | $C_6H_5-$ | 6 | H- | $CH_3-$ | 0 | 254.0 |
| 58-c | 20-c | H- | -CH=CH- | H- | 7 | H- | $CH_3-$ | 0 | 297.6 |
| 59-c | 20-c | H- | -CH=CH- | H- | 6 | H- | $CH_3-$ | 0 | 271.3 |
| 60-c | 20-c | H- | -CH=CH- | $C_6H_5-$ | 7 | H- | $CH_3-$ | 0 | 218.4 |
| 61-c | 22-c | H- | -CH=CH- | H- | 7 | H- | H- | 0 | 253.6 |
| 62-c | 22-c | H- | -CH=CH- | H- | 6 | H- | H- | 0 | 272.9 |

| Comp. No. | Ex. No. | R | $X^1=X^2$ | Y | p | $R^{26}$ | $R^{27}$ | n | mp.(°C) / base/salt |
|---|---|---|---|---|---|---|---|---|---|
| 63-c | 34-c | H- | -CH=CH- | H- | 7 | $NH_2$- | $CH_3$- | 0 | 178.8 |
| 64-c | 20-c | H- | -CH=CH- | 3-$CF_3$-$C_6H_4$- | 6 | H- | $CH_3$- | 0 | >300 (dec.) |
| 65-c | 20-c | H- | -CH=CH- | $CH_3$- | 6 | H- | $CH_3$- | 0 | 268.2 |
| 66-c | 20-c | H- | -CH=CH- | H- | 7 | H- | $C_6H_5$- | 0 | 293.8 |
| 67-c | 20-c | H- | -CH=CH- | H- | 6 | H- | $C_6H_5$- | 0 | 203.1 |
| 68-c | 20-c | H- | -CH=CH- | 2F-$C_6H_4$- | 6 | H- | $CH_3$- | 0 | 273.5 |
| 69-c | 20-c | H- | -CH=CH- | 3F-$C_6H_4$- | 6 | H- | $CH_3$- | 0 | 275.0 |
| 70-c | 20-c | H- | -CH=CH- | 4F-$C_6H_4$- | 6 | H- | $CH_3$- | 0 | 271.7 |
| 71-c | 20-c | H- | -CH=CH- | i-$C_3H_7$- | 7 | H- | $CH_3$- | 0 | 249.8 |
| 72-c | 33-c | H- | -CH=CH- | H- | 6 | $CH_3$- | $CH_3$- | 0 | 191.0 |
| 73-c | 20-c | H- | -CH=CH- | 3Cl-$C_6H_4$- | 6 | H- | $CH_3$- | 0 | 270.9 |
| 74-c | 33-c | H- | -CH=CH- | H- | 7 | $C_4H_9$- | $CH_3$- | 0 | 116.0 |
| 75-c | 33-c | H- | -CH=CH- | $C_6H_5$- | 6 | $C_4H_9$- | $CH_3$- | 0 | 139.9 |
| 76-c | 33-c | H- | -CH=CH- | $C_6H_5$- | 6 | $CH_3$- | $CH_3$- | 0 | 214.5 |
| 77-c | 20-c | H- | -CH=CH- | i-$C_3H_7$- | 6 | H- | $CH_3$- | 0 | 192.5 |
| 78-c | 20-c | H- | -CH=CH- | $C_3H_7$- | 6 | H- | $CH_3$- | 0 | 234.0 |
| 79-c | 33-c | H- | -CH=CH- | H- | 7 | $CH_3$- | $CH_3$- | 0 | 128.6/0.5$H_2O$ |
| 80-c | 33-c | H- | -CH=CH- | H- | 6 | $C_4H_9$- | $CH_3$- | 0 | 128.4 |
| 81-c | 20-c | H- | -CH=CH- | 3Cl-$C_6H_4$- | 7 | H- | $CH_3$- | 0 | 201.9/0.5$H_2O$ |
| 82-c | 20-c | H- | -CH=CH- | $CH_3$- | 7 | H- | $CH_3$- | 0 | 228.8 |
| 83-c | 20-c | H- | -CH=CH- | 3F-$C_6H_4$- | 7 | H- | $CH_3$- | 0 | 209.9 |
| 84-c | 20-c | H- | -CH=CH- | 4F-$C_6H_4$- | 7 | H- | $CH_3$- | 0 | 177.3/0.5$H_2O$ $(COOH)_2$ |
| 85-c | 34-c | H- | -CH=CH- | $C_6H_5$- | 6 | $NH_2$- | $CH_3$- | 0 | 192.8 |
| 86-c | 22-c | H- | -CH=CH- | $C_6H_5$- | 6 | H- | H- | 0 | 225.0 |
| 87-c | 20-c | H- | -CH=CH- | H- | 6 | H- | * | 0 | 208.1 |
| 88-c | 20-c | H- | -CH=CH- | H- | 7 | H- | * | 0 | 223.4 |
| 89-c | 20-c | H- | -CH=CH- | i-$C_3H_7$- | 6 | H- | $C_6H_5$- | 0 | 209.6 |
| 90-c | 22-c | H- | -CH=CH- | i-$C_3H_7$- | 7 | H- | H- | 0 | 246.3 |
| 91-c | 20-c | H- | -CH=CH- | i-$C_3H_7$- | 7 | H- | $C_6H_5$- | 0 | 281.0 |
| 92-c | 34-c | H- | -CH=CH- | H- | 6 | $NH_2$- | $CH_3$- | 0 | 220.7 |
| 93-c | 20-c | H- | -CH=CH- | 3-pyridinyl | 6 | H- | $CH_3$- | 0 | 237.4/0.5$H_2O$ |

* = $-CH(CH_3)COOC_2H_5$

| Comp. No. | Ex. No. | R | $-X^1=X^2-$ | Y | p | $R^{26}$ | $R^{27}$ | n | mp.(°C) / base / salt |
|---|---|---|---|---|---|---|---|---|---|
| 94-c | 22-c | H- | -CH=CH- | $i-C_3H_7-$ | 6 | H- | H- | 0 | 198.2 |
| 95-c | 22-c | H- | -CH=CH- | $C_6H_5-$ | 7 | H- | H- | 0 | 187.6 |
| 96-c | 20-c | H- | -CH=CH- | $C_6H_5-$ | 6 | H- | $C_6H_5-$ | 0 | 251.0 |
| 97-c | 20-c | H- | -CH=CH- | $c.C_3H_5-$ | 6 | H- | $CH_3-$ | 0 | 275.1 |
| 98-c | 20-c | H- | -CH=CH- | $i-C_4H_9-$ | 6 | H- | $CH_3-$ | 0 | 205.4 |
| 99-c | 20-c | H- | -CH=CH- | $c.C_3H_5-$ | 7 | H- | $CH_3-$ | 0 | 203.0 |
| 100-c | 23-c | H- | -CH=CH- | $i-C_3H_7-$ | 6 | H- | $C_3H_7-$ | 0 | 259.7 |
| 101-c | 20-c | H- | -CH=CH- | $i-C_3H_7-$ | 6 | H- | $i-C_4H_9-$ | 0 | 197.4 |
| 102-c | 20-c | H- | -CH=CH- | $i-C_3H_7-$ | 7 | H- | $i-C_4H_9-$ | 0 | 173.5 |
| 103-c | 20-c | H- | -CH=CH- | $C_2H_5-$ | 6 | H- | $CH_3-$ | 0 | 211.9 |
| 104-c | 23-c | H- | -CH=CH- | $i-C_3H_7-$ | 6 | H- | $C_2H_5-$ | 0 | 203.7 |
| 105-c | 20-c | H- | -CH=CH- | $i-C_4H_9-$ | 7 | H- | $CH_3-$ | 0 | 189.0 |
| 106-c | 20-c | H- | -CH=CH- | $i-C_3H_7-$ | 7 | H- | $i-C_3H_7-$ | 0 | 186.7 |
| 107-c | 24-c | H- | -CH=CH- | $i-C_3H_7-$ | 6 | H- | $-COOC_2H_5$ | 0 | 229.7 |
| 108-c | 20-c | H- | -CH=CH- | $i-C_3H_7-$ | 6 | $CH_3-$ | $CH_3-$ | 0 | $132.6/0.5H_2O$ |
| 109-c | 20-c | H- | -CH=CH- | $C_2H_5-$ | 7 | H- | $CH_3-$ | 0 | 197.4 |
| 110-c | 20-c | H- | -CH=CH- | $C_4H_9-$ | 6 | H- | $CH_3-$ | 0 | 201.6 |
| 111-c | 20-c | H- | -CH=CH- | $4-Cl-C_6H_4-$ | 6 | H- | $CH_3-$ | 0 | 228.7 |
| 112-c | 20-c | H- | -CH=CH- | $4-Cl-C_6H_4-$ | 7 | H- | $CH_3-$ | 0 | 160.8 |
| 113-c | 20-c | H- | -CH=CH- | $4-CH_3O-C_6H_4-$ | 6 | H- | $CH_3-$ | 0 | 199.7 |
| 114-c | 23-c | H- | -CH=CH- | $C_6H_5-$ | 6 | H- | $C_2H_5-$ | 0 | 280.0 |
| 115-c | 23-c | H- | -CH=CH- | $C_6H_5-$ | 7 | H- | $C_2H_5-$ | 0 | 211.2 |
| 116-c | 24-c | H- | -CH=CH- | $i-C_3H_7-$ | 7 | H- | $-COOC_2H_5$ | 0 | 184.8 |
| 117-c | 20-c | H- | -CH=CH- | $i-C_3H_7-$ | 6 | H- | $i-C_3H_7-$ | 0 | 187.4 |
| 118-c | 22-c | H- | -CH=CH- | $i-C_4H_9-$ | 7 | H- | H- | 0 | 203.6 |
| 119-c | 20-c | H- | -CH=CH- | $4-CH_3-C_6H_4-$ | 7 | H- | $CH_3-$ | 0 | 150.4 |
| 120-c | 20-c | H- | -CH=CH- | $4-CH_3-C_6H_4-$ | 6 | H- | $CH_3-$ | 0 | 222.5 |
| 121-c | 20-c | H- | -CH=CH- | $C_4H_9-$ | 7 | H- | $CH_3-$ | 0 | 143.1 |
| 122-c | 22-c | H- | -CH=CH- | 2-thienyl | 7 | H- | H- | 0 | 223.1 |
| 123-c | 20-c | H- | -CH=CH- | 2-thienyl | 6 | H- | $CH_3-$ | 0 | 254.5 |
| 124-c | 20-c | H- | -CH=CH- | $4-CH_3O-C_6H_4-$ | 7 | H- | $CH_3-$ | 0 | 203.5 |
| 125-c | 20-c | H- | -CH=N- | $i-C_3H_7-$ | 7 | H- | $CH_3-$ | 0 | 204.8 |
| 126-c | 20-c | H- | -CH=CH- | $c.C_6H_{11}-$ | 6 | H- | $CH_3-$ | 0 | 174.8 |

| Comp. No. | Ex. No. | R | $-X^1=X^2-$ | Y | p | $R^{26}$ | $R^{27}$ | n | mp.(°C) / base / salt |
|---|---|---|---|---|---|---|---|---|---|
| 127-c | 20-c | H- | -CH=CH- | $c.C_3H_5-$ | 6 | H- | $C_6H_5-$ | 0 | >300 |
| 128-c | 20-c | H- | -CH=CH- | 2-thienyl | 7 | H- | $CH_3-$ | 0 | 229.6 |
| 129-c | 35-c | H- | -CH=CH- | $i-C_3H_7-$ | 6 | H- | HOOC- | 0 | 237.5 |
| 130-c | 22-c | H- | -CH=CH- | $c.C_3H_5-$ | 7 | H- | H- | 0 | 234.6 |
| 131-c | 22-c | H- | -CH=CH- | $C_3H_7-$ | 7 | H- | H- | 0 | 168.5 |
| 132-c | 20-c | H- | -CH=CH- | $CH_3-$ | 6 | H- | $C_6H_5-$ | 0 | >300 |
| 133-c | 22-c | H- | -CH=CH- | $c.C_3H_5-$ | 6 | H- | H- | 0 | 224.2 |
| 134-c | 20-c | H- | -CH=CH- | $c.C_5H_9-$ | 6 | H- | $C_6H_5-$ | 0 | 127.9 |
| 135-c | 20-c | H- | -CH=CH- | $c.C_6H_{11}-$ | 6 | H- | $C_6H_5-$ | 0 | 193.4 |
| 136-c | 22-c | H- | -CH=CH- | $CH_3-$ | 7 | H- | H- | 0 | 252.4 |
| 137-c | 20-c | H- | -CH=CH- | $C_2H_5-$ | 6 | H- | $C_6H_5-$ | 0 | >300 |
| 138-c | 22-c | H- | -CH=CH- | $C_3H_7-$ | 6 | H- | H- | 0 | 161.9 |
| 139-c | 22-c | H- | -CH=CH- | $c.C_6H_{11}-$ | 7 | H- | H- | 0 | 278.9 |
| 140-c | 23-c | H- | -CH=CH- | $i-C_3H_7-$ | 7 | H- | $C_3H_7-$ | 0 | 176.0 |
| 141-c | 20-c | H- | -CH=CH- | $i-C_4H_9-$ | 6 | H- | $C_6H_5-$ | 0 | 245.0 |
| 142-c | 22-c | H- | -CH=CH- | $C_2H_5-$ | 7 | H- | H- | 0 | 201.3 |
| 143-c | 22-c | H- | -CH=CH- | $c.C_5H_9-$ | 7 | H- | H- | 0 | >300 |
| 144-c | 22-c | H- | -CH=CH- | $C_4H_9-$ | 7 | H- | H- | 0 | 170.0 |
| 145-c | 20-c | H- | -CH=CH- | $C_4H_9-$ | 7 | H- | $C_6H_5-$ | 0 | 198.4 |
| 146-c | 20-c | H- | -CH=CH- | $c.C_5H_9-$ | 6 | H- | $CH_3-$ | 0 | 221.1 |
| 147-c | 20-c | H- | -CH=CH- | $C_3H_7-$ | 6 | H- | $C_6H_5-$ | 0 | 249.4 |
| 148-c | 22-c | H- | -CH=N- | $i-C_3H_7-$ | 6 | H- | H- | 0 | 209.2 |
| 149-c | 20-c | H- | -CH=N- | $i-C_3H_7-$ | 6 | H- | $CH_3-$ | 0 | 211.0 |
| 150-c | 20-c | H- | -CH=CH- | $C_2H_5-$ | 7 | H- | $C_6H_5-$ | 0 | 272.2 |
| 151-c | 26-c | H- | -CH=CH- | $i-C_3H_7-$ | 6 | H- | H- | 1 | 208.7 |
| 152-c | 25-c | H- | -CH=CH- | $CH_3-$ | 6 | H- | $C_6H_5-CO-$ | 1 | 260.4 |
| 153-c | 25-c | H- | -CH=CH- | H- | 6 | H- | $C_6H_5-CO-$ | 1 | 250.4 |
| 154-c | 25-c | H- | -CH=CH- | $i-C_3H_7-$ | 6 | H- | $C_6H_5-CO-$ | 1 | $210.0/H_2O$ |
| 155-c | 28-c | H- | -CH=CH- | $i-C_3H_7-$ | 6 | H- | $4Cl-C_6H_4-$ | 1 | 181.3 |
| 156-c | 28-c | H- | -CH=CH- | $i-C_3H_7-$ | 6 | H- | $4F-C_6H_4-$ | 1 | 205.3 |
| 157-c | 30-c | H- | -CH=CH- | $i-C_3H_7-$ | 6 | H- | $4F-C_6H_4-$ | 0 | 240.8 |
| 158-c | 28-c | H- | -CH=N- | $i-C_3H_7-$ | 6 | H- | $C_6H_5-$ | 1 | - |
| 159-c | 30-c | H- | -CH=N- | $i-C_3H_7-$ | 6 | H- | $C_6H_5-$ | 0 | 249.4 |

| Comp. No. | Ex. No. | R | $-X^1=X^2-$ | Y | p | $R^{26}$ | $R^{27}$ | n | mp.(°C) / base / salt |
|---|---|---|---|---|---|---|---|---|---|
| 160-c | 30-c | H- | -CH=CH- | $i-C_3H_7-$ | 6 | H- | $4Cl-C_6H_4-$ | 0 | 236.6 |
| 161-c | 28-c | H- | -CH=CH- | $C_4H_9-$ | 6 | H- | $C_6H_5-$ | 1 | 240.5 |
| 162-c | 30-c | H- | -CH=CH- | $C_4H_9-$ | 6 | H- | $C_6H_5-$ | 0 | 192.4 |
| 163-c | 30-c | H- | -CH=CH- | $i-C_3H_7-$ | 6 | H- | $C_6H_5-CO-$ | 0 | $161.5/0.5H_2O$ |
| 164-c | 28-c | H- | -CH=CH- | $i-C_3H_7-$ | 6 | H- | $3Cl-C_6H_4-$ | 1 | 177.4 |
| 165-c | 30-c | H- | -CH=CH- | $i-C_3H_7-$ | 6 | H- | $3Cl-C_6H_4-$ | 0 | $179.7/H_2O$ |
| 166-c | 28-c | H- | -CH=CH- | $i-C_3H_7-$ | 6 | H- | $3F-C_6H_4-$ | 1 | 212.9 |
| 167-c | 30-c | H- | -CH=CH- | $i-C_3H_7-$ | 6 | H- | $3F-C_6H_4-$ | 0 | 255.6 |
| 168-c | 30-c | H- | -CH=CH- | H- | 6 | H- | $C_6H_5-CO-$ | 0 | 268.9 |
| 169-c | 30-c | H- | -CH=CH- | $CH_3-$ | 6 | H- | $C_6H_5-CO-$ | 0 | 192.8 |
| 170-c | 28-c | H- | -CH=CH- | $4Cl-C_6H_4-$ | 6 | H- | $C_6H_5-$ | 1 | 186.2 |
| 171-c | 26-c | H- | -CH=CH- | H- | 6 | H- | H- | 1 | - |
| 172-c | 30-c | H- | -CH=CH- | $4Cl-C_6H_4-$ | 6 | H- | $C_6H_5-$ | 0 | $166.3/0.5H_2O$ |
| 173-c | 20-c | H- | -CH=CH- | $4-iC_3H_7-C_6H_4-$ | 6 | H- | $CH_3-$ | 0 | 237.6 |
| 174-c | 20-c | H- | -CH=CH- | $4-iC_3H_7-C_6H_4-$ | 7 | H- | $CH_3-$ | 0 | 210.4 |
| 175-c | 26-c | H- | -CH=CH- | $3Cl-C_6H_4-$ | 6 | H- | H- | 1 | - |
| 176-c | 31-c | H- | -CH=CH- | $3Cl-C_6H_4-$ | 6 | H- | H- | 0 | 253.0 |
| 177-c | 21-c | H- | -CH=CH- | $3Cl-C_6H_4-$ | 7 | H- | $i-C_4H_9-$ | 0 | 215.2 |
| 178-c | 21-c | H- | -CH=CH- | $3Cl-C_6H_4-$ | 6 | H- | $i-C_4H_9-$ | 0 | 209.7 |
| 179-c | 23-c | H- | -CH=CH- | $3Cl-C_6H_4-$ | 7 | H- | $C_3H_7-$ | 0 | 187.5 |
| 180-c | 23-c | H- | -CH=CH- | $3Cl-C_6H_4-$ | 6 | H- | $C_3H_7-$ | 0 | 204.1 |
| 181-c | 21-c | H- | -CH=CH- | $3Cl-C_6H_4-$ | 6 | H- | $i-C_3H_7-$ | 0 | 188.8 |
| 182-c | 26-c | H- | -CH=CH- | $c.C_6H_{11}-$ | 6 | H- | H- | 1 | - |
| 183-c | 31-c | H- | -CH=CH- | $c.C_6H_{11}-$ | 6 | H- | H- | 0 | 275.7 |
| 184-c | 26-c | H- | -CH=CH- | $4-Cl-C_6H_4-$ | 6 | H- | H- | 1 | - |
| 185-c | 31-c | H- | -CH=CH- | $4-Cl-C_6H_4-$ | 6 | H- | H- | 0 | 149.2 |
| 186-c | 26-c | H- | -CH=CH- | $c.C_5H_9-$ | 6 | H- | H- | 1 | - |
| 187-c | 31-c | H- | -CH=CH- | $c.C_5H_9-$ | 6 | H- | H- | 0 | 229.5 |
| 188-c | 28-c | H- | -CH=CH- | $3-Cl-C_6H_4-$ | 6 | H- | $C_6H_5-$ | 1 | 236.1 |
| 189-c | 26-c | H- | -CH=CH- | $CH_3-$ | 6 | H- | H- | 1 | - |
| 190-c | 31-c | H- | -CH=CH- | $CH_3-$ | 6 | H- | H- | 0 | 263.8 |
| 191-c | 23-c | H- | -CH=CH- | $4Cl-C_6H_4-$ | 6 | H- | $C_3H_7-$ | 0 | 216.5 |
| 192-c | 23-c | H- | -CH=CH- | $4Cl-C_6H_4-$ | 7 | H- | $C_3H_7-$ | 0 | 222.0 |

| Comp. No. | Ex. No. | R | $-X^1=X^2-$ | Y | p | $R^{26}$ | $R^{27}$ | n | mp. °C base/salt |
|---|---|---|---|---|---|---|---|---|---|
| 193-c | 21-c | H- | -CH=CH- | $4Cl-C_6H_4-$ | 6 | H- | $-CH_2-CH(CH_3)_2$ | 0 | 200.3 |
| 194-c | 21-c | H- | -CH=CH- | $4Cl-C_6H_4-$ | 7 | H- | $-CH_2-CH(CH_3)_2$ | 0 | 203.9 |
| 195-c | 21-c | H- | -CH=CH- | $4Cl-C_6H_4-$ | 6 | H- | $i-C_3H_7-$ | 0 | 200.9 |
| 196-c | 30-c | H- | -CH=CH- | $3Cl-C_6H_4-$ | 6 | H- | $C_6H_5-$ | 0 | 245.5 |
| 197-c | 26-c | H- | -CH=CH- | $C_2H_5-$ | 6 | H- | H- | 1 | - |
| 198-c | 31-c | H- | -CH=CH- | $C_2H_5-$ | 6 | H- | H- | 0 | 200.6 |
| 199-c | 25-c | H- | -CH=CH- | $C_6H_5-$ | 6 | H- | $C_6H_5-CO-$ | 1 | $186.5/0.5H_2O$ |
| 200-c | 26-c | H- | -CH=CH- | $4F-C_6H_4-$ | 6 | H- | H- | 1 | - |
| 201-c | 31-c | H- | -CH=CH- | $4F-C_6H_4-$ | 6 | H- | H- | 0 | 259.5 |
| 202-c | 26-c | H- | -CH=CH- | $2F-C_6H_4-$ | 6 | H- | H- | 1 | - |
| 203-c | 31-c | H- | -CH=CH- | $2F-C_6H_4-$ | 6 | H- | H- | 0 | 220.3 |
| 204-c | 26-c | H- | -CH=CH- | $3F-C_6H_4-$ | 6 | H- | H- | 1 | - |
| 205-c | 31-c | H- | -CH=CH- | $3F-C_6H_4-$ | 6 | H- | H- | 0 | $135.4/0.5H_2O$ |
| 206-c | 34-c | H- | -CH=CH- | $i-C_3H_7-$ | 6 | $NH_2-$ | $CH_3-$ | 0 | - |
| 207-c | 28-c | H- | -CH=N- | $C_6H_5-$ | 6 | H- | $C_6H_5-$ | 1 | - |
| 208-c | 30-c | H- | -CH=N- | $C_6H_5-$ | 6 | H- | $C_6H_5-$ | 0 | 259.3 |
| 209-c | 28-c | H- | -CH=CH- | $i-C_3H_7-$ | 6 | H- | $2CH_3-C_6H_4-$ | 1 | - |
| 210-c | 30-c | H- | -CH=CH- | $i-C_3H_7-$ | 6 | H- | $2CH_3-C_6H_4-$ | 0 | $154.2/0.5H_2O$ |
| 211-c | 27-c | H- | -CH=CH- | $i-C_3H_7-$ | 6 | H- | $3-OCH_3-C_6H_4-$ | 1 | - |
| 212-c | 30-c | H- | -CH=CH- | $i-C_3H_7-$ | 6 | H- | $3-OCH_3-C_6H_4-$ | 0 | 225.0 |
| 213-c | 27-c | H- | -CH=CH- | $i-C_3H_7-$ | 6 | H- | $2F-C_6H_4-$ | 1 | - |
| 214-c | 30-c | H- | -CH=CH- | $i-C_3H_7-$ | 6 | H- | $2F-C_6H_4-$ | 0 | 230.1 |
| 215-c | 27-c | H- | -CH=N- | $i-C_3H_7-$ | 6 | H- | $4F-C_6H_4-$ | 1 | - |
| 216-c | 30-c | H- | -CH=N- | $i-C_3H_7-$ | 6 | H- | $4F-C_6H_4-$ | 0 | 268.0 |
| 217-c | 28-c | H- | -CH=CH- | $i-C_3H_7-$ | 6 | H- | $4-CH_3-C_6H_4-$ | 1 | - |
| 218-c | 30-c | H- | -CH=CH- | $i-C_3H_7-$ | 6 | H- | $4-CH_3-C_6H_4-$ | 0 | 221.9 |
| 219-c | 27-c | H- | -CH=N- | $i-C_3H_7-$ | 6 | H- | $3F-C_6H_4-$ | 1 | - |
| 220-c | 30-c | H- | -CH=N- | $i-C_3H_7-$ | 6 | H- | $3F-C_6H_4-$ | 0 | 202.3 |
| 221-c | 27-c | H- | -CH=N- | $i-C_3H_7-$ | 6 | H- | $4Cl-C_6H_4-$ | 1 | - |
| 222-c | 30-c | H- | -CH=N- | $i-C_3H_7-$ | 6 | H- | $4Cl-C_6H_4-$ | 0 | 274.6 |
| 223-c | 27-c | $CH_3-$ | -CH=CH- | $i-C_3H_7-$ | 6 | H- | $C_6H_5-$ | 1 | - |
| 224-c | 30-c | $CH_3-$ | -CH=CH- | $i-C_3H_7-$ | 6 | H- | $C_6H_5-$ | 0 | 252.5 |
| 225-c | 27-c | H- | -CH=CH- | $i-C_3H_7-$ | 6 | H- | $4-Br-C_6H_4-$ | 1 | - |

| Comp No. | Ex. No. | R | $-X^1=X^2-$ | Y | p | $R^{26}$ | $R^{27}$ | n | mp. °C base/salt |
|---|---|---|---|---|---|---|---|---|---|
| 226-c | 30-c | H- | -CH=CH- | $i-C_3H_7-$ | 6 | H- | $4-Br-C_6H_4-$ | 0 | 226.0 |
| 227-c | 27-c | H- | -CH=CH- | $i-C_3H_7-$ | 6 | H- | $3,4-F_2-C_6H_3-$ | 1 | - |
| 228-c | 30-c | H- | -CH=CH- | $i-C_3H_7-$ | 6 | H- | $3,4-F_2-C_6H_3-$ | 0 | 218.0 |
| 229-c | 27-c | H- | -CH=CH- | $i-C_3H_7-$ | 6 | H- | $3CH_3-C_6H_4-$ | 1 | - |
| 230-c | 30-c | H- | -CH=CH- | $i-C_3H_7-$ | 6 | H- | $3CH_3-C_6H_4-$ | 0 | 230.4 |
| 231-c | 27-c | H- | -CH=CH- | $i-C_3H_7-$ | 6 | H- | $4-OCH_3-C_6H_4-$ | 1 | - |
| 232-c | 30-c | H- | -CH=CH- | $i-C_3H_7-$ | 6 | H- | $4-OCH_3-C_6H_4-$ | 0 | $157.8/H_2O$ |
| 233-c | 27-c | H- | -CH=CH- | $3,4Cl_2-C_6H_3-$ | 6 | H- | $C_6H_5-$ | 1 | - |
| 234-c | 30-c | H- | -CH=CH- | $3,4Cl_2-C_6H_3-$ | 6 | H- | $C_6H_5-$ | 0 | 262.5 |
| 235-c | 27-c | H- | -CH=CH- | $i-C_3H_7-$ | 6 | H- | $3,4(OCH_3)_2-C_6H_3-$ | 1 | - |
| 236-c | 32-c | H- | -CH=CH- | $i-C_3H_7-$ | 6 | H- | $3,4(OCH_3)_2-C_6H_3-$ | 0 | 242.6 |
| 237-c | 27-c | H- | -CH=CH- | $i-C_3H_7-$ | 6 | H- | $2,4Cl_2-C_6H_3-$ | 1 | - |
| 238-c | 32-c | H- | -CH=CH- | $i-C_3H_7-$ | 6 | H- | $2,4Cl_2-C_6H_3-$ | 0 | 178.0 |
| 239-c | 27-c | H- | -CH=CH- | $i-C_3H_7-$ | 6 | H- | 2-naphthalenyl- | 1 | - |
| 240-c | 32-c | H- | -CH=CH- | $i-C_3H_7-$ | 6 | H- | 2-naphthalenyl- | 0 | 274.9 |
| 241-c | 27-c | H- | -CH=CH- | $i-C_3H_7-$ | 6 | H- | $3,4,5(OCH_3)_3-C_6H_2-$ | 1 | - |
| 242-c | 32-c | H- | -CH=CH- | $i-C_3H_7-$ | 6 | H- | $3,4,5(OCH_3)_3-C_6H_2-$ | 0 | 266.6 |
| 243-c | 27-c | H- | -CH=N- | $i-C_3H_7-$ | 6 | H- | 3-thienyl- | 1 | - |
| 244-c | 32-c | H- | -CH=N- | $i-C_3H_7-$ | 6 | H- | 3-thienyl- | 0 | 269.9 |
| 245-c | 27-c | H- | -CH=CH- | $i-C_3H_7-$ | 6 | H- | 2-thienyl- | 1 | - |
| 246-c | 32-c | H- | -CH=CH- | $i-C_3H_7-$ | 6 | H- | 2-thienyl- | 0 | 276.0 |
| 247-c | 27-c | H- | -CH=CH- | $i-C_3H_7-$ | 6 | H- | $2-OCH_3-C_6H_4-$ | 1 | - |
| 248-c | 32-c | H- | -CH=CH- | $i-C_3H_7-$ | 6 | H- | $2-OCH_3-C_6H_4-$ | 0 | 169.8 |
| 249-c | 27-c | H- | -CH=CH- | $i-C_3H_7-$ | 6 | H- | 1-naphthalenyl- | 1 | - |
| 250-c | 32-c | H- | -CH=CH- | $i-C_3H_7-$ | 6 | H- | 1-naphthalenyl- | 0 | 183.5 |
| 251-c | 27-c | H- | -CH=CH- | $i-C_3H_7-$ | 6 | H- | $4-OC_2H_5-C_6H_4-$ | 1 | - |
| 252-c | 32-c | H- | -CH=CH- | $i-C_3H_7-$ | 6 | H- | $4-OC_2H_5-C_6H_4-$ | 0 | 129.5 |
| 253-c | 27-c | H- | -CH=CH- | $i-C_3H_7-$ | 6 | H- | $2-Cl-C_6H_4-$ | 1 | - |
| 254-c | 32-c | H- | -CH=CH- | $i-C_3H_7-$ | 6 | H- | $2-Cl-C_6H_4-$ | 0 | 172.5 |
| 255-c | 27-c | H- | -CH=CH- | $i-C_3H_7-$ | 6 | H- | $3-OH-C_6H_4-$ | 1 | - |
| 256-c | 32-c | H- | -CH=CH- | $i-C_3H_7-$ | 6 | H- | $3-OH-C_6H_4-$ | 0 | 252.6 |
| 257-c | 27-c | H- | -CH=CH- | $i-C_3H_7-$ | 6 | H- | $3-Br-C_6H_4-$ | 1 | - |
| 258-c | 32-c | H- | -CH=CH- | $i-C_3H_7-$ | 6 | H- | $3-Br-C_6H_4-$ | 0 | $157.8/\frac{1}{2}H_2O$ |

| Comp. No. | Ex. No. | R | $-X^1=X^2-$ | Y | p | $R^{26}$ | $R^{27}$ | n | mp. °C base/salt |
|---|---|---|---|---|---|---|---|---|---|
| 259-c | 20-c | H- | -CH=N- | i-$C_3H_7$- | 7 | H- | 3-pyridinyl- | 0 | 298.7 |
| 260-c | 23-c | H- | -CH=N- | i-$C_3H_7$- | 7 | H- | $C_3H_7$- | 0 | 188.5 |
| 261-c | 27-c | H- | -CH=CH- | i-$C_3H_7$- | 6 | H- | 3-thienyl- | 1 | - |
| 262-c | 32-c | H- | -CH=CH- | i-$C_3H_7$- | 6 | H- | 3-thienyl- | 0 | 273.1 |
| 263-c | 23-c | H- | -CH=N- | i-$C_3H_7$- | 6 | H- | $C_3H_7$- | 0 | 187.3 |
| 264-c | 23-c | H- | -CH=N- | i-$C_3H_7$- | 6 | H- | $C_2H_5$- | 0 | 194.8 |
| 265-c | 27-c | H- | -CH=N- | i-$C_3H_7$- | 6 | H- | 2-thienyl- | 1 | - |
| 266-c | 32-c | H- | -CH=N- | i-$C_3H_7$- | 6 | H- | 2-thienyl- | 0 | >300 (dec.) |
| 267-c | 27-c | H- | -CH=CH- | i-$C_3H_7$- | 6 | H- | 2Cl-6F-$C_6H_3$- | 1 | - |
| 268-c | 32-c | H- | -CH=CH- | i-$C_3H_7$- | 6 | H- | 2Cl-6F-$C_6H_3$- | 0 | 165.4 |
| 269-c | 27-c | H- | -CH=CH- | i-$C_3H_7$- | 6 | H- | 3Br-4OH-$C_6H_3$- | 1 | - |
| 270-c | 32-c | H- | -CH=CH- | i-$C_3H_7$- | 6 | H- | 3Br-4OH-$C_6H_3$- | 0 | 241.4 |
| 271-c | 20-c | H- | -CH=CH- | i-$C_3H_7$- | 7 | H- | 3-pyridinyl- | 0 | 294.2 |
| 272-c | 27-c | H- | -CH=N- | $C_2H_5$- | 6 | H- | $C_6H_5$- | 1 | - |
| 273-c | 32-c | H- | -CH=N- | $C_2H_5$- | 6 | H- | $C_6H_5$- | 0 | 272.0 |
| 274-c | 27-c | H- | -CH=N- | i-$C_4H_9$- | 6 | H- | $C_6H_5$- | 1 | - |
| 275-c | 32-c | H- | -CH=N- | i-$C_4H_9$- | 6 | H- | $C_6H_5$- | 0 | 220.1 |
| 276-c | 27-c | H- | -N=CH- | i-$C_3H_7$- | 6 | H- | 4F-$C_6H_4$- | 1 | - |
| 277-c | 32-c | H- | -N=CH- | i-$C_3H_7$- | 6 | H- | 4F-$C_6H_4$- | 0 | 250.0 |
| 278-c | 27-c | H- | -CH=N- | $C_4H_9$- | 6 | H- | 3F-$C_6H_4$- | 1 | - |
| 279-c | 32-c | H- | -CH=N- | $C_4H_9$- | 6 | H- | 3F-$C_6H_4$- | 0 | 132.9 |
| 280-c | 23-c | H- | -CH=N- | i-$C_3H_7$- | 7 | H- | $C_2H_5$- | 0 | 163.9/0.5$H_2O$ |
| 281-c | 27-c | H- | -CH=CH- | i-$C_3H_7$- | 6 | H- | 3Cl-4OH-$C_6H_3$- | 1 | - |
| 282-c | 32-c | H- | -CH=CH- | i-$C_3H_7$- | 6 | H- | 3Cl-4OH-$C_6H_3$- | 0 | 237.0 |
| 283-c | 20-c | H- | -CH=N- | i-$C_3H_7$- | 6 | H- | 3-pyridinyl- | 0 | 236.3 |
| 284-c | 27-c | H- | -N=CH- | i-$C_3H_7$- | 6 | H- | $C_6H_5$- | 1 | - |
| 285-c | 32-c | H- | -N=CH- | i-$C_3H_7$- | 6 | H- | $C_6H_5$- | 0 | 210.2 |
| 286-c | 27-c | H- | -CH=N- | $C_3H_7$- | 6 | H- | 2-$CH_3$-$C_6H_4$- | 1 | - |
| 287-c | 32-c | H- | -CH=N- | $C_3H_7$- | 6 | H- | 2-$CH_3$-$C_6H_4$- | 0 | 230.8 |
| 288-c | 27-c | H- | -N=CH- | i-$C_3H_7$- | 6 | H- | 3Cl-$C_6H_4$- | 1 | - |
| 289-c | 32-c | H- | -N=CH- | i-$C_3H_7$- | 6 | H- | 3Cl-$C_6H_4$- | 0 | 176.7 |
| 290-c | 27-c | H- | -CH=N- | i-$C_4H_9$- | 6 | H- | 2$CH_3$-$C_6H_4$- | 1 | - |
| 291-c | 32-c | H- | -CH=N- | i-$C_4H_9$- | 6 | H- | 2$CH_3$-$C_6H_4$- | 0 | 168.3 |

| Comp. No. | Ex. No. | R | $-X^1=X^2-$ | Y | p | $R^{26}$ | $R^{27}$ | n | mp. °C base/salt |
|---|---|---|---|---|---|---|---|---|---|
| 292-c | 27-c | H- | -CH=N- | $C_2H_5$- | 6 | H- | $2CH_3$-$C_6H_4$- | 1 | - |
| 293-c | 32-c | H- | -CH=N- | $C_2H_5$- | 6 | H- | $2CH_3$-$C_6H_4$- | 0 | 187.0 |
| 294-c | 27-c | H- | -CH=N- | i-$C_3H_7$- | 6 | H- | 3Cl-$C_6H_4$- | 1 | - |
| 295-c | 32-c | H- | -CH=N- | i-$C_3H_7$- | 6 | H- | 3Cl-$C_6H_4$- | 0 | 183.6 |
| 296-c | 27-c | H- | -N=CH- | i-$C_3H_7$- | 6 | H- | 3F-$C_6H_4$- | 1 | - |
| 297-c | 32-c | H- | -N=CH- | i-$C_3H_7$- | 6 | H- | 3F-$C_6H_4$- | 0 | 213.2 |
| 298-c | 27-c | H- | -CH=N- | i-$C_3H_7$- | 6 | H- | 2F-$C_6H_4$- | 1 | - |
| 299-c | 32-c | H- | -CH=N- | i-$C_3H_7$- | 6 | H- | 2F-$C_6H_4$- | 0 | 125.8 |
| 300-c | 27-c | H- | -CH=N- | $C_4H_9$- | 6 | H- | $C_6H_5$- | 1 | - |
| 301-c | 32-c | H- | -CH=N- | $C_4H_9$- | 6 | H- | $C_6H_5$- | 0 | 183.3 |
| 302-c | 27-c | H- | -CH=N- | $C_4H_9$- | 6 | H- | 2-$CH_3$-$C_6H_4$- | 1 | - |
| 303-c | 32-c | H- | -CH=N- | $C_4H_9$- | 6 | H- | 2-$CH_3$-$C_6H_4$- | 0 | 176.4 |
| 304-c | 27-c | H- | -CH=N- | $C_3H_7$- | 6 | H- | 3F-$C_6H_4$- | 1 | - |
| 305-c | 32-c | H- | -CH=N- | $C_3H_7$- | 6 | H- | 3F-$C_6H_4$- | 1 | 210.5 |
| 306-c | 27-c | H- | -CH=N- | $C_3H_7$- | 6 | H- | $C_6H_5$- | 1 | - |
| 307-c | 32-c | H- | -CH=N- | $C_3H_7$- | 6 | H- | $C_6H_5$- | 0 | 206.3 |
| 308-c | 27-c | H- | -CH=CH- | $C_2H_5$- | 6 | H- | 2-$CH_3$-$C_6H_4$- | 1 | - |
| 309-c | 32-c | H- | -CH=CH- | $C_2H_5$- | 6 | H- | 2-$CH_3$-$C_6H_4$- | 0 | 202.2 |
| 310-c | - | H- | -N=CH- | i-$C_3H_7$- | 6 | H- | $CH_3$- | 0 | - |
| 311-c | - | H- | -CH=CH- | 1<u>H</u>-imidazolyl | 6 | H- | $CH_3$- | 0 | - |
| 312-c | - | H- | -CH=CH- | i-$C_3H_7$- | 6 | $C_6H_5$-$CH_2$- | $CH_3$- | 0 | - |
| 313-c | - | H- | -CH=CH- | 3Cl-$C_6H_4$- | 7 | H- | i-$C_3H_7$- | 0 | - |
| 314-c | - | H- | -CH=CH- | i-$C_3H_7$- | 7 | H- | $C_2H_5$- | 0 | - |

Table 10-c

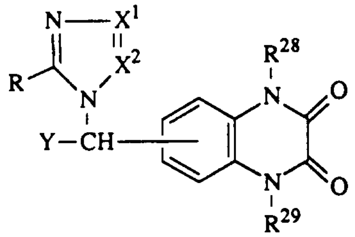

| Comp. No. | Ex. No. | R | $X^1$=$X^2$ | Y | p | $R^{28}$ | $R^{29}$ | mp.(°C) / base / salt |
|---|---|---|---|---|---|---|---|---|
| 315-c | 29-c | H- | CH=CH | H- | 6 | H- | H- | >300 |
| 316-c | - | H- | CH=CH | $C_6H_5$- | 6 | $CH_3$- | $CH_3$- | - |
| 317-c | - | H- | CH=CH | i-$C_3H_7$- | 6 | $CH_3$- | $CH_3$- | - |

C-c) Pharmacological Examples

The useful pharmacological properties of the compounds of the present invention can for example be demonstrated by the following experiment.

Example 39-c

Metabolism of exogenous all-trans-retinoic acid

Male Wistar rats weighing 200~210 g were orally treated with vehicle (PEG 200) or with 40 mg/kg of a compound of formula (I-c). One hour later, the animals were anesthetized with ether and injected intrajugularly with 0.50 ml saline solution containing 20 μg of all-trans-retinoic acid. Two hours after this injection, rats were killed by decapitation and blood was collected on heparin. Blood samples were centrifuged (1000 g, 15 min) and plasma was recovered to determine the quantity of plasmatic all-trans-retinoic acid. The samples were analyzed by means of HPLC with UV-detection at 350 nm. Quatification was achieved by peak area integration and external standardization. Under the conditions used, plasma concentrations of the retinoic acid in vehicle-pretreated animals were not detectable (<0.5 ng/ml), whereas compound nos. 5-c, 9-c, 11-c, 12-c, 13-c, 15-c, 16-c, 18-c, 57-c, 68-c, 69-c, 70-c, 86-c, 89-c, 94-c, 97-c, 103-c, 123-c, 132-c, 133-c, 134-c, 141-c, 146-c, 147-c, 148-c, 149-c, 151-c, 157-c, 161-c, 181-c, 183-c, 187-c, 198-c, 201-c, 210-c, 262-c, 263-c, 264-c, 295-c and 299-c enhanced the recovery of all-trans-retinoic acid from the plasma to a least 10 ng/ml after dosing with 40 mg/kg. The following compounds even enhanced the recovery of all trans-retinoic acid from the plasma to at least 20 ng/ml after dosing with 40 mg/kg : compound nos. 12-c, 70-c, 77-c, 86-c, 138-c and 146-c.

Example 40-c

Metabolism of endogenous all-trans-retinoic acid

Male Wistar rats weighing 200~210 g were orally treated with vehicle (PEG 200) or with 40 mg/kg of a compound of formula (I-c). Two hours after drug administration, the rats were killed by decapitation and blood was collected on heparin. Blood samples were centrifuged (1000 g, 15 min) and plasma was recovered to determine the quantity of plasmatic all-trans-retinoic acid. The samples were analyzed by means of HPLC with UV-detection at 350 nm. Quatification was achieved by peak area integration and external standardization. Under the conditions used, plasma concentrations of the retinoic acid in vehicle-pretreated animals were not detectable (<0.5 ng/ml), whereas compound nos. 5-c, 77-c, 94-c, 127-c, 151-c, 170-c, 183-c, 187-c, 190-c, 197-c, 201-c, 205-c, 208-c, 210-c, 212-c, 216-c, 218-c, 232-c, 246-c, 259-c, 260-c, 262-c, 263-c, 264-c, 266-c, 271-c, 273-c, 275-c, 277-c, 279-c, 280-c, 285-c, 287-c, 289-c, 291-c, 293-c, 295-c, 299-c, 301-c, 307-c and 309-c enhanced the recovery of all-trans-retinoic acid from the plasma to a least 1 ng/ml.

D) Composition Examples

The following formulations exemplify typical pharmaceutical compositions in dosage unit form suitable for systemic administra-tion to animal and human subjects in accordance with the present invention. "Active ingredient" (A.I.) as used throughout these examples relates to a compound of formula (I), a N-oxide form, a

pharmaceutically acceptable acid addition salt or a stereochemically isomeric form thereof.

Example 41 : ORAL DROPS

500 g of the A.I. was dissolved in 0.5 l of 2-hydroxypropanoic acid and 1.5 l of the polyethylene glycol at 60~80 °C. After cooling to 30~40 °C there were added 35 l of polyethylene glycol and the mixture was stirred well. Then there was added a solution of 1750 g of sodium saccharin in 2.5 l of purified water and while stirring there were added 2.5 l of cocoa flavor and polyethylene glycol q.s. to a volume of 50 l, providing an oral drop solution comprising 0.01 g of the A.I. per ml. The resulting solution was filled into suitable containers.

Example 42 : ORAL SOLUTION

9 g of methyl 4-hydroxybenzoate and 1 part of propyl 4-hydroxybenzoate were dissolved in 4 l of boiling purified water. In 3 l of this solution were dissolved first 10 g of 2,3-dihydroxybutanedioic acid and thereafter 20 g of the A.I. The latter solution was combined with the remaining part of the former solution and 12 l 1,2,3-propanetriol and 3 l of sorbitol 70% solution were added thereto. 40 g of sodium saccharin were dissolved in 0.5 l of water and 2 ml of raspberry and 2 ml of gooseberry essence were added. The latter solution was combined with the former, water was added q.s. to a volume of 20 l providing an oral solution comprising 0.005 g of the A.I. per teaspoonful (5 ml). The resulting solution was filled in suitable containers.

Example 43: CAPSULES

20 g of the A.I., 6 g sodium lauryl sulfate, 56 g starch, 56 g lactose, 0.8 g colloidal silicon dioxide, and 1.2 g magnesium stearate were vigorously stirred together. The resulting mixture was subsequently filled into 1000 suitable hardened gelatin capsules, each comprising 0.02 g of the A.I.

Example 44 : FILM-COATED TABLETS

Preparation of tablet core

A mixture of 100 g of the A.I., 570 g lactose and 200 g starch was mixed well and thereafter humidified with a solution of 5 g sodium dodecyl sulfate and 10 g polyvinylpyrrolidone (Kollidon-K 90 ®) in about 200 ml of water. The wet powder mixture was sieved, dried and sieved again. Then there was added 100 g microcrystalline cellulose (Avicel ®) and 15 g hydrogenated vegetable oil (Sterotex ®). The whole was mixed well and compressed into tablets, giving 10.000 tablets, each comprising 0.01 g of the active ingredient.

Coating

To a solution of 10 g methyl cellulose (Methocel 60 HG ®) in 75 ml of denaturated ethanol there was added a solution of 5 g of ethyl cellulose (Ethocel 22 cps ®) in 150 ml of dichloromethane. Then there were added 75 ml of dichloromethane and 2.5 ml 1,2,3-propanetriol. 10 g of polyethylene glycol was molten and dissolved in 75 ml of dichloromethane. The latter solution was added to the former and then there were added 2.5 g of magnesium octadecanoate, 5 g of polyvinylpyrrolidone and 30 ml of concentrated color suspension (Opaspray K-1-2109 ®) and the whole was homogenated. The tablet cores were coated with the thus obtained mixture in a coating apparatus.

Example 45 : INJECTABLE SOLUTION

1.8 g methyl 4-hydroxybenzoate and 0.2 g propyl 4-hydroxybenzoate were dissolved in about 0.5 l of boiling water for injection. After cooling to about 50 °C there were added while stirring 4 g lactic acid, 0.05 g propylene glycol and 4 g of the A.I. The solution was cooled to room temperature and supplemented with water for injection q.s. ad 1 l volume, giving a solution of 0.004 g A.I. per ml. The solution was sterilized by filtration (U.S.P. XVII p. 811) and filled in sterile containers.

Example 46 : SUPPOSITORIES

3 g A.I. was dissolved in a solution of 3 g 2,3-dihydroxy-butanedioic acid in 25 ml polyethylene glycol 400. 12 G surfactant (SPAN ®) and triglycerides (Witepsol 555 ®) q.s. ad 300 g were molten together. The latter mixture was mixed well with the former solution. The thus obtained mixture was poured into moulds at a temperature of 37~38 ° C to form 100 suppositories each containing 0.03 g of the active ingredient.

Example 47 : 2% cream

75 mg Stearyl alcohol, 2 mg cetyl alcohol, 20 mg sorbitan monostearate and 10 mg isopropyl myristate are introduced into a doublewall jacketed vessel and heated until the mixture has completely molten. This mixture is added to a separately prepared mixture of purified water, 200 mg propylene glycol and 15 mg polysorbate 60 having a temperature of 70 to 75 ° C while using a homogenizer for liquids. The resulting emulsion is allowed to cool to below 25 ° C while continuously mixing. A solution of 20 mg A.I., 1 mg polysorbate 80 and purified water and a solution of 2 mg sodium sulfite anhydrous in purified water are next added to the emulsion while continuously mixing. The cream, 1 g of the A.I. is homogenized and filled into suitable tubes.

Example 48 : 2% topical gel

To a solution of 200 mg ydroxypropyl β-cyclodextrine in purified water is added 20 mg of A.I. while stirring. Hydrochloric acid is added until complete solution and then sodium hydroxide is added until pH 6.0. This solution is added to a dispersion of 10 mg carrageenan PJ in 50 mg propylene glycol while mixing. While mixing slowly the mixture is heated to 50 ° C and allowed to cool to about 35 ° C whereupon the 50 mg ethyl alcohol 95% (v/v) is added. The rest of the purified water q.s. ad 1 g is added and the mixture is mixed to homogenous.

Example 49 : 2% topical cream

To a solution of 200 mg hydroxypropyl β-cyclodextrine in purified water is added 20 mg of A.I. while stirring. Hydrochloric acid is added until complete solution and next sodium hydroxide is added until pH 6.0. While stirring, 50 mg glycerol and 35 mg polysorbate 60 are added and the mixture is heated to 70 ° C. The resulting mixture is added to a mixture of 100 mg mineral oil, 20 mg stearyl alcohol, 20 mg cetyl alcohol, 20 mg glycerol monostearate and 15 mg sorbate 60 having a temperature of 70 ° C while mixing slowly. After cooling down to below 25 ° C, the rest of the purified water q.s. ad 1 g is added and the mixture is mixed to homogenous.

Example 50 : 2% liposome formulation

A mixture of 2 g A.I. microfine, 20 g phosphatidyl choline, 5 g cholesterol and 10 g ethyl alcohol is stirred and heated at 55-60 ° C until complete solution and is added to a solution of 0.2 g methyl paraben, 0.02 g propyl paraben, 0.15 g disodium edetate and 0.3 g sodium chloride in purified water while homogenizing. 0.15 g Hydroxypropylmethylcellulose in purified water ad 100 g is added and the mixing is continued until swelling is complete.

Example 51 : 2% liposome formulation

A mixture of 10 g phosphatidyl choline and 1 g cholesterol in 7.5 g ethyl alcohol is stirred and heated at 40 ° C until complete solution. 2 g A.I. microfine is dissolved in purified water by mixing while heating at 40 ° C. The alcoholic solution is added slowly to the aqueous solution while homogenizing during 10 minutes. 1.5 g Hydroxypropylmethylcellulose in purified water is added while mixing until swelling is complete. The resulting solution is adjusted to pH 5.0 with sodium hydroxide 1 N and diluted with the rest of the purified water ad 100 g

## Claims

**Claims for the following Contracting States : AT, BE, CH, DE, FR, GB, IT, LI, LU, NL, SE**

**1.** A chemical compound having the formula

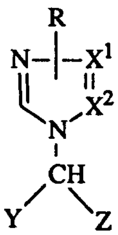

(I)

a pharmaceutical acceptable acid addition salt thereof or a stereochemically isomeric form thereof, wherein

-$X^1$ = $X^2$- is a bivalent radical having the formula

-CH = CH- (x),

-CH = N- (y), or

-N = CH- (z);

R is hydrogen or $C_{1-6}$alkyl;

Y is hydrogen, $C_{1-10}$alkyl, $C_{3-7}$cycloalkyl, $Ar^1$, $Ar^2$-$C_{1-6}$alkyl, $C_{2-6}$alkenyl or $C_{2-6}$alkynyl;

Z is a radical of formula

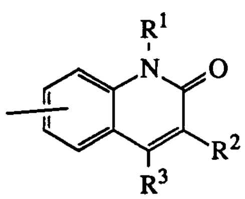

(a-1) ,

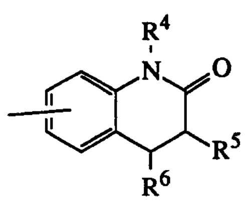

(a-2) ,

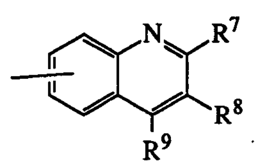

(a-3) or

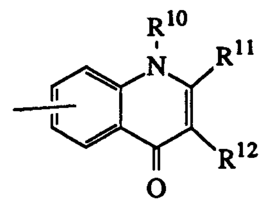

(a-4)

wherein

$R^1$, $R^4$ and $R^{10}$ each independently are hydrogen, $C_{1-6}$alkyl or $Ar^2$-$C_{1-6}$alkyl;

$R^2$, $R^5$, $R^8$ and $R^{12}$ each independently are hydrogen, $C_{1-6}$alkyl or $Ar^2$;

$R^3$, $R^6$ and $R^{11}$ each independently are hydrogen or $C_{1-6}$alkyl;

$R^7$ and $R^9$ each independently are hydrogen, $C_{1-6}$alkyl, $C_{1-6}$alkyloxy, halo, amino, or mono or di-($C_{1-6}$alkyl)amino; or

Z is a radical of formula

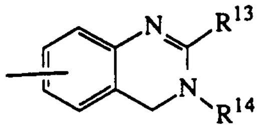, 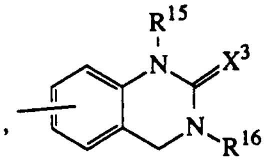, 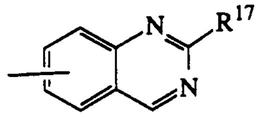,

(b-1) (b-2) (b-3)

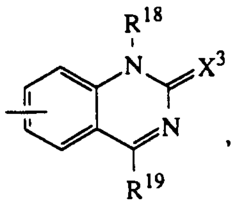, 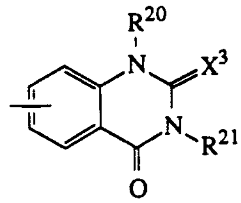 or 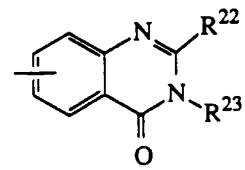

(b-4) (b-5) (b-6)

wherein

$R^{13}$, $R^{17}$ and $R^{22}$ each independently are hydrogen, halo, $C_{1-6}$alkyl, trifluoromethyl, $C_{1-6}$alkyloxy, $Ar^2$, $Ar^2$-$C_{1-6}$alkyl, amino, or mono- or di($C_{1-6}$alkyl)amino;

$R^{14}$, $R^{16}$ and $R^{21}$ each independently are hydrogen, $C_{1-6}$alkyl, $Ar^2$ or $Ar^2$-$C_{1-6}$alkyl;

$R^{15}$, $R^{18}$ and $R^{20}$ each independently are hydrogen, $C_{1-6}$alkyl or $Ar^2$-$C_{1-6}$alkyl;

$R^{19}$ is hydrogen or $C_{1-6}$alkyl;

$R^{23}$ is hydrogen, $C_{1-6}$alkyl, $Ar^2$-$C_{1-6}$alkyl, amino or mono($C_{1-6}$alkyl)amino;

$X^3$ is O or S; or

Z is a radical of formula

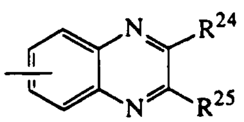, 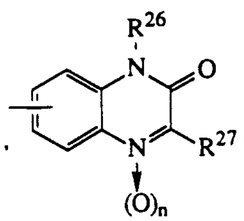, 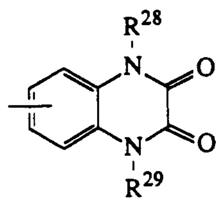, 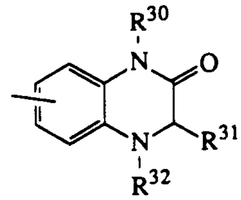, 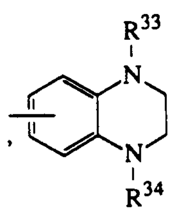

(c-1) (c-2) (c-3) (c-4) (c-5)

wherein

$R^{24}$ is hydrogen, halo, $C_{1-6}$alkyl, $C_{1-6}$alkyloxy, amino, mono- or di($C_{1-6}$alkyl)amino, $Ar^2$ or imidazolyl;

$R^{25}$ is hydrogen, $C_{1-6}$alkyl or $Ar^1$;

$R^{26}$ and $R^{30}$ each independently are hydrogen, $C_{1-6}$alkyl, $Ar^2$-$C_{1-6}$alkyl, amino or mono($C_{1-6}$alkyl)amino;

$R^{27}$ and $R^{31}$ each independently are hydrogen, $C_{1-6}$alkyl, $Ar^1$, $C_{1-6}$alkylcarbonyl, $Ar^2$-carbonyl, $C_{1-6}$alkyloxycarbonyl, carboxyl, $C_{1-6}$alkyloxycarbonyl-$C_{1-4}$alkyl, aminocarbonyl or cyano;

$R^{28}$, $R^{29}$, $R^{32}$, $R^{33}$ and $R^{34}$ each independently are hydrogen, $C_{1-6}$alkyl or $Ar^2$-$C_{1-6}$alkyl;

n is 0 or 1; and

$Ar^1$ is phenyl, substituted phenyl, naphthalenyl, pyridinyl, imidazolyl, triazolyl, thienyl, furanyl or thiazolyl and $Ar^2$ is phenyl or substituted phenyl; said substituted phenyl in $Ar^1$ or $Ar^2$ being phenyl substituted with 1, 2 or 3 substituents each independently selected from halo, hydroxy, trifluoromethyl, $C_{1-6}$alkyl, $C_{1-6}$alkyloxy, cyano, amino, mono- and di($C_{1-6}$alkyl)amino, nitro, carboxyl, formyl and $C_{1-6}$alkyloxycarbonyl.

**2.** A compound according to claim 1 wherein R is hydrogen or $C_{1-4}$alkyl; Y is hydrogen, $C_{1-6}$alkyl, $C_{3-7}$cycloalkyl, phenyl, substituted phenyl, pyridinyl, imidazolyl or thienyl; and Z is a radical of formula (a-1), (a-2), (a-3) or (a-4) wherein $R^1$, $R^2$, $R^3$, $R^4$, $R^5$, $R^6$, $R^8$, $R^{10}$, $R^{11}$ and $R^{12}$ each independently are hydrogen or $C_{1-4}$alkyl, and $R^7$ and $R^9$ each independently are hydrogen, $C_{1-4}$alkyl, $C_{1-4}$alkyloxy or halo; or Z is a radical of formula (b-1), (b-2), (b-3), (b-4), (b-5), or (b-6) wherein $R^{13}$ is hydrogen, $C_{1-4}$alkyl, trifluoromethyl or phenyl, $R^{14}$ is hydrogen, $C_{1-4}$alkyl, phenyl or phenyl$C_{1-4}$alkyl, $R^{15}$ is hydrogen or $C_{1-4}$alkyl substituted with phenyl, $R^{16}$, $R^{17}$, $R^{18}$, $R^{19}$ and $R^{20}$ each independently are hydrogen or $C_{1-4}$alkyl, $R^{21}$ is hydrogen, $C_{1-4}$alkyl or phenyl$C_{1-4}$alkyl, $R^{22}$ is hydrogen, amino or mono or di($C_{1-4}$alkylamino), and $R^{23}$ is hydrogen; or Z is a radical of formula (c-1), (c-2), (c-3), (c-4) or (c-5) wherein $R^{24}$ is hydrogen, $C_{1-4}$alkyl, halo, $C_{1-4}$alkyloxy, amino, mono- or di($C_{1-4}$alkyl)amino, phenyl, substituted phenyl or imidazolyl, $R^{25}$ is hydrogen, $C_{1-4}$alkyl, phenyl or substituted phenyl, $R^{26}$ is hydrogen, $C_{1-4}$alkyl, amino, $C_{1-4}$alkylamino or $C_{1-4}$alkyl substituted with phenyl or substituted phenyl; $R^{27}$ is hydrogen, $C_{1-4}$alkyl, $C_{1-4}$alkyloxycarbonyl$C_{1-4}$alkyl, phenyl, substituted phenyl, carboxyl, $C_{1-4}$alkyloxycarbonyl, phenylcarbonyl, substituted phenylcarbonyl, naphthalenyl, thienyl, furanyl, pyridinyl or imidazolyl; $R^{28}$ and $R^{29}$ each independently are hydrogen or $C_{1-4}$alkyl; $R^{30}$ is hydrogen, $C_{1-4}$alkyl, amino or $C_{1-4}$alkyl substituted with phenyl or substituted phenyl; $R^{31}$ is hydrogen, $C_{1-4}$alkyl, phenyl, substituted phenyl, $C_{3-7}$cycloalkyl, naphthalenyl, thienyl, pyridinyl or imidazolyl; $R^{32}$ is hydrogen or $C_{1-4}$alkyl and $R^{33}$ and $R^{34}$ are both hydrogen.

**3.** A compound according to claim 2 wherein $-X^1=X^2-$ is a radical of formula (x) or (y); and Y is hydrogen, $C_{1-4}$alkyl, cyclopropyl, cyclopentyl, cyclohexyl, imidazolyl, pyridinyl, thienyl or phenyl optionally substituted with one or two substituents each independently selected from halo, $C_{1-4}$alkyl, $C_{1-4}$alkyloxy and trifluoromethyl.

**4.** A compound according to claim 1 wherein

Z is a radical of formula (a-1) wherein $R^1$ and $R^2$ are hydrogen, $R^3$ is hydrogen or $C_{1-4}$alkyl and Y is hydrogen, $C_{1-4}$alkyl or phenyl optionally substituted with one or two halo atoms; or

Z is a radical of formula (a-2) wherein $R^4$, $R^5$ and $R^6$ all being hydrogen, and Y is hydrogen, $C_{1-4}$alkyl, cyclopropyl or phenyl optionally substituted with one or two halo atoms; or

Z is a radical of formula (a-3) wherein $R^7$ is hydrogen, halo or $C_{1-4}$alkyloxy, $R^8$ is hydrogen, $R^9$ is hydrogen, $C_{1-4}$alkyl or $C_{1-4}$alkyloxy and Y is hydrogen, $C_{1-4}$alkyl, cyclopropyl, cyclohexyl, imidazolyl, thienyl or phenyl optionally substituted with one or two substituents each independently selected from halo, $C_{1-4}$alkyl, $C_{1-4}$alkyloxy and trifluoromethyl; or

Z is a radical having the formula (a-4) wherein $R^{10}$ and $R^{12}$ are hydrogen, $R^{11}$ is $C_{1-4}$alkyl and Y is hydrogen; or

Z is a radical of formula (b-2) wherein $R^{15}$ is hydrogen, $R^{16}$ is hydrogen or $C_{1-4}$alkyl and Y is hydrogen, $C_{1-4}$alkyl, pyridinyl or phenyl optionally substituted with one or two halo atoms; or

Z is a radical of formula (b-3) wherein $R^{17}$ is $C_{1-4}$alkyl and Y is phenyl or halophenyl; or

Z is a radical of formula (b-5) wherein $R^{20}$ is hydrogen, $R^{21}$ is hydrogen, $C_{1-4}$alkyl or phenyl$C_{1-4}$alkyl and Y is hydrogen, phenyl or halophenyl; or

Z is a radical of formula (b-6) wherein $R^{22}$ is hydrogen or amino, $R^{23}$ is hydrogen and Y is hydrogen, phenyl or halophenyl; or

Z is a radical of formula (c-1) wherein $R^{24}$ is hydrogen, $C_{1-4}$alkyl, $C_{1-4}$alkyloxy, halo, amino, di($C_{1-4}$alkyl)amino, phenyl or imidazolyl, $R^{25}$ is hydrogen, $C_{1-4}$alkyl or phenyl and Y is hydrogen, $C_{1-4}$alkyl, thienyl, imidazolyl or phenyl optionally substituted with one or two substituents selected from halo, $C_{1-4}$alkyl, $C_{1-4}$alkyloxy or trifluoromethyl; or

Z is a radical of formula (c-2) wherein $R^{26}$ is hydrogen, $C_{1-4}$alkyl, amino or $C_{1-4}$alkyl substituted with phenyl and $R^{27}$ is hydrogen, $C_{1-4}$alkyl, carboxyl, $C_{1-4}$alkyloxycarbonyl, naphthalenyl, thienyl, pyridinyl, imidazolyl, phenyl or phenyl substituted with 1, 2 or 3 substituents each independently selected from $C_{1-4}$alkyl,

$C_{1-4}$alkyloxy, halo, hydroxy and trifluoromethyl and Y is hydrogen, $C_{1-4}$alkyl, cyclopropyl, cyclopentyl, cyclohexyl, imidazolyl, thienyl, pyridinyl or phenyl optionally substituted with one or two substituents selected from halo, $C_{1-4}$alkyl, $C_{1-4}$alkyloxy and trifluoromethyl.

**5.** A compound according to claim 1 wherein Z is a radical of formula (a-1); R is hydrogen; $-X^1=X^2-$ is a radical of formula (x) or (y); Y is isopropyl, phenyl or halophenyl; $R^1$ and $R^2$ are both hydrogen; and $R^3$ is methyl; or wherein Z is a radical of formula (a-2); R is hydrogen; $-X^1=X^2-$ is a radical of formula (x) or (y); Y is cyclopropyl, phenyl or halophenyl and $R^4$, $R^5$ and $R^6$ are all hydrogen; or wherein Z is a

radical of formula (a-3); R is hydrogen; $X^1=X^2$- is a radical of formula (x) or (y); Y is phenyl, halophenyl, dihalophenyl, methoxyphenyl or cyclohexyl.

**6.** A compound according to claim 1 wherein Z is a radical of formula (b-2); $-X^1=X^2$-is a radical of formula (x) or (y); R is hydrogen; $R^{15}$ is hydrogen; $R^{16}$ is hydrogen; and Y is phenyl, halophenyl or propyl; or wherein Z is a radical of formula (b-5); $-X^1=X^2$-is a radical of formula (x) or (y); R is hydrogen; $R^{20}$ is hydrogen; $R^{21}$ is hydrogen, methyl or $C_{1-4}$alkylphenyl; and Y is phenyl or halophenyl; or wherein Z is radical of formula (b-6); $-X^1=X^2$- is a radical of formula (x) or (y); R is hydrogen; $R^{22}$ is hydrogen; $R^{23}$ is hydrogen; and Y is phenyl or halophenyl.

**7.** A compound according to claim 1 wherein Z is a radical of formula (c-1); $-X^1=X^2$-is a radical of formula (x) or (y); R is hydrogen; $R^{24}$ and $R^{25}$ are both hydrogen and Y is phenyl or halophenyl; or wherein Z is a radical of formula (c-2); $-X^1=X^2$- is a radical of formula (x) or (y); R is hydrogen; Y is hydrogen, $C_{1-4}$alkyl, cyclopropyl, cyclopentyl or cyclohexyl; $R^{26}$ is hydrogen; $R^{27}$ is hydrogen, $C_{1-4}$alkyl, naphthalenyl, thienyl, pyridinyl, imidazolyl, phenyl or phenyl substituted with 1 or 2 substituents each independently selected from methyl, halo, hydroxy and methoxy; and n is 0; or wherein Z is a radical of formula (c-2); $-X^1=X^2$- is a radical of formula (x) or (y); Y is phenyl or halophenyl; $R^{26}$ is hydrogen; $R^{27}$ is hydrogen or $C_{1-4}$alkyl; and n is 0.

**8.** A pharmaceutical composition comprising a suitable pharmaceutical carrier and as active ingredient a therapeutically effective amount of a chemical compound as claimed in any of claims 1 to 7.

**9.** A method of preparing a pharmaceutical composition as claimed in claim 8, characterized in that a therapeutically effective amount of a compound as claimed in any of claims 1 to 7 is intimately mixed with a suitable pharmaceutical carrier.

**10.** A compound as claimed in any one of claims 1 to 7 for use as a medicine.

**11.** A process for preparing a chemical compound as claimed in any of claims 1 to 7, characterized by
a) N-alkylating an azole of formula (II) or an alkali metal salt thereof

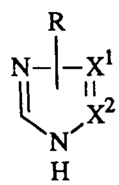 (II)

wherein R, $X^1$, $X^2$ are as defined under formula (I) with a quinoline, quinolinone, quinazoline or quinoxaline derivative of formula

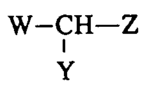 (III)

wherein Y and Z are as defined under formula (I) and W represents a reactive leaving group;
b) N-alkylating an azole of formula

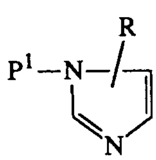 (II-x)

wherein R is as defined under formula (I) and $P^1$ represents a protective group with a quinoline, quinolinone, quinazoline or quinoxaline derivative of formula

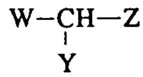 (III)

wherein Y and Z are as defined under formula (I) and W represents a reactive leaving group, thus obtaining a compound of formula

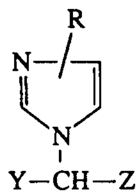 (I-x) ;

c) endo-N-alkylating an azole of formula

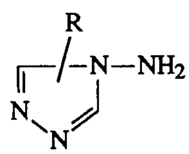 (II-y)

wherein R is as defined under formula (I) with a quinoline, quinolinone, quinazoline or quinoxaline derivative of formula

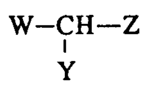 (III)

wherein Y and Z are as defined under formula (I) and W represents a reactive leaving group, subsequently deaminating the thus obtained triazolium salt,

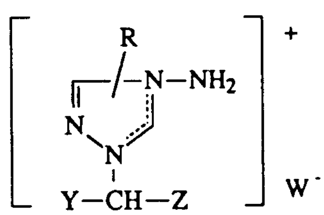

thus preparing a compound of formula

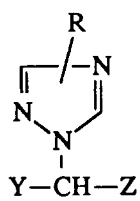 (I-y) ;

d) reacting a compound of formula

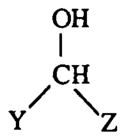

(V)

with a reagent of formula

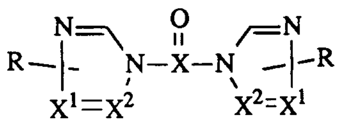

(VI)

in a suitable solvent;
e) reductively alkylating of a ketone or aldehyde of formula

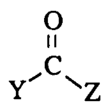

(VII)

with an azole of formula

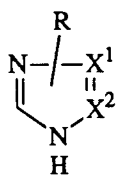

(II)

by stirring and heating the reagents in formic acid or formamides in the presence of an acid catalyst;
f) cyclizing an intermediate of formula

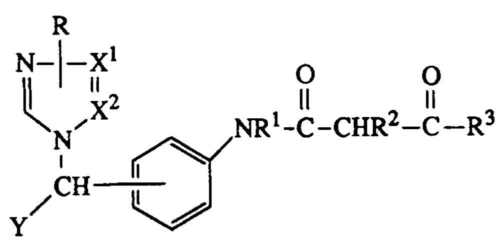

(IX)

in the presence of an acid, thus obtaining a compound of formula

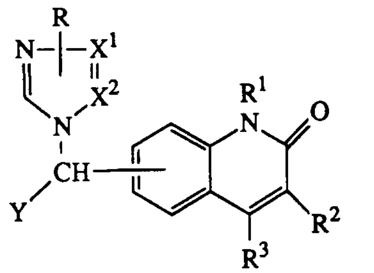

(I-a-1) ;

g) cyclizing an intermediate of formula

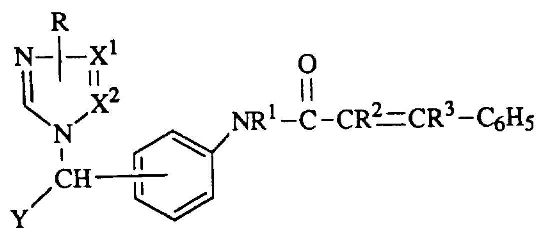

(X)

in the presence of a suitable Lewis acid, thus obtaining a compound of formula (I-a-1);
h) cyclizing an intermediate of formula

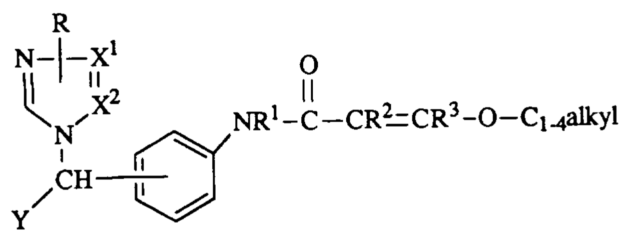

(XI)

in the presence of an acid, thus obtaining a compound of formula (I-a-1);
i) cyclizing an intermediate of formula

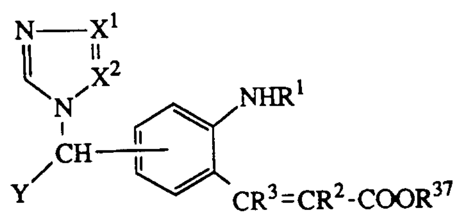

(XII)

thus obtaining a compound of formula (I-a-1);

j) cyclizing an intermediate of formula

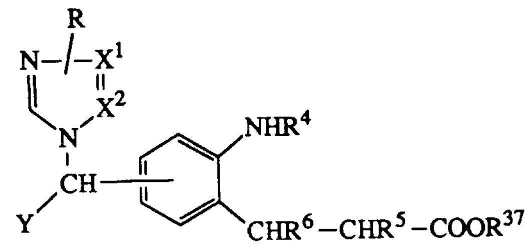

(XIII)

thus obtaining a compound of formula

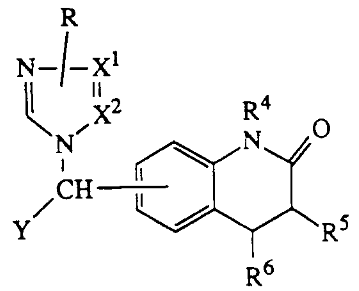

(I-a-2)

k) cyclizing an intermediate of formula

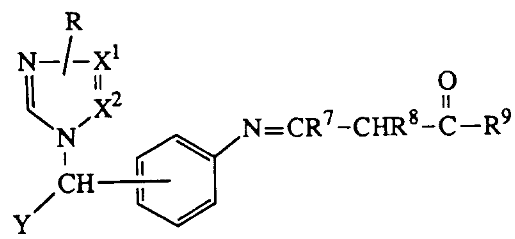

(XIV)

in the presence of a dehydrating agent thus obtaining a compound of formula

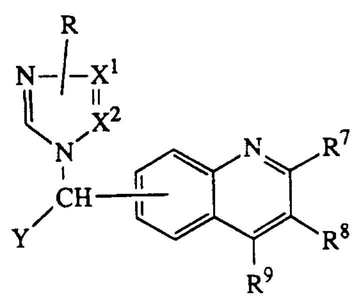

(I-a-3) ;

l) condensing an aniline of formula

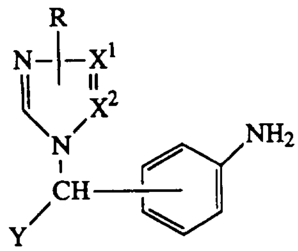

(VIII)

with an $\alpha,\beta$-unsaturated carbonyl synthon of formula

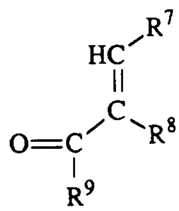

(XV)

in the presence of an acid and a mild oxidizing agent, thus obtaining a compound of formula (I-a-3);
m) condensing an ortho-acyl aniline of formula

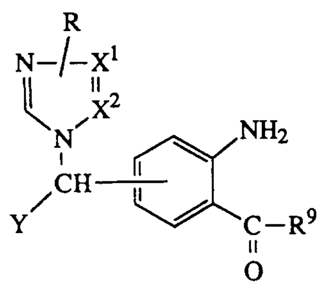

(XVI)

with a ketone or aldehyde of formula

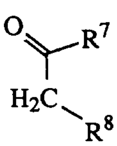

(XVII)

thus obtaining a compound of formula (I-a-3);
n) cyclizing an intermediate of formula

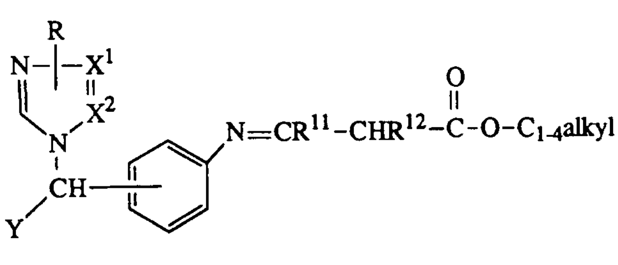

(XVIII)

in the presence of a suitable dehydrating agent, thus obtaining a compound of formula

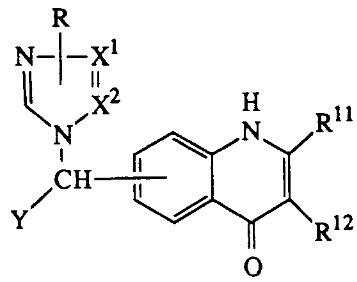

(I-a-4-a) ;

o) cyclizing an intermediate of formula

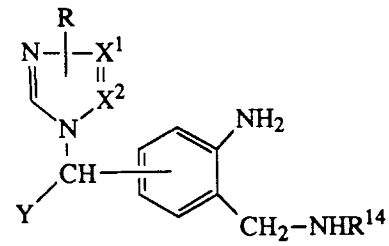

(XIX)

with a carboxylic acid of formula

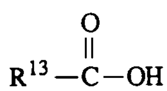

(XX)

or a functional derivative thereof in a reaction inert solvent, thus obtaining a compound of formula

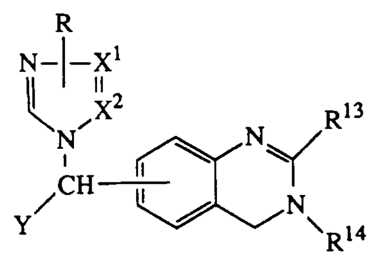

(I-b-1) ;

p) cyclizing an intermediate of formula

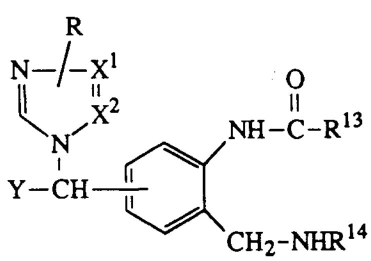

(XXI)

in a reaction-inert solvent, thus obtaining a compound of formula (I-b-1);

q) cyclizing an intermediate of formula

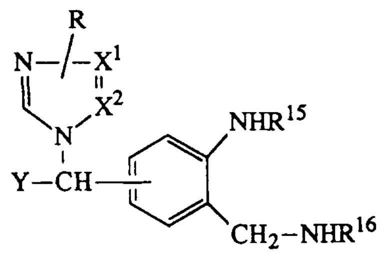

(XIX-a)

with a reagent of formula L-C(=$X^3$)-L (XXII) wherein L represents a reactive leaving group and $X^3$ is oxygen or sulfur, thus obtaining a compound of formula

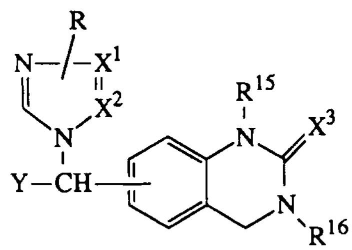

(I-b-2) ;

r) reducing and condensing an intermediate of formula

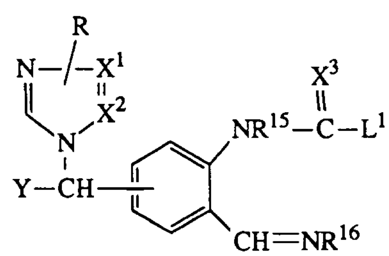

(XXIII)

wherein $L^1$ represents a reactive leaving group in a reaction-inert solvent and in the presence of a reducing agent, thus obtaining a compound of formula (I-b-2);

s) reacting an intermediate of formula

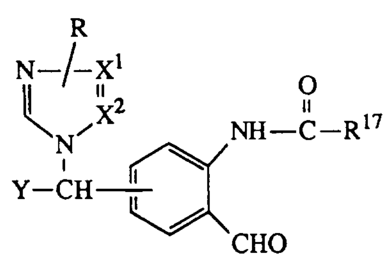

(XXIV)

with ammonia in a reaction-inert solvent, thus obtaining a compound of formula

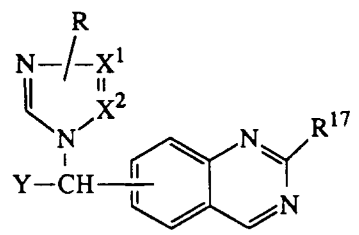

(I-b-3) ;

t) condensing an intermediate of formula

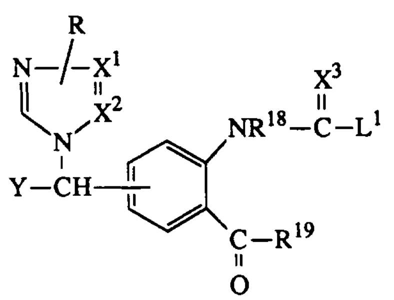

(XXV)

wherein $L^1$ represents a leaving group with ammonia, thus obtaining a compound of formula

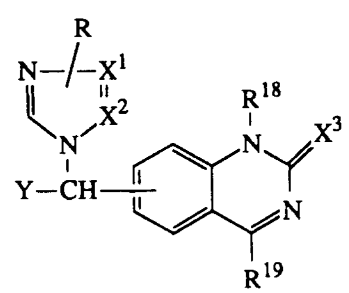

(I-b-4) ;

u) cyclizing an intermediate of formula

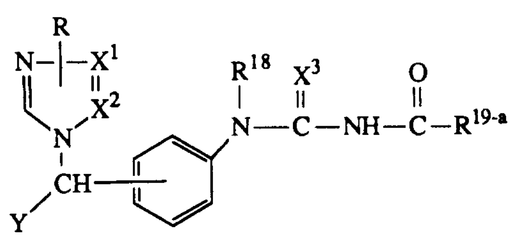

(XXVI)

in the presence of a dehydrating agent, thus obtaining a compound of formula (I-b-4);

v) condensing an intermediate of formula

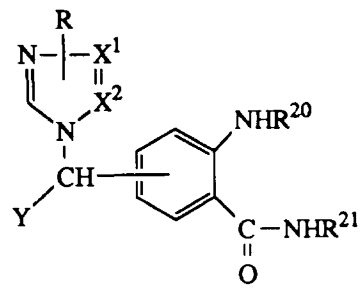

(XXVII)

with a reagent of formula L-C(=$X^3$)-L (XXII), in a reaction-inert solvent, thus obtaining a compound of formula

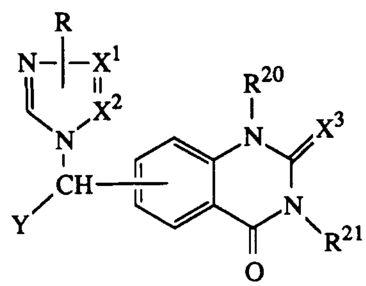

(I-b-5) ;

w) cyclizing an intermediate of formula

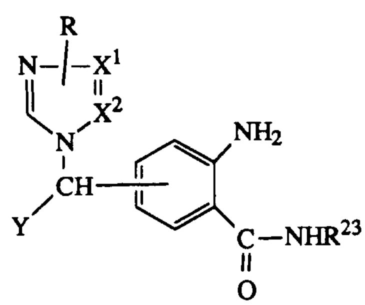

(XXVIII)

with a carboxylic acid of formula $R^{22}$-COOH (XXIX) or a functional derivative thereof, thus obtaining a compound of formula

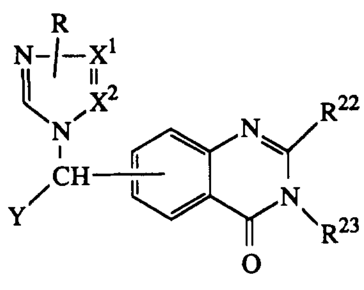

(I-b-6) ;

x) condensing an ortho-benzenediamine of formula

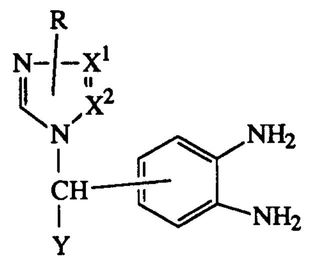

(XXX-a)

with a 1,2-diketone of formula $R^{24-a}$-C(=O)-C(=O)-$R^{25}$ (XXXI) in a reaction-inert solvent thus obtaining a compound of formula

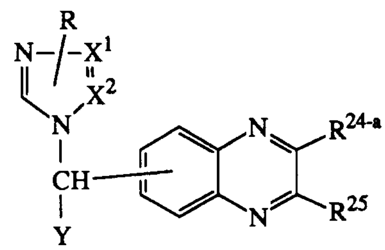

(I-c-1-a) ;

y) cyclizing an (1H-azol-1ylmethyl) substituted ortho-diamine of formula

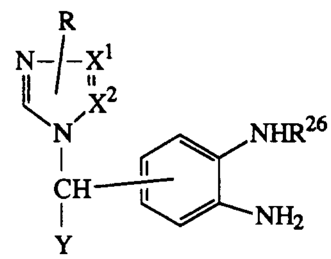

(XXX-b)

with a α-keto acid of formula HO-C(=O)-C(=O)-$R^{27}$ or a functional derivative thereof in a reaction-inert solvent, thus obtaining a compound of formula

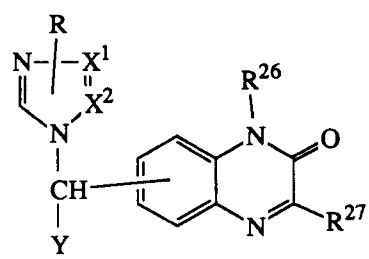

(I-c-2-a) ;

z) reducing and cyclizing an intermediate of formula

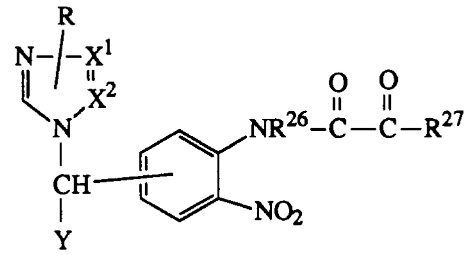

(XXXIII-b)

in a reaction-inert solvent, thus obtaining a compound of formula (I-c-2-a);
aa) cyclizing an ortho-nitroanilide containing a suitable activated methylene group of formula

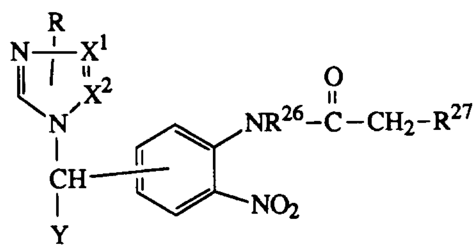

(XXXIV-a)

in a reaction-inert solvent, thus obtaining a compound of formula

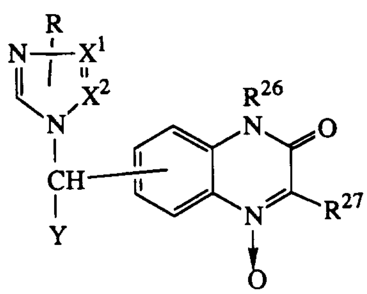

(I-c-2-b) ;

bb) cyclizing an ortho-anilide of formula

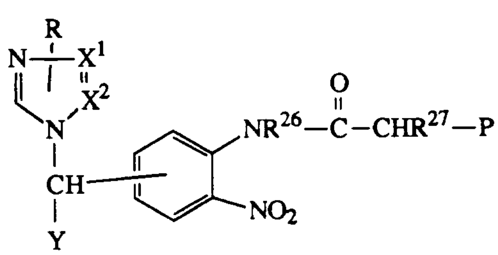

(XXXIV-b)

wherein P is a activating group, in a reaction-inert solvent, thus obtaining a compound of formula (I-c-2-b);

cc) condensing an intermediate of formula

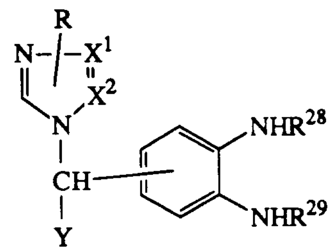

(XXX-c)

with oxalic acid HO-C( = O)-C( = O)-OH or a functional derivative thereof, thus obtaining a compound of formula

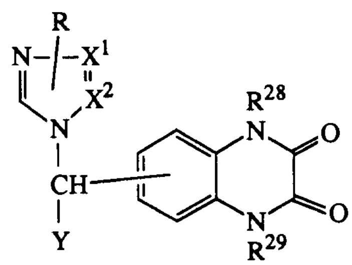

(I-c-3) ;

dd) condensing an ortho diamine of formula

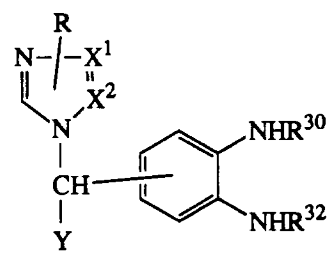

(XXX-d)

with an α-halo acid of formula

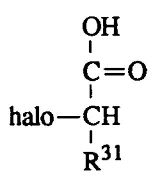

(XXXVI)

or a functional derivative thereof in a reaction-inert solvent, thus obtaining a compound of formula

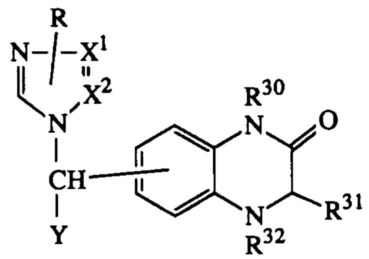

(I-c-4) ;

ee) reducing a substituted ortho-nitrophenyl glycine of formula

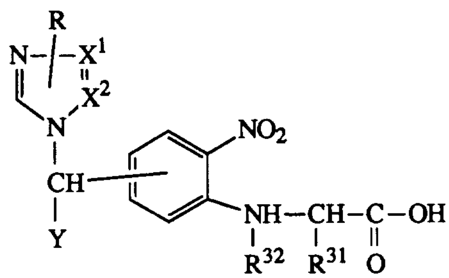

(XXXVII)

in a reaction inert solvent, thus obtaining a compound of formula

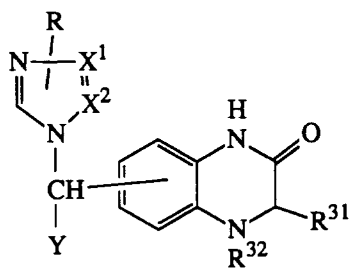

(I-c-4-a) ;

ff) cyclizing an intermediate of formula

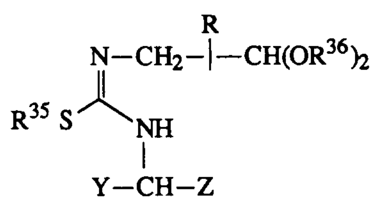

(XXXVIII)

and desulfurating the thus obtained intermediate of formula

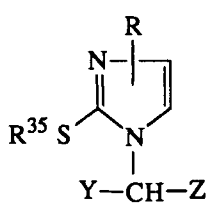

(IXL) ,

thus obtaining a compound of formula

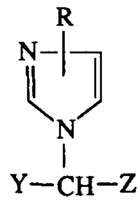

(I-x) ;

gg) reacting an intermediate of formula

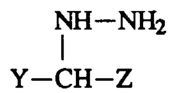

(XL)

with s-triazine, thus obtaining a compound of formula

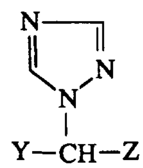

(I-y) ;

or optionally converting the compounds of formula (I) into each other following art-known grouptransformation procedures, and, if desired, converting the compounds of formula (I) into a therapeutically active non-toxic acid-addition salt form by treatment with an appropriate acid or, conversely, converting the acid-addition salt into the free base form with alkali; and/or preparing stereochemically isomeric forms thereof.

**Claim for the following Contracting States : ES, GR**

**1.** A process for preparing a chemical compound having the formula

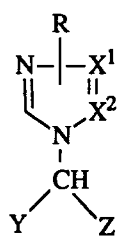

(I)

a pharmaceutical acceptable acid addition salt thereof or a stereochemically isomeric form thereof, wherein

$-X^1=X^2-$ is a bivalent radical having the formula

-CH=CH- (x),

-CH=N- (y), or

-N=CH- (z);

R is hydrogen or $C_{1-6}$alkyl;

Y is hydrogen, $C_{1-10}$alkyl, $C_{3-7}$cycloalkyl, $Ar^1$, $Ar^2$-$C_{1-6}$alkyl, $C_{2-6}$alkenyl or $C_{2-6}$alkynyl;

Z is a radical of formula

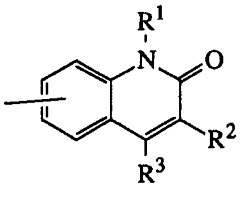 , 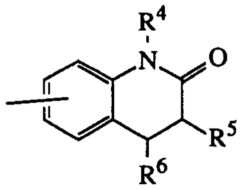 , 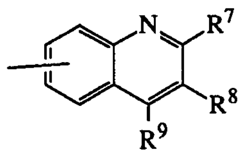 or 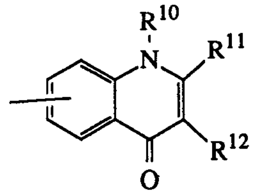

(a-1) (a-2) (a-3) (a-4)

wherein

$R^1$, $R^4$ and $R^{10}$ each independently are hydrogen, $C_{1-6}$alkyl or $Ar^2$-$C_{1-6}$alkyl;

$R^2$, $R^5$, $R^8$ and $R^{12}$ each independently are hydrogen, $C_{1-6}$alkyl or $Ar^2$;

$R^3$, $R^6$ and $R^{11}$ each independently are hydrogen or $C_{1-6}$alkyl;

$R^7$ and $R^9$ each independently are hydrogen, $C_{1-6}$alkyl, $C_{1-6}$alkyloxy, halo, amino, or mono or di-($C_{1-6}$alkyl)amino; or

Z is a radical of formula

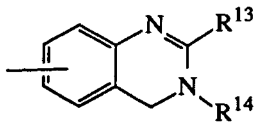 , 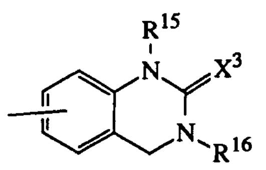 , 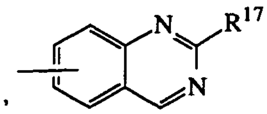 ,

(b-1) (b-2) (b-3)

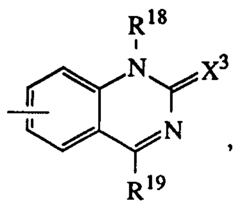 , 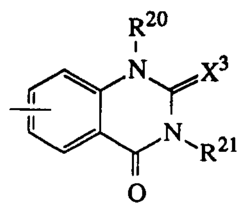 or 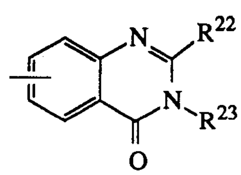

(b-4) (b-5) (b-6)

wherein

$R^{13}$, $R^{17}$ and $R^{22}$ each independently are hydrogen, halo, $C_{1-6}$alkyl, trifluoromethyl, $C_{1-6}$alkyloxy, $Ar^2$, $Ar^2$-$C_{1-6}$alkyl, amino, or mono- or di($C_{1-6}$alkyl)amino;

$R^{14}$, $R^{16}$ and $R^{21}$ each independently are hydrogen, $C_{1-6}$alkyl, $Ar^2$ or $Ar^2$-$C_{1-6}$alkyl;

$R^{15}$, $R^{18}$ and $R^{20}$ each independently are hydrogen, $C_{1-6}$alkyl or $Ar^2$-$C_{1-6}$alkyl;

$R^{19}$ is hydrogen or $C_{1-6}$alkyl;

$R^{23}$ is hydrogen, $C_{1-6}$alkyl, $Ar^2$-$C_{1-6}$alkyl, amino or mono($C_{1-6}$alkyl)amino;

$X^3$ is O or S; or

Z is a radical of formula

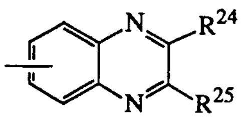(c-1), 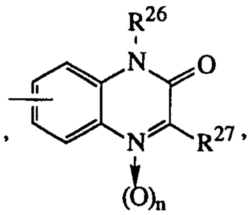(c-2), 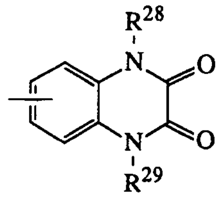(c-3), 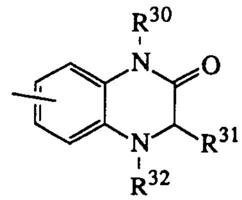(c-4), 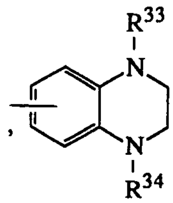(c-5)

wherein

$R^{24}$ is hydrogen, halo, $C_{1-6}$alkyl, $C_{1-6}$alkyloxy, amino, mono- or di($C_{1-6}$alkyl)amino, $Ar^2$ or imidazolyl;

$R^{25}$ is hydrogen, $C_{1-6}$alkyl or $Ar^1$;

$R^{26}$ and $R^{30}$ each independently are hydrogen, $C_{1-6}$alkyl, $Ar^2$-$C_{1-6}$alkyl, amino or mono-($C_{1-6}$alkyl)amino;

$R^{27}$ and $R^{31}$ each independently are hydrogen, $C_{1-6}$alkyl, $Ar^1$, $C_{1-6}$alkylcarbonyl, $Ar^2$-carbonyl, $C_{1-6}$alkyloxycarbonyl, carboxyl, $C_{1-6}$alkyloxycarbonyl-$C_{1-4}$alkyl, aminocarbonyl or cyano;

$R^{28}$, $R^{29}$, $R^{32}$, $R^{33}$ and $R^{34}$ each independently are hydrogen, $C_{1-6}$alkyl or $Ar^2$-$C_{1-6}$alkyl;

n is 0 or 1; and

$Ar^1$ is phenyl, substituted phenyl, naphthalenyl, pyridinyl, imidazolyl, triazolyl, thienyl, furanyl or thiazolyl and $Ar^2$ is phenyl or substituted phenyl; said substituted phenyl in $Ar^1$ or $Ar^2$ being phenyl substituted with 1, 2 or 3 substituents each independently selected from halo, hydroxy, trifluoromethyl, $C_{1-6}$alkyl, $C_{1-6}$alkyloxy, cyano, amino, mono- and di($C_{1-6}$alkyl)amino, nitro, carboxyl, formyl and $C_{1-6}$alkyloxycarbonyl.

characterized by

a) N-alkylating an azole of formula (II) or an alkali metal salt thereof

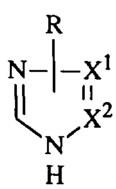 (II)

wherein R, $X^1$, $X^2$ are as defined under formula (I) with a quinoline, quinolinone, quinazoline or quinoxaline derivative of formula

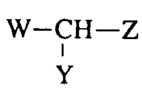 (III)

wherein Y and Z are as defined under formula (I) and W represents a reactive leaving group;

b) N-alkylating an azole of formula

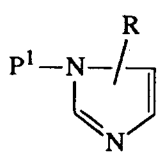 (II-x)

wherein R is as defined under formula (I) and $P^1$ represents a protective group with a quinoline, quinolinone, quinazoline or quinoxaline derivative of formula

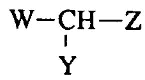 (III)

wherein Y and Z are as defined under formula (I) and W represents a reactive leaving group, thus obtaining a compound of formula

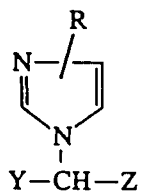 (I-x) ;

c) endo-N-alkylating an azole of formula

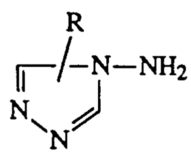 (II-y)

wherein R is as defined under formula (I) with a quinoline, quinolinone, quinazoline or quinoxaline derivative of formula

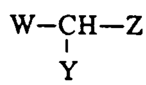 (III)

wherein Y and Z are as defined under formula (I) and W represents a reactive leaving group, subsequently deaminating the thus obtained triazolium salt,

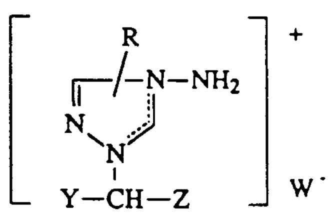

thus preparing a compound of formula

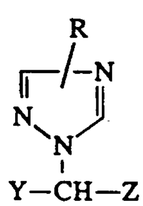 (I-y) ;

d) reacting a compound of formula

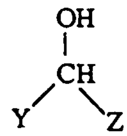

(V)

with a reagent of formula

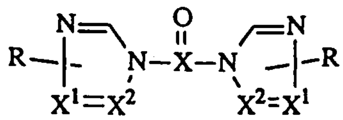

(VI)

in a suitable solvent;

e) reductively alkylating of a ketone or aldehyde of formula

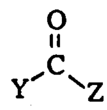

(VII)

with an azole of formula

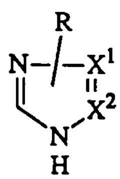

(II)

by stirring and heating the reagents in formic acid or formamides in the presence of an acid catalyst;

f) cyclizing an intermediate of formula

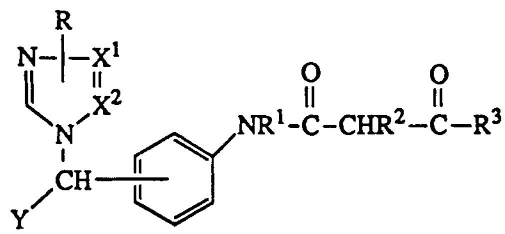

(IX)

in the presence of an acid, thus obtaining a compound of formula

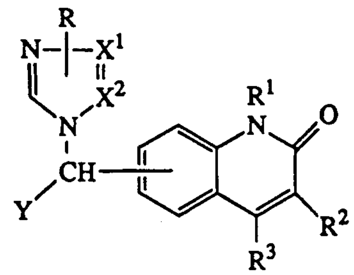

(I-a-1) ;

g) cyclizing an intermediate of formula

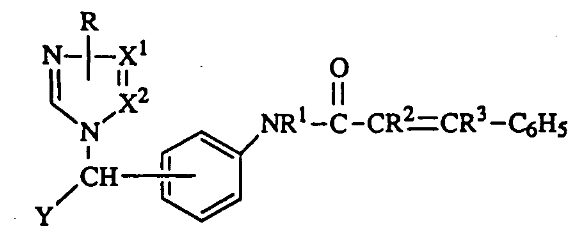

(X)

in the presence of a suitable Lewis acid, thus obtaining a compound of formula (I-a-1);
h) cyclizing an intermediate of formula

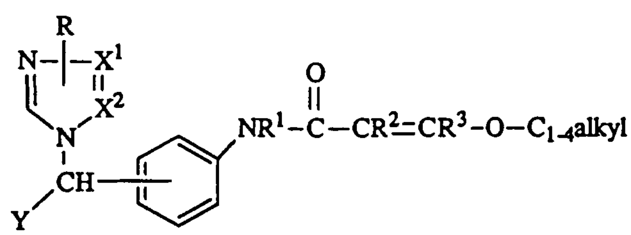

(XI)

in the presence of an acid, thus obtaining a compound of formula (I-a-1);
i) cyclizing an intermediate of formula

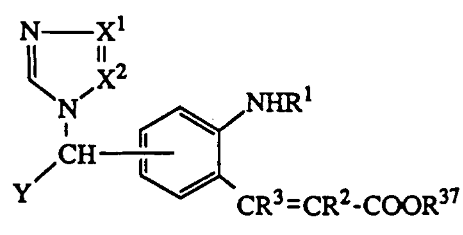

(XII)

thus obtaining a compound of formula (I-a-1);

j) cyclizing an intermediate of formula

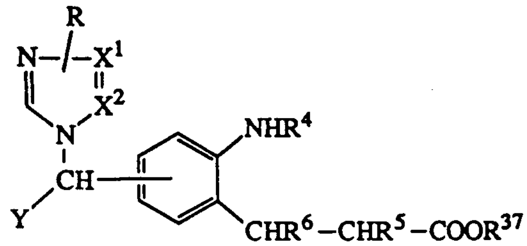

(XIII)

thus obtaining a compound of formula

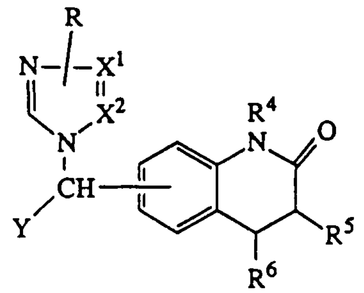

(I-a-2)

k) cyclizing an intermediate of formula

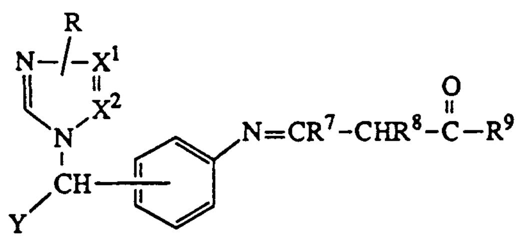

(XIV)

in the presence of a dehydrating agent, thus obtaining a compound of formula

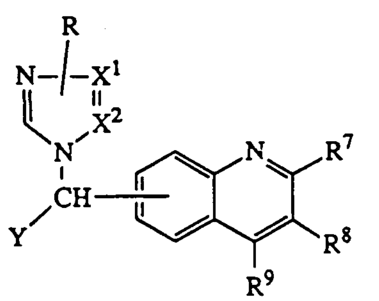

(I-a-3) ;

l) condensing an aniline of formula

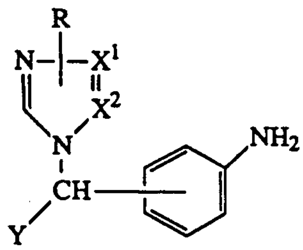

(VIII)

with an α,β-unsaturated carbonyl synthon of formula

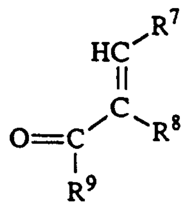

(XV)

in the presence of an acid and a mild oxidizing agent, thus obtaining a compound of formula (I-a-3);
m) condensing an ortho-acyl aniline of formula

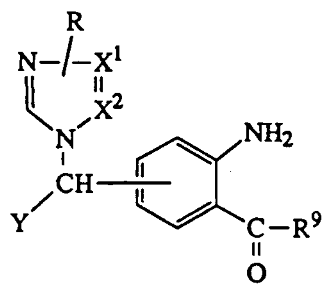

(XVI)

with a ketone or aldehyde of formula

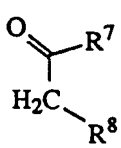

(XVII)

thus obtaining a compound of formula (I-a-3);
n) cyclizing an intermediate of formula

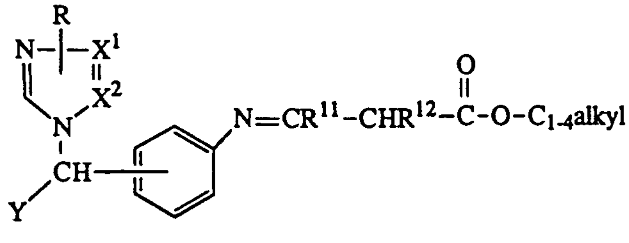

(XVIII)

in the presence of a suitable dehydrating agent, thus obtaining a compound of formula

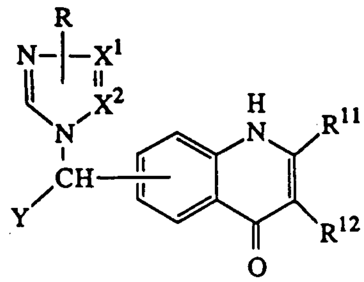

(I-a-4-a) ;

o) cyclizing an intermediate of formula

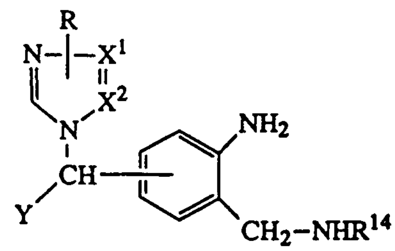

(XIX)

with a carboxylic acid of formula

$$R^{13}-\overset{O}{\overset{\|}{C}}-OH \qquad (XX)$$

or a functional derivative thereof in a reaction inert solvent, thus obtaining a compound of formula

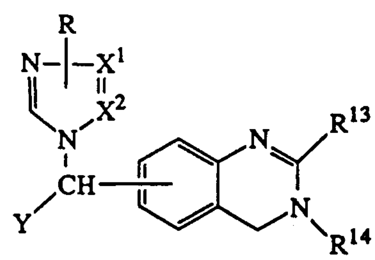

(I-b-1) ;

p) cyclizing an intermediate of formula

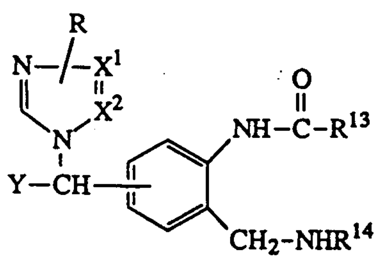

(XXI)

in a reaction-inert solvent, thus obtaining a compound of formula (I-b-1);

q) cyclizing an intermediate of formula

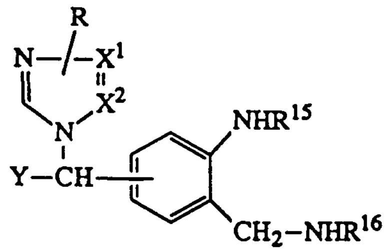

(XIX-a)

with a reagent of formula L-C(=$X^3$)-L (XXII) wherein L represents a reactive leaving group and $X^3$ is oxygen or sulfur, thus obtaining a compound of formula

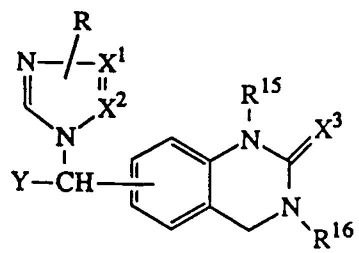

(I-b-2) ;

r) reducing and condensing an intermediate of formula

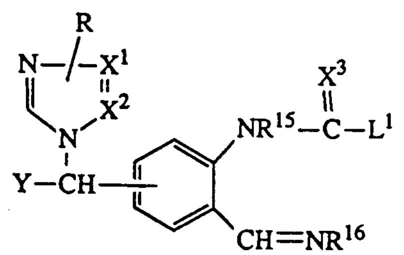

(XXIII)

wherein $L^1$ represents a reactive leaving group in a reaction-inert solvent and in the presence of a reducing agent, thus obtaining a compound of formula (I-b-2);

s) reacting an intermediate of formula

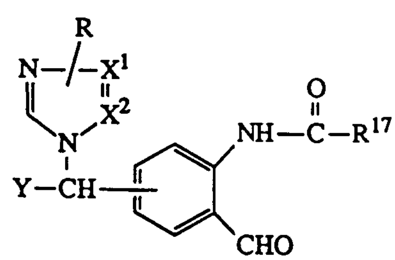

(XXIV)

with ammonia in a reaction-inert solvent, thus obtaining a compound of formula

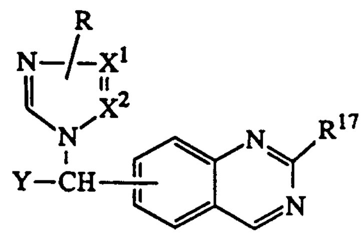

(I-b-3) ;

t) condensing an intermediate of formula

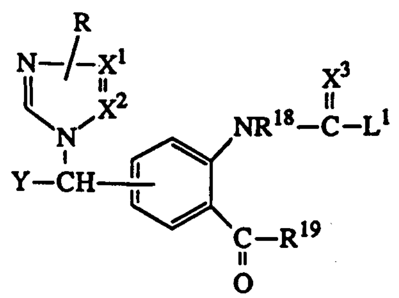

(XXV)

wherein $L^1$ represents a leaving group with ammonia, thus obtaining a compound of formula

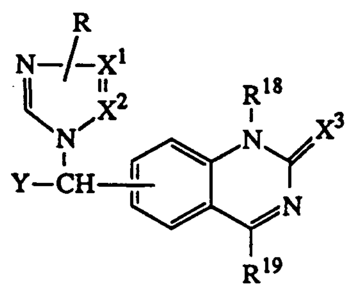

(I-b-4) ;

u) cyclizing a intermediate of formula

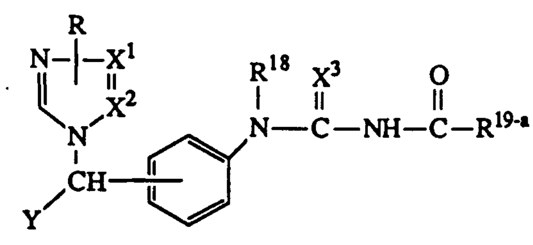

(XXVI)

in the presence of a dehydrating agent, thus obtaining a compound of formula (I-b-4);

v) condensing a intermediate of formula

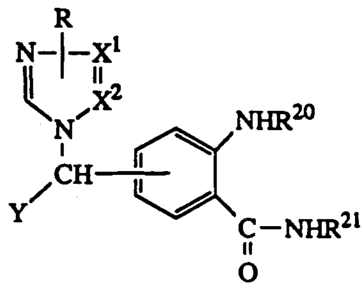

(XXVII)

with a reagent of formula L-C(=$X^3$)-L (XXII), in a reaction-inert solvent, thus obtaining a compound of formula

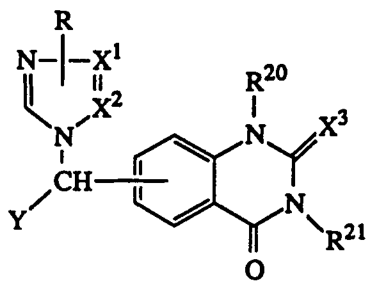

(I-b-5) ;

w) cyclizing a intermediate of formula

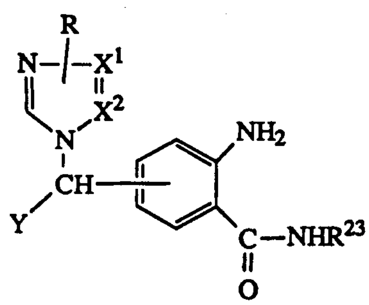

(XXVIII)

with a carboxylic acid of formula $R^{22}$-COOH (XXIX) or a functional derivative thereof, thus obtaining a compound of formula

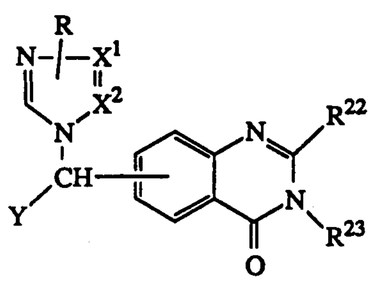

(I-b-6) ;

x) condensing an ortho-benzenediamine of formula

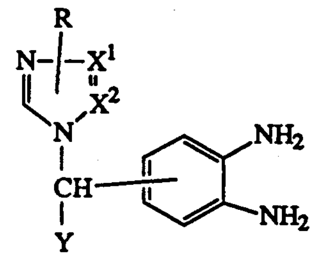

(XXX-a)

with a 1,2-diketone of formula $R^{24-a}$-C(=O)-C(=O)-$R^{25}$ (XXXI) in a reaction-inert solvent thus obtaining a compound of formula

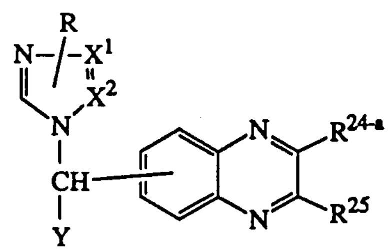

(I-c-1-a) ;

y) cyclizing an (1H-azol-1ylmethyl) substituted ortho-diamine of formula

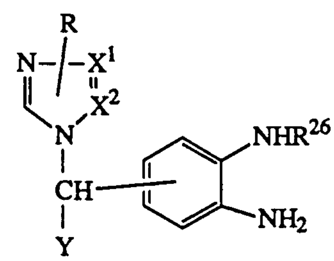

(XXX-b)

with a α-keto acid of formula HO-C(=O)-C(=O)-$R^{27}$ or a functional derivative thereof in a reaction-inert solvent, thus obtaining a compound of formula

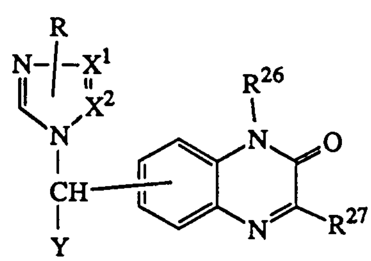

(I-c-2-a) ;

z) reducing and cyclizing an intermediate of formula

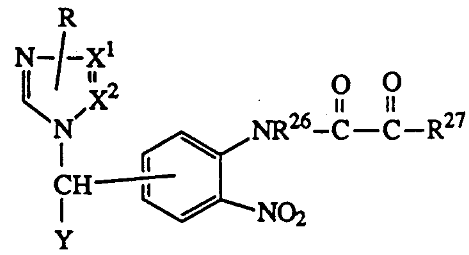

(XXXIII-b)

in a reaction-inert solvent, thus obtaining a compound of formula (I-c-2-a);

aa) cyclizing an ortho-nitroanilide containing a suitable activated methylene group of formula

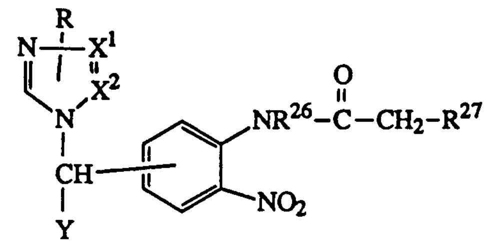

(XXXIV-a)

in a reaction-inert solvent, thus obtaining a compound of formula

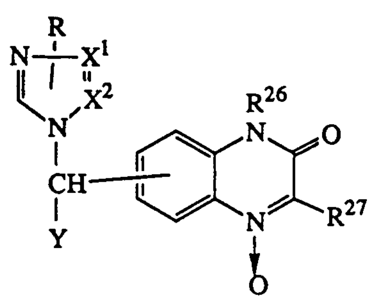

(I-c-2-b) ;

bb) cyclizing an ortho-anilide of formula

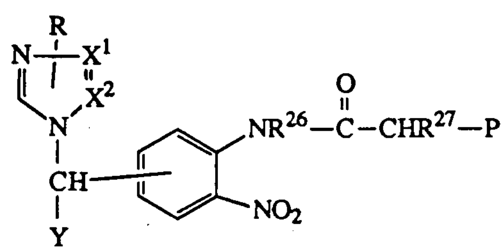

(XXXIV-b)

wherein P is a activating group, in a reaction-inert solvent; thus obtaining a compound of formula (I-c-2-b);

cc) condensing an intermediate of formula

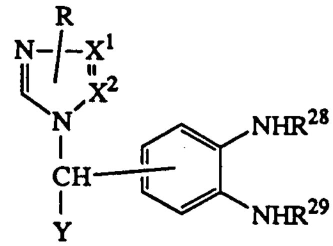

(XXX-c)

with oxalic acid HO-C( = O)-C( = O)-OH or a functional derivative thereof, thus obtaining a compound of formula

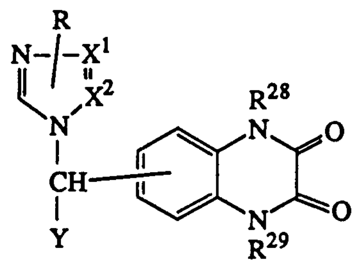

(I-c-3) ;

dd) condensing a ortho diamine of formula

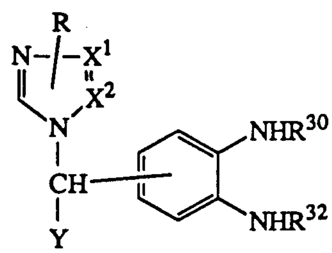

(XXX-d)

with an α-halo acid of formula

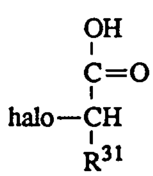

(XXXVI)

or a functional derivative thereof in a reaction-inert solvent, thus obtaining a compound of formula

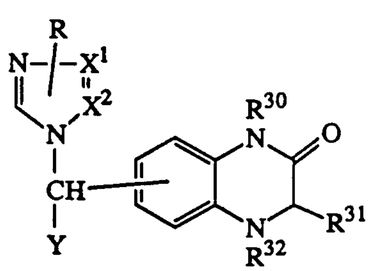

(I-c-4) ;

ee) reducing a substituted ortho-nitrophenyl glycine of formula

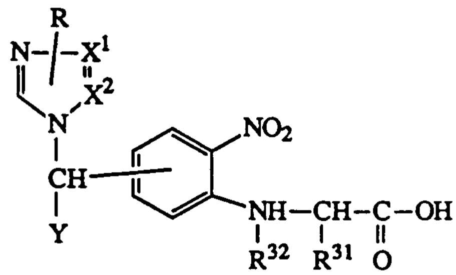

(XXXVII)

in a reaction inert solvent, thus obtaining a compound of formula

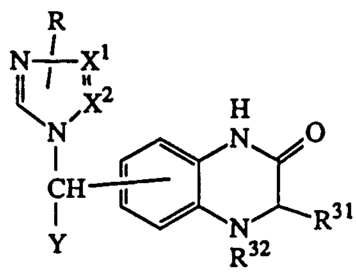

(I-c-4-a) ;

ff) cyclizing a intermediate of formula

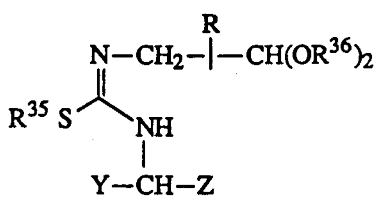

(XXXVIII)

and desulfurating the thus obtained intermediate of formula

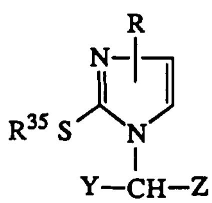

(IXL) ,

thus obtaining a compound of formula

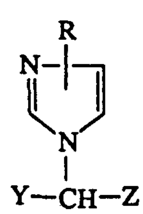

(I-x) ;

gg) reacting an intermediate of formula

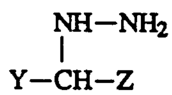

(XL)

with s-triazine, thus obtaining a compound of formula

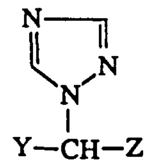

(I-y);

or optionally converting the compounds of formula (I) into each other following art-known grouptransformation procedures, and, if desired, converting the compounds of formula (I) into a therapeutically active non-toxic acid-addition salt form by treatment with an appropriate acid or, conversely, converting the acid-addition salt into the free base form with alkali; and/or preparing stereochemically isomeric forms thereof.

**Patentansprüche**

**Patentansprüche für folgende Vertragsstaaten : AT, BE, CH, DE, FR, GB, IT, LI, LU, NL, SE**

**1.** Chemische Verbindung mit der Formel

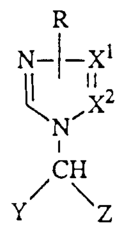

(I) ,

ein pharmazeutisch annehmbares Säureadditionssalz hievon oder eine stereochemisch isomere Form hievon, worin

$-X^1=X^2-$ einen zweiwertigen Rest mit der Formel

-CH=CH- (x),

-CH=N- (y) oder

-N=CH- (z)

bedeutet;

R für Wasserstoff oder $C_{1-6}$-Alkyl steht;

Y für Wasserstoff, $C_{1-10}$-Alkyl, $C_{3-6}$-Cycloalkyl, $Ar^1$, $Ar^2$-$C_{1-6}$-Alkyl, $C_{2-6}$-Alkenyl oder $C_{2-6}$-Alkinyl steht;

Z einen Rest der Formel

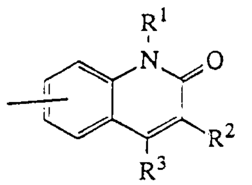

(a-1) ,

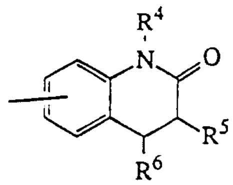

(a-2) ,

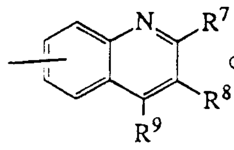

(a-3) oder

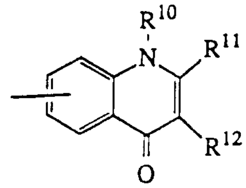

(a-4)

darstellt, worin

$R^1$, $R^4$ und $R^{10}$ jeweils unabhängig Wasserstoff, $C_{1-6}$-Alkyl oder $Ar^2$-$C_{1-6}$-Alkyl bedeuten;

$R^2$, $R^5$, $R^8$ und $R^{12}$ jeweils unabhängig Wasserstoff, $C_{1-6}$-Alkyl oder $Ar^2$ bedeuten;

$R^3$, $R^6$ und $R^{11}$ jeweils unabhängig Wasserstoff oder $C_{1-6}$-Alkyl bedeuten;

$R^7$ und $R^9$ jeweils unabhängig Wasserstoff, $C_{1-6}$-Alkyl, $C_{1-6}$-Alkyloxy, Halogen, Amino oder Mono- oder Di($C_{1-6}$-alkyl)amino bedeuten; oder

Z einen Rest der Formel

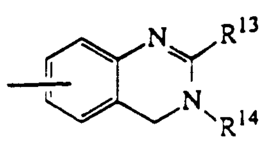

(b-1) ,

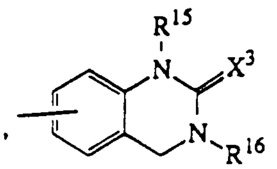

(b-2) ,

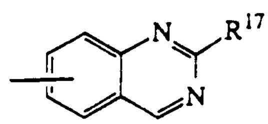

(b-3) ,

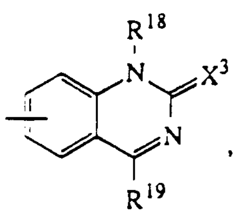

(b-4) ,

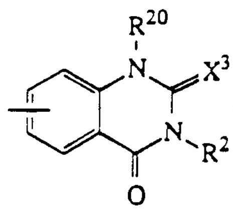

(b-5) oder

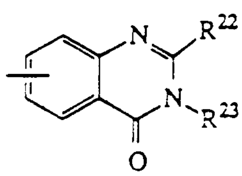

(b-6)

darstellt, worin

$R^{13}$, $R^{17}$ und $R^{22}$ jeweils unabhängig Wasserstoff, Halogen, $C_{1-6}$-Alkyl, Trifluormethyl, $C_{1-6}$-Alkyloxy, $Ar^2$, $Ar^2$-$C_{1-6}$-Alkyl, Amino oder Mono- oder Di($C_{1-6}$-alkyl)amino bedeuten;

$R^{14}$, $R^{16}$ und $R^{21}$ jeweils unabhängig Wasserstoff, $C_{1-6}$-Alkyl, $Ar^2$ oder $Ar^2$-$C_{1-6}$-Alkyl bedeuten;

$R^{15}$, $R^{18}$ und $R^{20}$ jeweils unabhängig Wasserstoff, $C_{1-6}$-Alkyl oder $Ar^2$-$C_{1-6}$-Alkyl bedeuten;

$R^{19}$ Wasserstoff oder $C_{1-6}$-Alkyl bedeutet;

$R^{23}$ Wasserstoff, $C_{1-6}$-Alkyl, $Ar^2$-$C_{1-6}$-Alkyl, Amino oder Mono($C_{1-6}$-alkyl)amino bedeutet;

$X^3$ für O oder S steht; oder

Z einen Rest der Formel

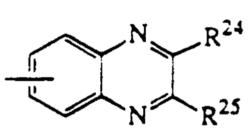

(c-1) ,

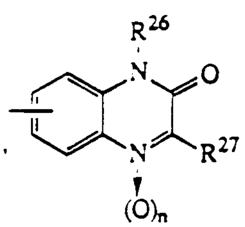

(c-2) ,

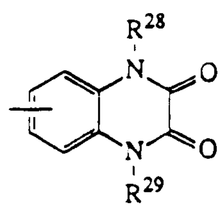

(c-3) ,

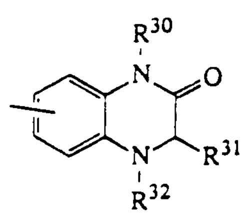

(c-4) ,

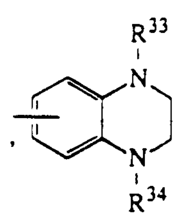

(c-5)

darstellt, worin

$R^{24}$ Wasserstoff, Halogen, $C_{1-6}$-Alkyl, $C_{1-6}$-Alkyloxy, Amino, Mono- oder Di($C_{1-6}$-alkyl)amino, $Ar^2$ oder Imidazolyl bedeutet;

$R^{25}$ Wasserstoff, $C_{1-6}$-Alkyl oder $Ar^1$ bedeutet;

$R^{26}$ und $R^{30}$ jeweils unabhängig Wasserstoff, $C_{1-6}$-Alkyl, $Ar^2$-$C_{1-6}$-Alkyl, Amino oder Mono($C_{1-6}$-alkyl)amino bedeuten;

$R^{27}$ und $R^{31}$ jeweils unabhängig Wasserstoff, $C_{1-6}$-Alkyl, $Ar^1$, $C_{1-6}$-Alkylcarbonyl, $Ar^2$-Carbonyl, $C_{1-6}$-Alkyloxycarbonyl, Carboxyl, $C_{1-6}$-Alkyloxycarbonyl-$C_{1-4}$-alkyl, Aminocarbonyl oder Cyano bedeuten;

$R^{28}$, $R^{29}$, $R^{32}$, $R^{33}$ und $R^{34}$ jeweils unabhängig Wasserstoff, $C_{1-6}$-Alkyl oder $Ar^2$-$C_{1-6}$-Alkyl bedeuten;

n den Wert 0 oder 1 aufweist; und

$Ar^1$ für Phenyl, substituiertes Phenyl, Naphthalinyl, Pyridinyl, Imidazolyl, Triazolyl, Thienyl, Furanyl oder Thiazolyl steht und $Ar^2$ Phenyl oder substituiertes Phenyl bedeutet; wobei das genannte substituierte Phenyl in $Ar^1$ oder $Ar^2$ ein Phenyl ist, das durch 1, 2 oder 3 Substituenten substituiert ist, die jeweils unabhängig unter Halogen, Hydroxy, Trifluormethyl, $C_{1-6}$-Alkyl, $C_{1-6}$-Alkyloxy, Cyano, Amino, Mono- und Di($C_{1-6}$-alkyl)amino, Nitro, Carboxyl, Formyl und $C_{1-6}$-Alkyloxycarbonyl ausgewählt sind.

**2.** Verbindung nach Anspruch 1, worin R Wasserstoff oder $C_{1-4}$-Alkyl darstellt; Y für Wasserstoff, $C_{1-6}$-Alkyl, $C_{3-7}$-Cycloalkyl, Phenyl, substituiertes Phenyl, Pyridinyl, Imidazolyl oder Thienyl steht; und Z einen Rest der Formel (a-1), (a-2), (a-3) oder (a-4) darstellt, worin $R^1$, $R^2$, $R^3$, $R^4$, $R^5$, $R^6$, $R^8$, $R^{10}$, $R^{11}$ und $R^{12}$ jeweils unabhängig Wasserstoff oder $C_{1-4}$-Alkyl bedeuten und $R^7$ und $R^9$ jeweils unabhängig Wasserstoff, $C_{1-4}$-Alkyl, $C_{1-4}$-Alkyloxy oder Halogen bedeuten; oder Z einen Rest der Formel (b-1), (b-2), (b-3), (b-4), (b-5) oder (b-6) bedeutet, worin $R^{13}$ Wasserstoff, $C_{1-4}$-Alkyl, Trifluormethyl oder Phenyl darstellt, $R^{14}$ für Wasserstoff, $C_{1-4}$-Alkyl, Phenyl oder Phenyl-$C_{1-4}$-alkyl steht, $R^{15}$ Wasserstoff oder durch Phenyl substituiertes $C_{1-4}$-Alkyl bedeutet, $R^{16}$, $R^{17}$, $R^{18}$, $R^{19}$ und $R^{20}$ jeweils unabhängig Wasserstoff oder $C_{1-4}$-Alkyl darstellen, $R^{21}$ für Wasserstoff, $C_{1-4}$-Alkyl oder Phenyl-$C_{1-4}$-alkyl steht, $R^{22}$ Wasserstoff, Amino oder Mono- oder Di($C_{1-4}$-alkylamino) bedeutet und $R^{23}$ Wasserstoff darstellt; oder Z einen Rest der Formel (c-1), (c-2), (c-3), (c-4) oder (c-5) bedeutet, worin $R^{24}$ Wasserstoff, $C_{1-4}$-Alkyl, Halogen, $C_{1-4}$-Alkyloxy, Amino, Mono- oder Di($C_{1-4}$-alkyl)amino, Phenyl, substituiertes Phenyl oder Imidazolyl bedeutet, $R^{25}$ für Wasserstoff, $C_{1-4}$-Alkyl, Phenyl oder substituiertes Phenyl steht, $R^{26}$ Wasserstoff, $C_{1-4}$-Alkyl, Amino, $C_{1-4}$-Alkylamino oder durch Phenyl oder substituiertes Phenyl substituiertes $C_{1-4}$-Alkyl darstellt; $R^{27}$ Wasserstoff, $C_{1-4}$-Alkyl, $C_{1-4}$-Alkyloxycarbonyl-$C_{1-4}$-alkyl, Phenyl, substituiertes Phenyl, $C_{1-4}$-Alkyloxycarbonyl, Carboxyl, Phenylcarbonyl, substituiertes Phenylcarbonyl, Naphthalinyl, Thienyl, Furanyl, Pyridinyl oder Imidazolyl bedeutet; $R^{28}$ und $R^{29}$ jeweils unabhängig Wasserstoff oder $C_{1-4}$-Alkyl bedeuten; $R^{30}$ für Wasserstoff, $C_{1-4}$-Alkyl, Amino oder durch Phenyl oder substituiertes Phenyl substituiertes $C_{1-4}$-Alkyl steht; $R^{31}$ Wasserstoff, $C_{1-4}$-Alkyl, Phenyl, substituiertes Phenyl, $C_{3-7}$-Cycloalkyl, Naphthalinyl, Thienyl, Pyridinyl oder Imidazolyl bedeutet; $R^{32}$ Wasserstoff oder $C_{1-4}$-Alkyl darstellt und $R^{33}$ und $R^{34}$ beide Wasserstoff bedeuten.

**3.** Verbindung nach Anspruch 2, worin $-X^1=X^2-$ einen Rest der Formel (x) oder (y) bedeutet; und Y für Wasserstoff, $C_{1-4}$-Alkyl, Cyclopropyl, Cyclopentyl, Cyclohexyl, Imidazolyl, Pyridinyl, Thienyl oder gegebenenfalls durch einen oder zwei Substituenten substituiertes Phenyl steht, welche Substituenten jeweils unabhängig unter Halogen, $C_{1-4}$-Alkyl, $C_{1-4}$-Alkyloxy und Trifluormethyl ausgewählt sind.

**4.** Verbindung nach Anspruch 1, worin

Z für einen Rest der Formel (a-1) steht, worin $R^1$ und $R^2$ Wasserstoff bedeuten, $R^3$ Wasserstoff oder $C_{1-4}$-Alkyl darstellt und Y für Wasserstoff, $C_{1-4}$-Alkyl oder Phenyl steht, das gegebenenfalls durch ein oder zwei Halogenatome substituiert ist; oder

Z für einen Rest der Formel (a-2) steht, worin $R^4$, $R^5$ und $R^6$ jeweils Wasserstoff bedeuten und Y für Wasserstoff, $C_{1-4}$-Alkyl, Cyclopropyl oder Phenyl steht, das gegebenenfalls durch ein oder zwei Halogenatome substituiert ist; oder

Z für einen Rest der Formel (a-3) steht, worin $R^7$ Wasserstoff, Halogen oder $C_{1-4}$-Alkyloxy bedeutet, $R^8$ Wasserstoff darstellt, $R^9$ Wasserstoff, $C_{1-4}$-Alkyl oder $C_{1-4}$-Alkyloxy bedeutet und Y für Wasserstoff, $C_{1-4}$-Alkyl, Cyclopropyl, Cyclohexyl, Imidazolyl, Thienyl oder Phenyl steht, das gegebenenfalls durch einen oder zwei Substituenten, jeweils unabhängig ausgewählt unter Halogen, $C_{1-4}$-Alkyl, $C_{1-4}$-Alkyloxy und Trifluormethyl, substituiert ist; oder

Z für einen Rest mit der Formel (a-4) steht, worin $R^{10}$ und $R^{12}$ Wasserstoff bedeuten, $R^{11}$ für $C_{1-4}$-

Alkyl steht und Y Wasserstoff bedeutet; oder

Z für einen Rest der Formel (b-2) steht, worin $R^{15}$ Wasserstoff darstellt, $R^{16}$ Wasserstoff oder $C_{1-4}$-Alkyl bedeutet und Y für Wasserstoff, $C_{1-4}$-Alkyl, Pyridinyl oder gegebenenfalls durch ein oder zwei Halogenatome substituiertes Phenyl steht; oder

Z für einen Rest der Formel (b-3) steht, worin $R^{17}$ für $C_{1-4}$-Alkyl steht und Y Phenyl oder Halogenphenyl bedeutet; oder

Z für einen Rest der Formel (b-5) steht, worin $R^{20}$ Wasserstoff bedeutet, $R^{21}$ Wasserstoff, $C_{1-4}$-Alkyl oder Phenyl-$C_{1-4}$-alkyl bedeutet und Y für Wasserstoff, Phenyl oder Halogenphenyl steht; oder

Z für einen Rest der Formel (b-6) steht, worin $R^{22}$ Wasserstoff oder Amino bedeutet, $R^{23}$ Wasserstoff darstellt und Y für Wasserstoff, Phenyl oder Halogenphenyl steht; oder

Z für einen Rest der Formel (c-1) steht, worin $R^{24}$ Wasserstoff,$C_{1-4}$-Alkyl, $C_{1-4}$-Alkyloxy, Halogen, Amino, Di($C_{1-4}$-alkyl)amino, Phenyl oder Imidazolyl bedeutet, $R^{25}$ Wasserstoff, $C_{1-4}$-Alkyl oder Phenyl darstellt und Y für Wasserstoff, $C_{1-4}$-Alkyl, Thienyl, Imidazolyl oder Phenyl steht, das gegebenenfalls durch ein oder zwei Substituenten, ausgewählt unter Halogen, $C_{1-4}$-Alkyl, $C_{1-4}$-Alkyloxy oder Trifluormethyl, substituiert ist; oder

Z für einen Rest der Formel (c-2) steht, worin $R^{26}$ Wasserstoff,$C_{1-4}$-Alkyl, Amino oder durch Phenyl substituiertes $C_{1-4}$-Alkyl darstellt und $R^{27}$ Wasserstoff, $C_{1-4}$-Alkyl, Carboxyl, $C_{1-4}$-Alkyloxycarbonyl, Naphthalinyl, Thienyl, Pyridinyl, Imidazolyl, Phenyl oder durch 1, 2 oder 3 Substituenten substituiertes Phenyl darstellt, die jeweils unabhängig unter $C_{1-4}$-Alkyl, $C_{1-4}$-Alkyloxy, Halogen, Hydroxy und Trifluormethyl ausgewählt sind, und Y für Wasserstoff, $C_{1-4}$-Alkyl, Cyclopropyl, Cyclopentyl, Cyclohexyl, Imidazolyl, Thienyl, Pyridinyl oder Phenyl steht, das gegebenenfalls durch einen oder zwei Substituenten, ausgewählt unter Halogen, $C_{1-4}$-Alkyl, $C_{1-4}$-Alkyloxy und Trifluormethyl, substituiert ist.

**5.** Verbindung nach Anspruch 1, worin Z für einen Rest der Formel (a-1) steht; R Wasserstoff bedeutet; $-X^1=X^2-$ für einen Rest der Formel (x) oder (y) steht; Y Isopropyl, Phenyl oder Halogenphenyl bedeutet; $R^1$ und $R^2$ beide Wasserstoff darstellen; und $R^3$ für Methyl steht; oder worin Z für einen Rest der Formel (a-2) steht; R Wasserstoff ist; $-X^1=X^2-$ für einen Rest der Formel (x) oder (y) steht; Y Cyclopropyl, Phenyl oder Halogenphenyl ist und $R^4$, $R^5$ und $R^6$ jeweils Wasserstoff bedeuten; oder worin Z für einen Rest der Formel (a-3) steht; R Wassserstoff bedeutet; $-X^1=X^2-$ für einen Rest der Formel (x) oder (y) steht; Y Phenyl, Halogenphenyl, Dihalogenphenyl, Methoxyphenyl oder Cyclohexyl bedeutet.

**6.** Verbindung nach Anspruch 1, worin Z für einen Rest der Formel (b-2) steht; $-X^1=X^2-$ einen Rest der Formel (x) oder (y) bedeutet; R Wasserstoff ist; $R^{15}$ Wasserstoff ist; $R^{16}$ Wasserstoff ist; und Y für Phenyl, Halogenphenyl oder Propyl steht; oder worin Z für einen Rest der Formel (b-5) steht; $-X^1=X^2-$ einen Rest der Formel (x) oder (y) bedeutet; R Wasserstoff ist; $R^{20}$ Wasserstoff ist; $R^{21}$ für Wasserstoff, Methyl oder $C_{1-4}$-Alkylphenyl steht; und Y Phenyl oder Halogenphenyl bedeutet; oder worin Z für einen Rest der Formel (b-6) steht; $-X^1=X^2-$ einen Rest der Formel (x) oder (y) darstellt; R Wasserstoff ist; $R^{22}$ Wasserstoff ist; $R^{23}$ Wasserstoff ist; und Y für Phenyl oder Halogenphenyl steht.

**7.** Verbindung nach Anspruch 1, worin Z für einen Rest der Formel (c-1) steht; $-X^1=X^2-$ einen Rest der Formel (x) oder (y) darstellt; R Wasserstoff ist; $R^{24}$ und $R^{25}$ beide Wasserstoff bedeuten und Y Phenyl oder Halogenphenyl darstellt; oder worin Z für einen Rest der Formel (c-2) steht; $-X^1=X^2-$ einen Rest der Formel (x) oder (y) darstellt; R Wasserstoff ist; Y für Wasserstoff, $C_{1-4}$-Alkyl, Cyclopropyl, Cyclopentyl oder Cyclohexyl steht; $R^{26}$ Wasserstoff ist; $R^{27}$ für Wasserstoff, $C_{1-4}$-Alkyl, Naphthalinyl, Thienyl, Pyridinyl, Imidazolyl, Phenyl oder durch 1 oder 2 Substituenten substituiertes Phenyl steht, welche Substituenten jeweils unabhängig unter Methyl, Halogen, Hydroxy und Methoxy ausgewählt sind; und n den Wert 0 hat; oder worin Z für einen Rest der Formel (c-2) steht; $-X^1=X^2-$ einen Rest der Formel (x) oder (y) bedeutet; Y für Phenyl oder Halogenphenyl steht; $R^{26}$ Wasserstoff ist; $R^{27}$ Wasserstoff oder $C_{1-4}$-Alkyl darstellt; und n den Wert 0 hat.

**8.** Pharmazeutische Zusammensetzung, umfassend einen geeigneten pharmazeutischen Träger und als wirksamen Bestandteil eine therapeutisch wirksame Menge einer chemischen Verbindung, wie in einem der Ansprüche 1 bis 7 beansprucht.

**9.** Verfahren zur Herstellung einer pharmazeutischen Zusammensetzung nach Anspruch 8, dadurch gekennzeichnet, daß eine therapeutisch wirksame Menge einer Verbindung nach einem der Ansprüche

1 bis 7 innig mit einem geeigneten pharmazeutischen Träger vermischt wird.

**10.** Verbindung nach einem der Ansprüche 1 bis 7 zur Verwendung als ein Medikament.

**11.** Verfahren zur Herstellung einer chemischen Verbindung nach einem der Ansprüche 1 bis 7, gekennzeichnet durch

a) N-Alkylieren eines Azols der Formel (II) oder eines Alkalimetallsalzes hievon

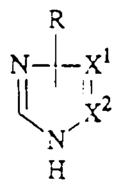 (II) ,

worin R, $X^1$, $X^2$ wie unter Formel (I) definiert sind, mit einem Chinolin-, Chinolinon-, Chinazolin- oder Chinoxalinderivat der Formel

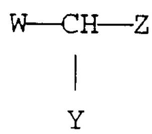 (III),

worin Y und Z wie unter Formel (I) definiert sind und W eine reaktionsfähige Leaving-Gruppe bezeichnet;

b) N-Alkylieren eines Azols der Formel

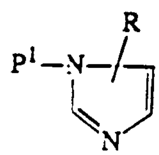 (II-x) ,

worin R wie unter Formel (I) definiert ist und $P^1$ eine Schutzgruppe bezeichnet, mit einem Chinolin-, Chinolinon-, Chinazolin- oder Chinoxalinderivat der Formel

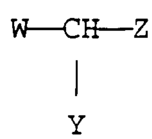 (III),

worin Y und Z wie unter Formel (I) definiert sind und W eine reaktionsfähige Leaving-Gruppe bezeichnet, unter Ausbildung einer Verbindung der Formel

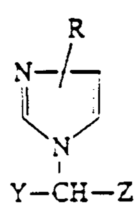 (I-x) ;

c) Endo-N-alkylieren eines Azols der Formel

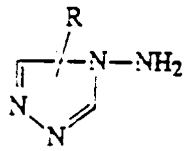 (II-y) ,

worin R wie unter Formel (I) definiert ist, mit einem Chinolin-, Chinolinon-, Chinazolin- oder Chinoxalinderivat der Formel

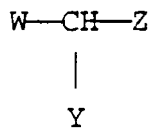 (III),

worin Y und Z wie unter Formel (I) definiert sind und W eine reaktionsfähige Leaving-Gruppe bezeichnet, und anschließendes Deaminieren des so erhaltenen Triazoliumsalzes

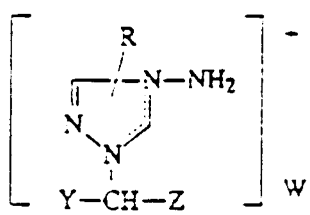 ,

unter Ausbildung einer Verbindung der Formel

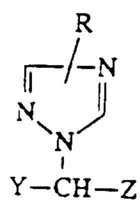 (I-y) ;

d) Umsetzen einer Verbindung der Formel

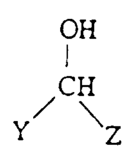 (V)

mit einem Reagens der Formel

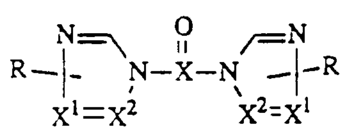 (VI)

in einem geeigneten Lösungsmittel;

e) reduktives Alkylieren eines Ketons oder Aldehyds der Formel

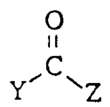

(VII)

mit einem Azol der Formel

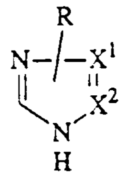

(II)

durch Rühren und Erwärmen der Reagenzien in Ameisensäure oder Formamiden in Anwesenheit eines sauren Katalysators;

f) Cyclisieren eines Zwischenprodukts der Formel

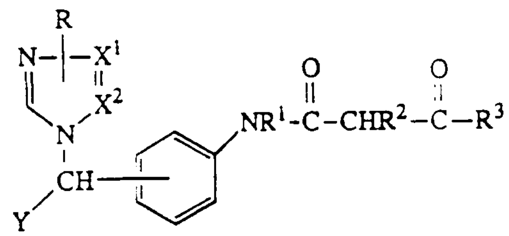

(IX)

in Anwesenheit einer Säure, unter Ausbildung einer Verbindung der Formel

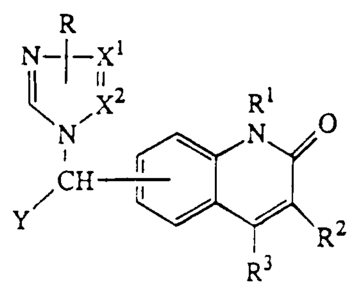

(I-a-1) ;

g) Cyclisieren eines Zwischenprodukts der Formel

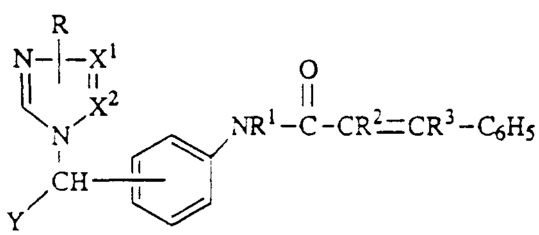

(X)

in Anwesenheit einer geeigneten Lewis-Säure, unter Ausbildung einer Verbindung der Formel (I-a-1);

h) Cyclisieren eines Zwischenprodukts der Formel

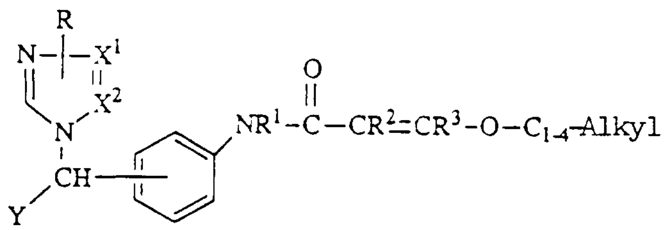

(XI)

in Anwesenheit einer Säure, unter Ausbildung einer Verbindung der Formel (I-a-1);

i) Cyclisieren eines Zwischenprodukts der Formel

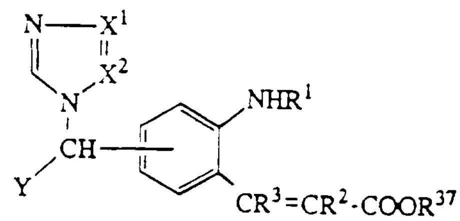

(XII) ,

unter Ausbildung einer Verbindung der Formel (I-a-1);

j) Cyclisieren eines Zwischenprodukts der Formel

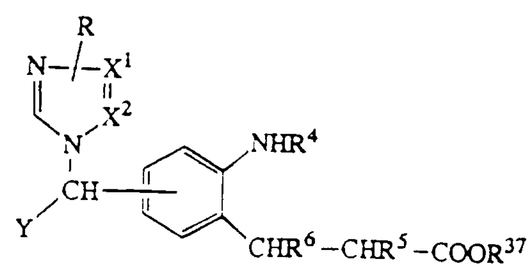

(XIII) ,

unter Ausbildung einer Verbindung der Formel

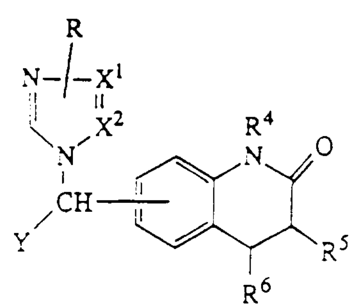

(I-a-2) ;

k) Cyclisieren eines Zwischenprodukts der Formel

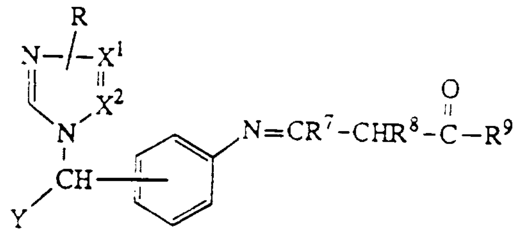

(XIV)

in Anwesenheit eines Dehydratisierungsmittels, unter Ausbildung einer Verbindung der Formel

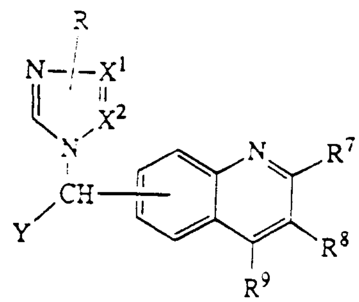

(I-a-3) ;

l) Kondensieren eines Anilins der Formel

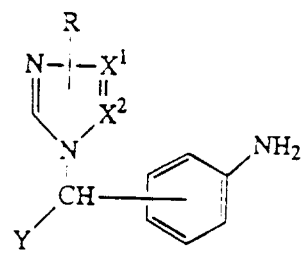

(VIII)

mit einem $\alpha,\beta$-ungesättigten Carbonylsynthon der Formel

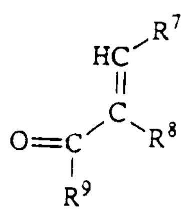

(XV)

in Anwesenheit einer Säure und eines milden Oxidationsmittels, unter Ausbildung einer Verbindung der Formel (I-a-3);

m) Kondensieren eines ortho-Acylanilins der Formel

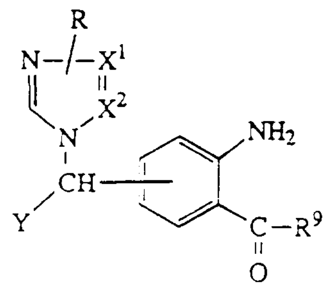

(XVI)

mit einem Keton oder Aldehyd der Formel

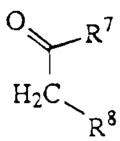

(XVII) ,

unter Ausbildung einer Verbindung der Formel (I-a-3);
n) Cyclisieren eines Zwischenprodukts der Formel

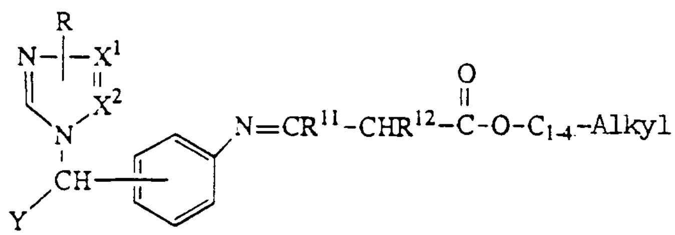

(XVIII)

in Anwesenheit eines geeigneten Dehydratisierungsmittels, unter Ausbildung einer Verbindung der Formel

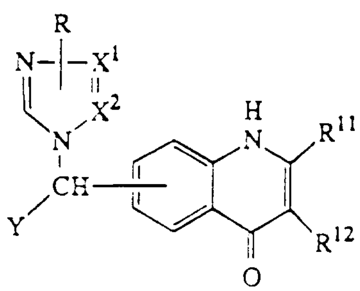

(I-a-4-a) ;

o) Cyclisieren eines Zwischenprodukts der Formel

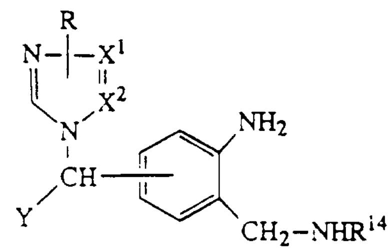

(XIX)

mit einer Carbonsäure der Formel

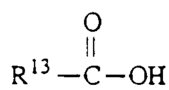

(XX)

oder einem funktionellen Derivat hievon in einem reaktionsinerten Lösungsmittel, unter Ausbildung einer Verbindung der Formel

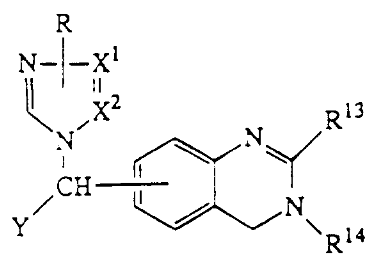

(I-b-1) ;

p) Cyclisieren eines Zwischenprodukts der Formel

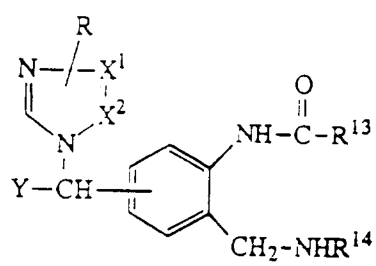

(XXI)

in einem reaktionsinerten Lösungsmittel, unter Ausbildung einer Verbindung der Formel (I-b-1);
q) Cyclisieren eines Zwischenprodukts der Formel

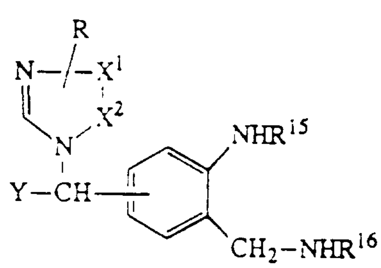

(XIX-a)

mit einem Reagens der Formel L-C(=$X^3$)-L (XXII), worin L eine reaktionsfähige Leaving-Gruppe bezeichnet und $X^3$ Sauerstoff oder Schwefel darstellt, unter Ausbildung einer Verbindung der Formel

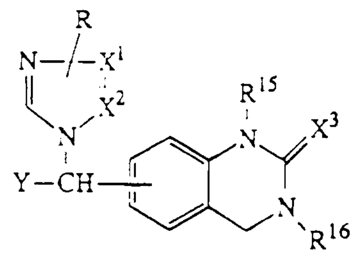

(I-b-2) ;

r) Reduzieren und Kondensieren eines Zwischenprodukts der Formel

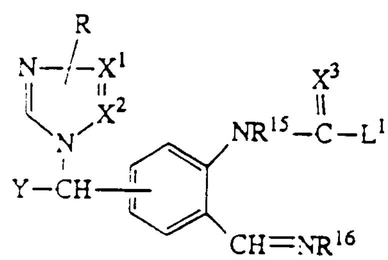

(XXIII) ,

worin $L^1$ eine reaktionsfähige Leaving-Gruppe bezeichnet, in einem reaktionsinerten Lösungsmittel und in Anwesenheit eines Reduktionsmittels, unter Ausbildung einer Verbindung der Formel (I-b-2);
s) Umsetzen eines Zwischenprodukts der Formel

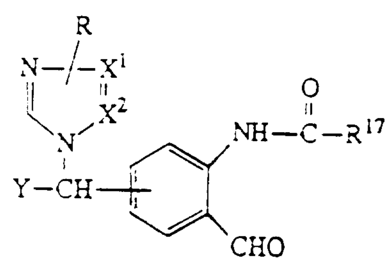

(XXIV)

mit Ammoniak in einem reaktionsinerten Lösungsmittel, unter Ausbildung einer Verbindung der Formel

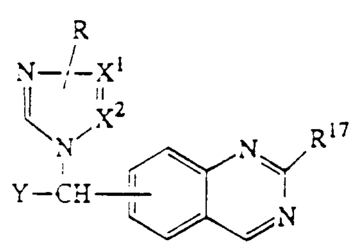

(I-b-3) ;

t) Kondensieren eines Zwischenprodukts der Formel

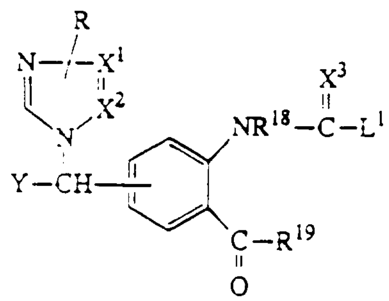

(XXV) ,

worin $L^1$ eine Leaving-Gruppe darstellt, mit Ammoniak, unter Ausbildung einer Verbindung der Formel

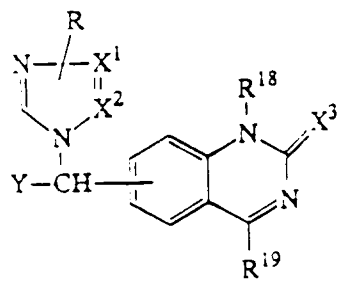

(I-b-4) ;

u) Cyclisieren eines Zwischenprodukts der Formel

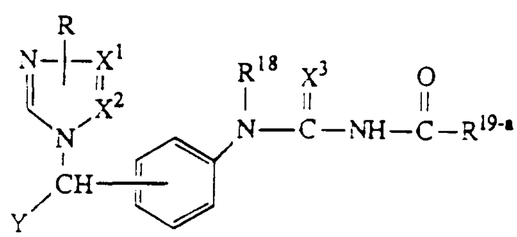

(XXVI)

in Anwesenheit eines Dehydratisierungsmittels, unter Ausbildung einer Verbindung der Formel (I-b-4);

v) Kondensieren eines Zwischenprodukts der Formel

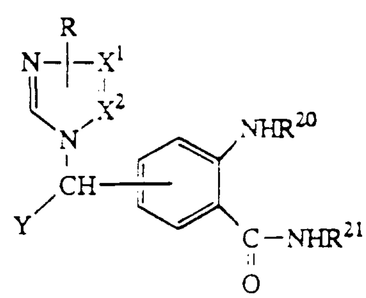

(XXVII)

mit einem Reagens der Formel L-C(=$X^3$)-L (XXII) in einem reaktionsinerten Lösungsmittel, unter Ausbildung einer Verbindung der Formel

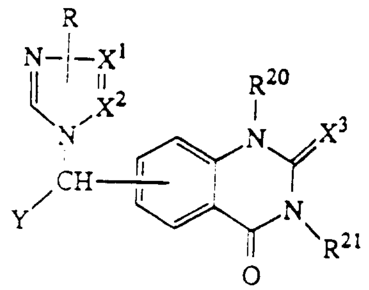

(I-b-5) ;

w) Cyclisieren eines Zwischenprodukts der Formel

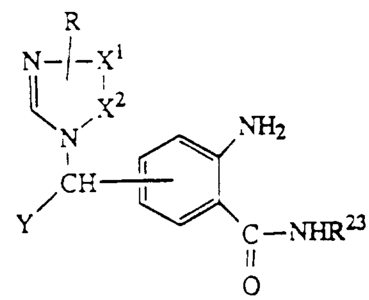

(XXVIII)

mit einer Carbonsäure der Formel $R^{22}$-COOH (XXIX) oder einem funktionellen Derivat hievon, unter Ausbildung einer Verbindung der Formel

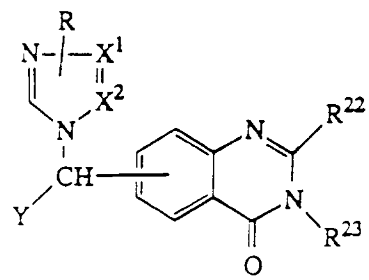

(I-b-6) ;

x) Kondensieren eines ortho-Benzoldiamins der Formel

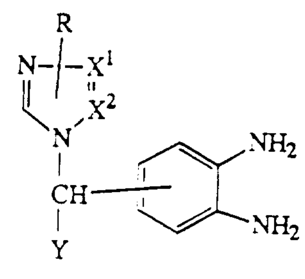

(XXX-a)

mit einem 1,2-Diketon der Formel $R^{24-a}$-C(=O)-C(=O)-$R^{25}$ (XXXI) in einem reaktionsinerten Lösungsmittel, unter Ausbildung einer Verbindung der Formel

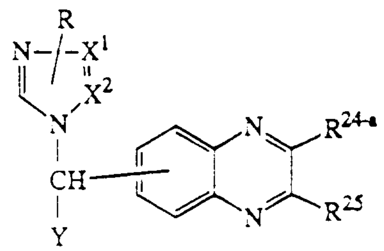

(I-c-1-a) ;

y) Cyclisieren eines (1H-Azol-1-ylmethyl)-substituierten ortho-Diamins der Formel

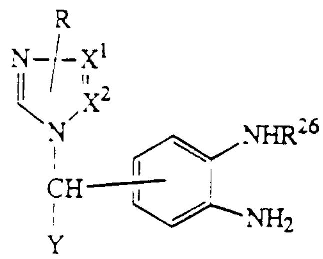

(XXX-b)

mit einer α-Ketosäure der Formel HO-C( = O)-C( = O)-$R^{27}$ oder einem funktionellen Derivat hievon in einem reaktionsinerten Lösungsmittel, unter Ausbildung einer Verbindung der Formel

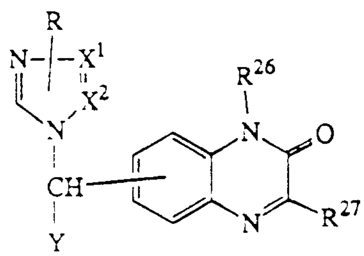

(I-c-2-a) ;

z) Reduzieren und Cyclisieren eines Zwischenprodukts der Formel

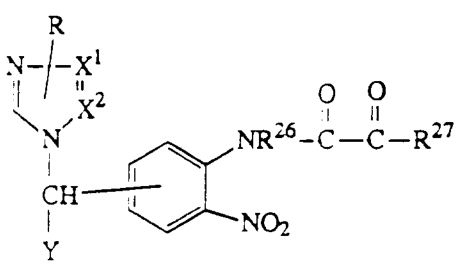

(XXXIII-b)

in einem reaktionsinerten Lösungsmittel, unter Ausbildung einer Verbindung der Formel (I-c-2-a);

aa) Cyclisieren eines ortho-Nitroanilids mit einem Gehalt an einer geeignet aktivierten Methylengruppe mit der Formel

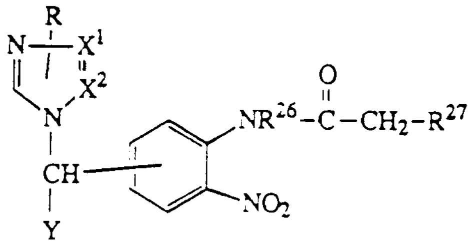

(XXXIV-a)

in einem reaktionsinerten Lösungsmittel, unter Ausbildung einer Verbindung der Formel

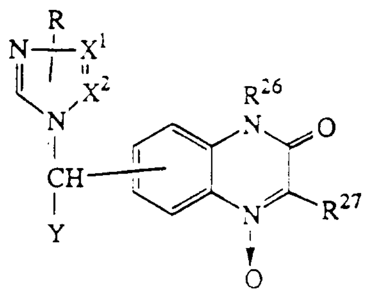

(I-c-2-b) ;

bb) Cyclisieren eines ortho-Anilids der Formel

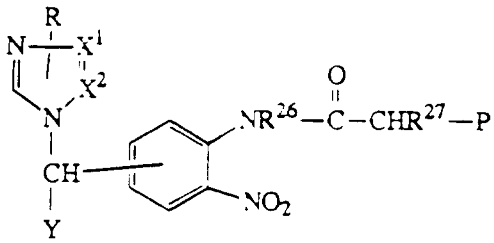

(XXXIV-b) ,

worin P eine Aktivierungsgruppe bedeutet, in einem reaktionsinerten Lösungsmittel, unter Ausbildung einer Verbindung der Formel (I-c-2-b);
cc) Kondensieren eines Zwischenprodukts der Formel

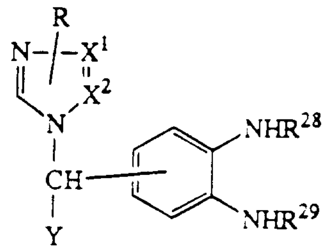

(XXX-c)

mit Oxalsäure HO-C( = O)-C( = O)-OH oder einem funktionellen Derivat hievon, unter Ausbildung einer Verbindung der Formel

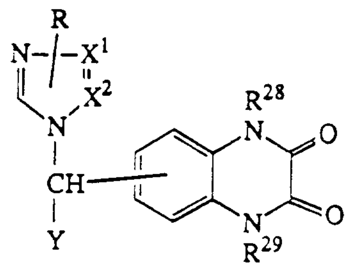

(I-c-3) ;

dd) Kondensieren eines ortho-Diamins der Formel

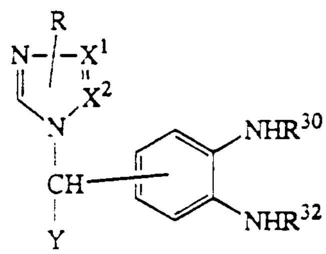

(XXX-d)

mit einer $\alpha$-Halogensäure der Formel

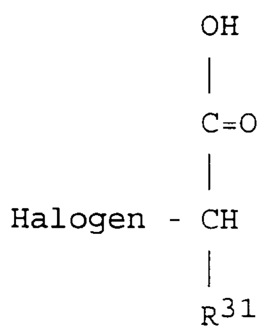

(XXXVI)

oder einem funktionellen Derivat hievon in einem reaktionsinerten Lösungsmittel, unter Ausbildung einer Verbindung der Formel

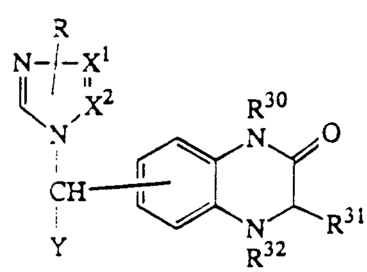

(I-c-4) ;

ee) Reduzieren eines substituierten ortho-Nitrophenylglycins der Formel

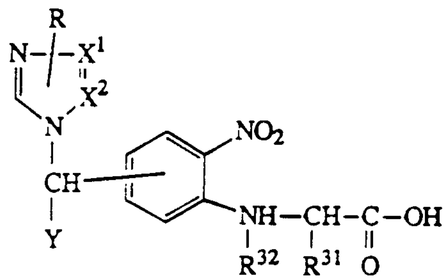

(XXXVII)

in einem reaktionsinerten Lösungsmittel unter Ausbildung einer Verbindung der Formel

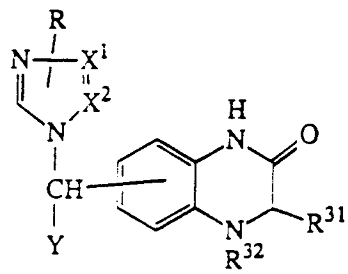

(I-c-4-a) ;

ff) Cyclisieren eines Zwischenprodukts der Formel

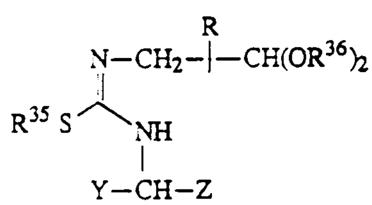

(XXXVIII)

und Desulfurieren des so erhaltenen Zwischenprodukts der Formel

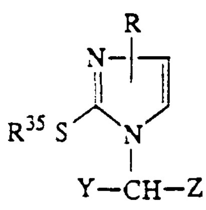

(IXL) ,

unter Ausbildung einer Verbindung der Formel

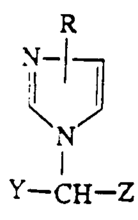

(I-x) ;

gg) Umsetzen eines Zwischenprodukts der Formel

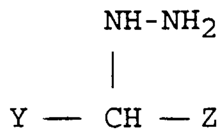 (XL)

mit s-Triazin, unter Ausbildung einer Verbindung der Formel

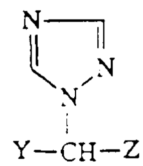 (I-y) ;

oder gegebenenfalls Überführen der Verbindungen der Formel (I) ineinander nach bekannten Gruppentransformationsverfahren, und gewünschtenfalls Überführen der Verbindungen der Formel (I) in eine therapeutisch wirksame nicht-toxische Säureadditionssalzform durch Behandlung mit einer entsprechenden Säure, oder umgekehrt Überführen des Säureadditionssalzes in die freie Basenform mit Alkali; und/oder Bereiten stereochemisch isomerer Formen hievon.

**Patentanspruch für folgende Vertragsstaaten : ES, GR**

**1.** Verfahren zur Herstellung einer chemischen Verbindung mit der Formel

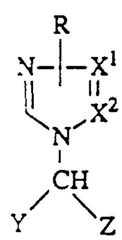 (I) ,

eines pharmazeutisch annehmbaren Säureadditionssalzes hievon oder einer stereochemisch isomeren Form hievon, worin

$-X^1=X^2-$ einen zweiwertigen Rest mit der Formel

-CH = CH- (x),

-CH = N- (y) oder

-N = CH- (z)

bedeutet;

R für Wasserstoff oder $C_{1-6}$-Alkyl steht;

Y für Wasserstoff, $C_{1-10}$-Alkyl, $C_{3-6}$-Cycloalkyl, $Ar^1$, $Ar^2$-$C_{1-6}$-Alkyl, $C_{2-6}$-Alkenyl oder $C_{2-6}$-Alkinyl steht;

Z einen Rest der Formel

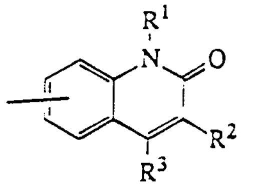 , 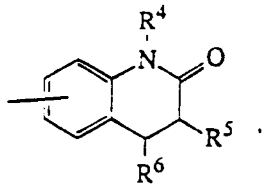 , 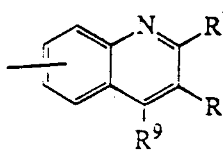 oder 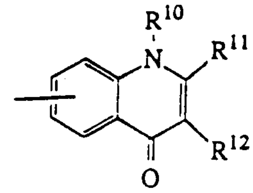

(a-1) (a-2) (a-3) (a-4)

darstellt, worin

$R^1$, $R^4$ und $R^{10}$ jeweils unabhängig Wasserstoff, $C_{1-6}$-Alkyl oder $Ar^2$-$C_{1-6}$-Alkyl bedeuten;

$R^2$, $R^5$, $R^8$ und $R^{12}$ jeweils unabhängig Wasserstoff, $C_{1-6}$-Alkyl oder $Ar^2$ bedeuten;

$R^3$, $R^6$ und $R^{11}$ jeweils unabhängig Wasserstoff oder $C_{1-6}$-Alkyl bedeuten;

$R^7$ und $R^9$ jeweils unabhängig Wasserstoff, $C_{1-6}$-Alkyl, $C_{1-6}$-Alkyloxy, Halogen, Amino oder Mono- oder Di($C_{1-6}$-alkyl)amino bedeuten; oder

Z einen Rest der Formel

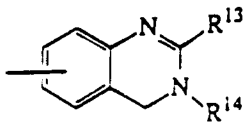 , 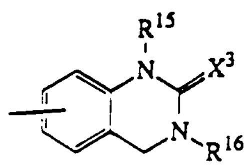 , 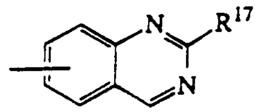 ,

(b-1) (b-2) (b-3)

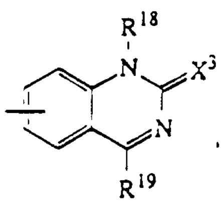 , 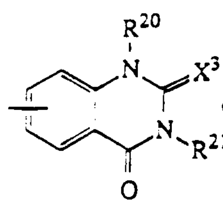 oder 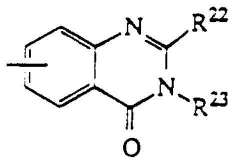

(b-4) (b-5) (b-6)

darstellt, worin

$R^{13}$, $R^{17}$ und $R^{22}$ jeweils unabhängig Wasserstoff, Halogen, $C_{1-6}$-Alkyl, Trifluormethyl, $C_{1-6}$-Alkyloxy, $Ar^2$, $Ar^2$-$C_{1-6}$-Alkyl, Amino oder Mono- oder Di($C_{1-6}$-alkyl)amino bedeuten;

$R^{14}$, $R^{16}$ und $R^{21}$ jeweils unabhängig Wasserstoff, $C_{1-6}$-Alkyl, $Ar^2$ oder $Ar^2$-$C_{1-6}$-Alkyl bedeuten;

$R^{15}$, $R^{18}$ und $R^{20}$ jeweils unabhängig Wasserstoff, $C_{1-6}$-Alkyl oder $Ar^2$-$C_{1-6}$-Alkyl bedeuten;

$R^{19}$ Wasserstoff oder $C_{1-6}$-Alkyl bedeutet;

$R^{23}$ Wasserstoff, $C_{1-6}$-Alkyl, $Ar^2$-$C_{1-6}$-Alkyl, Amino oder Mono($C_{1-6}$-alkyl)amino bedeutet;

$X^3$ für O oder S steht; oder

Z einen Rest der Formel

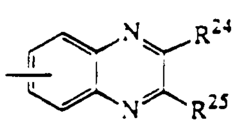 , 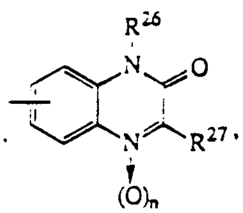 , 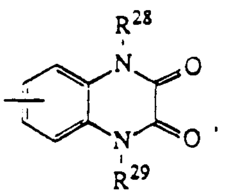 , 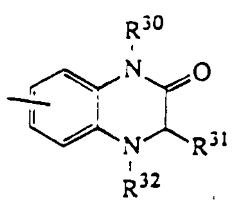 , 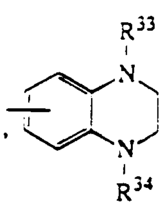

(c-1) (c-2) (c-3) (c-4) (c-5)

darstellt, worin

$R^{24}$ Wasserstoff, Halogen, $C_{1-6}$-Alkyl, $C_{1-6}$-Alkyloxy, Amino, Mono- oder Di($C_{1-6}$-alkyl)amino, $Ar^2$ oder Imidazolyl bedeutet;

$R^{25}$ Wasserstoff, $C_{1-6}$-Alkyl oder $Ar^1$ bedeutet;

$R^{26}$ und $R^{30}$ jeweils unabhängig Wasserstoff, $C_{1-6}$-Alkyl, $Ar^2$-$C_{1-6}$-Alkyl, Amino oder Mono($C_{1-6}$-alkyl)amino bedeuten;

$R^{27}$ und $R^{31}$ jeweils unabhängig Wasserstoff, $C_{1-6}$-Alkyl, $Ar^1$, $C_{1-6}$-Alkylcarbonyl, $Ar^2$-Carbonyl, $C_{1-6}$-Alkyloxycarbonyl, Carboxyl, $C_{1-6}$-Alkyloxycarbonyl-$C_{1-4}$-alkyl, Aminocarbonyl oder Cyano bedeuten;

$R^{28}$, $R^{29}$, $R^{32}$, $R^{33}$ und $R^{34}$ jeweils unabhängig Wasserstoff, $C_{1-6}$-Alkyl oder $Ar^2$-$C_{1-6}$-Alkyl bedeuten;

n den Wert 0 oder 1 aufweist; und

$Ar^1$ für Phenyl, substituiertes Phenyl, Naphthalinyl, Pyridinyl, Imidazolyl, Triazolyl, Thienyl, Furanyl oder Thiazolyl steht und $Ar^2$ Phenyl oder substituiertes Phenyl bedeutet; wobei das genannte substituierte Phenyl in $Ar^1$ oder $Ar^2$ ein Phenyl ist, das durch 1, 2 oder 3 Substituenten substituiert ist, die jeweils unabhängig unter Halogen, Hydroxy, Trifluormethyl, $C_{1-6}$-Alkyl, $C_{1-6}$-Alkyloxy, Cyano, Amino, Mono- und Di($C_{1-6}$-alkyl)amino, Nitro, Carboxyl, Formyl und $C_{1-6}$-Alkyloxycarbonyl ausgewählt sind, gekennzeichnet durch

a) N-Alkylieren eines Azols der Formel (II) oder eines Alkalimetallsalzes hievon

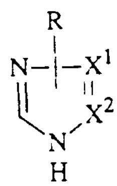 (II) ,

worin R, $X^1$, $X^2$ wie unter Formel (I) definiert sind, mit einem Chinolin-, Chinolinon-, Chinazolin- oder Chinoxalinderivat der Formel

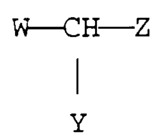 (III),

worin Y und Z wie unter Formel (I) definiert sind und W eine reaktionsfähige Leaving-Gruppe bezeichnet;

b) N-Alkylieren eines Azols der Formel

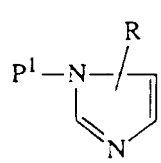 (II-x) ,

worin R wie unter Formel (I) definiert ist und $P^1$ eine Schutzgruppe bezeichnet, mit einem Chinolin-, Chinolinon-, Chinazolin- oder Chinoxalinderivat der Formel

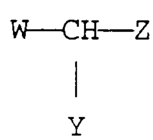 (III),

worin Y und Z wie unter Formel (I) definiert sind und W eine reaktionsfähige Leaving-Gruppe

bezeichnet, unter Ausbildung einer Verbindung der Formel

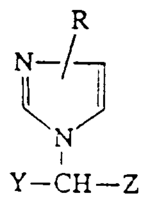

(I-x) ;

c) Endo-N-alkylieren eines Azols der Formel

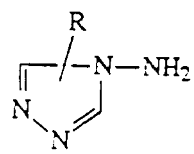

(II-y) ,

worin R wie unter Formel (I) definiert ist, mit einem Chinolin-, Chinolinon-, Chinazolin- oder Chinoxalinderivat der Formel

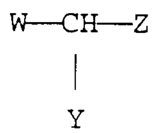

(III),

worin Y und Z wie unter Formel (I) definiert sind und W eine reaktionsfähige Leaving-Gruppe bezeichnet, und anschließendes Deaminieren des so erhaltenen Triazoliumsalzes

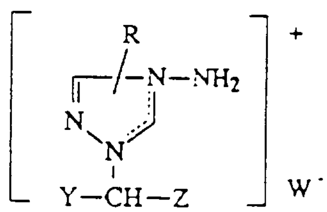

,

unter Ausbildung einer Verbindung der Formel

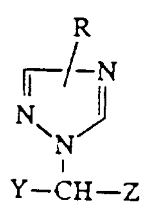

(I-y) ;

d) Umsetzen einer Verbindung der Formel

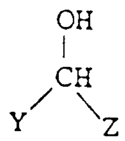

(V)

mit einem Reagens der Formel

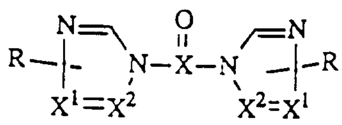

(VI)

in einem geeigneten Lösungsmittel;

e) reduktives Alkylieren eines Ketons oder Aldehyds der Formel

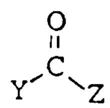

(VII)

mit einem Azol der Formel

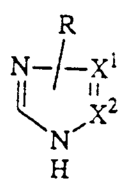

(II)

durch Rühren und Erwärmen der Reagenzien in Ameisensäure oder Formamiden in Anwesenheit eines sauren Katalysators;

f) Cyclisieren eines Zwischenprodukts der Formel

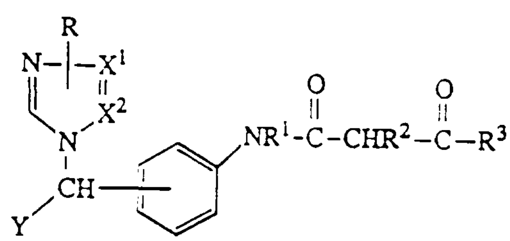

(IX)

in Anwesenheit einer Säure, unter Ausbildung einer Verbindung der Formel

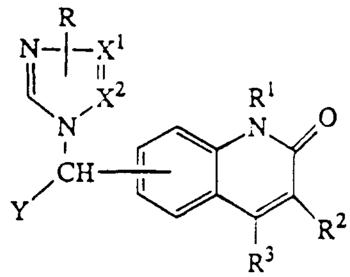

(I-a-1) ;

g) Cyclisieren eines Zwischenprodukts der Formel

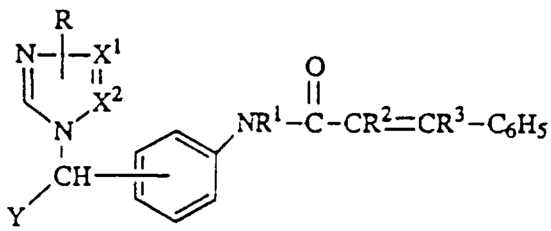

(X)

in Anwesenheit einer geeigneten Lewis-Säure, unter Ausbildung einer Verbindung der Formel (I-a-1);

h) Cyclisieren eines Zwischenprodukts der Formel

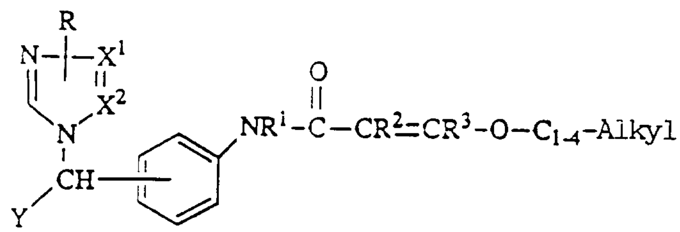

(XI)

in Anwesenheit einer Säure, unter Ausbildung einer Verbindung der Formel (I-a-1);

i) Cyclisieren eines Zwischenprodukts der Formel

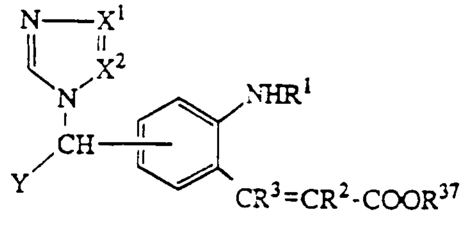

(XII) ,

unter Ausbildung einer Verbindung der Formel (I-a-1);

j) Cyclisieren eines Zwischenprodukts der Formel

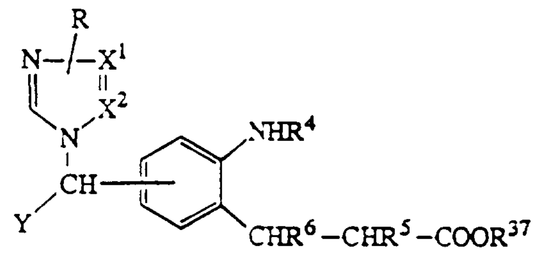

(XIII) ,

unter Ausbildung einer Verbindung der Formel

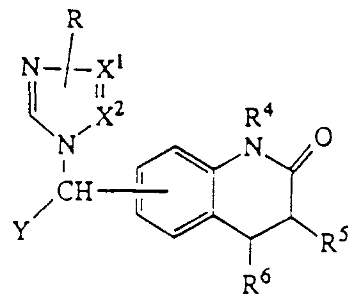

(I-a-2) ;

k) Cyclisieren eines Zwischenprodukts der Formel

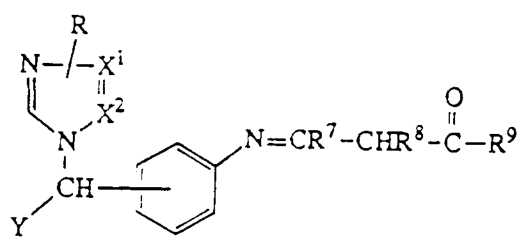

(XIV)

in Anwesenheit eines Dehydratisierungsmittels, unter Ausbildung einer Verbindung der Formel

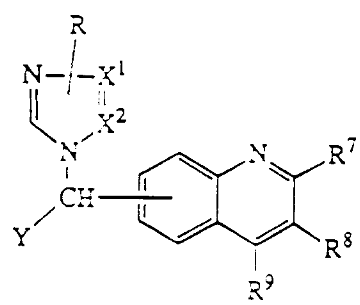

(I-a-3) ;

l) Kondensieren eines Anilins der Formel

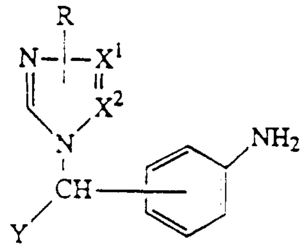

(VIII)

mit einem $\alpha,\beta$-ungesättigten Carbonylsynthon der Formel

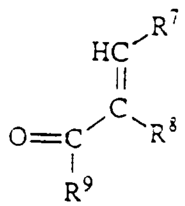

(XV)

in Anwesenheit einer Säure und eines milden Oxidationsmittels, unter Ausbildung einer Verbindung der Formel (I-a-3);

m) Kondensieren eines ortho-Acylanilins der Formel

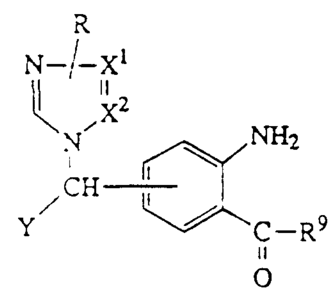

(XVI)

mit einem Keton oder Aldehyd der Formel

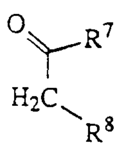

(XVII) ,

unter Ausbildung einer Verbindung der Formel (I-a-3);

n) Cyclisieren eines Zwischenprodukts der Formel

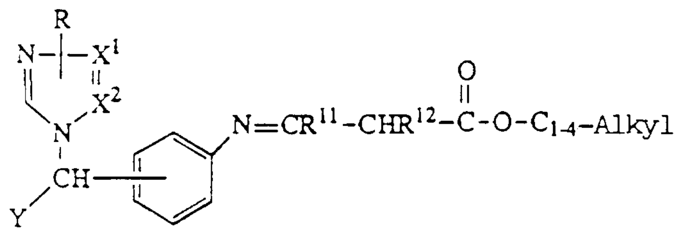

(XVIII)

in Anwesenheit eines geeigneten Dehydratisierungsmittels, unter Ausbildung einer Verbindung der Formel

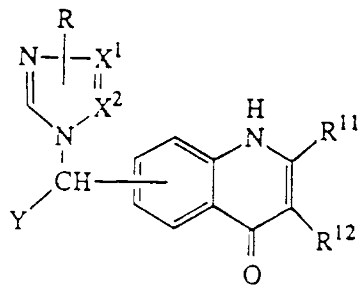

(I-a-4-a) ;

o) Cyclisieren eines Zwischenprodukts der Formel

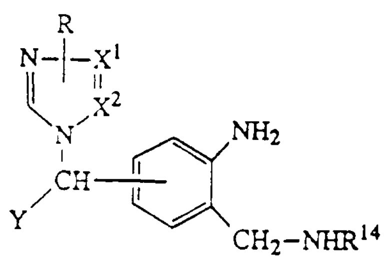

(XIX)

mit einer Carbonsäure der Formel

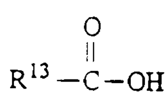

(XX)

oder einem funktionellen Derivat hievon in einem reaktionsinerten Lösungsmittel, unter Ausbildung einer Verbindung der Formel

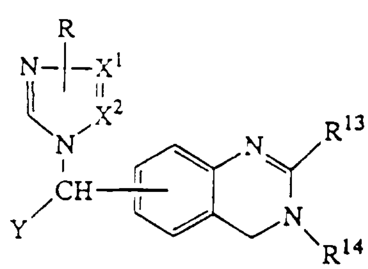

(I-b-1) ;

p) Cyclisieren eines Zwischenprodukts der Formel

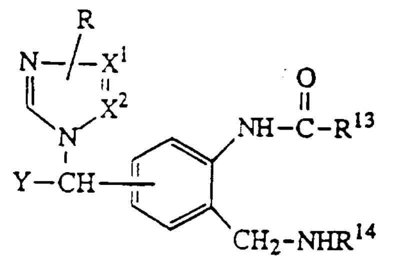

(XXI)

in einem reaktionsinerten Lösungsmittel, unter Ausbildung einer Verbindung der Formel (I-b-1);
q) Cyclisieren eines Zwischenprodukts der Formel

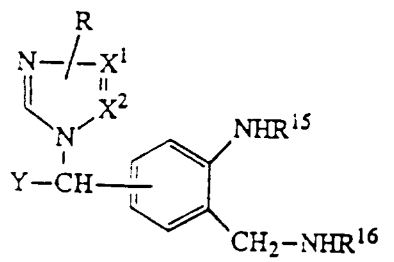

(XIX-a)

mit einem Reagens der Formel L-C(=$X^3$)-L (XXII), worin L eine reaktionsfähige Leaving-Gruppe bezeichnet und $X^3$ Sauerstoff oder Schwefel darstellt, unter Ausbildung einer Verbindung der Formel

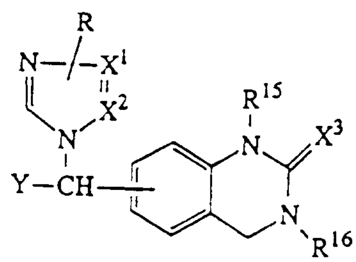

(I-b-2) ;

r) Reduzieren und Kondensieren eines Zwischenprodukts der Formel

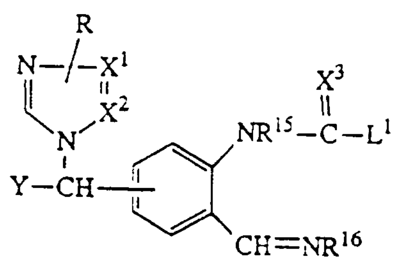

(XXIII) ,

worin $L^1$ eine reaktionsfähige Leaving-Gruppe bezeichnet, in einem reaktionsinerten Lösungsmittel und in Anwesenheit eines Reduktionsmittels, unter Ausbildung einer Verbindung der Formel (I-b-2);

s) Umsetzen eines Zwischenprodukts der Formel

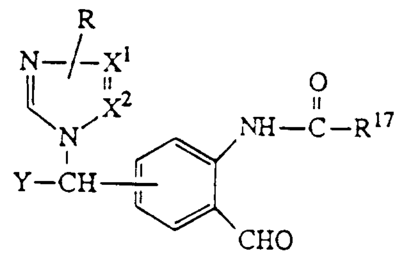

(XXIV)

mit Ammoniak in einem reaktionsinerten Lösungsmittel, unter Ausbildung einer Verbindung der Formel

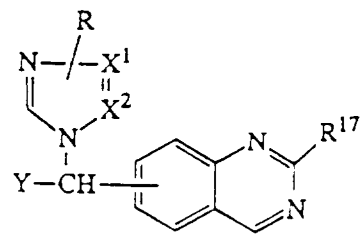

(I-b-3) ;

t) Kondensieren eines Zwischenprodukts der Formel

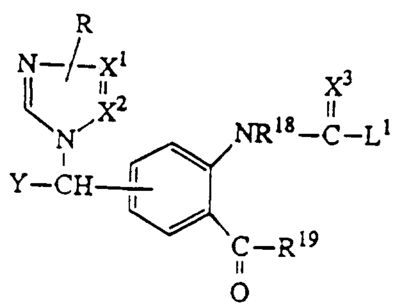

(XXV) ,

worin $L^1$ eine Leaving-Gruppe darstellt, mit Ammoniak, unter Ausbildung einer Verbindung der Formel

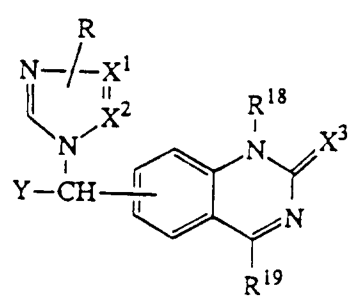

(I-b-4) ;

u) Cyclisieren eines Zwischenprodukts der Formel

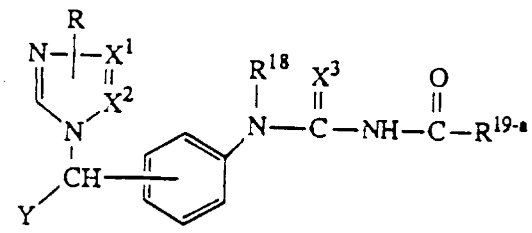

(XXVI)

in Anwesenheit eines Dehydratisierungsmittels, unter Ausbildung einer Verbindung der Formel (I-b-4);

v) Kondensieren eines Zwischenprodukts der Formel

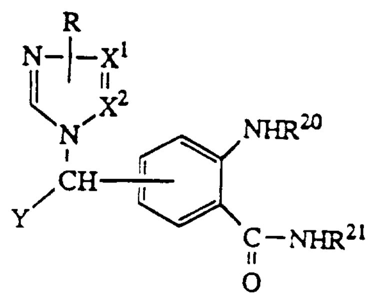

(XXVII)

mit einem Reagens der Formel L-C(=$X^3$)-L (XXII) in einem reaktionsinerten Lösungsmittel, unter Ausbildung einer Verbindung der Formel

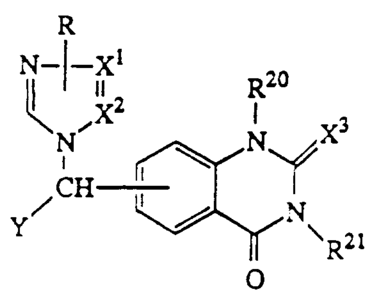

(I-b-5) ;

w) Cyclisieren eines Zwischenprodukts der Formel

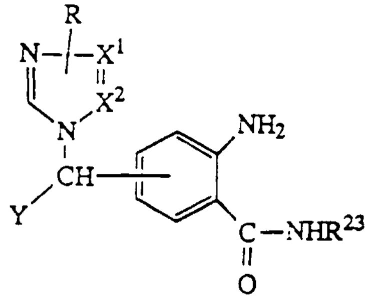

(XXVIII)

mit einer Carbonsäure der Formel $R^{22}$-COOH (XXIX) oder einem funktionellen Derivat hievon, unter Ausbildung einer Verbindung der Formel

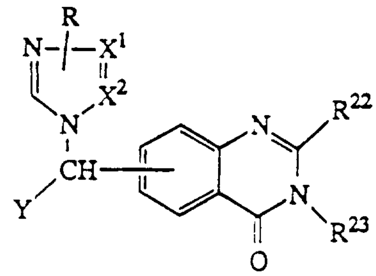

(I-b-6) ;

x) Kondensieren eines ortho-Benzoldiamins der Formel

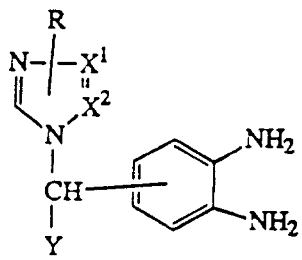

(XXX-a)

mit einem 1,2-Diketon der Formel $R^{24-a}$-C(=O)-C(=O)-$R^{25}$ (XXXI) in einem reaktionsinerten Lösungsmittel, unter Ausbildung einer Verbindung der Formel

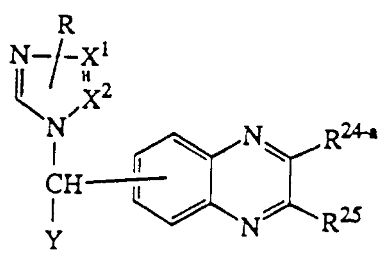

(I-c-1-a) ;

y) Cyclisieren eines (1H-Azol-1-ylmethyl)-substituierten ortho-Diamins der Formel

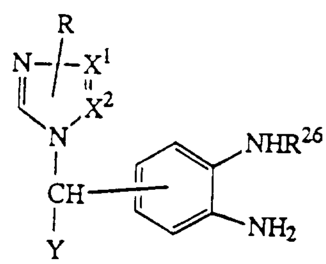

(XXX-b)

mit einer α-Ketosäure der Formel HO-C(=O)-C(=O)-$R^{27}$ oder einem funktionellen Derivat hievon in einem reaktionsinerten Lösungsmittel, unter Ausbildung einer Verbindung der Formel

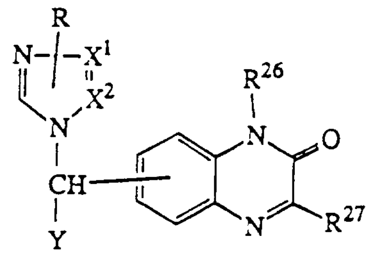

(I-c-2-a) ;

z) Reduzieren und Cyclisieren eines Zwischenprodukts der Formel

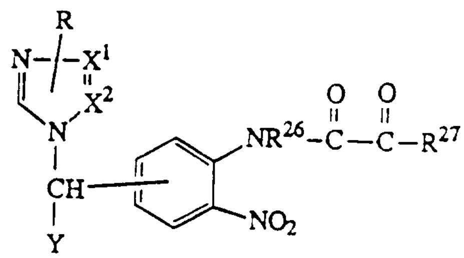

(XXXIII-b)

in einem reaktionsinerten Lösungsmittel, unter Ausbildung einer Verbindung der Formel (I-c-2-a);
aa) Cyclisieren eines ortho-Nitroanilids mit einem Gehalt an einer geeignet aktivierten Methylengruppe mit der Formel

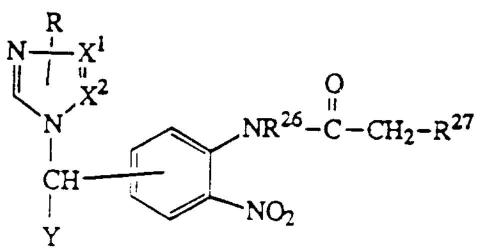

(XXXIV-a)

in einem reaktionsinerten Lösungsmittel, unter Ausbildung einer Verbindung der Formel

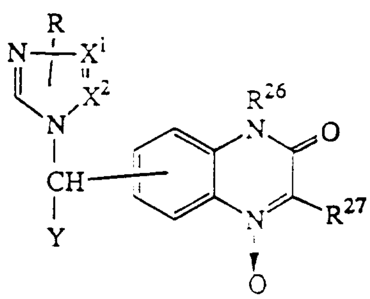

(I-c-2-b) ;

bb) Cyclisieren eines ortho-Anilids der Formel

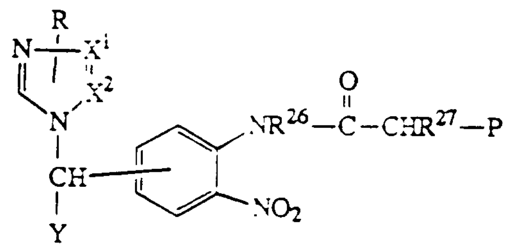

(XXXIV-b) ,

worin P eine Aktivierungsgruppe bedeutet, in einem reaktionsinerten Lösungsmittel, unter Ausbildung einer Verbindung der Formel (I-c-2-b);

cc) Kondensieren eines Zwischenprodukts der Formel

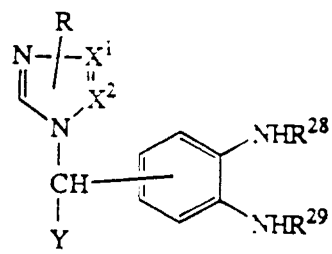

(XXX-c)

mit Oxalsäure HO-C( = O)-C( = O)-OH oder einem funktionellen Derivat hievon, unter Ausbildung einer Verbindung der Formel

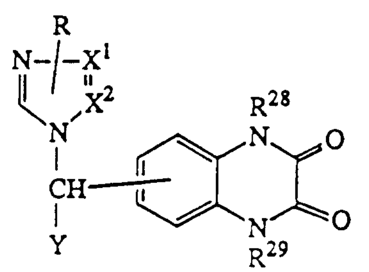

(I-c-3) ;

dd) Kondensieren eines ortho-Diamins der Formel

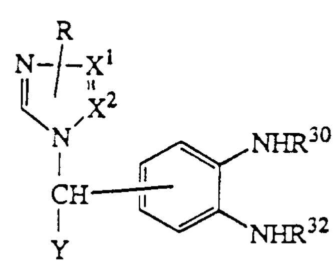

(XXX-d)

mit einer $\alpha$-Halogensäure der Formel

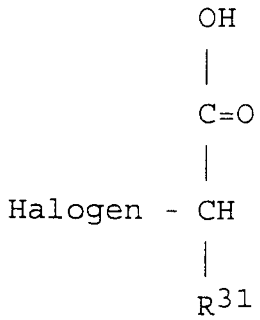 (XXXVI)

oder einem funktionellen Derivat hievon in einem reaktionsinerten Lösungsmittel, unter Ausbildung einer Verbindung der Formel

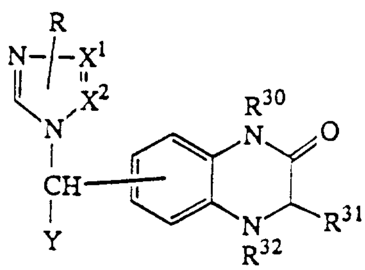 (I-c-4) ;

ee) Reduzieren eines substituierten ortho-Nitrophenylglycins der Formel

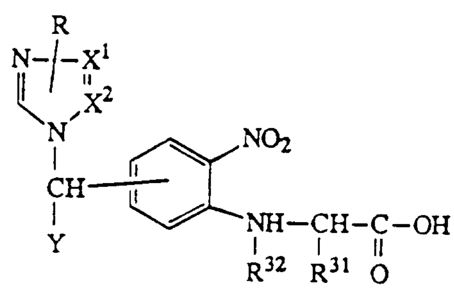 (XXXVII)

in einem reaktionsinerten Lösungsmittel unter Ausbildung einer Verbindung der Formel

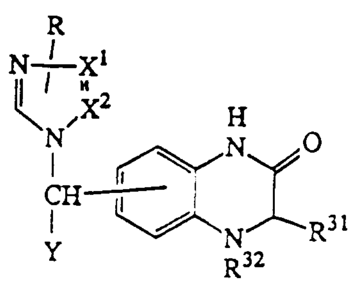 (I-c-4-a) ;

ff) Cyclisieren eines Zwischenprodukts der Formel

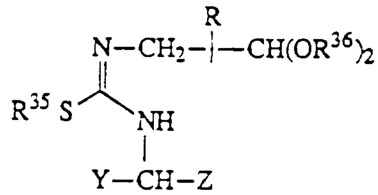 (XXXVIII)

und Desulfurieren des so erhaltenen Zwischenprodukts der Formel

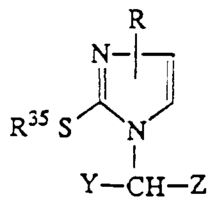 (IXL) ,

unter Ausbildung einer Verbindung der Formel

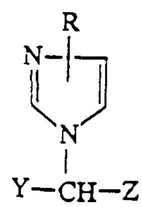 (I-x) ;

gg) Umsetzen eines Zwischenprodukts der Formel

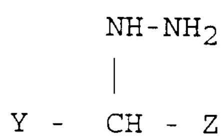 (XL)

mit s-Triazin, unter Ausbildung einer Verbindung der Formel

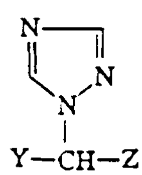 (I-y) ;

oder gegebenenfalls Überführen der Verbindungen der Formel (I) ineinander nach bekannten Gruppentransformationsverfahren, und gewünschtenfalls Überführen der Verbindungen der Formel (I) in eine therapeutisch wirksame nicht-toxische Säureadditionssalzform durch Behandlung mit einer entsprechenden Säure, oder umgekehrt Überführen des Säureadditionssalzes in die freie Basenform mit Alkali; und/oder Bereiten stereochemisch isomerer Formen hievon.

## Revendications

**Revendications pour les Etats contractants suivants : AT, BE, CH, DE, FR, GB, IT, LI, LU, NL, SE**

**1.** Composé chimique ayant la formule

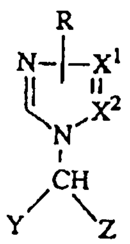

(I)

un sel d'addition d'acide acceptable sur le plan pharmaceutique de celui-ci ou une forme stéréochimiquement isomère de celui-ci, dans lequel
$-X^1=X^2-$ est un radical bivalent ayant la formule

-CH = CH- (x),

-CH = N- (y), ou

-N = CH- (z);

R est un atome d'hydrogène ou un groupe alkyle en $C_1$ à $C_6$;
Y est un atome d'hydrogène, un groupe alkyle en $C_1$ à $C_{10}$, cycloalkyle en $C_3$ à $C_7$, $Ar^1$, $Ar^2$-alkyle(en $C_1$ à $C_6$), alcényle en $C_2$ à $C_6$ ou alcynyle en $C_2$ à $C_6$;
Z est un radical de formule

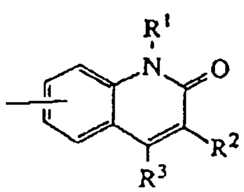

(a-1)

,

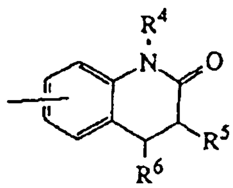

(a-2)

,

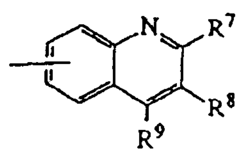

(a-3)

ou

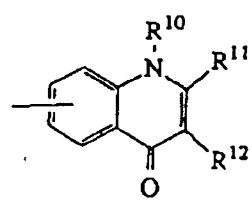

(a-4)

dans laquelle
$R^1$, $R^4$ et $R^{10}$ sont chacun indépendamment un atome d'hydrogène, un groupe alkyle en $C_1$ à $C_6$ ou $Ar^2$-alkyle(en $C_1$ à $C_6$);
$R^2$, $R^5$, $R^8$ et $R^{12}$ sont chacun indépendamment un atome d'hydrogène, un groupe alkyle en $C_1$ à $C_6$ ou $Ar^2$ ;
$R^3$, $R^6$ et $R^{11}$ sont chacun indépendamment un atome d'hydrogène, ou un groupe alkyle en $C_1$ à $C_6$ ;
$R^7$ et $R^9$ sont chacun indépendamment un atome d'hydrogène, un radical alkyle en $C_1$ à $C_6$, alkyloxy en $C_1$ à $C_6$, halogéno, amino, ou mono ou di(alkyl en $C_1$ à $C_6$) amino; ou
Z est un radical de formule

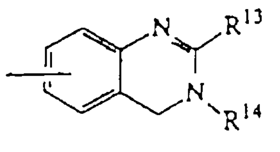,
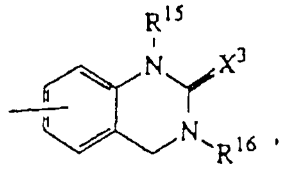,
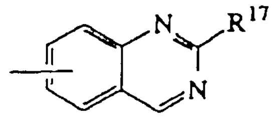

(b-1) (b-2) (b-3)

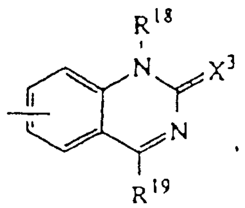,
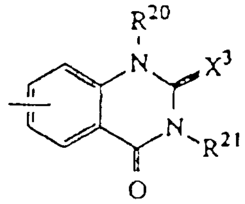
or
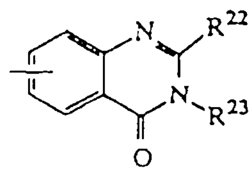

(b-4) (b-5) (b-6)

dans laquelle

$R^{13}$, $R^{17}$ et $R^{22}$ sont chacun indépendamment un atome d'hydrogène, un radical halogéno, alkyle en $C_1$ à $C_6$, trifluorométhyle, alkyloxy en $C_1$ à $C_6$, $Ar^2$, $Ar^2$ -alkyle(en $C_1$ à $C_6$), amino ou mono- ou di(alkyl en $C_1$ à $C_6$) amino;

$R^{14}$, $R^{16}$ et $R^{21}$ sont chacun indépendamment un atome d'hydrogène, un groupe alkyle en $C_1$ à $C_6$, $Ar^2$ ou $Ar^2$-alkyle(en $C_1$ à $C_6$ );

$R^{15}$, $R^{18}$ et $R^{20}$ sont chacun indépendamment un atome d'hydrogène, un groupe alkyle en $C_1$ à $C_6$ ou $Ar^2$-alkyle(en $C_1$ à $C_6$ );

$R^{19}$ est un atome d'hydrogène ou un groupe alkyle en $C_1$ à $C_6$;

$R^{23}$ est un atome d'hydrogène, un groupe alkyle en $C_1$ à $C_6$ , $Ar^2$-alkyle(en $C_1$ à $C_6$), amino ou mono-(alkyl en $C_1$ à $C_6$) amino;

$X^3$ est O ou S ; ou

Z est un radical de formule

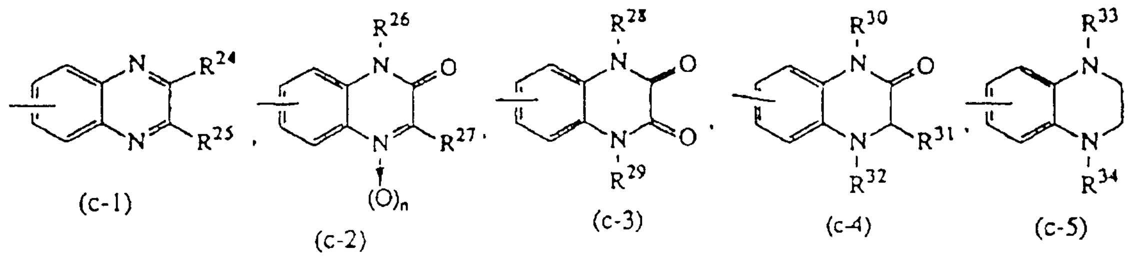

(c-1) (c-2) (c-3) (c-4) (c-5)

dans laquelle

$R^{24}$ est un atome d'hydrogène, d'halogène, un groupe alkyle en $C_1$ à $C_6$, alkyloxy en $C_1$ en $C_6$, amino, mono- ou di(alkyl en $C_1$ à $C_6$)amino, $Ar^2$ ou imidazolyle;

$R^{25}$ est un atome d'hydrogène, un groupe alkyle en $C_1$ à $C_6$ ou $Ar^1$;

$R^{26}$ et $R^{30}$ sont chacun indépendamment un atome d'hydrogène, un groupe alkyle en $C_1$ à $C_6$, $Ar^2$-alkyle(en $C_1$ à $C_6$), un groupe amino, ou mono(alkyl en $C_1$ à $C_6$) amino;

$R^{27}$ et $R^{31}$ sont chacun indépendamment un atome d'hydrogène, un groupe alkyle en $C_1$ à $C_6$, $Ar^1$, alkyl(en $C_1$ à $C_6$)carbonyle, $Ar^2$-carbonyle, alkyl(en $C_1$ à $C_6$ )oxycarbonyle, carboxyle, alkyl(en $C_1$ à $C_6$ )oxycarbonyle-alkyle(en $C_1$ à C4), aminocarbonyle ou cyano;

$R^{28}$, $R^{29}$, $R^{32}$ et $R^{34}$ sont chacun indépendamment un atome d'hydrogène, un groupe alkyle en $C_1$ à $C_6$ ou $Ar^2$-alkyle(en $C_1$ à $C_6$ );

n vaut O ou 1; et

$Ar^1$ est un groupe phényle, phényle substitué, naphtalényle, pyridinyle, imidazolyle, triasolyle, thiényle,

furanyle ou thiazolyle et $Ar^2$ est un groupe phényle ou phényle substitué; ledit groupe phényle substitué en $Ar^1$ ou $Ar^2$ étant un groupe phényle substitué avec 1, 2 ou 3 substituants choisis chacun indépendamment parmi les radicaux halogéno, hydroxy, trifluorométhyle, alkyle en $C_1$ à $C_6$, alkyloxy en $C_1$ à $C_6$, cyano, amino, mono- et di(alkyle en $C_1$ à $C_6$)amino, nitro, carboxyle, formyle et alkyl(en $C_1$ à $C_6$)oxycarbonyle.

**2.** Compose selon la revendication 1, dans lequel R est un atome d'hydrogène ou un groupe alkyle en $C_1$ à $C_4$; Y est un atome d'hydrogène, un groupe alkyle en $C_1$ à $C_6$, un groupe cycloalkyle en $C_3$ à $C_7$, un groupe phényle, phényle substitué, pyridinyle, imidazolyle ou thiényle; et Z est un radical de formule (a-1), (a-2), (a-3) ou (a-4) dans lequel $R^1$, $R^2$, $R^3$, $R^4$, $R^5$, $R^6$, $R^8$, $R^{10}$, $R^{11}$ et $R^{12}$ sont chacun indépendamment un atome d'hydrogène ou un groupe alkyle en $C_1$ à $C_4$, et $R^7$ et $R^9$ sont chacun indépendamment un atome d hydrogène un groupe alkyle en $C_1$ à $C_4$, alkyloxy en $C_1$ à $C_4$ ou un atome d'halogène; ou Z est un radical de formule (b-1), (b-2), (b-3), (b-4), (b-5) ou (b-6) dans laquelle $R^{13}$ est un atome d'hydrogène, un groupe alkyle en $C_1$ à $C_4$, un groupe trifluorométhyle ou phényle, R14 est un atome d'hydrogène, un groupe alkyle en $C_1$ à $C_4$, un groupe phényle ou phénylalkyle en $C_1$ à $C_4$, $R^{15}$ est un atome d hydrogène ou un groupe alkyle en $C_1$ à $C_4$ substitué avec un phényle, $R^{16}$, $R^{17}$, $R^{18}$, $R^{19}$ et $R^{20}$ sont chacun indépendamment un atome d hydrogène ou un groupe alkyle en $C_1$ à $C_4$, $R^{21}$ est un atome d'hydrogène, un groupe alkyle en $C_1$ à $C_4$ ou un groupe phénylalkyle en $C_1$ à $C_4$, $R^{22}$ est un atome d'hydrogène, un groupe amino ou mono ou di(alkyl(en $C_1$ à $C_1$))amino, et $R^{23}$ est un atome d'hydrogène; ou Z est un radical de formule (c-1), (c-2), (c-3), (c-4) ou (c-5) dans laquelle $R^{24}$ est un atome d'hydrogène, d'halogène, un groupe alkyle en $C_1$ à $C_4$, alkyloxy en $C_1$ à $C_4$, amino, mono- ou di(alkyl(en $C_1$ à $C_4$))amino, un groupe phényle, phényle substitué ou imidazolyle, $R^{25}$ est un atome d'hydrogène, un groupe alkyle en $C_1$ à $C_4$, phényle ou phényle substitué, $R^{26}$ est un atome d'hydrogène, un groupe alkyle en $C_1$ à $C_4$, amino, alkyl(en $C_1$ à $C_4$)amino ou alkyle en $C_1$ à $C_4$ substitué avec un groupe phényle ou phényle substitué; $R^{27}$ est un atome d'hydrogène, un groupe alkyle en $C_1$ à $C_4$, alkyl(en $C_1$ à $C_4$)oxycarbonyle-alkyle en $C_1$ à $C_4$, phényle, phényle substitué,

alkyloxycarbonyle en $C_1$ à $C_4$, carboxyle, phénylcarbonyle, phénylcarbonyle substitué, naphtalényle, thiényle, furanyle, pyridinyle ou imidazolyle; $R^{28}$ et $R^{29}$ sont chacun indépendamment un atome d'hydrogène ou un groupe alkyle en $C_1$ à $C_4$; $R^{30}$ est un atome d'hydrogène, un alkyle en $C_1$ à $C_4$, un groupe amino ou alkyle en $C_1$ à $C_4$ substitué avec un groupe phényle ou phényle substitue; $R^{31}$ est un atome d'hydrogène, un groupe alkyle en $C_1$ à $C_4$, phényle, phényle substitué, cycloalkyle en $C_3$ à $C_7$, naphtalényle, thiényle, pyridinyle ou imidazolyle; $R^{32}$ est un atome d'hydrogène ou un groupe alkyle en $C_1$ à $C_4$ et $R^{33}$ et $R^{34}$ sont tous deux des atomes d'hydrogène.

**3.** Composé selon la revendication 2, dans lequel $-X^1=X^2-$ est un radical de formule (x) ou (y); et Y est un atome d'hydrogène, un groupe alkyle en $C_1$ à $C_4$, cyclopropyle, cyclopentyle, cyclohexyle, imidazolyle, pyridinyle, thiényle ou phényle éventuellement substitué avec un ou substituant deux chacun choisi indépendamment parmi les radicaux halogéno, alkyle en $C_1$ à $C_4$, alkyloxy en $C_1$ à $C_4$ et trifluorométhyle.

**4.** Composé selon la revendication 1, dans lequel Z est un radical de formule (a-1) dans laquelle $R^1$ et $R^2$ sont un atome d'hydrogène, $R^3$ est un atome d'hydrogène ou un groupe alkyle en $C_1$ à $C_4$ et Y est un atome d'hydrogène, un groupe alkyle en $C_1$ à $C_4$ ou phényle éventuellement substitué avec un ou deux atomes d'halogène; ou

Z est un radical de formule (a-2) dans laquelle $R^4$, $R^5$ et $R^6$ sont tous des atomes d'hydrogène, et Y est un atome d'hydrogène, un groupe alkyle en $C_1$ à $C_4$, cyclopropyle ou phényle éventuellement substitué avec un ou deux atomes d'halogène; ou

Z est un radical de formule (a-3) dans laquelle $R^7$ est un atome d'hydrogène, d'halogène ou un groupe alkyloxy en $C_1$ à $C_4$, $R^8$ est un atome d'hydrogène, $R^9$ est un atome d'hydrogène, un groupe alkyle en $C_1$ à $C_4$ ou un groupe alkyloxy en $C_1$ à $C_4$ et Y est un atome d'hydrogène, un groupe alkyle en $C_1$ à $C_4$, cyclopropyle, cyclohexyle, imidazolyle, thiényle ou phényle éventuellement substitué avec un ou deux substituants chacun choisi indépendamment parmi les radicaux halogéno, alkyle en $C_1$ à $C_4$, alkyloxy en $C_1$ à $C_4$ et trifluorométhyle; ou

Z est un radical ayant la formule (a-4) dans laquelle $R^{10}$ et $R^{12}$ sont des atomes d'hydrogène, $R^{11}$ est un groupe alkyle en $C_1$ à $C_4$ et Y est un atome d'hydrogène; ou

Z est un radical de formule (b-2) dans laquelle $R^{15}$ est un atome d'hydrogène, $R^{16}$ est un atome d'hydrogène ou un groupe alkyle en $C_1$ à $C_4$ et Y est un atome d'hydrogène, un groupe alkyle en $C_1$ à $C_4$, pyridinyle ou phényle éventuellement substitué avec un ou deux atomes d'halogène; ou

Z est un radical de formule (b-3) dans laquelle $R^{17}$ est un groupe alkyle en $C_1$ à $C_4$ et Y est un groupe phényle ou halogénophényle; ou
Z est un radical de formule (b-5) dans laquelle $R^{20}$ est un atome d'hydrogène, $R^{21}$ est un atome d'hydrogène, un groupe alkyle en $C_1$ à $C_4$ ou phénylalkyle en $C_1$ à $C_4$ et Y est un atome d'hydrogène, un groupe phényle ou halogénophényle; ou
Z est un radical de formule (b-6) dans laquelle $R^{22}$ est un atome d'hydrogène ou un groupe amino, $R^{23}$ est un atome d'hydrogène et Y est un atome d'hydrogène, un groupe phényle ou halogénophényle; ou
Z est un radical de formule (c-1) dans laquelle $R^{24}$ est un atome d'hydrogène, un groupe alkyle en $C_1$ à $C_4$, un groupe alkyloxy en $C_1$ à $C_4$, un groupe halogéno, amino, di(alkyl(en $C_1$ à $C_4$))amino, phényle ou imidazolyle, $R^{25}$ est un atome d'hydrogène, un groupe alkyle en $C_1$ à $C_4$ ou phényle et Y est un atome d'hydrogène, un groupe alkyle en $C_1$ à $C_4$, thiényle, imidazolyle ou phényle éventuellement substitué avec un ou deux substituants choisis parmi les radicaux halogéno, alkyle en $C_1$ à $C_4$; alkyloxy en $C_1$ à $C_4$ ou trifluorométhyle; ou
Z est un radical de formule (c-2) dans laquelle $R^{26}$ est un atome d'hydrogène, un groupe alkyle en $C_1$ à $C_4$, amino ou un groupe alkyle en $C_1$ à $C_4$ substitué avec un groupe phényle et $R^{27}$ est un atome d'hydrogène, un groupe alkyle en $C_1$ à $C_4$ carboxyle, alkyl(en $C_1$ à $C_4$)oxycarbonyle, naphtalényle, thiényle, pyridinyle, imidazolyle, phényle ou phényle substitué avec 1, 2 ou 3 substituants chacun choisi indépendamment parmi les radicaux alkyle en $C_1$ à $C_4$, alkyloxy en $C_1$ à $C_4$, halogéno, hydroxy et trifluorométhyle et Y est un atome d'hydrogène, un groupe alkyle en $C_1$ à $C_4$, un groupe cyclopropyle,cyclopentyle,cyclohexyle,imidazolyle,thiényle, pyridinyle ou phényle éventuellement substitué avec un ou deux substituants choisis parmi les groupes halogène, alkyle en $C_1$ à $C_4$, alkyloxy en $C_1$ à $C_4$ et trifluorométhyle.

**5.** Composé selon la revendication 1 dans lequel Z est un radical de formule (a-1); R est un atome d'hydrogène; $-X^1=X^2-$ est un radical de formule (x) ou (y); Y est un groupe isopropyle, phényle ou halogénophényle; $R^1$ et $R^2$ sont tous deux des atomes d'hydrogène; et $R^3$ est un groupe méthyle; ou dans lequel Z est un radical de formule (a-2); R est un atome d'hydrogène; $-X^1=X^2-$ est un radical de formule (x) ou (y); Y est un groupe cyclopropyle, phényle ou halogénophényle et $R^4$, $R^5$ et $R^6$ sont tous des atomes d'hydrogène; ou dans lequel Z est un radical de formule (a-3); R est un atome d'hydrogène; $-X^1=X^2-$ est un radical de formule (x) ou (y); Y est un groupe phényle, halogénophényle, dihalogénophényle, méthoxyphényle ou cyclohexyle.

**6.** Composé selon la revendication 1, dans lequel Z est un radical de formule (b-2); $-X^1=X^2-$ est un radical de formule (x) ou (y); R est un atome d'hydrogène; $R^{15}$ est un atome d'hydrogène; $R^{16}$ est un atome d'hydrogène; et Y est un groupe phényle, halogénophényle ou propyle; ou dans lequel Z est un radical de formule (b-5); $-X^1=X^2-$ est un radical de formule (x) ou (y); R est un atome d'hydrogène, $R^{20}$ est un atome d'hydrogène; $R^{21}$ est un atome d'hydrogène, un groupe méthyle ou alkyl(en $C_1$ à $C_4$) phényle; et Y est un groupe phényle ou halogénophényle; ou dans lequel Z est un radical de formule (b-6); $-X^1=X^2-$ est un radical de formule (x) ou (y); R est un atome d'hydrogène; $R^{22}$ est un atome d'hydrogène; $R^{23}$ est un atome d'hydrogène; et Y est un groupe phényle ou halogénophényle.

**7.** Composé selon la revendication 1 dans lequel Z est un radical de formule (c-1); $-X^1=X^2-$ est un radical de formule (x) ou (y); R est un atome d'hydrogène; $R^{24}$ et $R^{25}$ sont tous deux des atomes d'hydrogène et Y est un groupe phényle ou halogénophényle; ou dans lequel Z est un radical de formule (c-2); $-X^1=X^2-$ est un radical de formule (x) ou (y); R est un atome d'hydrogène; Y est un atome d'hydrogène, un groupe alkyle en $C_1$ à $C_4$, cyclopropyle, cyclopentyle ou cyclohexyle; $R^{27}$ est un atome d'hydrogène est un atome d'hydrogène, un groupe alkyle en $C_1$ à $C_4$ , un groupe naphtalényle, thiényle, pyridinyle, imidazolyle, phényle ou phényle substitué avec 1 ou 2 substituants chacun choisi indépendamment parmi les radicaux méthyle, halogéno, hydroxy et méthoxy; et n vaut O; ou dans lequel Z est un radical de formule (c-2); $-X^1=X^2-$ est un radical de formule (x) ou (y); Y est un groupe phényle ou halogénophényle; $R^{26}$ est un atome d'hydrogène; $R^{27}$ est un atome d'hydrogène ou un groupe alkyle en $C_1$ à $C_4$ ; et n vaut 0.

**8.** Composition pharmaceutique comprenant un véhicule pharmaceutique approprié et en tant qu'ingrédient actif une quantité efficace sur le plan thérapeutique d'un composé chimique selon l'une quelconque des revendications 1 à 7.

**9.** Procédé de préparation d'une composition pharmaceutique selon la revendication 8, caractérisé en ce qu'une quantité efficace sur le plan thérapeutique d'un composé selon l'une quelconque des revendications 1 à 7 est intimement mélangée avec un véhicule pharmaceutique approprié.

**10.** Composé selon l'une quelconque des revendications 1 à 7 pour une utilisation en tant que médicament.

**11.** Procédé pour la préparation d'un composé chimique selon l'une quelconque des revendications 1 à 7, caractérisé par:
a) la N-alkylation d'un azole de formule (II) ou d'un sel de métal alcalin de celui-ci

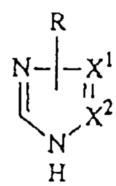
(II)

dans laquelle R, $X^1$, $X^2$ sont tels que définis sous la formule (I) avec un dérivé de quinoline, de quinolinone, de quinazoline ou de quinoxaline de formule

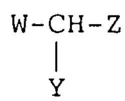
(III)

dans laquelle Y et Z sont tels que définis sous la formule (I) et W représente un groupe quittant réactif;
b) la N-alkylation d'un azole de formule

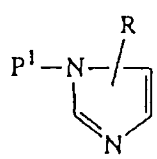
(II-x)

dans laquelle R est tel que défini sous la formule (I) et $P^1$ représente un groupe protecteur avec un dérivé de quinoline, quinolinone, quinazoline ou quinoxaline de formule

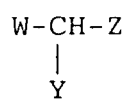
(III)

dans laquelle Y et Z sont tels que définis sous la formule (I) et W représente un groupe quittant réactif, obtenant ainsi un composé de formule

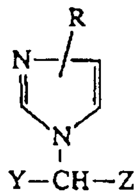

(I-x) ;

c) l'endo-N-alkylation d'un azole de formule

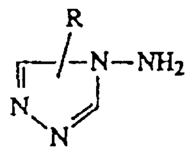

(II-y)

dans laquelle R est tel que défini sous la formule (I) avec un dérivé de quinoline, quinolinone, quinazoline ou quinoxaline de formule

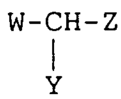

(III)

dans laquelle Y et Z sont tels que définis sous la formule (I) et W représente un groupe quittant réactif, la déamination ultérieure du sel de triazolium ainsi obtenu,

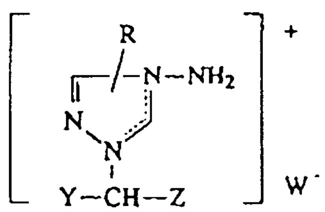

préparant ainsi un composé de formule

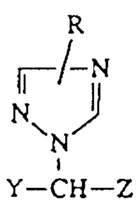

(I-y) ;

d) la réaction d'un composé de formule

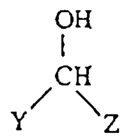

(V)

avec un réactif de formule

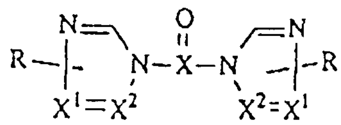

(VI)

dans un solvant approprié;

e) l'alkylation réductrice d'une cétone ou d'un aldéhyde de formule

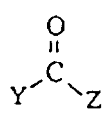

(VII)

avec un azole de formule

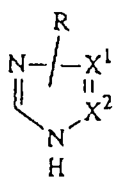

(II)

en agitant et en chauffant les réactifs dans de l'acide formique ou des formamides en présence d'un catalyseur acide;

f) la cyclisation d'un intermédiaire de formule

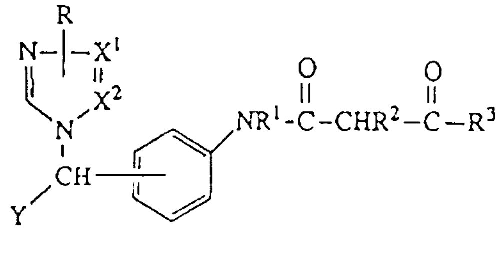

(IX)

en présence d'un acide, obtenant ainsi un composé de formule

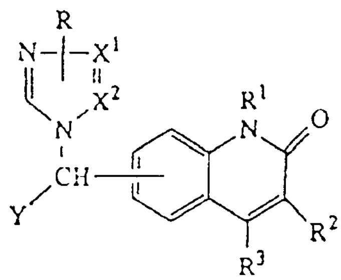

(I-a-1) ;

g) la cyclisation d'un intermédiaire de formule

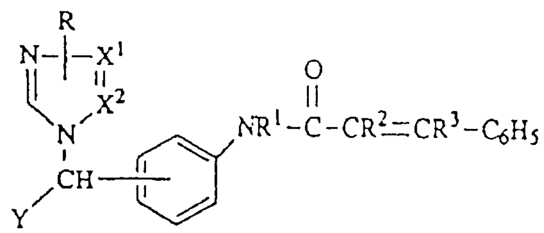

(X)

en présence d'un acide Lewis approprié, obtenant ainsi un composé de formule (I-a-1);
h) la cyclisation d'un intermédiaire de formule

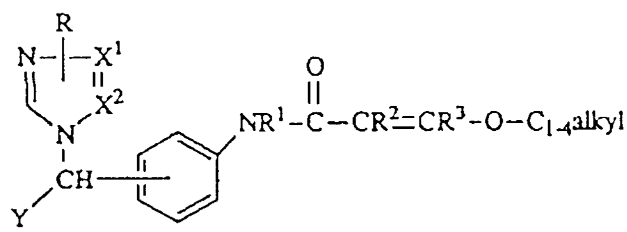

(XI)

en présence d'un acide, obtenant ainsi un composé de formule (I-a-1);
i) la cyclisation d'un intermédiaire de formule

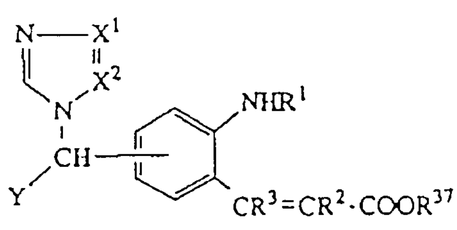

(XII)

obtenant ainsi un composé de formule (I-a-1);

j) la cyclisation d'un intermédiaire de formule

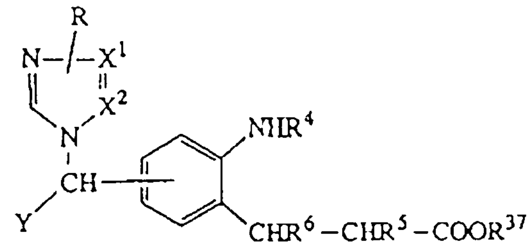

(XIII)

obtenant ainsi un composé de formule

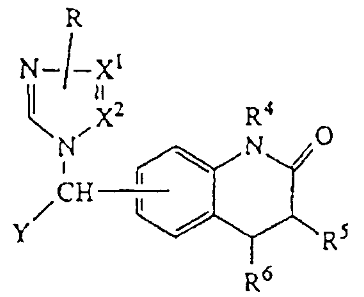

(I-a-2)

k) la cyclisation d'un intermédiaire de formule

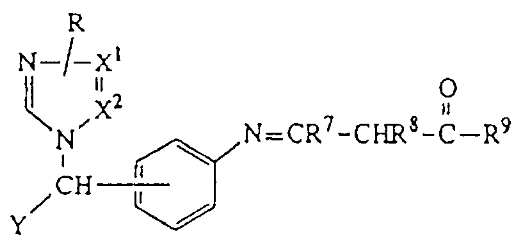

(XIV)

en présence d'un agent de déshydratation, obtenant ainsi un composé de formule

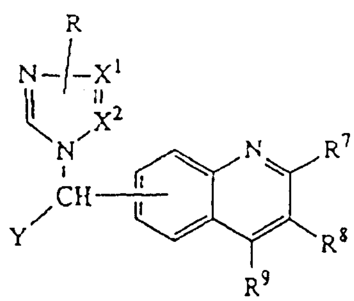

(I-a-3) ;

l) la condensation d'une aniline de formule

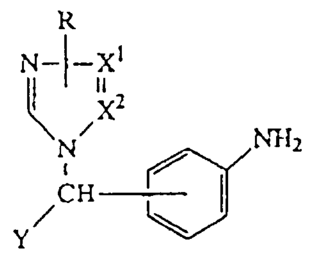

(VIII)

avec un élément de synthèse non saturé en $\alpha$-$\beta$ de formule

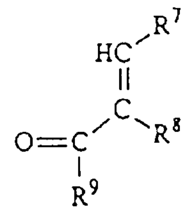

(XV)

en présence d'un acide et d'un agent oxydant doux, obtenant ainsi un composé de formule (I-a-3);
m) la condensation d'une ortho-acylaniline de formule

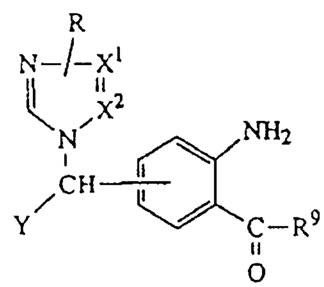

(XVI)

avec une cétone ou un aldéhyde de formule

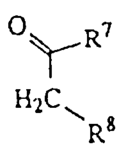

(XVII)

obtenant ainsi un composé de formule (I-a-3);

n) la cyclisation d'un intermédiaire de formule

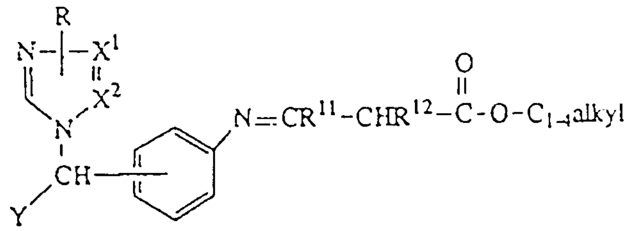

(XVIII)

en présence d'un agent de déshydratation approprié, obtenant ainsi un composé de formule

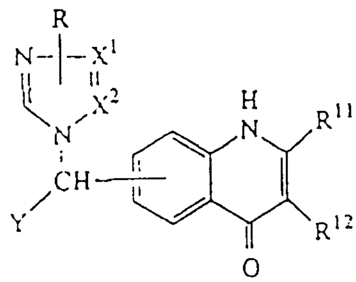

(I-a-4-a) ;

o) la cyclisation d'un intermédiaire de formule

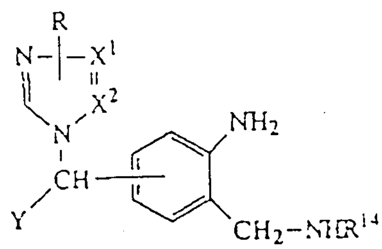

(XIX)

avec un acide carboxylique de formule

$$R^{13} - \overset{\overset{\displaystyle O}{\|}}{C} - OH \qquad (XX)$$

ou un dérivé fonctionnel de celui-ci dans un solvant inerte à l'égard de la réaction, obtenant ainsi un composé de formule

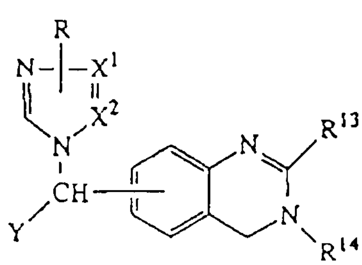

(I-b-1) ;

p) la cyclisation d'un intermédiaire de formule

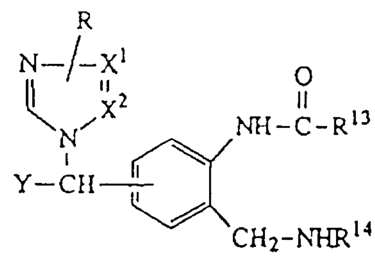

(XXI)

dans un solvant inerte à l'égard de la réaction, obtenant ainsi un composé de formule (I(b-1);
q) la cyclisation d'un intermédiaire de formule

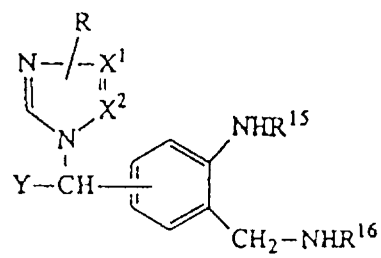

(XIX-a)

avec un réactif de formule L-C(=$X^3$)-L (XXII) dans laquelle L représente un groupe réactif quittant et $X^3$ est un atome d'oxygène ou de soufre, obtenant ainsi un composé de formule

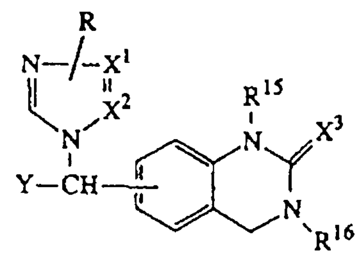

(I-b-2) ;

r) la réaction et la condensation d'un intermédiaire de formule

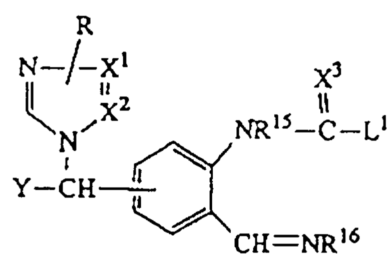

(XXIII)

dans laquelle $L^1$ représente un groupe quittant réactif dans un solvant inerte à l'égard de la réaction et en présence d'un agent réducteur, obtenant ainsi un composé de formule (I-b-2);

s) la réaction d'un intermédiaire de formule

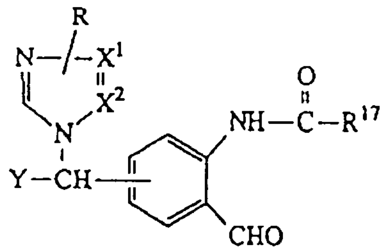

(XXIV)

avec de l'ammoniac dans un solvant inerte à l'égard de la réaction, obtenant ainsi un composé de formule

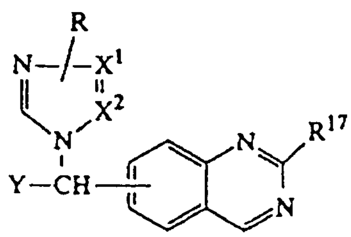

(I-b-3) ;

t) la condensation d'un intermédiaire de formule

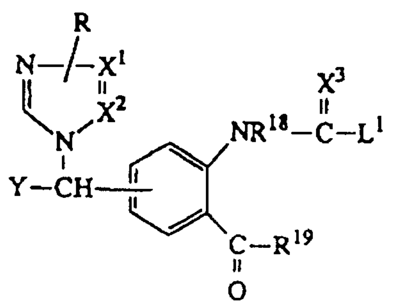

(XXV)

dans laquelle $L^1$ représente un groupe quittant avec de l'ammoniac, obtenant ainsi un composé de formule

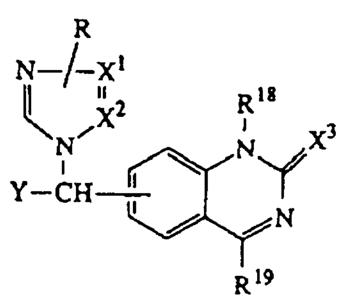

(I-b-4) ;

u) la cyclisation d'un intermédiaire de formule

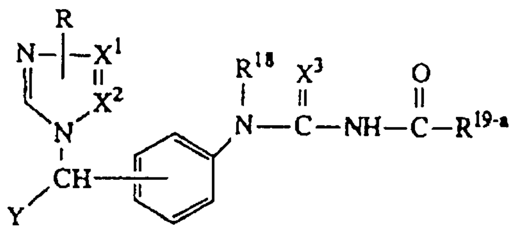

(XXVI)

en présence d'un agent de déshydratation, obtenant un composé de formule (I-b-4);

v) la condensation d'un intermédiaire de formule

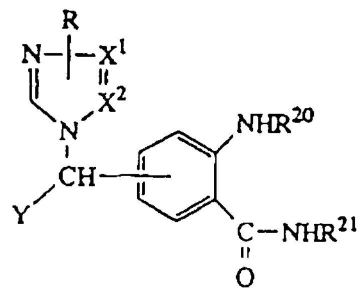

(XXVII)

avec un réactif de formule L-C(=$X^3$)-L (XXII) dans un solvant inerte à l'égard de la réaction, obtenant ainsi un composé de formule

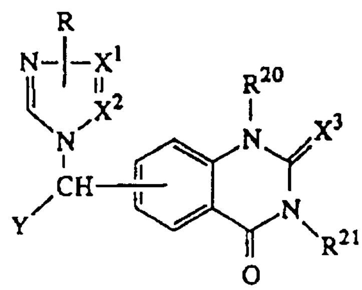

(I-b-5) ;

w) la cyclisation d'un intermédiaire de formule

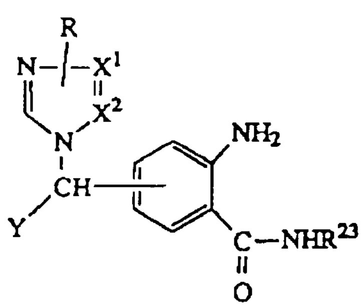

(XXVIII)

avec un acide carboxylique de formule $R^{22}$-COOH (XXIX) ou un dérivé fonctionnel de celui-ci; obtenant ainsi un composé de formule

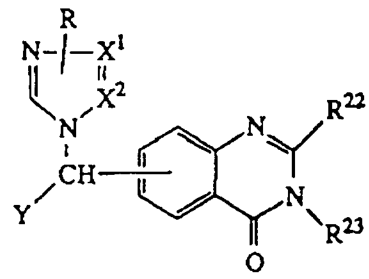

(I-b-6) ;

x) la condensation d'une ortho-benzènediamine de formule

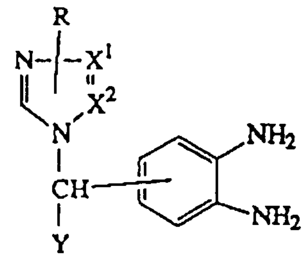

(XXX-a)

avec une 1,2-dicétone de formule $R^{24-a}$-C(=O)-C(=O)-$R^{25}$ (XXXI) dans un solvant inerte à l'égard de la réaction obtenant ainsi un composé de formule

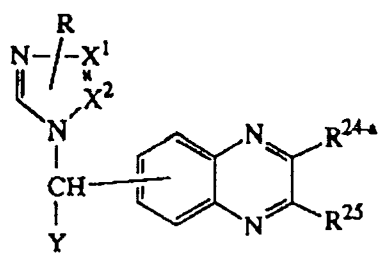

(I-c-1-a) ;

y) la cyclisation d'une ortho-diamine substituée (1H-azol-lylméthyl) de formule

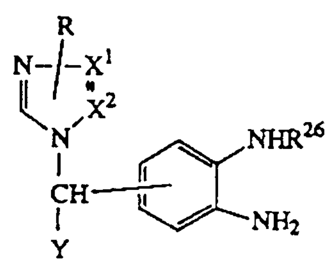

(XXX-b)

avec un α-céto-acide de formule HO-C(=O)-C(=O)-$R^{27}$ ou un dérivé fonctionnel de celui-ci dans un solvant inerte à l'égard de la réaction, obtenant ainsi un composé de formule

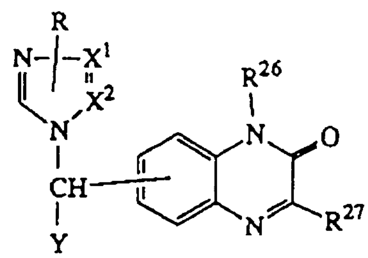

(I-c-2-a) ;

z) la réduction et la cyclisation d'un intermédiaire de formule

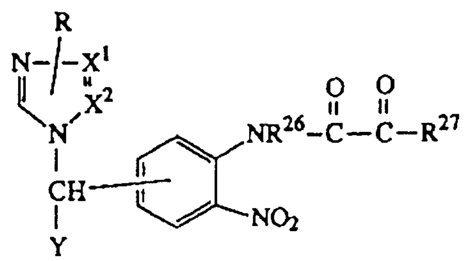

(XXXIII-b)

dans un solvant inerte à l'égard de la réaction, obtenant ainsi un composé de formule (I-c-2-a);
aa) la cyclisation d'un ortho-nitroanilide contenant un groupe méthylène activé approprié de formule

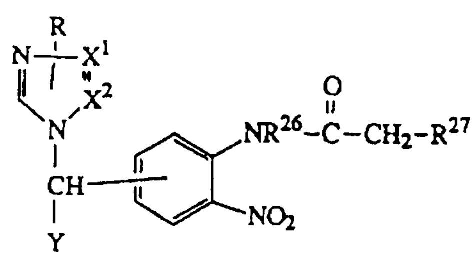

(XXXIV-a)

dans un solvant inerte à l'égard de la réaction, obtenant ainsi un composé de formule

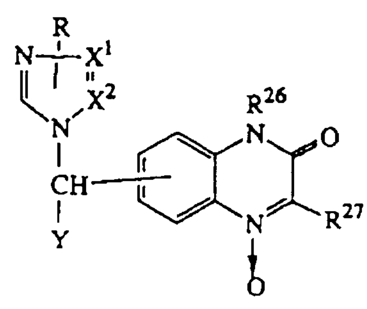

(I-c-2-b) ;

bb) la cyclisation d'un ortho-anilide de formule

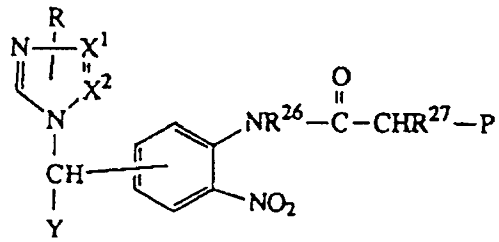

(XXXIV-b)

dans laquelle P est un groupe activant, dans un solvant inerte à l'égard de la réaction, obtenant ainsi un composé de formule (I-c-2-b);

cc) la condensation d'un intermédiaire de formule

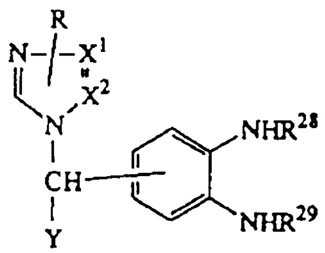

(XXX-c)

avec un acide oxalique HO-C( = O)-C( = O)-OH ou un dérivé fonctionnel de celui-ci, obtenant ainsi un composé de formule

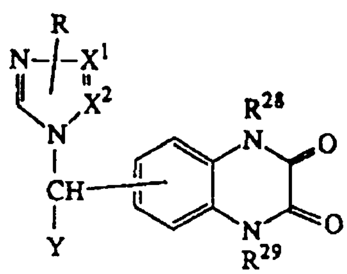

(I-c-3) ;

dd) la condensation d'un orthodiamine de formule

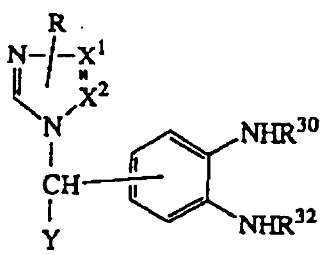

(XXX-d)

avec un acide $\alpha$-halogéno-acide de formule

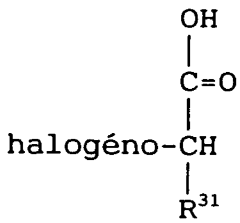

(XXXVI)

ou un dérivé fonctionnel de celui-ci dans un solvant inerte à l'égard de la réaction, obtenant ainsi un composé de formule

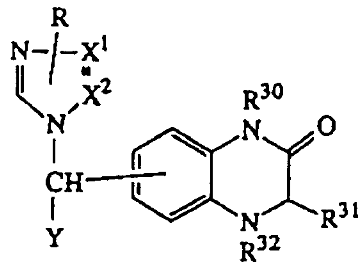

(I-c-4) ;

ee) la réduction d'une ortho-nitrophénylglycine substituée de formule

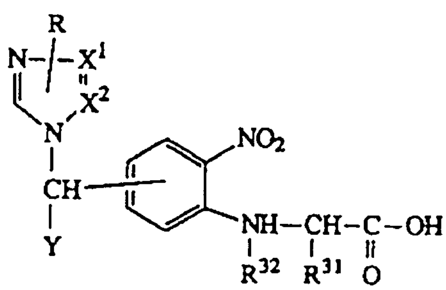

(XXXVII)

dans un solvant inerte à l'égard de la réaction, obtenant ainsi un composé de formule

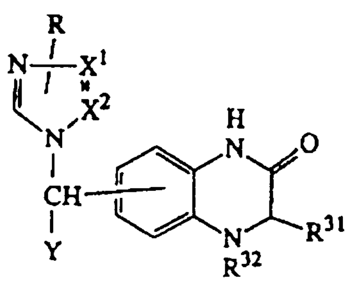

(I-c-4-a) ;

ff) la cyclisation d'un intermédiaire de formule

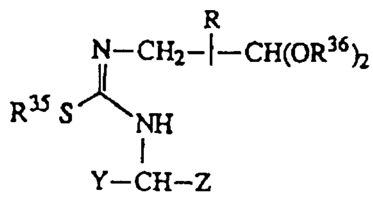

(XXXVIII)

et la désulfuration de l'intermédiaire ainsi obtenu de formule

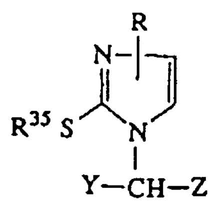

(IXL) ,

obtenant ainsi un composé de formule

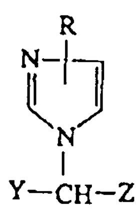

(I-x) ;

gg) la réaction d'un intermédiaire de formule

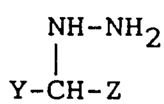

(XL)

avec la s-triazine, obtenant ainsi un composé de formule

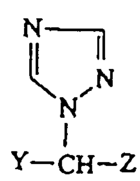

(I-y) ;

ou éventuellement la conversion des composés de formule (I) en l'un ou l'autre des groupes suivant connus dans la technique des procédés de transformation, et, si souhaité, la conversion des composés de formule (I) en une forme de sel d'addition d'acide non toxique active sur le plan thérapeutique par traitement avec un acide approprié ou, au contraire, la conversion du sel d'addition d'acide en la forme basique libre avec une base; et/ou la préparation de formes stéréochimiquement isomères de celui-ci.

**Revendication pour les Etats contractants suivants : ES, GR**

**1.** Procédé pour préparer un composé chimique ayant la formule

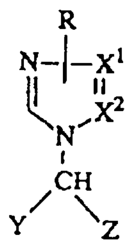

(I)

un sel d'addition d'acide acceptable sur le plan pharmaceutique de celui-ci ou une forme stéréochimiquement isomère de celui-ci, dans lequel
$-X^1=X^2-$ est un radical bivalent ayant la formule

-CH=CH- (x),

-CH=N- (y), ou

-N=CH- (z);

R est un atome d'hydrogène ou un groupe alkyle en $C_1$ à $C_6$;
Y est un atome d'hydrogène, un groupe alkyle en $C_1$ à $C_{10}$, cycloalkyle en $C_3$ à $C_7$ $Ar^1$, $Ar^2$-alkyle(en $C_1$ à $C_6$), alcényle en $C_2$ à $C_6$ ou alcynyle en $C_2$ à $C_6$;
Z est un radical de formule

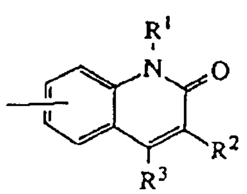

(a-1)

,

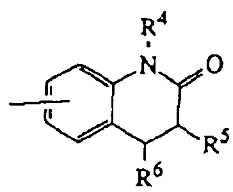

(a-2)

,

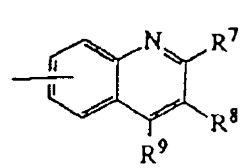

(a-3)

ou

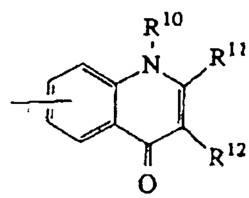

(a-4)

dans laquelle
$R^1$, $R^4$ et $R^{10}$ sont chacun indépendamment un atome d'hydrogène, un groupe alkyle en $C_1$ à $C_6$ ou $Ar^2$-alkyle(en $C_1$ à $C_6$);
$R^2$, $R^5$, $R^8$ et $R^{12}$ sont chacun indépendamment un atome d'hydrogène, un groupe alkyle en $C_1$ à $C_6$ ou $Ar^2$;
$R^3$, $R^6$ et $R^{11}$ sont chacun indépendamment un atome d'hydrogène, ou un groupe alkyle en $C_1$ à $C_6$;
$R^7$ et $R^9$ sont chacun indépendamment un atome d'hydrogène, un radical alkyle en $C_1$ à $C_6$, alkyloxy en $C_1$ à $C_6$, halogéno, amino, ou mono ou di(alkyl en $C_1$ à $C_6$)amino; ou
Z est un radical de formule

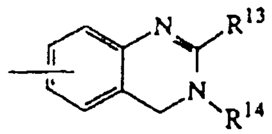,
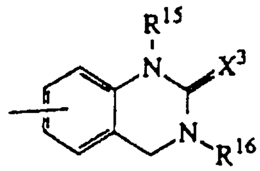,
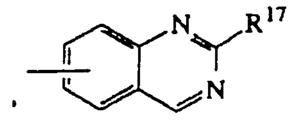.

(b-1) (b-2) (b-3)

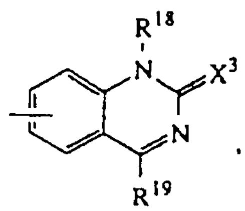,
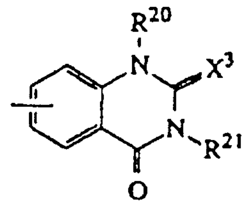
or
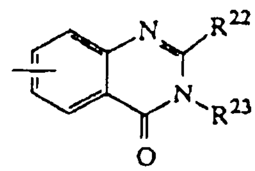

(b-4) (b-5) (b-6)

dans laquelle

$R^{13}$, $R^{17}$ et $R^{22}$ sont chacun indépendamment un atome d'hydrogène, un radical halogéno, alkyle en $C_1$ à $C_6$, trifluorométhyle, alkyloxy en $C_1$ à $C_6$, $Ar^2$, $Ar^2$-alkyle(en $C_1$ à $C_6$), amino ou mono- ou di(alkyl en $C_1$ à $C_6$)amino;

$R^{14}$, $R^{16}$ et $R^{21}$ sont chacun indépendamment un atome d'hydrogène, un groupe alkyle en $C_1$ à $C_6$, $Ar^2$ ou $Ar^2$-alkyle(en $C_1$ à $C_6$);

$R^{15}$ , $R^{18}$ et $R^{20}$ sont chacun indépendamment un atome d'hydrogène, un groupe alkyle en $C_1$ à $C_6$ ou $Ar^2$-alkyle(en $C_1$ à $C_6$);

$R^{19}$ est un atome d'hydrogène $R^{23}$ est un atome d'hydrogène, un groupe alkyle en $C_1$ à $C_6$ , $Ar^2$-alkyle-(en $C_1$ à $C_6$), amino ou mono(alkyl en $C_1$ à $C_6$) amino;

$X^3$ est O ou S ; ou

Z est un radical de formule

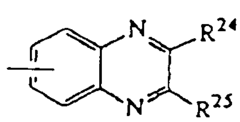,
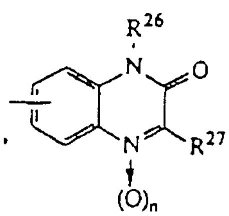,
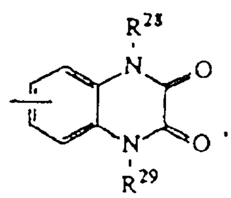,
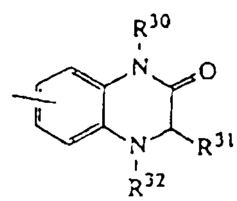,
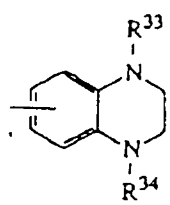

(c-1) (c-2) (c-3) (c-4) (c-5)

dans laquelle

$R^{24}$ est un atome d'hydrogène, d'halogène, un groupe alkyle en $C_1$ a $C_6$, alkyloxy en $C_1$ en $C_6$, amino, mono- ou di(alkyl en $C_1$ à $C_6$)amino, $Ar^2$ ou imidazolyle;

$R^{25}$ est un atome d'hydrogène, un groupe alkyle en $C_1$ à $C_6$ ou $Ar^1$;

$R^{26}$ et $R^{30}$ sont chacun indépendamment un atome d'hydrogène, un groupe alkyle en $C_1$ à $C_6$, $Ar^2$-alkyle(en $C_1$ à $C_6$), un groupe amino, ou mono(alkyl en $C_1$ à $C_6$) amino;

$R^{27}$ et $R^{31}$ sont chacun indépendamment un atome d'hydrogène, un groupe alkyle en $C_1$ à $C_6$, $Ar^1$, alkyl(en $C_1$ à $C_6$)carbonyle, $Ar^2$-carbonyle, alkyl(en $C_1$ à $C_6$)oxycarbonyle, carboxyle, alkyl(en $C_1$ à $C_6$)-oxycarbonyle-alkyle(en $C_1$ à $C_4$), aminocarbonyle ou cyano;

$R^{28}$, $R^{29}$, $R^{32}$ et $R^{34}$ sont chacun indépendamment un atome d'hydrogène, un groupe alkyle en $C_1$ à $C_6$ ou $Ar^2$-alkyle(en $C_1$ à $C_6$ );

n vaut O ou 1; et

$Ar^1$ est un groupe phényle, phényle substitué, naphtalényle, pyridinyle, imidazolyle, triasolyle, thiényle,

furanyle ou thiazolyle et $Ar^2$ est un groupe phényle ou phényle substitué; ledit groupe phényle substitué en $Ar^1$ ou $Ar^2$ étant un groupe phényle substitué avec 1, 2 ou 3 substituants choisis chacun indépendamment parmi les radicaux halogéno, hydroxy, trifluorométhyle, alkyle en $C_1$ à $C_6$, alkyloxy en $C_1$ à $C_6$, cyano, amino, mono- et di(alkyle en $C_1$ à $C_6$)amino, nitro, carboxyle, formyle et alkyl(en $C_1$ à $C_6$)oxycarbonyle, caractérisé par:

a) la N-alkylation d'un azole de formule (II) ou d'un sel de métal alcalin de celui-ci

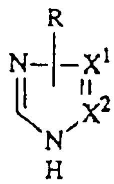 (II)

dans laquelle R, $X^1$, $X^2$ sont tels que définis sous la formule (I) avec un dérivé de quinoline, de quinolinone, de quinazoline ou de quinoxaline de formule

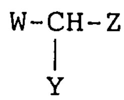 (III)

dans laquelle Y et Z sont tels que définis sous la formule (I) et W représente un groupe quittant réactif;

b) la N-alkylation d'un azole de formule

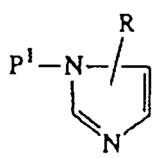 (II-x)

dans laquelle R est tel que défini sous la formule (I) et $P^1$ représente un groupe protecteur avec un dérivé de quinoline, quinolinone, quinazoline ou quinoxaline de formule

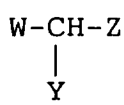 (III)

dans laquelle Y et Z sont tels que définis sous la formule (I) et W représente un groupe quittant réactif, obtenant ainsi un composé de formule

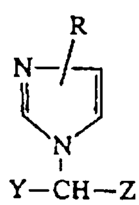 (I-x) ;

c) l'endo-N-alkylation d'un azole de formule

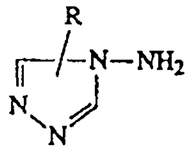 (II-y)

dans laquelle R est tel que défini sous la formule (I) avec un dérivé de quinoline, quinolinone, quinazoline ou quinoxaline de formule

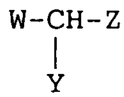 (III)

dans laquelle Y et Z sont tels que définis sous la formule (I) et W représente un groupe quittant réactif, la déamination ultérieure du sel de triazolium ainsi obtenu,

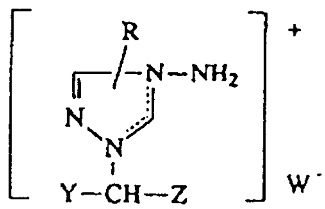

préparant ainsi un composé de formule

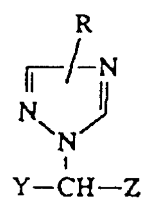 (I-y) ;

d) la réaction d'un composé de formule

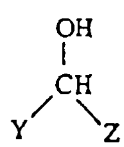 (V)

avec un réactif de formule

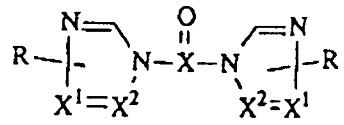 (VI)

dans un solvant approprié;

e) l'alkylation réductrice d'une cétone ou d'un aldéhyde de formule

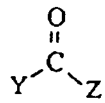 (VII)

avec un azole de formule

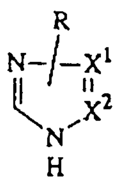 (II)

en agitant et en chauffant les réactifs dans de l'acide formique ou des formamides en présence d'un catalyseur acide;

f) la cyclisation d'un intermédiaire de formule

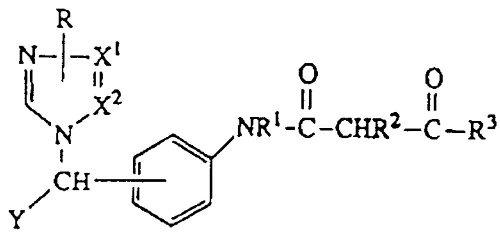 (IX)

en présence d'un acide, obtenant ainsi un composé de formule

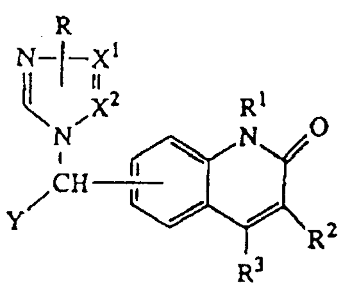 (I-a-1) ;

g) la cyclisation d'un intermédiaire de formule

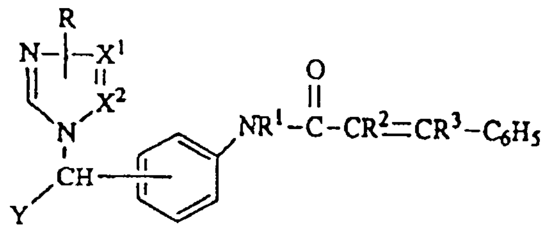

(X)

en présence d'un acide Lewis approprié, obtenant ainsi un composé de formule (I-a-1);
h) la cyclisation d'un intermédiaire de formule

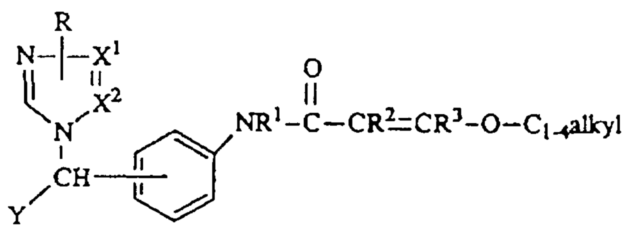

(XI)

en présence d'un acide, obtenant ainsi un composé de formule (I-a-1);
i) la cyclisation d'un intermédiaire de formule

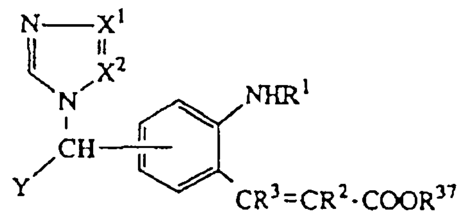

(XII)

obtenant ainsi un composé de formule (I-a-1);
j) la cyclisation d'un intermédiaire de formule

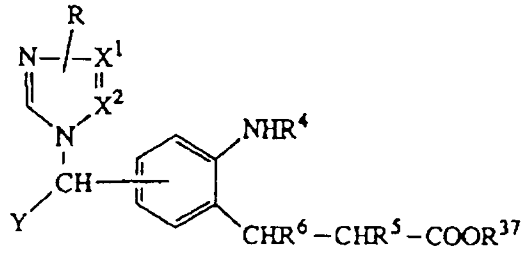

(XIII)

obtenant ainsi un composé de formule

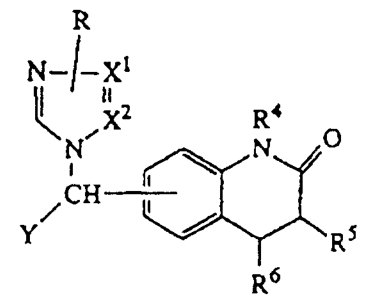

(I-a-2)

k) la cyclisation d'un intermédiaire de formule

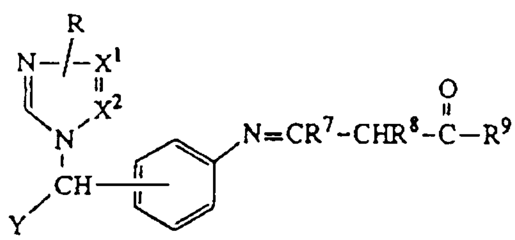

(XIV)

en présence d'un agent de déshydratation, obtenant ainsi un composé de formule

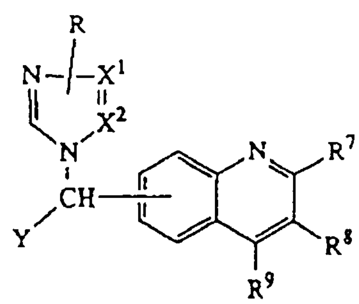

(I-a-3) ;

l) la condensation d'une aniline de formule

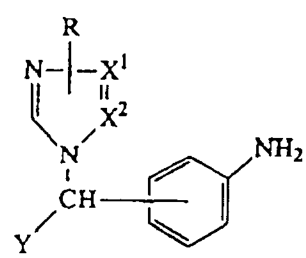

(VIII)

avec un élément de synthèse non saturé en $\alpha$-$\beta$ de formule

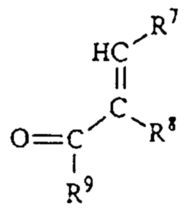

(XV)

en présence d'un acide et d'un agent oxydant doux, obtenant ainsi un composé de formule (I-a-3);

m) la condensation d'une ortho-acylaniline de formule

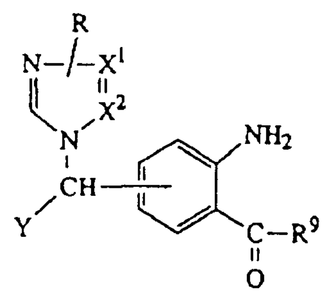

(XVI)

avec une cétone ou un aldéhyde de formule

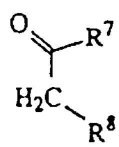

(XVII)

obtenant ainsi un composé de formule (I-a-3);

n) la cyclisation d'un intermédiaire de formule

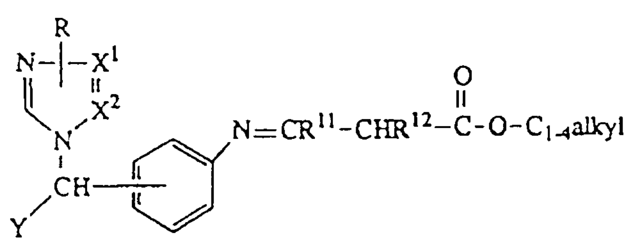

(XVIII)

en présence d'un agent de déshydratation approprié, obtenant ainsi un composé de formule

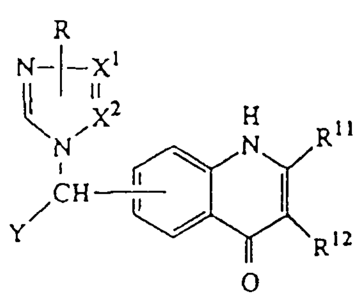

(I-a-4-a) ;

o) la cyclisation d'un intermédiaire de formule

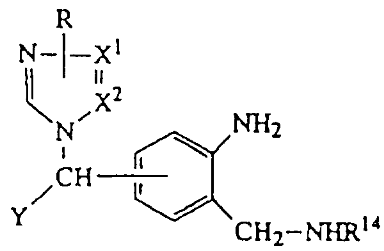

(XIX)

avec un acide carboxylique de formule

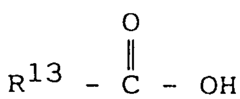

(XX)

ou un dérivé fonctionnel de celui-ci dans un solvant inerte à l'égard de la réaction, obtenant ainsi un composé de formule

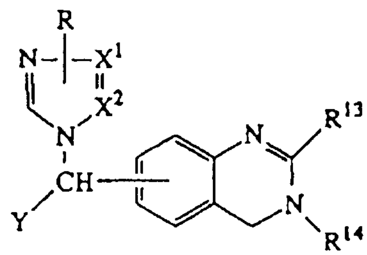

(I-b-1) ;

p) la cyclisation d'un intermédiaire de formule

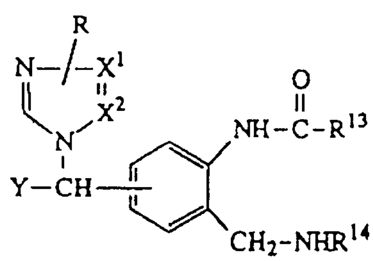

(XXI)

dans un solvant inerte à l'égard de la réaction, obtenant ainsi un composé de formule (I(b-1);

q) la cyclisation d'un intermédiaire de formule

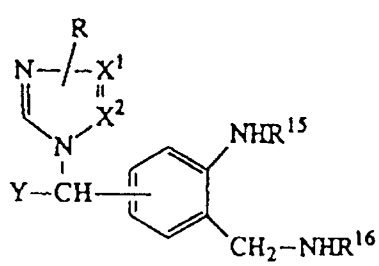

(XIX-a)

avec un réactif de formule L-C(=$X^3$)-L (XXII) dans laquelle L représente un groupe réactif quittant

et $X^3$ est un atome d'oxygène ou de soufre, obtenant ainsi un composé de formule

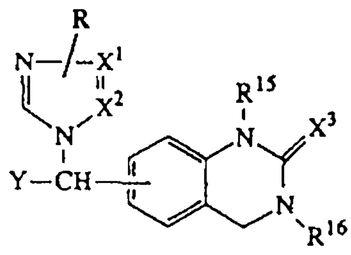

(I-b-2) ;

r) la réaction et la condensation d'un intermédiaire de formule

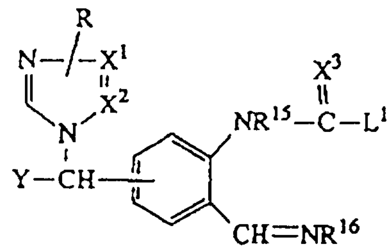

(XXIII)

dans laquelle $L^1$ représente un groupe quittant réactif dans un solvant inerte à l'égard de la réaction et en présence d'un agent réducteur, obtenant ainsi un composé de formule (I-b-2);
s) la réaction d'un intermédiaire de formule

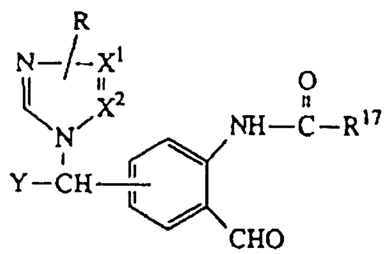

(XXIV)

avec de l'ammoniac dans un solvant inerte à l'égard de la réaction, obtenant ainsi un composé de formule

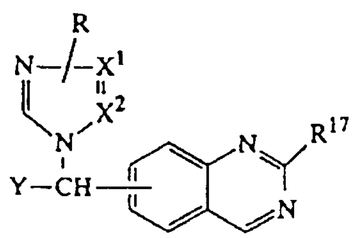

(I-b-3) ;

t) la condensation d'un intermédiaire de formule

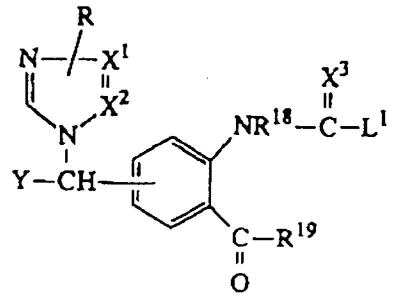

(XXV)

dans laquelle $L^1$ représente un groupe quittant avec de l'ammoniac, obtenant ainsi un composé de formule

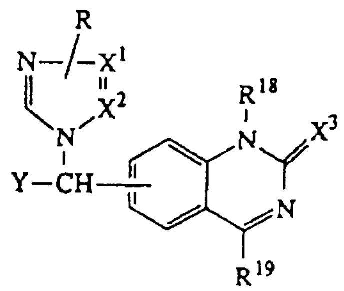

(I-b-4) ;

u) la cyclisation d'un intermédiaire de formule

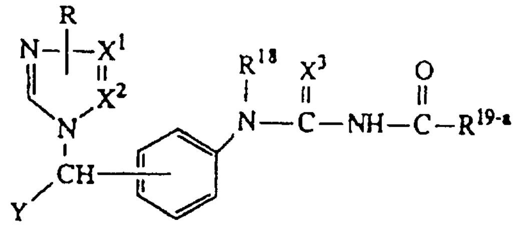

(XXVI)

en présence d'un agent de déshydratation, obtenant un composé de formule (I-b-4);
v) la condensation d'un intermédiaire de formule

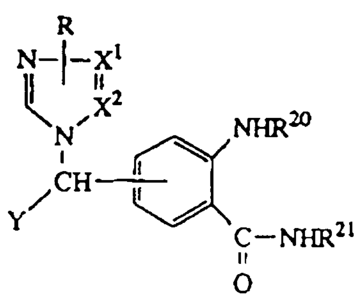

(XXVII)

avec un réactif de formule L-C(=$X^3$)-L (XXII) dans un solvant inerte à l'égard de la réaction, obtenant ainsi un composé de formule

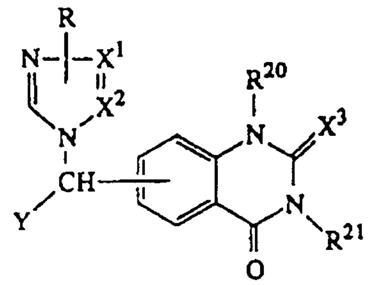

(I-b-5) ;

w) la cyclisation d'un intermédiaire de formule

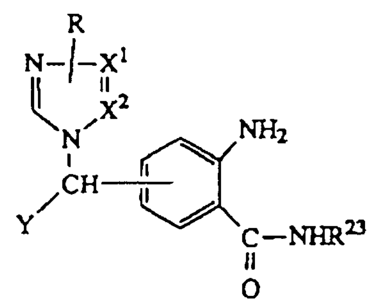

(XXVIII)

avec un acide carboxylique de formule $R^{22}$-COOH (XXIX) ou un dérivé fonctionnel de celui-ci; obtenant ainsi un composé de formule

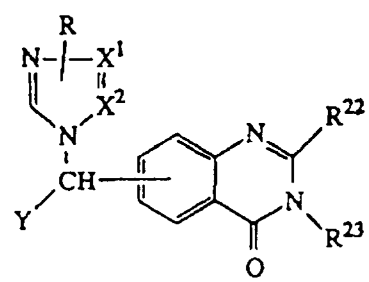

(I-b-6) ;

x) la condensation d'une ortho-benzènediamine de formule

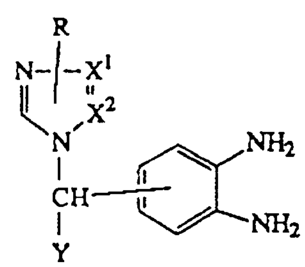

(XXX-a)

avec une 1,2-dicétone de formule $R^{24-a}$-C(=O)-C(=O)-$R^{25}$ (XXXI) dans un solvant inerte à l'égard de la réaction obtenant ainsi un composé de formule

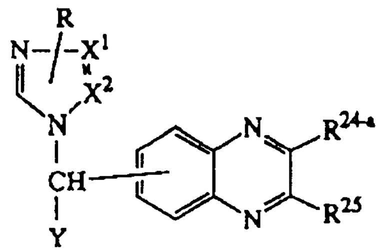

(I-c-1-a) ;

y) la cyclisation d'une ortho-diamine substituée (1H-azol-lylméthyl) de formule

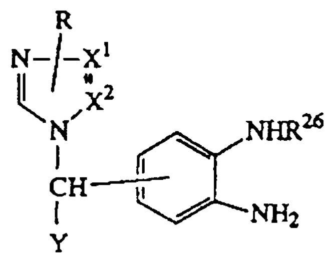

(XXX-b)

avec un α-céto-acide de formule HO-C( = O)-C( = O)-$R^{27}$ ou un dérivé fonctionnel de celui-ci dans un solvant inerte à l'égard de la réaction, obtenant ainsi un composé de formule

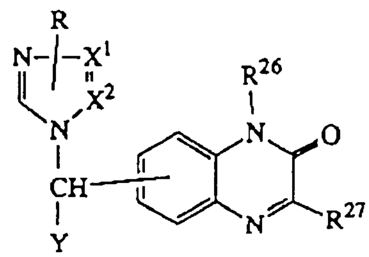

(I-c-2-a) ;

z) la réduction et la cyclisation d'un intermédiaire de formule

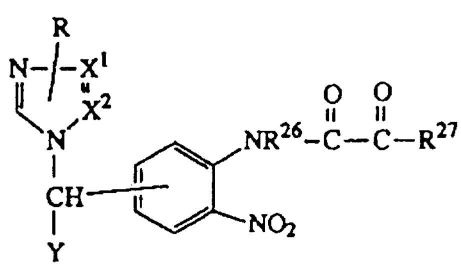

(XXXIII-b)

dans un solvant inerte à l'égard de la réaction, obtenant ainsi un composé de formule (I-c-2-a);

aa) la cyclisation d'un ortho-nitroanilide contenant un groupe méthylène activé approprié de formule

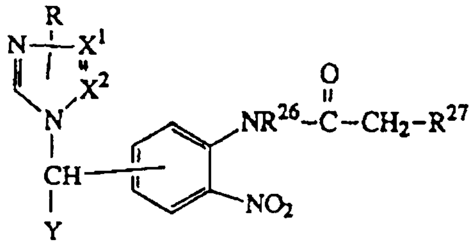

(XXXIV-a)

dans un solvant inerte à l'égard de la réaction, obtenant ainsi un composé de formule

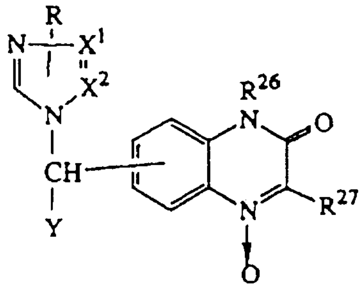

(I-c-2-b) ;

bb) la cyclisation d'un ortho-anilide de formule

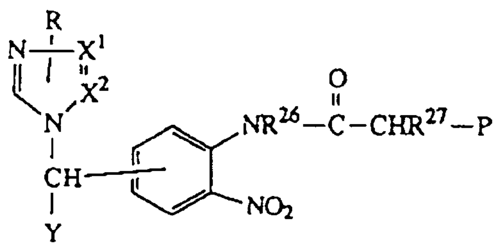

(XXXIV-b)

dans laquelle P est un groupe activant, dans un solvant inerte à l'égard de la réaction, obtenant ainsi un composé de formule (I-c-2-b);

cc) la condensation d'un intermédiaire de formule

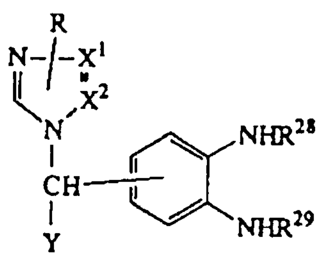

(XXX-c)

avec un acide oxalique HO-C( = O)-C( = O)-OH ou un dérivé fonctionnel de celui-ci, obtenant ainsi un composé de formule

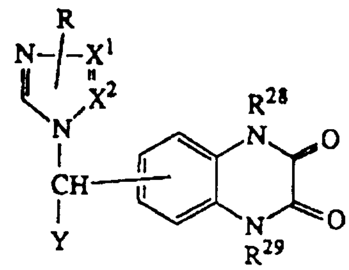

(I-c-3) ;

dd) la condensation d'un orthodiamine de formule

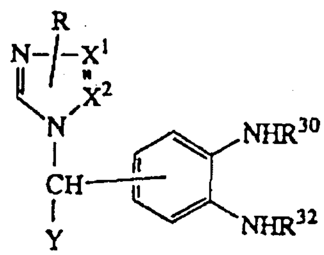

(XXX-d)

avec un acide α-halogéno-acide de formule

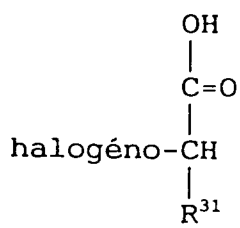

(XXXVI)

ou un dérivé fonctionnel de celui-ci dans un solvant inerte à l'égard de la réaction, obtenant ainsi un composé de formule

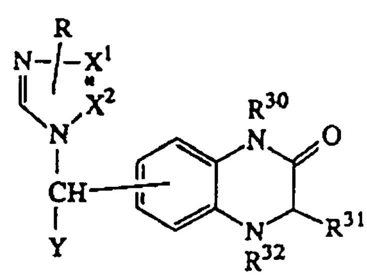

(I-c-4) ;

ee) la réduction d'une ortho-nitrophénylglycine substituée de formule

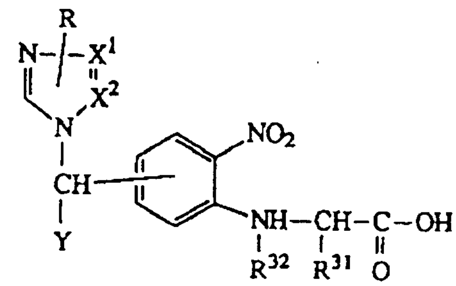

(XXXVII)

dans un solvant inerte à l'égard de la réaction, obtenant ainsi un composé de formule

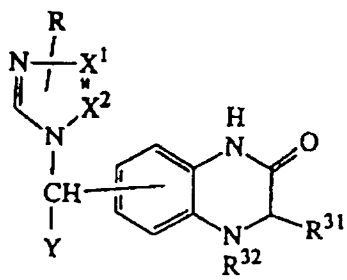

(I-c-4-a) ;

ff) la cyclisation d'un intermédiaire de formule

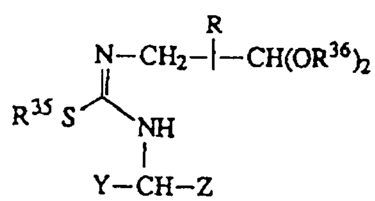

(XXXVIII)

et la désulfuration de l'intermédiaire ainsi obtenu de formule

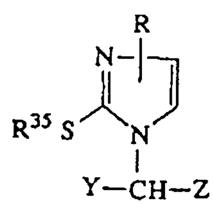

(IXL) ,

obtenant ainsi un composé de formule

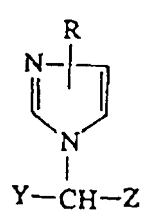

(I-x) ;

gg) la réaction d'un intermédiaire de formule

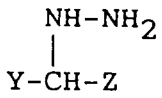

(XL)

avec la s-triazine, obtenant ainsi un composé de formule

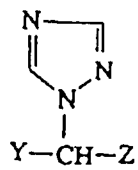

(I-y) ;

ou éventuellement la conversion des composés de formule (I) en l'un ou l'autre des groupes suivant connus dans la technique des procédés de transformation, et, si souhaité, la conversion des composés de formule (I) en une forme de sel d'addition d'acide non toxique active sur le plan thérapeutique par traitement avec un acide approprié ou, au contraire, la conversion du sel d'addition d'acide en la forme basique libre avec une base; et/ou la préparation de formes stéréochimiquement isomères de celui-ci.